(19) Europäisches Patentamt
European Patent Office
Office européen des brevets

(11) EP 3 118 200 A1

(12) **EUROPEAN PATENT APPLICATION**
published in accordance with Art. 153(4) EPC

(43) Date of publication:
**18.01.2017 Bulletin 2017/03**

(21) Application number: **15761818.2**

(22) Date of filing: **11.03.2015**

(51) Int Cl.:
*C07D 413/14* (2006.01) *A61K 31/501* (2006.01)
*A61P 35/00* (2006.01) *A61P 43/00* (2006.01)
*C07D 401/14* (2006.01) *C07D 417/14* (2006.01)

(86) International application number:
**PCT/JP2015/057122**

(87) International publication number:
**WO 2015/137385 (17.09.2015 Gazette 2015/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **12.03.2014 JP 2014049420**

(71) Applicant: **Takeda Pharmaceutical Company Limited**
**Osaka-shi, Osaka 541-0045 (JP)**

(72) Inventors:
- **IMAMURA Keisuke**
**Fujisawa-shi**
**Kanagawa 251-0012 (JP)**
- **TOMITA Naoki**
**Tokyo 103-0027 (JP)**
- **ITO Yoshiteru**
**Fujisawa-shi**
**Kanagawa 251-0012 (JP)**
- **ONO Koji**
**Fujisawa-shi**
**Kanagawa 251-0012 (JP)**
- **MAEZAKI Hironobu**
**Fujisawa-shi**
**Kanagawa 251-0012 (JP)**
- **NII Noriyuki**
**Fujisawa-shi**
**Kanagawa 251-0012 (JP)**

(74) Representative: **Huenges, Martin**
**Maiwald Patentanwalts GmbH**
**Elisenhof**
**Elisenstrasse 3**
**80335 München (DE)**

(54) **PYRIDAZINE COMPOUND**

(57) The present provides a pyridazine compound having an inhibiting effect on Stearoyl-CoA desaturase (SCD) (in particular, SCD1). The present provides a compound represented by formula

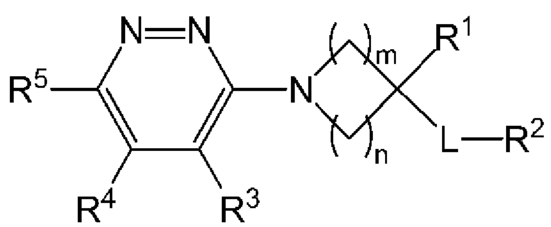

(I)

(where each symbol is as defined as in the Specification) or a salt thereof

EP 3 118 200 A1

**Description**

Technical Field

The present invention relates to a novel compound or a salt thereof which has an inhibitory effect on stearoyl-CoA desaturase (hereinafter, also referred to as SCD), particularly, SCD1, a subtype of SCD. The present invention further relates to a medicament for the prevention or treatment of SCD1-related diseases including cancer and the like, comprising the compound or a salt thereof

[Background of the invention]

SCD, which is an enzyme localized in the endoplasmic reticulum, is a rate-limiting enzyme of monovalent unsaturated fatty acid synthesis and introduces a double bond to the Δ9-Δ10 position of a saturated fatty acid. SCD has selectivity for palmitic acid and stearic acid and converts these acids to palmitoleic acid and oleic acid. Because of the diverse functions of monovalent unsaturated fatty acids, variations in SCD activity might influence various metabolisms or signal pathways involved in the malignant transformation of cells or the growth of cancer cells, etc.

Two types of SCD genes have been cloned in rats (SCD1 and SCD2) (GenBank ACCESSION NO.: NM_139192; GenBank ACCESSION NO.: NM_031841), while four types of SCD genes have been cloned in mice (SCD1, SCD2, SCD3 and SCD4) (GenBank ACCESSION NO.: NM_009127; GenBank ACCESSION NO.: NM_009128; GenBank ACCESSION NO.: NM_024450; GenBank ACCESSION NO.: NM_183216).

In humans, two types of genes (SCD1 and SCD5) have been cloned (GenBank ACCESSION NO.: NM_005063; GenBank ACCESSION NO.: NM_001037582). The amino acid sequences of human SCD1 and mouse SCD1 have a homology as high as 85%. On the other hand, SCD5 is expressed in the brain and the pancreas, and its involvement in the regulation of nerve cell growth and differentiation has been suggested.

From the knockdown experiments of human urinary bladder cancer cells, it has been found that the knockdown of SCD1 inhibits cell growth and induces apoptosis. It has also been found that the inhibition of cell growth by the SCD1 knockdown is performed in a manner depending on fatty acid unsaturation. Thus, the possibility has been suggested that SCD1 inhibitors are useful for the treatment or prevention of cancer (Patent Literature 10).

Patent Literature 9 discloses pyridazine compounds as compounds having a SCD inhibitory effect.

Patent Literatures 3, 4, 8, 10 and 11 and Non Patent Literatures 1 and 2 disclose pyridazine compounds as compounds having a SCD1 inhibitory effect. Patent Literatures 3, 4 and 10 suggest that the compounds disclosed in these literatures have an anticancer effect.

Patent Literatures 1, 2, 5, 6 and 7 disclose pyridazine compounds having pharmacological activity. Patent Literature 1 suggests that the compounds disclosed in this literature have an anticancer effect through the inhibition of Hedgehog/Smo (Smoothened) signaling. Patent Literature 5 suggests that the compounds disclosed in this literature have a suppressive effect on cytokine-mediated diseases including sarcoidosis.

Citation List

Patent Literature

Patent Literature 1: International Publication No. WO2010/007120
Patent Literature 2: U.S. Patent No. 5001125
Patent Literature 3: International Publication No. WO2008/062276
Patent Literature 4: International Publication No. WO2006/034338
Patent Literature 5: International Publication No. WO01/42241
Patent Literature 6: International Publication No. WO00/23444
Patent Literature 7: European Patent Application Publication No. EP320032
Patent Literature 8: International Publication No. WO2007/009236
Patent Literature 9: International Publication No. WO2008/096746
Patent Literature 10: International Publication No. WO2013/056148
Patent Literature 11: International Publication No. WO2013/134546


Non Patent Literature

Non Patent Literature 1: Liu et al., Journal of Medicinal Chemistry, 2007, 50 (13), pp 3086-3100
Non Patent Literature 2: Isabel et al., Bioorganic & Medicinal Chemistry Letters, 21 (1), 1 January 2011, Pages 479-483

Summary of Invention

Technical Problem

An object of the present invention is to provide a compound that has an excellent SCD1 inhibitory effect and is sufficiently satisfactory as a medicament.

Solution to Problem

The present inventors have conducted diligent studies in light of the background described above and consequently completed the present invention by finding that a compound represented by the formula given below has the activity of selectively inhibiting SCD1. Thus, the present invention is as follows.

[1] A compound represented by a formula:

[Formula 1]

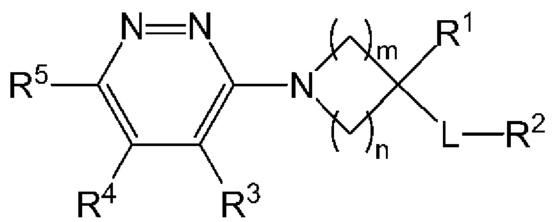

(I)

wherein

$R^1$ represents $R^6$, CN or $OR^7$;
$R^2$ represents an optionally substituted aromatic hydrocarbon group or an optionally substituted aromatic heterocyclic group;
one of $R^3$ and $R^4$ represents a hydrogen atom, and the other represents a hydrogen atom or a substituent;
$R^5$ represents an optionally substituted aromatic heterocyclic group;
$R^6$ represents an optionally halogenated $C_{1-6}$ alkyl group, an optionally substituted $C_{3-6}$ cycloalkyl group, an optionally substituted $C_{2-6}$ alkenyl group or an optionally substituted $C_{2-6}$ alkynyl group;
$R^7$ represents an optionally substituted $C_{3-6}$ cycloalkyl group or an optionally substituted $C_{1-6}$ alkyl group;
L represents a bond or a chain linker with a principal chain having 1 to 3 atoms; and
m and n are the same or different and each represents 1 or 2,

or a salt thereof (herein, the compound or a salt thereof may be also abbreviated as "compound (I)").
[2] The compound or a salt thereof according to [1], wherein $R^1$ is an optionally halogenated $C_{1-6}$ alkyl group.
[3] The compound or a salt thereof according to [1] or [2], wherein $R^2$ is a $C_{6-14}$ aryl group optionally substituted by 1 to 3 substituents selected from

(1) a halogen atom,
(2) a $C_{1-6}$ alkyl group optionally substituted by 1 to 3 halogen atoms,
(3) a $C_{2-6}$ alkynyl group,
(4) a $C_{1-6}$ alkoxy group optionally substituted by 1 to 3 halogen atoms, and
(5) a sulfanyl group optionally substituted by 1 to 5 halogen atoms.

[4] The compound or a salt thereof according to any of [1] to [3], wherein both of $R^3$ and $R^4$ are a hydrogen atom.
[5] The compound or a salt thereof according to any of [1] to [4], wherein $R^5$ is a nitrogen-containing 5-membered aromatic heterocyclic group optionally substituted by 1 to 3 substituents selected from

(1) a $C_{1-6}$ alkyl group,

(2) a hydroxy-$C_{1-6}$ alkyl group,
(3) a $C_{1-6}$ alkyl-carbonyloxy-$C_{1-6}$ alkyl group,
(4) a $C_{6-14}$ aryl-carbonyloxy-$C_{1-6}$ alkyl group,
(5) a $C_{1-6}$ alkoxy-$C_{7-16}$ aralkyloxy-$C_{1-6}$ alkyl group, and
(6) a $C_{1-6}$ alkoxy-$C_{1-6}$ alkyl group.

[6] The compound or a salt thereof according to any of [1] to [5], wherein L is a bond.
[7] The compound or a salt thereof according to any of [1] to [6], wherein both of m and n are 2.
[8] The compound or a salt thereof according to [1], wherein
$R^1$ is CN, $R^6$ or $OR^7$;
$R^2$ is a $C_{6-14}$ aryl group optionally substituted by 1 to 3 substituents selected from

(1) a halogen atom,
(2) a $C_{1-6}$ alkyl group optionally substituted by 1 to 3 halogen atoms,
(3) a $C_{2-6}$ alkynyl group,
(4) a $C_{1-6}$ alkoxy group optionally substituted by 1 to 3 halogen atoms, and
(5) a sulfanyl group optionally substituted by 1 to 5 halogen atoms;

each of $R^3$ and $R^4$ is a hydrogen atom;
$R^5$ is a nitrogen-containing 5-membered aromatic heterocyclic group optionally substituted by 1 to 3 substituents selected from

(1) a $C_{1-6}$ alkyl group,
(2) a hydroxy-$C_{1-6}$ alkyl group,
(3) a $C_{1-6}$ alkyl-carbonyloxy-$C_{1-6}$ alkyl group,
(4) a $C_{6-14}$ aryl-carbonyloxy-$C_{1-6}$ alkyl group,
(5) a $C_{1-6}$ alkoxy-$C_{7-16}$ aralkyloxy-$C_{1-6}$ alkyl group, and
(6) a $C_{1-6}$ alkoxy-$C_{1-6}$ alkyl group;

$R^6$ is

(1) a $C_{1-6}$ alkyl group optionally substituted by 1 to 3 halogen atoms, or
(2) a $C_{2-6}$ alkynyl group;

$R^7$ is a $C_{1-6}$ alkyl group;
L is a bond or a chain linker having a principal chain formed from 1 to 3 atoms selected from the group consisting of carbon, oxygen, nitrogen and sulfur; and
m and n are the same or different and each represents 1 or 2.
[9] (5-(6-(4-(Trifluoromethyl)-4-(4-(trifluoromethyl)phenyl)piperidin-1-yl)pyridazin-3-yl)-1,3,4-oxadiazol-2-yl)methanol, or a salt thereof
[10] (5-(6-(4-(Trifluoromethyl)-4-(4-(trifluoromethyl)phenyl)piperidin-1-yl)pyridazin-3-yl)-1,2,4-thiadiazol-3-yl)methanol, or a salt thereof
[11] (5-(6-(4-(Trifluoromethyl)-4-(4-(trifluoromethyl)phenyl)piperidin-1-yl)pyridazin-3-yl)-1,3,4-thiadiazol-2-yl)methanol, or a salt thereof
[12] A medicament comprising a compound or a salt thereof according to any of [1] to [11].
[13] The medicament according to [12], wherein the medicament is a SCD1 inhibitor.
[14] The medicament according to [12] or [13], wherein the medicament is a prophylactic or therapeutic agent for cancer.
[15] A method of inhibiting SCD1 in a mammal, comprising administering an effective amount of a compound or a salt thereof according to any of [1] to [11] to the mammal.
[16] A method for preventing or treating cancer in a mammal, comprising administering an effective amount of a compound or a salt thereof according to any of [1] to [11] to the mammal.
[17] The compound or a salt thereof according to any of [1] to [11] for use in the prevention or treatment of cancer.
[18] Use of a compound or a salt thereof according to any of [1] to [11] for the production of a prophylactic or therapeutic agent for cancer.

Advantageous Effects of Invention

The compound or the medicament of the present invention has an effect of specifically inhibiting SCD1 with high selectivity. Thus, the compound or the medicament of the present invention can be used as a SCD1 inhibitor and is useful as a prophylactic or therapeutic agent for diseases that would be probably influenced by SCD1, for example, cancer.

(Detailed Description of the Invention)

Hereinafter, the compound of the present invention, a method for producing the same and use of the same will be described in detail.
The definition of each substituent used in the present specification is described in detail in the following. Unless otherwise specified, each substituent has the following definition.
In the present specification, examples of the "halogen atom" include fluorine, chlorine, bromine and iodine.
In the present specification, examples of the "$C_{1-6}$ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl.
In the present specification, examples of the "optionally halogenated $C_{1-6}$ alkyl group" include a $C_{1-6}$ alkyl group optionally having 1 to 7, preferably 1 to 5, halogen atoms. Specific examples thereof include methyl, chloromethyl, difluoromethyl, trichloromethyl, trifluoromethyl, ethyl, 2-bromoethyl, 2,2,2-trifluoroethyl, tetrafluoroethyl, pentafluoroethyl, propyl, 2,2-difluoropropyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl and 6,6,6-trifluorohexyl.
In the present specification, examples of the "$C_{2-6}$ alkenyl group" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl and 5-hexenyl.
In the present specification, examples of the "$C_{2-6}$ alkynyl group" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and 4-methyl-2-pentynyl.
In the present specification, examples of the "$C_{3-10}$ cycloalkyl group" include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl and adamantyl.
In the present specification, examples of the "optionally halogenated $C_{3-10}$ cycloalkyl group" include a $C_{3-10}$ cycloalkyl group optionally having 1 to 7, preferably 1 to 5, halogen atoms. Specific examples thereof include cyclopropyl, 2,2-difluorocyclopropyl, 2,3-difluorocyclopropyl, cyclobutyl, difluorocyclobutyl, cyclopentyl, cyclohexyl, cycloheptyl and cyclooctyl.
In the present specification, examples of the "$C_{3-10}$ cycloalkenyl group" include cyclopropenyl, cyclobutenyl, cyclopentenyl, cyclohexenyl, cycloheptenyl and cyclooctenyl.
In the present specification, examples of the "$C_{6-14}$ aryl group" include phenyl, 1-naphthyl, 2-naphthyl, 1-anthryl, 2-anthryl and 9-anthryl.
In the present specification, examples of the "$C_{7-16}$ aralkyl group" include benzyl, phenethyl, naphthylmethyl and phenylpropyl.
In the present specification, examples of the "$C_{1-6}$ alkoxy group" include methoxy, ethoxy, propoxy, isopropoxy, butoxy, isobutoxy, sec-butoxy, tert-butoxy, pentyloxy and hexyloxy.
In the present specification, examples of the "optionally halogenated $C_{1-6}$ alkoxy group" include a $C_{1-6}$ alkoxy group optionally having 1 to 7, preferably 1 to 5, halogen atoms. Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy and hexyloxy.
In the present specification, examples of the "$C_{3-10}$ cycloalkyloxy group" include cyclopropyloxy, cyclobutyloxy, cyclopentyloxy, cyclohexyloxy, cycloheptyloxy and cyclooctyloxy.
In the present specification, examples of the "$C_{1-6}$ alkylthio group" include methylthio, ethylthio, propylthio, isopropylthio, butylthio, sec-butylthio, tert-butylthio, pentylthio and hexylthio.
In the present specification, examples of the "optionally halogenated $C_{1-6}$ alkylthio group" include a $C_{1-6}$ alkylthio group optionally having 1 to 7, preferably 1 to 5, halogen atoms. Specific examples thereof include methylthio, difluoromethylthio, trifluoromethylthio, ethylthio, propylthio, isopropylthio, butylthio, 4,4,4-trifluorobutylthio, pentylthio and hexylthio.
In the present specification, examples of the "$C_{1-6}$ alkyl-carbonyl group" include acetyl, propanoyl, butanoyl, 2-methylpropanoyl, pentanoyl, 3-methylbutanoyl, 2-methylbutanoyl, 2,2-dimethylpropanoyl, hexanoyl and heptanoyl.
In the present specification, examples of the "optionally halogenated $C_{1-6}$ alkyl-carbonyl group" include a $C_{1-6}$ alkyl-carbonyl group optionally having 1 to 7, preferably 1 to 5, halogen atoms. Specific examples thereof include acetyl,

chloroacetyl, trifluoroacetyl, trichloroacetyl, propanoyl, butanoyl, pentanoyl and hexanoyl.

In the present specification, examples of the "$C_{1-6}$ alkoxy-carbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl and hexylo xycarbonyl.

In the present specification, examples of the "$C_{6-14}$ aryl-carbonyl group" include benzoyl, 1-naphthoyl and 2-naphthoyl.

In the present specification, examples of the "$C_{7-16}$ aralkyl-carbonyl group" include phenylacetyl and phenylpropionyl.

In the present specification, examples of the "5- to 14-membered aromatic heterocyclylcarbonyl group" include nicotinoyl, isonicotinoyl, thenoyl and furoyl.

In the present specification, examples of the "3- to 14-membered non-aromatic heterocyclylcarbonyl group" include morpholinylcarbonyl, piperidinylcarbonyl and pyrrolidinylcarbonyl.

In the present specification, examples of the "mono- or di-$C_{1-6}$ alkyl-carbamoyl group" include methylcarbamoyl, ethylcarbamoyl, dimethylcarbamoyl, diethylcarbamoyl and N-ethyl-N-methylcarbamoyl.

In the present specification, examples of the "mono- or di-$C_{7-16}$ aralkyl-carbamoyl group" include benzylcarbamoyl and phenethylcarbamoyl.

In the present specification, examples of the "$C_{1-6}$ alkylsulfonyl group" include methylsulfonyl, ethylsulfonyl, propylsulfonyl, isopropylsulfonyl, butylsulfonyl, sec-butylsulfonyl and tert-butylsulfonyl.

In the present specification, examples of the "optionally halogenated $C_{1-6}$ alkylsulfonyl group" include a $C_{1-6}$ alkylsulfonyl group optionally having 1 to 7, preferably 1 to 5, halogen atoms. Specific examples thereof include methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl and hexylsulfonyl.

In the present specification, examples of the "$C_{6-14}$ arylsulfonyl group" include phenylsulfonyl, 1-naphthylsulfonyl and 2-naphthylsulfonyl.

In the present specification, examples of the "substituent" include a halogen atom, a cyano group, a nitro group, an optionally substituted hydrocarbon group, an optionally substituted heterocyclic group, an acyl group, an optionally substituted amino group, an optionally substituted carbamoyl group, an optionally substituted thiocarbamoyl group, an optionally substituted sulfamoyl group, an optionally substituted hydroxy group, an optionally substituted sulfanyl (SH) group and an optionally substituted silyl group.

In the present specification, examples of the "hydrocarbon group" (including "hydrocarbon group" of "optionally substituted hydrocarbon group") include a $C_{1-6}$ alkyl group, a $C_{2-6}$ alkenyl group, a $C_{2-6}$ alkynyl group, a $C_{3-10}$ cycloalkyl group, a $C_{3-10}$ cycloalkenyl group, a $C_{6-14}$ aryl group and a $C_{7-16}$ aralkyl group.

In the present specification, examples of the "optionally substituted hydrocarbon group" include a hydrocarbon group optionally having substituent(s) selected from the following substituent group A.

[substituent group A]

(1) a halogen atom,
(2) a nitro group,
(3) a cyano group,
(4) an oxo group,
(5) a hydroxy group,
(6) an optionally halogenated $C_{1-6}$ alkoxy group,
(7) a $C_{6-14}$ aryloxy group (e.g., phenoxy, naphthoxy),
(8) a $C_{7-16}$ aralkyloxy group (e.g., benzyloxy),
(9) a 5- to 14-membered aromatic heterocyclyloxy group (e.g., pyridyloxy),
(10) a 3- to 14-membered non-aromatic heterocyclyloxy group (e.g., morpholinyloxy, piperidinyloxy),
(11) a $C_{1-6}$ alkyl-carbonyloxy group (e.g., acetoxy, propanoyloxy),
(12) a $C_{6-14}$ aryl-carbonyloxy group (e.g., benzoyloxy, 1-naphthoyloxy, 2-naphthoyloxy),
(13) a $C_{1-6}$ alkoxy-carbonyloxy group (e.g., methoxycarbonyloxy, ethoxycarbonyloxy, propoxycarbonyloxy, butoxycarbonyloxy),
(14) a mono- or di-$C_{1-6}$ alkyl-carbamoyloxy group (e.g., methylcarbamoyloxy, ethylcarbamoyloxy, dimethylcarbamoyloxy, diethylcarbamoyloxy),
(15) a $C_{6-14}$ aryl-carbamoyloxy group (e.g., phenylcarbamoyloxy, naphthylcarbamoyloxy),
(16) a 5- to 14-membered aromatic heterocyclylcarbonyloxy group (e.g., nicotinoyloxy),
(17) a 3- to 14-membered non-aromatic heterocyclylcarbonyloxy group (e.g., morpholinylcarbonyloxy, piperidinylcarbonyloxy),
(18) an optionally halogenated $C_{1-6}$ alkylsulfonyloxy group (e.g., methylsulfonyloxy, trifluoromethylsulfonylo xy),
(19) a $C_{6-14}$ arylsulfonyloxy group optionally substituted by a $C_{1-6}$ alkyl group (e.g., phenylsulfonyloxy, toluenesulfonyloxy),
(20) an optionally halogenated $C_{1-6}$ alkylthio group,
(21) a 5- to 14-membered aromatic heterocyclic group,
(22) a 3- to 14-membered non-aromatic heterocyclic group,
(23) a formyl group,
(24) a carboxy group,
(25) an optionally halogenated $C_{1-6}$ alkyl-carbonyl group,
(26) a $C_{6-14}$ aryl-carbonyl group,
(27) a 5- to 14-membered aromatic heterocyclylcarbonyl group,
(28) a 3- to 14-membered non-aromatic heterocyclylcarbonyl group,
(29) a $C_{1-6}$ alkoxy-carbonyl group,
(30) a $C_{6-14}$ aryloxy-carbonyl group (e.g., phenyloxycarbonyl, 1-naphthyloxycarbonyl, 2-naphthyloxycarbonyl),
(31) a $C_{7-16}$ aralkyloxy-carbonyl group (e.g., benzyloxycarbonyl, phenethyloxycarbonyl),
(32) a carbamoyl group,
(33) a thiocarbamoyl group,
(34) a mono- or di-$C_{1-6}$ alkyl-carbamoyl group,
(35) a $C_{6-14}$ aryl-carbamoyl group (e.g., phenylcarbamoyl),
(36) a 5- to 14-membered aromatic heterocyclylcarbamoyl group (e.g., pyridylcarbamoyl, thienylcarbamoyl),
(37) a 3- to 14-membered non-aromatic heterocyclylcarbamoyl group (e.g., morpholinylcarbamoyl, piperidinylcarbamoyl),
(38) an optionally halogenated $C_{1-6}$ alkylsulfonyl group,
(39) a $C_{6-14}$ arylsulfonyl group,
(40) a 5- to 14-membered aromatic heterocyclylsulfonyl group (e.g., pyridylsulfonyl, thienylsulfonyl),
(41) an optionally halogenated $C_{1-6}$ alkylsulfinyl group,
(42) a $C_{6-14}$ arylsulfinyl group (e.g., phenylsulfinyl, 1-naphthylsulfinyl, 2-naphthylsulfinyl),
(43) a 5- to 14-membered aromatic heterocyclylsulfinyl group (e.g., pyridylsulfinyl, thienylsulfinyl),
(44) an amino group,
(45) a mono- or di-$C_{1-6}$ alkylamino group (e.g., methylamino, ethylamino, propylamino, isopropylamino, butylamino, dimethylamino, diethylamino, dipropylamino, dibutylamino, N-ethyl-N-methylamino),
(46) a mono- or di-$C_{6-14}$ arylamino group (e.g., phenylamino),
(47) a 5- to 14-membered aromatic heterocyclylamino group (e.g., pyridylamino),
(48) a $C_{7-16}$ aralkylamino group (e.g., benzylamino),
(49) a formylamino group,
(50) a $C_{1-6}$ alkyl-carbonylamino group (e.g., acetylamino, propanoylamino, butanoylamino),
(51) a ($C_{1-6}$ alkyl)($C_{1-6}$ alkyl-carbonyl)amino group (e.g., N-acetyl-N-methylamino),
(52) a $C_{6-14}$ aryl-carbonylamino group (e.g., phenylcarbonylamino, naphthylcarbonylamino),
(53) a $C_{1-6}$ alkoxy-carbonylamino group (e.g., methoxycarbonylamino, ethoxycarbonylamino, propoxycarbonylamino, butoxycarbonylamino, tert-butoxycarbonylamino),
(54) a $C_{7-16}$ aralkyloxy-carbonylamino group (e.g., benzyloxycarbonylamino),
(55) a $C_{1-6}$ alkylsulfonylamino group (e.g., methylsulfonylamino, ethylsulfonylamino),
(56) a $C_{6-14}$ arylsulfonylamino group optionally substituted by a $C_{1-6}$ alkyl group (e.g., phenylsulfonylamino, toluenesulfonylamino),
(57) an optionally halogenated $C_{1-6}$ alkyl group,
(58) a $C_{2-6}$ alkenyl group,
(59) a $C_{2-6}$ alkynyl group,
(60) a $C_{3-10}$ cycloalkyl group,
(61) a $C_{3-10}$ cycloalkenyl group and
(62) a $C_{6-14}$ aryl group.

The number of the above-mentioned substituents in the "optionally substituted hydrocarbon group" is, for example, 1 to 5, preferably 1 to 3. When the number of the substituents is two or more, the respective substituents may be the same or different.

In the present specification, examples of the "heterocyclic group" (including "heterocyclic group" of "optionally substituted heterocyclic group") include (i) an aromatic heterocyclic group, (ii) a non-aromatic heterocyclic group and (iii) a 7- to 10-membered bridged heterocyclic group, each containing, as a ring-constituting atom besides carbon atom,

1 to 4 hetero atoms selected from a nitrogen atom, a sulfur atom and an oxygen atom.

In the present specification, examples of the "aromatic heterocyclic group" (including "5- to 14-membered aromatic heterocyclic group") include a 5- to 14-membered (preferably 5- to 10-membered) aromatic heterocyclic group containing, as a ring-constituting atom besides carbon atom, 1 to 4 hetero atoms selected from a nitrogen atom, a sulfur atom and an oxygen atom.

Preferable examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and 8- to 14-membered fused polycyclic (preferably bi or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl and the like.

In the present specification, examples of the "non-aromatic heterocyclic group" (including "3- to 14-membered non-aromatic heterocyclic group") include a 3- to 14-membered (preferably 4- to 10-membered) non-aromatic heterocyclic group containing, as a ring-constituting atom besides carbon atom, 1 to 4 hetero atoms selected from a nitrogen atom, a sulfur atom and an oxygen atom.

Preferable examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisooxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; and

9- to 14-membered fused polycyclic (preferably bi or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl and the like.

In the present specification, preferable examples of the "7- to 10-membered bridged heterocyclic group" include quinuclidinyl and 7-azabicyclo[2.2.1]heptanyl.

In the present specification, examples of the "nitrogen-containing heterocyclic group" include a "heterocyclic group" containing at least one nitrogen atom as a ring-constituting atom.

In the present specification, examples of the "optionally substituted heterocyclic group" include a heterocyclic group optionally having substituent(s) selected from the aforementioned substituent group A.

The number of the substituents in the "optionally substituted heterocyclic group" is, for example, 1 to 3. When the number of the substituents is two or more, the respective substituents may be the same or different.

In the present specification, examples of the "acyl group" include a formyl group, a carboxy group, a carbamoyl group, a thiocarbamoyl group, a sulfino group, a sulfo group, a sulfamoyl group and a phosphono group, each optionally having "1 or 2 substituents selected from a $C_{1-6}$ alkyl group, a $C_{2-6}$ alkenyl group, a $C_{3-10}$ cycloalkyl group, a $C_{3-10}$ cycloalkenyl group, a $C_{6-14}$ aryl group, a $C_{7-16}$ aralkyl group, a 5- to 14-membered aromatic heterocyclic group and a 3- to 14-membered non-aromatic heterocyclic group, each of which optionally has 1 to 3 substituents selected from a halogen atom, an optionally halogenated $C_{1-6}$ alkoxy group, a hydroxy group, a nitro group, a cyano group, an amino group and a carbamoyl group".

Examples of the "acyl group" also include a hydrocarbon-sulfonyl group, a heterocyclylsulfonyl group, a hydrocarbon-sulfinyl group and a heterocyclylsulfinyl group.

Here, the hydrocarbon-sulfonyl group means a hydrocarbon group-bonded sulfonyl group, the heterocyclylsulfonyl group means a heterocyclic group-bonded sulfonyl group, the hydrocarbon-sulfinyl group means a hydrocarbon group-bonded sulfinyl group and the heterocyclylsulfinyl group means a heterocyclic group-bonded sulfinyl group.

Preferable examples of the "acyl group" include a formyl group, a carboxy group, a $C_{1-6}$ alkyl-carbonyl group, a $C_{2-6}$ alkenyl-carbonyl group (e.g., crotonoyl), a $C_{3-10}$ cycloalkylcarbonyl group (e.g., cyclobutanecarbonyl, cyclopentanecarbonyl, cyclohexanecarbonyl, cycloheptanecarbonyl), a $C_{3-10}$ cycloalkenyl-carbonyl group (e.g., 2-cyclohexenecarbonyl), a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a $C_{6-14}$ aryloxy-

carbonyl group (e.g., phenyloxycarbonyl, naphthyloxycarbonyl), a $C_{7-16}$ aralkyloxy-carbonyl group (e.g., benzyloxycarbonyl, phenethyloxycarbonyl), a carbamoyl group, a mono- or di-$C_{1-6}$ alkyl-carbamoyl group, a mono- or di-$C_{2-6}$ alkenyl-carbamoyl group (e.g., diallylcarbamoyl), a mono- or di-$C_{3-10}$ cycloalkyl-carbamoyl group (e.g., cyclopropylcarbamoyl), a mono- or di-$C_{6-14}$ aryl-carbamoyl group (e.g., phenylcarbamoyl), a mono- or di-$C_{7-16}$ aralkyl-carbamoyl group, a 5- to 14-membered aromatic heterocyclylcarbamoyl group (e.g., pyridylcarbamoyl), a thiocarbamoyl group, a mono- or di-$C_{1-6}$ alkyl-thiocarbamoyl group (e.g., methylthiocarbamoyl, N-ethyl-N-methylthiocarbamoyl), a mono- or di-$C_{2-6}$ alkenyl-thiocarbamoyl group (e.g., diallylthiocarbamoyl), a mono- or di-$C_{3-10}$ cycloalkyl-thiocarbamoyl group (e.g., cyclopropylthiocarbamoyl, cyclohexylthiocarbamoyl), a mono- or di-$C_{6-14}$ aryl-thiocarbamoyl group (e.g., phenylthiocarbamoyl), a mono- or di-$C_{7-16}$ aralkyl-thiocarbamoyl group (e.g., benzylthiocarbamoyl, phenethylthiocarbamoyl), a 5- to 14-membered aromatic heterocyclylthiocarbamoyl group (e.g., pyridylthiocarbamoyl), a sulfino group, a $C_{1-6}$ alkylsulfinyl group (e.g., methylsulfinyl, ethylsulfinyl), a sulfo group, a $C_{1-6}$ alkylsulfonyl group, a $C_{6-14}$ arylsulfonyl group, a phosphono group and a mono- or di-$C_{1-6}$ alkylphosphono group (e.g., dimethylphosphono, diethylphosphono, diisopropylphosphono, dibutylphosphono).

In the present specification, examples of the "optionally substituted amino group" include an amino group optionally having "1 or 2 substituents selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group, a mono- or di-$C_{7-16}$ aralkyl-carbamoyl group, a $C_{1-6}$ alkylsulfonyl group and a $C_{6-14}$ arylsulfonyl group, each of which optionally has 1 to 3 substituents selected from substituent group A".

Preferable examples of the optionally substituted amino group include an amino group, a mono- or di-(optionally halogenated $C_{1-6}$ alkyl)amino group (e.g., methylamino, trifluoromethylamino, dimethylamino, ethylamino, diethylamino, propylamino, dibutylamino), a mono- or di-$C_{2-6}$ alkenylamino group (e.g., diallylamino), a mono- or di-$C_{3-10}$ cycloalkylamino group (e.g., cyclopropylamino, cyclohexylamino), a mono- or di-$C_{6-14}$ arylamino group (e.g., phenylamino), a mono- or di-$C_{7-16}$ aralkylamino group (e.g., benzylamino, dibenzylamino), a mono- or di-(optionally halogenated $C_{1-6}$ alkyl)-carbonylamino group (e.g., acetylamino, propionylamino), a mono- or di-$C_{6-14}$ aryl-carbonylamino group (e.g., benzoylamino), a mono- or di-$C_{7-16}$ aralkyl-carbonylamino group (e.g., benzylcarbonylamino), a mono- or di-5- to 14-membered aromatic heterocyclylcarbonylamino group (e.g., nicotinoylamino, isonicotinoylamino), a mono- or di-3- to 14-membered non-aromatic heterocyclylcarbonylamino group (e.g., piperidinylcarbonylamino), a mono- or di-$C_{1-6}$ alkoxy-carbonylamino group (e.g., tert-butoxycarbonylamino), a 5- to 14-membered aromatic heterocyclylamino group (e.g., pyridylamino), a carbamoylamino group, a (mono- or di-$C_{1-6}$ alkyl-carbamoyl)amino group (e.g., methylcarbamoylamino), a (mono- or di-$C_{7-16}$ aralkyl-carbamoyl)amino group (e.g., benzylcarbamoylamino), a $C_{1-6}$ alkylsulfonylamino group (e.g., methylsulfonylamino, ethylsulfonylamino), a $C_{6-14}$ arylsulfonylamino group (e.g., phenylsulfonylamino), a ($C_{1-6}$ alkyl)($C_{1-6}$ alkyl-carbonyl)amino group (e.g., N-acetyl-N-methylamino) and a ($C_{1-6}$ alkyl)($C_{6-14}$ aryl-carbonyl)amino group (e.g., N-benzoyl-N-methylamino).

In the present specification, examples of the "optionally substituted carbamoyl group" include a carbamoyl group optionally having "1 or 2 substituents selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group and a mono- or di-$C_{7-16}$ aralkyl-carbamoyl group, each of which optionally has 1 to 3 substituents selected from substituent group A".

Preferable examples of the optionally substituted carbamoyl group include a carbamoyl group, a mono- or di-$C_{1-6}$ alkyl-carbamoyl group, a mono- or di-$C_{2-6}$ alkenyl-carbamoyl group (e.g., diallylcarbamoyl), a mono- or di-$C_{3-10}$ cycloalkyl-carbamoyl group (e.g., cyclopropylcarbamoyl, cyclohexylcarbamoyl), a mono- or di-$C_{6-14}$ aryl-carbamoyl group (e.g., phenylcarbamoyl), a mono- or di-$C_{7-16}$ aralkyl-carbamoyl group, a mono- or di-$C_{1-6}$ alkyl-carbonyl-carbamoyl group (e.g., acetylcarbamoyl, propionylcarbamoyl), a mono- or di-$C_{6-14}$ aryl-carbonyl-carbamoyl group (e.g., benzoylcarbamoyl) and a 5- to 14-membered aromatic heterocyclylcarbamoyl group (e.g., pyridylcarbamoyl).

In the present specification, examples of the "optionally substituted thiocarbamoyl group" include a thiocarbamoyl group optionally having "1 or 2 substituents selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group and a mono- or di-$C_{7-16}$ aralkyl-carbamoyl group, each of which optionally has 1 to 3 substituents selected from substituent group A".

Preferable examples of the optionally substituted thiocarbamoyl group include a thiocarbamoyl group, a mono- or di-$C_{1-6}$ alkyl-thiocarbamoyl group (e.g., methylthiocarbamoyl, ethylthiocarbamoyl, dimethylthiocarbamoyl, diethylthiocarbamoyl, N-ethyl-N-methylthiocarbamoyl), a mono- or di-$C_{2-6}$ alkenyl-thiocarbamoyl group (e.g., diallylthiocarbamoyl), a mono- or di-$C_{3-10}$ cycloalkyl-thiocarbamoyl group (e.g., cyclopropylthiocarbamoyl, cyclohexylthiocarbamoyl), a mono- or di-$C_{6-14}$ aryl-thiocarbamoyl group (e.g., phenylthiocarbamoyl), a mono- or di-$C_{7-16}$ aralkyl-thiocarbamoyl group (e.g., benzylthiocarbamoyl, phenethylthiocarbamoyl), a mono- or di-$C_{1-6}$ alkyl-carbonyl-thiocarbamoyl group (e.g., acetylthiocarbamoyl, propionylthiocarbamoyl), a mono- or di-$C_{6-14}$ aryl-carbonyl-thiocarbamoyl group (e.g., benzoylthiocarbamoyl) and a 5- to 14-membered aromatic heterocyclylthiocarbamoyl group (e.g., pyridylthiocarbamoyl).

In the present specification, examples of the "optionally substituted sulfamoyl group" include a sulfamoyl group optionally having "1 or 2 substituents selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group and a mono- or di-$C_{7-16}$ aralkyl-carbamoyl group, each of which optionally has 1 to 3 substituents selected from substituent group A".

Preferable examples of the optionally substituted sulfamoyl group include a sulfamoyl group, a mono- or di-$C_{1-6}$ alkyl-sulfamoyl group (e.g., methylsulfamoyl, ethylsulfamoyl, dimethylsulfamoyl, diethylsulfamoyl, N-ethyl-N-methylsulfamoyl), a mono- or di-$C_{2-6}$ alkenyl-sulfamoyl group (e.g., diallylsulfamoyl), a mono- or di-$C_{3-10}$ cycloalkyl-sulfamoyl group (e.g., cyclopropylsulfamoyl, cyclohexylsulfamoyl), a mono- or di-$C_{6-14}$ aryl-sulfamoyl group (e.g., phenylsulfamoyl), a mono- or di-$C_{7-16}$ aralkyl-sulfamoyl group (e.g., benzylsulfamoyl, phenethylsulfamoyl), a mono- or di-$C_{1-6}$ alkyl-carbonyl-sulfamoyl group (e.g., acetylsulfamoyl, propionylsulfamoyl), a mono- or di-$C_{6-14}$ aryl-carbonyl-sulfamoyl group (e.g., benzoylsulfamoyl) and a 5- to 14-membered aromatic heterocyclylsulfamoyl group (e.g., pyridylsulfamoyl).

In the present specification, examples of the "optionally substituted hydroxy group" include a hydroxyl group optionally having "a substituent selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group, a mono- or di-$C_{7-16}$ aralkyl-carbamoyl group, a $C_{1-6}$ alkylsulfonyl group and a $C_{6-14}$ arylsulfonyl group, each of which optionally has 1 to 3 substituents selected from substituent group A".

Preferable examples of the optionally substituted hydroxy group include a hydroxy group, a $C_{1-6}$ alkoxy group, a $C_{2-6}$ alkenyloxy group (e.g., allyloxy, 2-butenyloxy, 2-pentenyloxy, 3-hexenyloxy), a $C_{3-10}$ cycloalkyloxy group (e.g., cyclohexyloxy), a $C_{6-14}$ aryloxy group (e.g., phenoxy, naphthyloxy), a $C_{7-16}$ aralkyloxy group (e.g., benzyloxy, phenethyloxy), a $C_{1-6}$ alkyl-carbonyloxy group (e.g., acetyloxy, propionyloxy, butyryloxy, isobutyryloxy, pivaloyloxy), a $C_{6-14}$ aryl-carbonyloxy group (e.g., benzoyloxy), a $C_{7-16}$ aralkyl-carbonyloxy group (e.g., benzylcarbonyloxy), a 5- to 14-membered aromatic heterocyclylcarbonyloxy group (e.g., nicotinoyloxy), a 3- to 14-membered non-aromatic heterocyclylcarbonyloxy group (e.g., piperidinylcarbonyloxy), a $C_{1-6}$ alkoxy-carbonyloxy group (e.g., tert-butoxycarbonyloxy), a 5- to 14-membered aromatic heterocyclyloxy group (e.g., pyridyloxy), a carbamoyloxy group, a $C_{1-6}$ alkyl-carbamoyloxy group (e.g., methylcarbamoyloxy), a $C_{7-16}$ aralkyl-carbamoyloxy group (e.g., benzylcarbamoyloxy), a $C_{1-6}$ alkylsulfonyloxy group (e.g., methylsulfonyloxy, ethylsulfonyloxy) and a $C_{6-14}$ arylsulfonyloxy group (e.g., phenylsulfonyloxy).

In the present specification, examples of the "optionally substituted sulfanyl group" include a sulfanyl group optionally having "a substituent selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group and a 5- to 14-membered aromatic heterocyclic group, each of which optionally has 1 to 3 substituents selected from substituent group A" and a halogenated sulfanyl group.

Preferable examples of the optionally substituted sulfanyl group include a sulfanyl (-SH) group, a $C_{1-6}$ alkylthio group, a $C_{2-6}$ alkenylthio group (e.g., allylthio, 2-butenylthio, 2-pentenylthio, 3-hexenylthio), a $C_{3-10}$ cycloalkylthio group (e.g., cyclohexylthio), a $C_{6-14}$ arylthio group (e.g., phenylthio, naphthylthio), a $C_{7-16}$ aralkylthio group (e.g., benzylthio, phenethylthio), a $C_{1-6}$ alkyl-carbonylthio group (e.g., acetylthio, propionylthio, butyrylthio, isobutyrylthio, pivaloylthio), a $C_{6-14}$ aryl-carbonylthio group (e.g., benzoylthio), a 5- to 14-membered aromatic heterocyclylthio group (e.g., pyridylthio) and a halogenated thio group (e.g., pentafluorothio).

In the present specification, examples of the "optionally substituted silyl group" include a silyl group optionally having "1 to 3 substituents selected from a $C_{1-6}$ alkyl group, a $C_{2-6}$ alkenyl group, a $C_{3-10}$ cycloalkyl group, a $C_{6-14}$ aryl group and a $C_{7-16}$ aralkyl group, each of which optionally has 1 to 3 substituents selected from substituent group A".

Preferable examples of the optionally substituted silyl group include a tri-$C_{1-6}$ alkylsilyl group (e.g., trimethylsilyl, tert-butyl(dimethyl)silyl).

In the present specification, examples of the "$C_{1-6}$ alkylene group" include $-CH_2-$, $-(CH_2)_2-$, $-(CH_2)_3-$, $-(CH_2)_4-$, $-(CH_2)_5-$, $-(CH_2)_6-$, $-CH(CH_3)-$, $-C(CH_3)_2-$, $-CH(C_2H_5)-$,$-CH(C_3H_7)-$, $-CH(CH(CH_3)_2)-$, $-(CH(CH_3))_2-$, $-CH_2-CH(CH_3)-$, $-CH(CH_3)-CH_2-$, $-CH_2-CH_2-C(CH_3)_2-$, $-C(CH_3)_2-CH_2-CH_2-$, $-CH_2-CH_2-CH_2-C(CH_3)_2-$ and $-C(CH_3)_2-CH_2-CH_2-CH_2-$.

In the present specification, examples of the "$C_{2-6}$ alkenylene group" include $-CH=CH-$, $-CH_2-CH=CH-$, $-CH=CH-CH_2-$, $-C(CH_3)_2-CH=CH-$, $-CH=CH-C(CH_3)_2-$, $-CH_2-CH=CH-CH_2-$, $-CH_2-CH_2-CH=CH-$, $-CH=CH-CH_2-CH_2-$, $-CH=CH-CH=CH-$, $-CH=CH-CH_2-CH_2-CH_2-$ and $-CH_2-CH_2-CH_2-CH=CH-$.

In the present specification, examples of the "$C_{2-6}$ alkynylene group" include - C≡C-, -$CH_2$-C≡C-, -C≡C-$CH_2$-, -$C(CH_3)_2$-C≡C-, -C≡C-$C(CH_3)_2$-, -$CH_2$-C≡C-$CH_2$-, -$CH_2$-$CH_2$-C≡C-, -C≡C-$CH_2$-$CH_2$-, -C≡C-C≡C-, -C≡C-$CH_2$-$CH_2$-$CH_2$- and -$CH_2$-$CH_2$-$CH_2$-C≡C-.

In the present specification, examples of the "hydrocarbon ring" include a $C_{6-14}$ aromatic hydrocarbon ring, $C_{3-10}$ cycloalkane and $C_{3-10}$ cycloalkene.

In the present specification, examples of the "$C_{6-14}$ aromatic hydrocarbon ring" include benzene and naphthalene.

In the present specification, examples of the "$C_{3-10}$ cycloalkane" include cyclopropane, cyclobutane, cyclopentane, cyclohexane, cycloheptane and cyclooctane.

In the present specification, examples of the "$C_{3-10}$ cycloalkene" include cyclopropene, cyclobutene, cyclopentene, cyclohexene, cycloheptene and cyclooctene.

In the present specification, examples of the "heterocycle" include an aromatic heterocycle and a non-aromatic heterocycle, each containing, as a ring-constituting atom besides carbon atom, 1 to 4 hetero atoms selected from a nitrogen atom, a sulfur atom and an oxygen atom.

In the present specification, examples of the "aromatic heterocycle" include a 5-to 14-membered (preferably 5- to 10-membered) aromatic heterocycle containing, as a ring-constituting atom besides carbon atom, 1 to 4 hetero atoms selected from a nitrogen atom, a sulfur atom and an oxygen atom. Preferable examples of the "aromatic heterocycle" include 5-or 6-membered monocyclic aromatic heterocycles such as thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine and the like; and

8- to 14-membered fused polycyclic (preferably bi or tricyclic) aromatic heterocycles such as benzothiophene, benzofuran, benzimidazole, benzoxazole, benzisoxazole, benzothiazole, benzisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, naphtho[2,3-b]thiophene, phenoxathiin, indole, isoindole, 1H-indazole, purine, isoquinoline, quinoline, phthalazine, naphthyridine, quinoxaline, quinazoline, cinnoline, carbazole, β-carboline, phenanthridine, acridine, phenazine, phenothiazine, phenoxazine and the like.

In the present specification, examples of the "non-aromatic heterocycle" include a 3- to 14-membered (preferably 4- to 10-membered) non-aromatic heterocycle containing, as a ring-constituting atom besides carbon atom, 1 to 4 hetero atoms selected from a nitrogen atom, a sulfur atom and an oxygen atom. Preferable examples of the "non-aromatic heterocycle" include 3- to 8-membered monocyclic non-aromatic heterocycles such as aziridine, oxirane, thiirane, azetidine, oxetane, thietane, tetrahydrothiophene, tetrahydrofuran, pyrroline, pyrrolidine, imidazoline, imidazolidine, oxazoline, oxazolidine, pyrazoline, pyrazolidine, thiazoline, thiazolidine, tetrahydroisothiazole, tetrahydrooxazole, tetrahydroisoxazole, piperidine, piperazine, tetrahydropyridine, dihydropyridine, dihydrothiopyran, tetrahydropyrimidine, tetrahydropyridazine, dihydropyran, tetrahydropyran, tetrahydrothiopyran, morpholine, thiomorpholine, azepanine, diazepane, azepine, azocane, diazocane, oxepane and the like; and

9- to 14-membered fused polycyclic (preferably bi or tricyclic) non-aromatic heterocycles such as dihydrobenzofuran, dihydrobenzimidazole, dihydrobenzoxazole, dihydrobenzothiazole, dihydrobenzisothiazole, dihydronaphtho[2,3-b]thiophene, tetrahydroisoquinoline, tetrahydroquinoline, 4H-quinolizine, indoline, isoindoline, tetrahydrothieno[2,3-c]pyridine, tetrahydrobenzazepine, tetrahydroquinoxaline, tetrahydrophenanthridine, hexahydrophenothiazine, hexahydrophenoxazine, tetrahydrophthalazine, tetrahydronaphthyridine, tetrahydroquinazoline, tetrahydrocinnoline, tetrahydrocarbazole, tetrahydro-β-carboline, tetrahydroacridine, tetrahydrophenazine, tetrahydrothioxanthene, octahydroisoquinoline and the like.

In the present specification, examples of the "nitrogen-containing heterocycle" include a "heterocycle" containing at least one nitrogen atom as a ring-constituting atom.

In the present specification, examples of the "optionally substituted hydrocarbon group" further include a hydrocarbon group optionally having the following substituent(s):

(63) a $C_{1-6}$ alkoxy-$C_{7-16}$ aralkyloxy group (e.g., methoxybenzyloxy).

In the present specification, the "linker" refers to an atomic group that intervenes between two atomic groups in order to link the two atomic groups. The linker has a principal chain formed from atom(s) selected from the group consisting of, for example, carbon, oxygen, nitrogen and sulfur. The atoms constituting the principal chain may be bonded with each other through a single bond, a double bond or a triple bond. Examples of the linker include an alkylene group, an alkenylene group, an alkynylene group, -O-, -S-, -NH-, -C(O)-, -C(S)-, - C(=NH)- and -CH=N- which are each optionally further substituted by 1 to 3 halogen atoms (particularly, a fluorine atom), and a group formed by the linkage of a combination thereof.

In the present specification, examples of the "chain linker with a principal chain having 1 to 3 atoms" include a

chain linker that is formed by each alone of $-CH_2-$, -CH=CH-, - C≡C-, -O-, -S-, -NH-, -C(O)-, -C(S)-, -C(=NH)- and -CH=N- which are each optionally further substituted by 1 to 3 halogen atoms (particularly, a fluorine atom), or formed by the linkage of a combination thereof, and has a principal chain having 1 to 3 atoms.

Preferable examples of $R^1$, $R^2$, $R^3$, $R^4$, $R^5$, L, m and n in the formula (I) are shown below.

$R^1$ is CN, $R^6$ or $OR^7$.

$R^1$ is preferably an optionally halogenated $C_{1-6}$ alkyl group, more preferably a halogenated $C_{1-6}$ alkyl group (particularly, difluoromethyl, trifluoromethyl, trifluoroethyl).

The "aromatic hydrocarbon group" in the "optionally substituted aromatic hydrocarbon group" represented by $R^2$ is preferably a $C_{6-14}$ aryl group (particularly, phenyl, naphthyl (e.g., 1-naphthyl, 2-naphthyl)).

$R^2$ is preferably

1. a $C_{6-14}$ aryl group (particularly, phenyl, naphthyl (e.g., 1-naphthyl, 2-naphthyl)) optionally substituted by 1 to 3 (particularly, 1 or 2) substituents selected from

    (1) a halogen atom (particularly, a fluorine atom, a chlorine atom, a bromine atom, an iodine atom),
    (2) a $C_{1-6}$ alkyl group (particularly, methyl, tert-butyl) optionally substituted by 1 to 3 halogen atoms (particularly, a fluorine atom),
    (3) a $C_{2-6}$ alkynyl group (particularly, ethynyl),
    (4) a $C_{1-6}$ alkoxy group (particularly, methoxy) optionally substituted by 1 to 3 halogen atoms (particularly, a fluorine atom),
    (5) a sulfanyl group optionally substituted by 1 to 5 halogen atoms (particularly, a fluorine atom),
    (6) a nitro group,
    (7) an amino group,
    (8) a sulfonyl group optionally substituted by a $C_{1-6}$ alkyl group (particularly, methyl) optionally substituted by 1 to 3 halogen atoms (particularly, a fluorine atom), and
    (9) a cyano group

or
2. a 5- or 6-membered monocyclic aromatic heterocyclic group (particularly, thienyl (e.g., 2-thienyl, 3-thienyl), pyridine (e.g., 2-pyridyl, 3-pyridyl, 4-pyridyl)) optionally substituted by 1 to 3 halogen atoms (particularly, a chlorine atom).

$R^2$ is more preferably a $C_{6-14}$ aryl group (particularly, phenyl, 1-naphthyl) optionally substituted by 1 to 3 (particularly, 1 or 2) substituents selected from

(1) a halogen atom (particularly, a fluorine atom, a chlorine atom, a bromine atom, an iodine atom),
(2) a $C_{1-6}$ alkyl group (particularly, methyl, tert-butyl) optionally substituted by 1 to 3 halogen atoms (particularly, a fluorine atom),
(3) a $C_{2-6}$ alkynyl group (particularly, ethynyl),
(4) a $C_{1-6}$ alkoxy group (particularly, methoxy) optionally substituted by 1 to 3 halogen atoms (particularly, a fluorine atom), and
(5) a sulfanyl group optionally substituted by 1 to 5 halogen atoms (particularly, a fluorine atom).

$R^2$ is further preferably a $C_{6-14}$ aryl group (particularly, phenyl, naphthyl (e.g., 1-naphthyl, 2-naphthyl)) optionally substituted by 1 to 3 (particularly, 1) substituents selected from

(1) a $C_{1-6}$ alkyl group (particularly, methyl) optionally substituted by 1 to 3 halogen atoms (particularly, a fluorine atom), and
(2) a sulfanyl group optionally substituted by 1 to 5 halogen atoms (particularly, a fluorine atom).

Each of $R^3$ and $R^4$ is preferably a hydrogen atom.

The "aromatic heterocyclic group" in the "optionally substituted aromatic heterocyclic group" represented by $R^5$ preferably includes a nitrogen-containing 5-membered aromatic heterocyclic group (particularly, imidazolyl, pyrazolyl, oxadiazolyl (e.g., 1,2,4-oxadiazolyl, 1,3,4-oxadiazolyl), thiadiazolyl (e.g., 1,2,4-thiadiazolyl, 1,3,4-thiadiazolyl), triazolyl (e.g., 1,2,3-triazolyl), thiazolyl (e.g., 1,3-thiazolyl)), more preferably imidazolyl, pyrazolyl, oxadiazolyl (particularly, 1,2,4-oxadiazolyl, 1,3,4-oxadiazolyl), thiadiazolyl (particularly, 1,2,4-thiadiazolyl, 1,3,4-thiadiazolyl) or triazolyl (particularly, 1,2,3-triazolyl), particularly preferably oxadiazolyl (particularly, 1,3,4-oxadiazolyl, ) or thiadiazolyl (particularly, 1,2,4-thiadiazolyl, 1,3,4-thiadiazolyl).

$R^5$ is preferably a nitrogen-containing 5-membered aromatic heterocyclic group (particularly, imidazolyl, pyrazolyl, oxadiazolyl (preferably 1,2,4-oxadiazolyl, 1,3,4-oxadiazolyl), thiadiazolyl (preferably 1,2,4-thiadiazolyl, 1,3,4-thiadiazolyl), triazolyl (preferably 1,2,3-triazolyl), thiazolyl (preferably 1,3-thiazolyl)) optionally substituted by 1 to 3 substituents selected from

(1) a $C_{1-6}$ alkyl group (particularly, methyl),
(2) a hydroxy-$C_{1-6}$ alkyl group (particularly, hydroxymethyl),
(3) a $C_{1-6}$ alkyl-carbonyloxy-$C_{1-6}$ alkyl group (particularly, methyl-carbonyloxy-methyl),
(4) a $C_{6-14}$ aryl-carbonyloxy-$C_{1-6}$ alkyl group (particularly, phenyl-carbonyloxy-methyl),
(5) a $C_{1-6}$ alkoxy-$C_{7-16}$ aralkyloxy-$C_{1-6}$ alkyl group (particularly, 4-methoxy-benzyloxy-methyl), and
(6) a $C_{1-6}$ alkoxy-$C_{1-6}$ alkyl group (particularly, methoxymethyl).

$R^5$ is more preferably a nitrogen-containing 5-membered aromatic heterocyclic group (particularly, imidazolyl, pyrazolyl, oxadiazolyl (preferably 1,2,4-oxadiazolyl, 1,3,4-oxadiazolyl), thiadiazolyl (preferably 1,2,4-thiadiazolyl, 1,3,4-thiadiazolyl), triazolyl (preferably 1,2,3-triazolyl)) optionally substituted by 1 to 3 (preferably 1) substituents selected from

(1) a $C_{1-6}$ alkyl group (particularly, methyl),
(2) a hydroxy-$C_{1-6}$ alkyl group (particularly, hydroxymethyl), and
(3) a $C_{1-6}$ alkyl-carbonyloxy-$C_{1-6}$ alkyl group (particularly, methyl-carbonyloxy-methyl).

$R^5$ is further preferably a nitrogen-containing 5-membered aromatic heterocyclic group (particularly, oxadiazolyl (preferably 1,3,4-oxadiazolyl), thiadiazolyl (e.g., 1,2,4-thiadiazolyl, 1,3,4-thiadiazolyl)) optionally substituted by 1 to 3 (preferably 1) substituents selected from
a hydroxy-$C_{1-6}$ alkyl group (particularly, hydroxymethyl).

$R^5$ is particularly preferably a nitrogen-containing 5-membered aromatic heterocyclic group (particularly, oxadiazolyl (preferably 1,3,4-oxadiazolyl), thiadiazolyl (e.g., 1,2,4-thiadiazolyl, 1,3,4-thiadiazolyl)) substituted by one hydroxy-$C_{1-6}$ alkyl group (particularly, hydroxymethyl).

$R^6$ is preferably

(1) a $C_{1-6}$ alkyl group (particularly, methyl, ethyl) optionally substituted by 1 to 3 halogen atoms (particularly, a fluorine atom), or
(2) a $C_{2-6}$ alkynyl group (particularly, ethynyl).

$R^6$ is more preferably a $C_{1-6}$ alkyl group (particularly, methyl, ethyl) optionally substituted by 1 to 3 halogen atoms (particularly, a fluorine atom).

$R^7$ is preferably a $C_{1-6}$ alkyl group (particularly, methyl, ethyl).

L is preferably a bond or a chain linker having a principal chain formed from 1 to 3 atoms selected from the group consisting of carbon, oxygen, nitrogen and sulfur (particularly, a bond, -O-, $-CH_2-$, $-CF_2-$, $-CH_2O-$, $-C{\equiv}C-$).

L is more preferably a bond, -O-, $-CH_2-$, $-CF_2-$ or $-CH_2O-$, further preferably a bond. Compound (I) wherein L is a bond is excellent in *in vivo* kinetics and has excellent *in vivo* effects.

m is preferably 2.

n is preferably 2.

Preferably, both of m and n are 2.

Preferable specific examples of the compound (I) include the following:

Compound (A-1):

Compound (I) wherein
$R^1$ is $R^6$, CN or $OR^7$;
$R^2$ is

1. a $C_{6-14}$ aryl group (particularly, phenyl, naphthyl (e.g., 1-naphthyl, 2-naphthyl)) optionally substituted by 1 to 3 (particularly, 1 or 2) substituents selected from

(1) a halogen atom (particularly, a fluorine atom, a chlorine atom, a bromine atom, an iodine atom),
(2) a $C_{1-6}$ alkyl group (particularly, methyl, tert-butyl) optionally substituted by 1 to 3 halogen atoms (particularly, a fluorine atom),
(3) a $C_{2-6}$ alkynyl group (particularly, ethynyl),
(4) a $C_{1-6}$ alkoxy group (particularly, methoxy) optionally substituted by 1 to 3 halogen atoms (particularly,

a fluorine atom),
(5) a sulfanyl group optionally substituted by 1 to 5 halogen atoms (particularly, a fluorine atom),
(6) a nitro group,
(7) an amino group,
(8) a sulfonyl group optionally substituted by a $C_{1-6}$ alkyl group (particularly, methyl) optionally substituted by 1 to 3 halogen atoms (particularly, a fluorine atom), and
(9) a cyano group

or
2. a 5- or 6-membered monocyclic aromatic heterocyclic group (particularly, thienyl (e.g., 2-thienyl, 3-thienyl), pyridyl (e.g., 2-pyridyl, 3-pyridyl, 4-pyridyl)) optionally substituted by 1 to 3 halogen atoms (particularly, a chlorine atom);

each of $R^3$ and $R^4$ is a hydrogen atom;
$R^5$ is a nitrogen-containing 5-membered aromatic heterocyclic group (particularly, imidazolyl, pyrazolyl, oxadiazolyl (e.g., 1,2,4-oxadiazolyl, 1,3,4-oxadiazolyl), thiadiazolyl (e.g., 1,2,4-thiadiazolyl, 1,3,4-thiadiazolyl), triazolyl (e.g., 1,2,3-triazolyl), thiazolyl (e.g., 1,3-thiazolyl)) optionally substituted by 1 to 3 substituents selected from

(1) a $C_{1-6}$ alkyl group (particularly, methyl),
(2) a hydroxy-$C_{1-6}$ alkyl group (particularly, hydroxymethyl),
(3) a $C_{1-6}$ alkyl-carbonyloxy-$C_{1-6}$ alkyl group (particularly, methyl-carbonyloxy-methyl),
(4) a $C_{6-14}$ aryl-carbonyloxy-$C_{1-6}$ alkyl group (particularly, phenyl-carbonyloxy-methyl),
(5) a $C_{1-6}$ alkoxy-$C_{7-16}$ aralkyloxy-$C_{1-6}$ alkyl group (particularly, 4-methoxy-benzyloxy-methyl), and
(6) a $C_{1-6}$ alkoxy-$C_{1-6}$ alkyl group (particularly, methoxymethyl);

$R^6$ is

(1) a $C_{1-6}$ alkyl group (particularly, methyl, ethyl) optionally substituted by 1 to 3 halogen atoms (particularly, a fluorine atom), or
(2) a $C_{2-6}$ alkynyl group (particularly, ethynyl);

$R^7$ is a $C_{1-6}$ alkyl group (particularly, methyl, ethyl);
L is a bond or a chain linker having a principal chain formed from 1 to 3 atoms selected from the group consisting of carbon, oxygen, nitrogen and sulfur (preferably a bond, - O-, $-CH_2-$, $-CF_2-$, $-CH_2O-$ or $-C{\equiv}C-$); and
m and n are the same or different and each represent 1 or 2.

Compound (A):

Compound (I) wherein
$R^1$ is CN, $R^6$ or $OR^7$;
$R^2$ is a $C_{6-14}$ aryl group (particularly, phenyl, naphthyl (e.g., 1-naphthyl, 2-naphthyl)) optionally substituted by 1 to 3 (particularly, 1 or 2) substituents selected from

(1) a halogen atom (particularly, a fluorine atom, a chlorine atom, a bromine atom, an iodine atom),
(2) a $C_{1-6}$ alkyl group (particularly, methyl, tert-butyl) optionally substituted by 1 to 3 halogen atoms (particularly, a fluorine atom),
(3) a $C_{2-6}$ alkynyl group (particularly, ethynyl),
(4) a $C_{1-6}$ alkoxy group (particularly, methoxy) optionally substituted by 1 to 3 halogen atoms (particularly, a fluorine atom), and
(5) a sulfanyl group optionally substituted by 1 to 5 halogen atoms (particularly, a fluorine atom);

each of $R^3$ and $R^4$ is a hydrogen atom;
$R^5$ is a nitrogen-containing 5-membered aromatic heterocyclic group (particularly, imidazolyl, pyrazolyl, oxadiazolyl (e.g., 1,2,4-oxadiazolyl, 1,3,4-oxadiazolyl), thiadiazolyl (e.g., 1,2,4-thiadiazolyl, 1,3,4-thiadiazolyl), triazolyl (e.g., 1,2,3-triazolyl), thiazolyl (e.g., 1,3-thiazolyl)) optionally substituted by 1 to 3 substituents selected from

(1) a $C_{1-6}$ alkyl group (particularly, methyl),
(2) a hydroxy-$C_{1-6}$ alkyl group (particularly, hydroxymethyl),

(3) a $C_{1-6}$ alkyl-carbonyloxy-$C_{1-6}$ alkyl group (particularly, methyl-carbonyloxy-methyl),
(4) a $C_{6-14}$ aryl-carbonyloxy-$C_{1-6}$ alkyl group (particularly, phenyl-carbonyloxy-methyl),
(5) a $C_{1-6}$ alkoxy-$C_{7-16}$ aralkyloxy-$C_{1-6}$ alkyl group (particularly, 4-methoxy-benzyloxy-methyl), and
(6) a $C_{1-6}$ alkoxy-$C_{1-6}$ alkyl group (particularly, methoxymethyl);

$R^6$ is

(1) a $C_{1-6}$ alkyl group (particularly, methyl, ethyl) optionally substituted by 1 to 3 halogen atoms (particularly, a fluorine atom), or
(2) a $C_{2-6}$ alkynyl group (particularly, ethynyl);

$R^7$ is a $C_{1-6}$ alkyl group (particularly, methyl, ethyl);
L is a bond or a chain linker having a principal chain formed from 1 to 3 atoms selected from the group consisting of carbon, oxygen, nitrogen and sulfur (preferably a bond, - O-, $-CH_2-$, $-CF_2-$ or $-CH_2O-$); and
m and n are the same or different and each represent 1 or 2.

Compound (B):

Compound (A) wherein
$R^5$ is a nitrogen-containing 5-membered aromatic heterocyclic group (particularly, imidazolyl, pyrazolyl, oxadiazolyl (e.g., 1,2,4-oxadiazolyl, 1,3,4-oxadiazolyl), thiadiazolyl (e.g., 1,2,4-thiadiazolyl, 1,3,4-thiadiazolyl), triazolyl (e.g., 1,2,3-triazolyl)) optionally substituted by 1 to 3 (particularly, 1) substituents selected from

(1) a $C_{1-6}$ alkyl group (particularly, methyl),
(2) a hydroxy-$C_{1-6}$ alkyl group (particularly, hydroxymethyl), and
(3) a $C_{1-6}$ alkyl-carbonyloxy-$C_{1-6}$ alkyl group (particularly, methyl-carbonyloxy-methyl);

L is a bond, -O-, $-CH_2-$, $-CF_2-$ or $-CH_2O-$; and
m and n are the same or different and each represent 1 or 2 (particularly, each of m and n is 2).

Compound (C):

Compound (A) wherein
$R^2$ is a $C_{6-14}$ aryl group (particularly, phenyl) optionally substituted by 1 to 3 (particularly, 1) substituents selected from

(1) a $C_{1-6}$ alkyl group (particularly, methyl) optionally substituted by 1 to 3 halogen atoms (particularly, a fluorine atom), and
(2) a sulfanyl group optionally substituted by 1 to 5 halogen atoms (particularly, a fluorine atom);

$R^5$ is a nitrogen-containing 5-membered aromatic heterocyclic group (particularly, oxadiazolyl (e.g., 1,3,4-oxadiazolyl), thiadiazolyl (e.g., 1,2,4-thiadiazolyl, 1,3,4-thiadiazolyl)) optionally substituted by 1 to 3 (preferably 1) substituents selected from a hydroxy-$C_{1-6}$ alkyl group (particularly, hydroxymethyl);
$R^6$ is a $C_{1-6}$ alkyl group (particularly, methyl) optionally substituted by 1 to 3 halogen atoms (particularly, a fluorine atom);
L is a bond; and
each of m and n is 2.

Compound (D):

Compound (I) wherein
$R^1$ is a halogenated $C_{1-6}$ alkyl group (particularly, difluoromethyl, trifluoromethyl, trifluoroethyl);
$R^2$ is a $C_{6-14}$ aryl group (particularly, phenyl) optionally substituted by 1 to 3 (particularly, 1 or 2) substituents selected from

(1) a halogen atom (particularly, a chlorine atom),
(2) a $C_{1-6}$ alkyl group (particularly, methyl) optionally substituted by 1 to 3 halogen atoms (particularly, a fluorine atom), and
(3) a sulfanyl group optionally substituted by 1 to 5 halogen atoms (particularly, a fluorine atom);

each of $R^3$ and $R^4$ is a hydrogen atom;

$R^5$ is a nitrogen-containing 5-membered aromatic heterocyclic group (particularly, oxadiazolyl (e.g., 1,2,4-oxadiazolyl, 1,3,4-oxadiazolyl), thiadiazolyl (e.g., 1,2,4-thiadiazolyl, 1,3,4-thiadiazolyl)) optionally substituted by 1 to 3 substituents selected from

(1) a $C_{1-6}$ alkyl group (particularly, methyl),
(2) a hydroxy-$C_{1-6}$ alkyl group (particularly, hydroxymethyl),
(3) a $C_{1-6}$ alkyl-carbonyloxy-$C_{1-6}$ alkyl group (particularly, methyl-carbonyloxy-methyl),
(4) a $C_{6-14}$ aryl-carbonyloxy-$C_{1-6}$ alkyl group (particularly, phenyl-carbonyloxy-methyl), and
(5) a $C_{1-6}$ alkoxy-$C_{7-16}$ aralkyloxy-$C_{1-6}$ alkyl group (particularly, 4-methoxy-benzyloxy-methyl);

L is a bond, -O-, $-CH_2-$ or $-CH_2O-$; and
one of m and n is 2, and the other is 1 or 2.

The salt of the compound (I) is preferably a pharmacologically acceptable salt. Examples thereof include a salt with an inorganic base, a salt with an organic base, a salt with an inorganic acid, a salt with an organic acid and a salt with a basic or acidic amino acid.

Preferable examples of the salt with an inorganic base include: an alkali metal salt such as sodium salt, potassium salt and the like; an alkaline earth metal salt such as calcium salt, magnesium salt and the like; and an aluminum salt and an ammonium salt.

Preferable examples of the salt with an organic base include a salt with trimethylamine, triethylamine, pyridine, picoline, ethanolamine, diethanolamine, triethanolamine, tromethamine [tris(hydroxymethyl)methylamine], tert-butylamine, cyclohexylamine, benzylamine, dicyclohexylamine or N,N-dibenzylethylenediamine.

Preferable examples of the salt with an inorganic acid include a salt with hydrochloric acid, hydrobromic acid, nitric acid, sulfuric acid or phosphoric acid.

Preferable examples of the salt with an organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid or p-toluenesulfonic acid.

Preferable examples of the salt with a basic amino acid include a salt with arginine, lysine or ornithine.

Preferable examples of the salt with an acidic amino acid include a salt with aspartic acid or glutamic acid.

A method for producing the compounds according to the present invention will be described below.

A starting material or a reagent used in each step in the production methods given below and an obtained compound may each form a salt. Examples of such a salt include the same as the aforementioned salt of the compound of the present invention, and the like.

When the compound obtained in each step is a free compound, this compound can be converted to a salt of interest by a method known per se in the art. On the contrary, when the compound obtained in each step is a salt, this salt can be converted to a free form or another type of salt of interest by a method known per se in the art.

The compound obtained in each step may be used in the next reaction in the form of its reaction solution or after being obtained as a crude product. Alternatively, the compound obtained in each step can be isolated and/or purified from the reaction mixture by a separation approach such as concentration, crystallization, recrystallization, distillation, solvent extraction, fractionation, chromatography or the like according to a routine method.

If a starting material or a reagent compound for each step is commercially available, the commercially available product can be used directly.

In the reaction of each step, the reaction time may vary depending on the reagent or the solvent used and is usually 1 minute to 48 hours, preferably 10 minutes to 8 hours, unless otherwise specified.

In the reaction of each step, the reaction temperature may vary depending on the reagent or the solvent used and is usually -78°C to 300°C, preferably -78°C to 150°C, unless otherwise specified.

In the reaction of each step, the pressure may differ depending on the reagent or the solvent used and is usually 1 atm to 20 atm, preferably 1 atm to 3 atm, unless otherwise specified.

In the reaction of each step, a microwave synthesis apparatus, for example, Initiator manufactured by Biotage Japan Ltd., may be used. The reaction temperature may vary depending on the reagent or the solvent used and is usually room temperature to 300°C, preferably 50°C to 250°C, unless otherwise specified. The reaction time may vary depending on the reagent or the solvent used and is usually 1 minute to 48 hours, preferably 1 minute to 8 hours, unless otherwise specified.

In the reaction of each step, a reagent is used at 0.5 equivalents to 20 equivalents, preferably 0.8 equivalents to 5 equivalents, with respect to the substrate, unless otherwise specified. In the case of using a reagent as a catalyst, the reagent is used at 0.001 equivalents to 1 equivalent, preferably 0.01 equivalents to 0.2 equivalents, with respect to a substrate. When the reagent also serves as a reaction solvent, the reagent is used in the amount of the solvent.

In the reaction of each step, this reaction is carried out without a solvent or by dissolution or suspension in an appropriate solvent, unless otherwise specified. Specific examples of the solvent include the solvents described in

Examples and the following:

alcohols: methanol, ethanol, tert-butyl alcohol, 2-methoxyethanol and the like;
ethers: diethyl ether, diphenyl ether, tetrahydrofuran, 1,2-dimethoxyethane and the like;
aromatic hydrocarbons: chlorobenzene, toluene, xylene and the like;
saturated hydrocarbons: cyclohexane, hexane and the like;
amides: N,N-dimethylformamide, N-methylpyrrolidone and the like;
halogenated hydrocarbons: dichloromethane, carbon tetrachloride and the like;
nitriles: acetonitrile and the like;
sulfoxides: dimethyl sulfoxide and the like;
aromatic organic bases: pyridine and the like;
acid anhydrides: acetic anhydride and the like;
organic acids: formic acid, acetic acid, trifluoroacetic acid and the like;
inorganic acids: hydrochloric acid, sulfuric acid and the like;
esters: ethyl acetate and the like;
ketones: acetone, methyl ethyl ketone and the like; and
water.

Two or more of these solvents may be used as a mixture at an appropriate ratio.
In the case of using a base in the reaction of each step, for example, the following bases or the bases described in Examples is used:

inorganic bases: sodium hydroxide, magnesium hydroxide, sodium carbonate, calcium carbonate, sodium bicarbonate and the like;
organic bases: triethylamine, diethylamine, pyridine, 4-dimethylaminopyridine, N,N-dimethylaniline, 1,4-diazabicyclo[2.2.2]octane, 1,8-diazabicyclo[5.4.0]-7-undecene, imidazole,
piperidine and the like;
metal alkoxides: sodium ethoxide, potassium tert-butoxide and the like;
alkali metal hydrides: sodium hydride and the like;
metal amides: sodium amide, lithium diisopropylamide, lithium hexamethyldisilazide and the like; and
organic lithiums: n-butyllithium and the like.

In the case of using an acid or an acidic catalyst in the reaction of each step, for example, the following acids or acidic catalysts or the acids or acidic catalysts described in Examples may be used:

inorganic acids: hydrochloric acid, sulfuric acid, nitric acid, hydrobromic acid, phosphoric acid and the like;
organic acids: acetic acid, trifluoroacetic acid, citric acid, p-toluenesulfonic acid, 10-camphorsulfonic acid and the like; and
Lewis acids: boron trifluoride-diethyl ether complex, zinc iodide, anhydrous aluminum chloride, anhydrous zinc chloride, anhydrous iron chloride and the like.

The reaction of each step is carried out according to a method known per se in the art, for example, the method described in The Fifth Series of Experimental Chemistry, Vol. 13 to Vol. 19 (edited by The Chemical Society of Japan); Shin Jikken Kagaku Koza (New Experimental Chemistry in English), Vol. 14 to Vol. 15 (edited by The Chemical Society of Japan); Syntheses in the Organic Chemistry Laboratory, Revised, 2nd Ed. (L. F. Tietze, Th. Eicher, Nankodo Co., Ltd.); Organic Name Reactions; The Reaction Mechanism and Essence, Revised (Hideo Tougo, Kodansha Ltd.); Organic Syntheses Collective Volume I to VII (John Wiley & Sons, Inc.); Modern Organic Synthesis in the Laboratory: A Collection of Standard Experimental Procedures (Jie Jack Li, Oxford University Press); Comprehensive Heterocyclic Chemistry III, Vol. 1 to Vol. 14 (Elsevier Japan KK); Strategic Applications of Named Reactions in Organic Synthesis (translated by Kiyoshi Tomioka, published by Kagaku-Dojin Publishing Company, Inc.); Comprehensive Organic Transformations (VCH Publishers, Inc.) (1989), etc.; or the methods described in Examples, unless otherwise specified.
In each step, the protection or deprotection reaction of a functional group is carried out according to a method known per se in the art, for example, the methods described in "Protective Groups in Organic Synthesis, 4th Ed." (Theodora W. Greene, Peter G. M. Wuts), Wiley-Interscience (2007); "Protecting Groups, 3rd Ed." (P.J. Kocienski) Thieme Medical Publishers (2004), etc., or the methods described in Examples.
Examples of a protective group for a hydroxy group or a phenolic hydroxy group in an alcohol or the like include: an ether-type protective group such as methoxymethyl ether, benzyl ether, t-butyldimethylsilyl ether, tetrahydropyranyl ether and the like; a carboxylic acid ester-type protective group such as acetic acid ester and the like; a sulfonic acid

ester-type protective group such as methanesulfonic acid ester and the like; a carbonic acid ester-type protective group such as t-butyl carbonate and the like; and the like.

Examples of a protective group for a carbonyl group in an aldehyde include: an acetal-type protective group such as dimethylacetal and the like; a cyclic acetal-type protective group such as cyclic 1,3-dioxane and the like; and the like.

Examples of a protective group for a carbonyl group in a ketone include: a ketal-type protective group such as dimethylketal and the like; a cyclic ketal-type protective group such as cyclic 1,3-dioxane and the like; an oxime-type protective group such as O-methyloxime and the like; a hydrazone-type protective group such as N,N-dimethylhydrazone and the like; and the like.

Examples of a protective group for a carboxyl group include: an ester-type protective group such as methyl ester and the like; an amide-type protective group such as N,N-dimethylamide and the like; and the like

Examples of a protective group for a thiol include: an ether-type protective group such as benzyl thioether and the like; an ester-type protective group such as thioacetic acid ester, thiocarbonate, thiocarbamate and the like; and the like.

Examples of a protective group for an amino group or an aromatic heterocycle such as imidazole, pyrrole, indole or the like include: a carbamate-type protective group such as benzyl carbamate and the like; an amide-type protective group such as acetamide and the like; an alkylamine-type protective group such as N-triphenylmethylamine and the like; a sulfonamide-type protective group such as methanesulfonamide and the like; and the like.

These protective groups can be removed by use of a method known per se in the art, for example, a method of using an acid, a base, ultraviolet light, hydrazine, phenylhydrazine, sodium N-methyldithiocarbamate, tetrabutylammonium fluoride, palladium acetate or trialkylsilyl halide (e.g., trimethylsilyl iodide, trimethylsilyl bromide) or a reduction method.

In the case of carrying out a reduction reaction in each step, examples of the reducing agent used include: metal hydrides such as lithium aluminum hydride, sodium triacetoxyborohydride, sodium cyanoborohydride, diisobutyl aluminum hydride (DIBAL-H), sodium borohydride, tetramethylammonium triacetoxyborohydride and the like; boranes such as a borane-tetrahydrofuran complex and the like; Raney nickel; Raney cobalt; hydrogen; formic acid; and the like. In the case of reducing a carbon-carbon double bond or triple bond, a method using a catalyst such as palladium-carbon, a Lindlar’s catalyst or the like can be used.

In the case of carrying out oxidation reaction in each step, examples of the oxidizing agent used include: peracids such as m-chloroperbenzoic acid (MCPBA), hydrogen peroxide, t-butyl hydroperoxide and the like; perchlorates such as tetrabutylammonium perchlorate and the like; chlorates such as sodium chlorate and the like; chlorites such as sodium chlorite and the like; periodates such as sodium periodate and the like; a high-valent iodine reagent such as iodosylbenzene and the like; a reagent having manganese, such as manganese dioxide, potassium permanganate and the like; lead compounds such as lead tetraacetate and the like; a reagent having chromium, such as pyridinium chlorochromate (PCC), pyridinium dichromate (PDC), Jones reagents and the like; halogen compounds such as N-bromosuccinimide (NBS) and the like; oxygen; ozone; a sulfur trioxide-pyridine complex; osmium tetroxide; selenium dioxide; 2,3-dichloro-5,6-dicyano-1,4-benzoquinone (DDQ); and the like.

In the case of carrying out a radical cyclization reaction in each step, examples of the radical initiator used include: an azo compound such as azobisisobutyronitrile (AIBN) and the like; a water-soluble radical initiator such as 4-4’-azobis-4-cyanopentanoic acid (ACPA) and the like; triethylboron in the presence of air or oxygen; benzoyl peroxide; and the like. Examples of the radical reaction agent used include tributylstannane, tris(trimethylsilyl)silane, 1,1,2,2-tetraphenyldisilane, diphenylsilane, samarium iodide and the like.

In the case of carrying out Wittig reaction in each step, examples of the Wittig reagent used include alkylidenephosphoranes and the like. The alkylidenephosphoranes can be prepared by a method known per se in the art, for example, the reaction between a phosphonium salt and a strong base.

In the case of carrying out Horner-Emmons reaction in each step, examples of the reagent used include: phosphonoacetic acid esters such as methyl dimethylphosphonoacetate, ethyl diethylphosphonoacetate and the like; and a base such as alkali metal hydrides, organic lithiums and the like.

In the case of carrying out Friedel-Crafts reaction in each step, examples of the reagent used include a Lewis acid and an acid chloride or an alkylating agent (e.g., alkyl halides, alcohols, olefins, etc.). Alternatively, an organic acid or an inorganic acid may be used instead of the Lewis acid, and an acid anhydride such as acetic anhydride or the like may be used instead of the acid chloride.

In the case of carrying out aromatic nucleophilic substitution reaction in each step, a nucleophile (e.g., amines, imidazole, etc.) and a base (e.g., organic bases, etc.) are used as reagents.

In the case of carrying out nucleophilic addition reaction using a carbanion, nucleophilic 1,4-addition reaction (Michael addition reaction) using a carbanion or nucleophilic substitution reaction using a carbanion in each step, examples of the base used for generating the carbanion include organic lithiums, metal alkoxides, inorganic bases, organic bases and the like.

In the case of carrying out Grignard reaction in each step, examples of the Grignard reagent include: aryl magnesium halides such as phenyl magnesium bromide and the like; and alkyl magnesium halides such as methyl magnesium bromide and the like. The Grignard reagent can be prepared by a method known per se in the art, for example, the reaction between alkyl halide or aryl halide and metal magnesium in the presence of ether or tetrahydrofuran as a solvent.

In the case of carrying out Knoevenagel condensation reaction in each step, an active methylene compound flanked by two electron-attracting groups (e.g., malonic acid, diethyl malonate, malononitrile, etc.) and a base (e.g., organic bases, metal alkoxides, inorganic bases) are used as reagents.

In the case of carrying out Vilsmeier-Haack reaction in each step, phosphoryl chloride and an amide derivative (e.g., N,N-dimethylformamide, etc.) are used as reagents.

In the case of carrying out an azidation reaction of alcohols, alkyl halides or sulfonic acid esters in each step, examples of the azidating agent used include diphenylphosphorylazide (DPPA), trimethylsilylazide, sodium azide and the like. In the case of azidating, for example, alcohols, a method using diphenylphosphorylazide and 1,8-diazabicyclo[5.4.0]undec-7-ene (DBU), a method using trimethylsilylazide and a Lewis acid, or the like can be used.

In the case of carrying out a reductive amination reaction in each step, examples of the reducing agent used include sodium triacetoxyborohydride, sodium cyanoborohydride, hydrogen, formic acid and the like. When the substrate is an amine compound, examples of the carbonyl compound used include p-formaldehyde as well as aldehydes such as acetaldehyde and the like, and ketones such as cyclohexanone and the like. When the substrate is a carbonyl compound, examples of the amines used include: ammonia; primary amine such as methylamine and the like; secondary amine such as dimethylamine and the like; and the like.

In the case of carrying out Mitsunobu reaction in each step, azodicarboxylic acid esters (e.g., diethyl azodicarboxylate (DEAD), diisopropyl azodicarboxylate (DIAD), etc.) and triphenylphosphine are used as reagents.

In the case of carrying out an esterification reaction, amidation reaction or ureation reaction in each step, examples of the reagent used include: an acyl halide form such as acid chloride, acid bromide and the like; and activated carboxylic acids such as an acid anhydride, an active ester form, a sulfuric acid ester form and the like. Examples of the activator for carboxylic acid include: a carbodiimide condensing agent such as 1-ethyl-3-(3-dimethylaminopropyl)carbodiimide hydrochloride (WSCD) and the like; a triazine condensing agent such as 4-(4,6-dimethoxy-1,3,5-triazin-2-yl)-4-methylmorpholinium chloride-n-hydrate (DMT-MM) and the like; a carbonic acid ester condensing agent such as 1,1-carbonyldiimidazole (CDI) and the like; diphenylphosphorylazide (DPPA); benzotriazol-1-yloxy-trisdimethylaminophosphonium salt (BOP reagent); 2-chloro-1-methyl-pyridinium iodide (Mukaiyama reagent); thionyl chloride; lower alkyl haloformate such as ethyl chloroformate and the like; O-(7-azabenzotriazol-1-yl)-N,N,N',N'-tetramethyluronium hexafluorophosphate (HATU); sulfuric acid; and combinations thereof; and the like. In the case of using a carbodiimide condensing agent, an additive such as 1-hydroxybenzotriazole (HOBt), N-hydroxysuccinimide (HOSu), dimethylaminopyridine (DMAP) or the like may be further added for the reaction.

In the case of carrying out a coupling reaction in each step, examples of the metal catalyst used include: a palladium compound such as palladium(II) acetate, tetrakis(triphenylphosphine)palladium(0), dichlorobis(triphenylphosphine)palladium(II), dichlorobis(triethylphosphine)palladium(II), tris(dibenzylideneacetone)dipalladium(0), 1,1'-bis(diphenylphosphino)ferrocene palladium(II) chloride, palladium(II) acetate and the like; a nickel compound such as tetrakis(triphenylphosphine)nickel(0) and the like; a rhodium compound such as tris(triphenylphosphine)rhodium(III) chloride and the like; a cobalt compound; a copper compound such as copper oxide, copper(I) iodide and the like; a platinum compound; and the like. A base may be further added for the reaction. Examples of such a base include inorganic bases and the like.

In the case of carrying out a thiocarbonylation reaction in each step, diphosphorus pentasulfide is typically used as a thiocarbonylating agent. A reagent having a 1,3,2,4-dithiadiphosphetane-2,4-disulfide structure such as 2,4-bis(4-methoxyphenyl-1,3,2,4-dithiadiphosphetane-2,4-disulfide (Lawesson's reagent) or the like may be used instead of diphosphorus pentasulfide.

In the case of carrying out Wohl-Ziegler reaction in each step, examples of the halogenating agent used include N-iodosuccinimide, N-bromosuccinimide (NBS), N-chlorosuccinimide (NCS), bromine, sulfuryl chloride and the like. The reaction can be accelerated by the further addition of a radical initiator such as heat, light, benzoyl peroxide, azobisisobutyronitrile or the like for the reaction.

In the case of carrying out a halogenation reaction of a hydroxy group in each step, examples of the halogenating agent used include a hydrohalic acid and an acid halide of an inorganic acid, specifically, hydrochloric acid, thionyl chloride, phosphorus oxychloride and the like for chlorination, and 48% hydrobromic acid and the like for bromination. Also, a method for obtaining an alkyl halide form from an alcohol by the action of triphenylphosphine and carbon tetrachloride or carbon tetrabromide or the like may be used. Alternatively, a method for synthesizing an alkyl halide form through 2-stage reactions involving the conversion of an alcohol to a sulfonic acid ester and the subsequent reaction of the ester with lithium bromide, lithium chloride or sodium iodide may be used.

In the case of carrying out Arbuzov reaction in each step, examples of the reagent used include: alkyl halides

such as ethyl bromoacetate and the like; and phosphites such as triethyl phosphite, tri(isopropyl) phosphite and the like.

In the case of carrying out sulfone-esterification reaction in each step, examples of the sulfonylating agent used include methanesulfonyl chloride, p-toluenesulfonyl chloride, methanesulfonic anhydride, p-toluenesulfonic anhydride and the like.

In the case of carrying out hydrolysis reaction in each step, an acid or a base is used as a reagent. In the case of carrying out acid hydrolysis reaction of t-butyl ester, formic acid, triethylsilane or the like may be added for reductively trapping a by-product t-butyl cation.

In the case of carrying out dehydration reaction in each step, examples of the dehydrating agent used include sulfuric acid, diphosphorus pentoxide, phosphorus oxychloride, N,N'-dicyclohexylcarbodiimide, alumina, polyphosphoric acid and the like.

In the case of carrying out alkylation reaction of alcohols in each step, examples of the alkylating agent include optionally substituted alkyl halide (e.g., iodomethane), optionally substituted alkyl having an optionally substituted $C_{1-6}$ alkylsulfonyloxy group as a leaving group, optionally substituted alkyl having a $C_{6-14}$ arylsulfonyloxy group optionally substituted by a $C_{1-6}$ alkyl group, and the like. Examples of the base used include organic lithiums, metal alkoxides, inorganic bases, organic bases and the like.

In the case of carrying out fluorination reaction in each step, examples of the fluorinating agent used include DAST (diethyl aminosulfur trifluoride), bis(2-methoxyethyl)aminosulfur trifluoride and the like.

In the case of carrying out imidation reaction in each step, examples of the reagent used include: an acyl halide form such as acid chloride, acid bromide and the like; and activated carboxylic acids such as an active ester form, a sulfuric acid ester form and the like. Examples of the activator for carboxylic acid include: an acid anhydride (e.g. acetic anhydride); a carbodiimide condensing agent such as 1-ethyl-3-(3-dimethylaminopropyl)carbodiimide hydrochloride (WSCD) and the like; a triazine condensing agent such as 4-(4,6-dimethoxy-1,3,5-triazin-2-yl)-4-methylmorpholinium chloride-n-hydrate (DMT-MM) and the like; a carbonic acid ester condensing agent such as 1,1-carbonyldiimidazole (CDI) and the like; diphenylphosphorylazide (DPPA); benzotriazol-1-yloxy-trisdimethylaminophosphonium salt (BOP reagent); 2-chloro-1-methyl-pyridinium iodide (Mukaiyama reagent); thionyl chloride; lower alkyl halo formate such as ethyl chloroformate and the like; O-(7-azabenzotriazol-1-yl)-N,N,N',N'-tetramethyluronium hexafluorophosphate (HATU); sulfuric acid; and combinations thereof; and the like. In the case of using a carbodiimide condensing agent, an additive such as 1-hydroxybenzotriazole (HOBt), N-hydroxysuccinimide (HOSu), dimethylaminopyridine (DMAP) or the like may be further added for the reaction.

Hereinafter, the method for producing the compound (I) will be described more specifically with reference to reaction schemes.

Each symbol in the reaction schemes given below represents the same meaning as above, unless otherwise specified.

Each starting compound for use in various production methods given below can be produced by a method known per se in the art, unless otherwise specified.

[Production method A-1]

The compound (I) and compounds (Ia), (Ib) and (Ic) included in the compound (I) can each be produced from compound (II) by the following method:

[Formula 2]

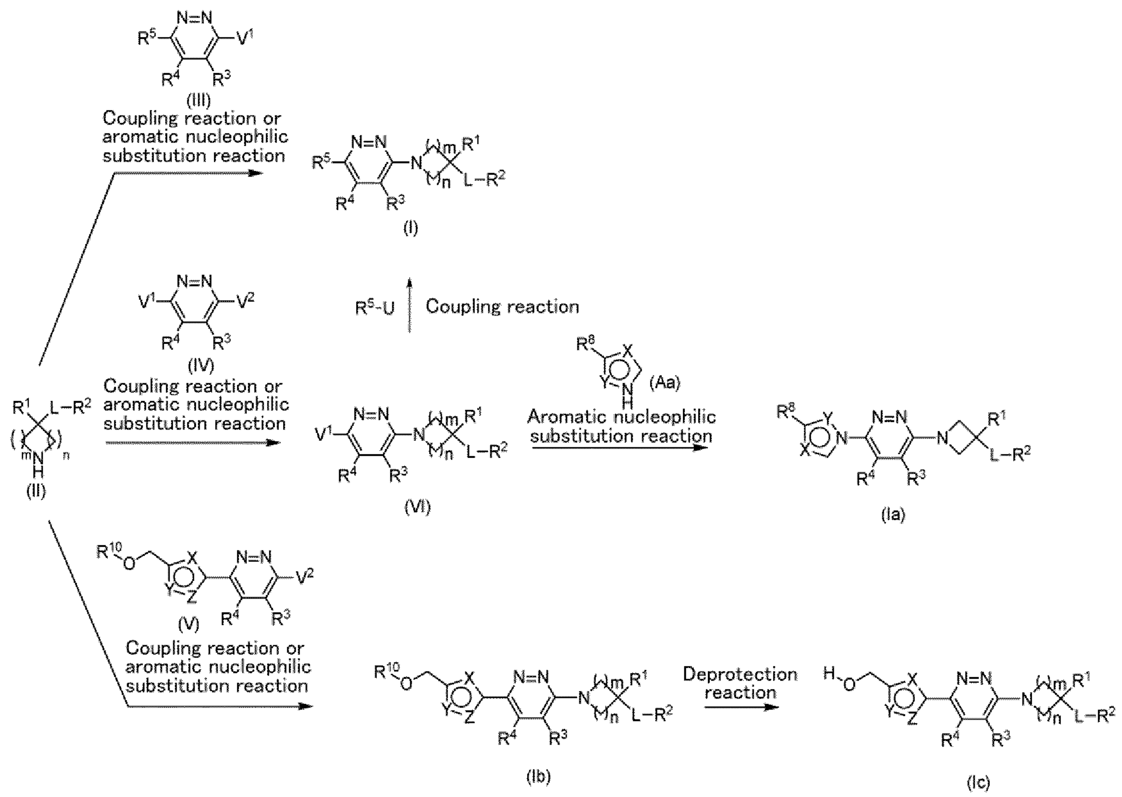


In the reaction scheme, X, Y and Z each independently represent $CR^9$, N, O or S except that at least one or more of X, Y and Z is N;
$R^8$ and $R^9$ each independently a hydrogen atom or a substituent; and
$R^{10}$ represents a protective group for a hydroxy group in an alcohol or the like.

In the reaction scheme, $V^1$ and $V^2$ each represent a leaving group. Examples thereof include hydrogen, an alkali metal (e.g., lithium, sodium), a halogen atom (e.g., fluorine, chlorine, bromine, iodine), a $C_{1-6}$ alkoxy group (e.g., methoxy), a $C_{6-14}$ aryloxy group (e.g., phenoxy), an optionally substituted acyl-oxy group (e.g., acetyloxy, benzoyloxy), an optionally substituted $C_{1-6}$ alkoxysulfonyloxy group (e.g., methoxysulfonyloxy), an optionally halogenated $C_{1-6}$ alkyl-sulfonyl-oxy group [e.g., methanesulfonyloxy, ethanesulfonyloxy, trichloromethanesulfonyloxy, trifluoromethanesulfonyloxy (triflate)] and an optionally substituted $C_{6-14}$ arylsulfonyl-oxy group [e.g., a $C_{6-14}$ arylsulfonyloxy group optionally having 1 to 3 substituents selected from a $C_{1-6}$ alkyl group (e.g., methyl, ethyl, propyl, isopropyl, butyl, isobutyl, sec-butyl, tert-butyl, pentyl, hexyl), a $C_{1-6}$ alkoxy group (e.g., methoxy, ethoxy, propoxy, isopropoxy, butoxy, isobutoxy, sec-butoxy, pentyloxy, hexyloxy) and a nitro group; specific examples thereof include benzenesulfonyloxy, m-nitrobenzenesulfonyloxy, p-toluenesulfonyloxy and naphthylsulfonyloxy].

In the case of carrying out coupling reaction in each step, a ligand may be added into the reaction system. Examples of such a ligand include a phosphine ligand [e.g., triphenylphosphine, 2,2'-bis(diphenylphosphino)-1,1'-binaphthyl, 2-dicyclohexylphosphino-2',6'-diisopropoxy-1,1'-biphenyl, 2-(dicyclohexylphosphino)-3,6-dimethoxy-2',4',6'-triisopropyl-1,1'-biphenyl, 2-di-tert-butylphosphino-3,4,5,6-tetramethyl-2',4',6'-triisopropyl-1,1'-biphenyl, 2-(di-tert-butylphosphino)biphenyl, 2-(dicyclohexylphosphino)biphenyl, 2-dicyclohexylphosphino-2',6'-dimethoxy-1,1'-biphenyl, 2-dicyclohexylphosphino-2',4',6'-triisopropyl-1,1'-biphenyl, 2-(dicyclohexylphosphino)-2'-(N,N-dimethylamino)biphenyl, 1,1'-bis(diphenylphosphino)ferrocene, tri-tert-butylphosphine, tricyclohexylphosphine, 4,5-bis(diphenylphosphino)-9,9-dimethylxanthene], an amine ligand (N,N'-dimethylethylenediamine, trans-1,2-diaminocyclohexane, trans-N,N'-dimethyl-1,2-cyclohexanediamine, 1,10-phenanthroline, 4,7-dimethoxy-1,10-phenanthroline, 3,4,7,8-tetramethyl-1,10-phenanthroline, etc.), a diketone ligand (2-acetylcyclohexanone, 2-isobutyrylhexanone, 2,2,6,6-tetramethyl-3,5-heptanedione, etc.), salicylaldoxime and proline.

In the reaction scheme, $R^5$-U represents a boron derivative or a stannyl derivative. Examples of U include a boryl group (e.g.,

[Formula 3]

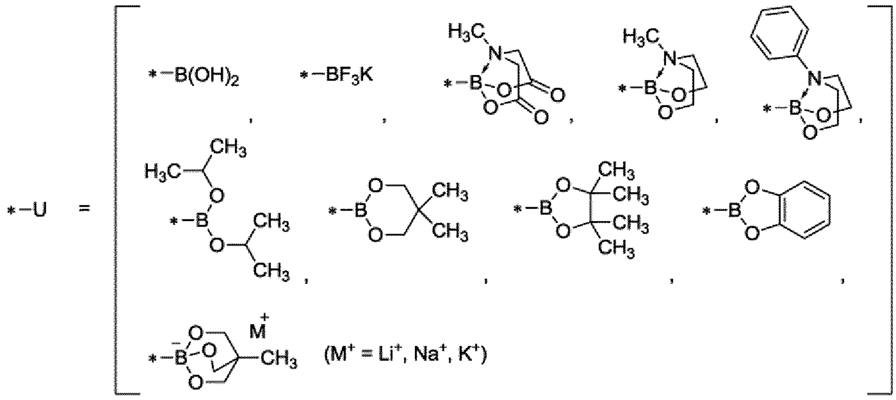

), an optionally substituted $C_{1-6}$ alkylstannyl group (e.g., tributylstannyl), an optionally substituted $C_{2-6}$ alkenylstannyl group and the like.

[Production method A-2]

Among the compounds (I), compounds (Id), (Ie), (If) and (Ig) can each be produced from compound (II) by the following method:

[Formula 4]

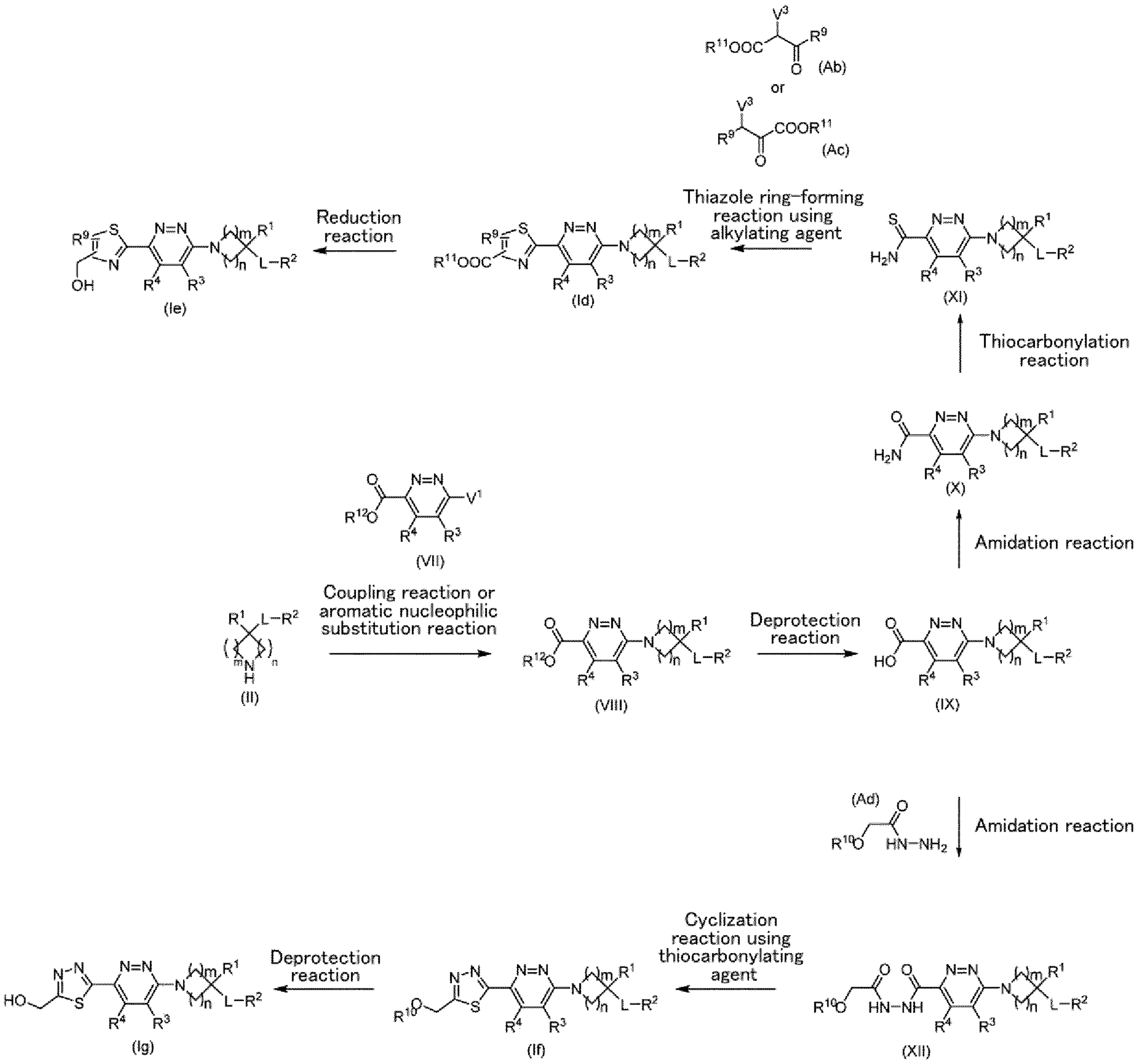


In the reaction scheme, $R^{11}$ and $R^{12}$ each represent a protective group for a carboxyl group.

In the reaction scheme, $V^3$ represents a leaving group. Examples of the leaving group include a halogen atom (e.g., a fluorine atom, a chlorine atom, a bromine atom, an iodine atom) and the like.

Among the compounds (I), compound (Id) can be produced through the thiazole ring-forming reaction between compound (XI) and compound (Ab) or compound (Ac). This reaction can be appropriately carried out according to a method known per se in the art [e.g., WO2010/142801, WO2011/066803, WO2007/115315, Bioorganic & Medicinal Chemistry Letters, 22 (20), 6373-6376 (2012), Journal of Medicinal Chemistry, 50 (4), 685-695 (2007), WO2007/115315, WO 2009/123316, WO 2012/122368, Journal of Medicinal Chemistry, 27 (12), 1559-65 (1984), Journal of Organic Chemistry, 75 (10), 3507-3510 (2010)] or a method equivalent thereto.

Among the compounds (I), compound (If) can be produced through the cyclization reaction of compound (XII) using a thiocarbonylating agent. In this respect, examples of the thiocarbonylating agent used include those listed as the aforementioned thiocarbonylating agent.

[Production method A-3]

Among the compounds (I), compounds (Ih) and (Ii) can each be produced from compound (II) by the following method:

[Formula 5]

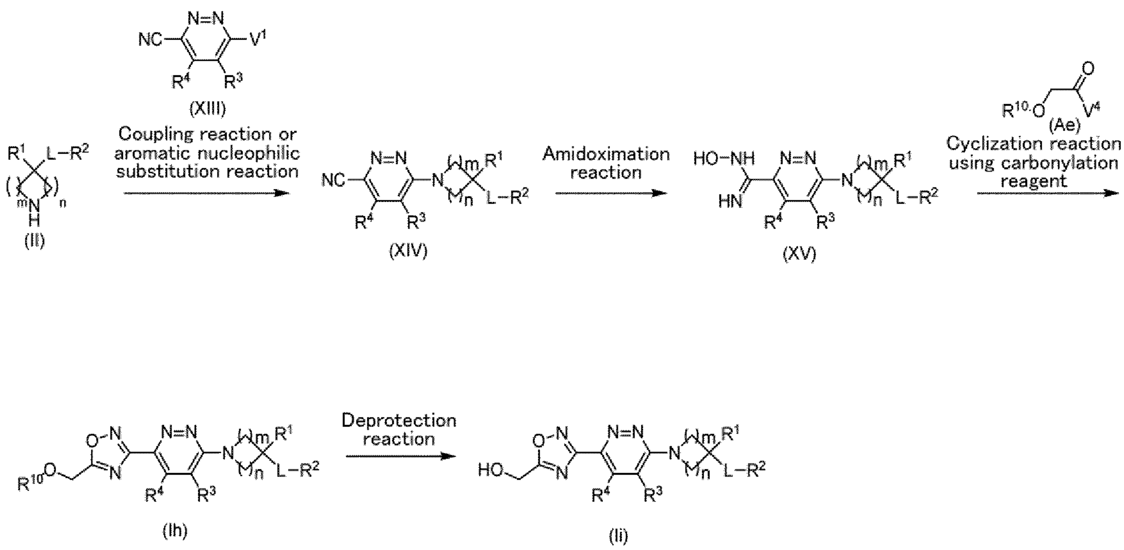


In the reaction scheme, $V^4$ represents a leaving group. Examples of the leaving group include a halogen atom (e.g., a chlorine atom, a bromine atom) and the like.

Compound (XV) can be produced through the amidoximation reaction of compound (XIV). Examples of the amidoximation reagent include hydroxylamine and the like. This reaction may be carried out by the addition of a base or the like.

Among the compounds (I), compound (Ih) can be produced through the cyclization reaction of compound (XV) using a carbonylation reagent. Examples of the carbonylation reagent include activated carboxylic acids such as an acyl halide and the like such as acid chloride, acid bromide and the like. This reaction may be carried out by the addition of a base or the like.

[Production method A-4]

Among the compounds (I), compound (Ij) can be produced from compound (XVI) by the following method:

[Formula 6]

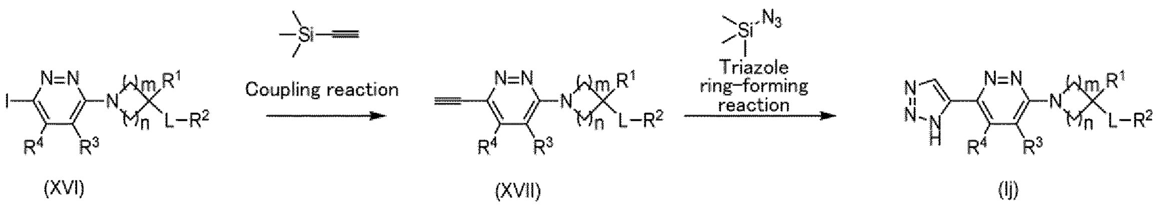


Among the compounds (I), compound (Ij) can be produced through the triazole ring-forming reaction between compound (XVII) and trimethylsilylazide. In this respect, examples of the reagent used include: a copper compound such as copper (I) iodide and the like; and the like. This reaction may be carried out by the addition of a base or the like.

Hereinafter, the method for producing the compound (II) will be described.

[Production method B-1]

Among the compounds (II), compound (IIa) can be produced from compound (XVIII) by the following method:

[Formula 7]

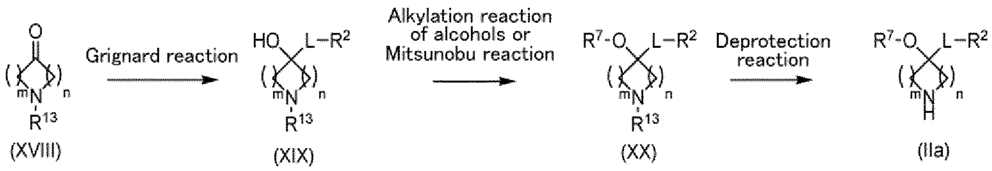


In the reaction scheme, $R^{13}$ represents a protective group for amines. Examples of the protective group for amines include the protective group mentioned above as the protective group for an amino group or an aromatic heterocycle such as imidazole, pyrrole, indole or the like.

[Production method B-2]

Among the compounds (II), compounds (IIb), (IIc), (IId) and (IIe) can each be produced from compound (XXI) or (XXIII) by the following method:

[Formula 8]

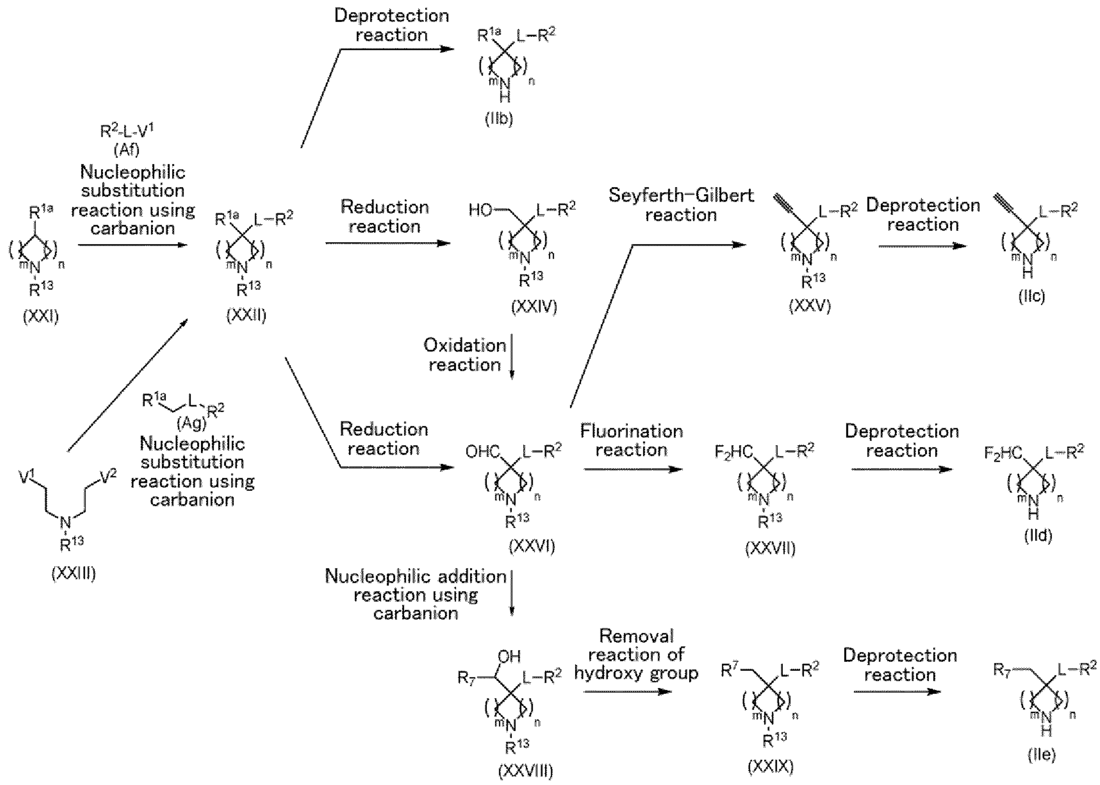


In the reaction scheme, $R^{1a}$ represents a cyano group or a $C_{1-6}$ alkoxy-carbonyl group.
Compound (XXV) can be produced through the Seyferth-Gilbert homologation reaction of compound (XXVI) in the presence of a base. In this respect, examples of the reagent used include dimethyl diazomethylphosphonate, dimethyl (1-diazo-2-oxopropyl)phosphonate and the like.
Compound (XXIX) can be produced through the removal reaction of a hydroxy group of compound (XXVIII). This reaction can be appropriately carried out according to a method known per se in the art [e.g., Tetrahedron Letters, 29 (47), 6125-6 (1988), Journal of Organic Chemistry, 66 (22), 7500-7504 (2001), Tetrahedron: Asymmetry, 20 (19), 2205-2210 (2009), Heterocycles, 39 (2), 801-9 (1994), Synlett, (3), 431-434 (2002)] or a method equivalent thereto.

[Production method B-3]

Compound (IIf) can be produced from compound (XXX) by the following method:

[Formula 9]

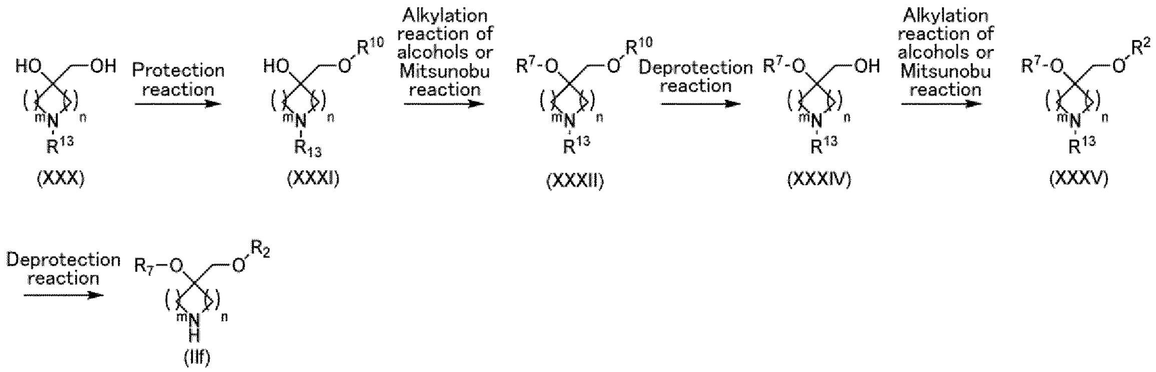

(XXX) (XXXI) (XXXII) (XXXIV) (XXXV) (IIf)

[Production method B-4]

Among the compounds (II), compound (IIg) can be produced from compound (XXXIX) by the following method:

[Formula 10]

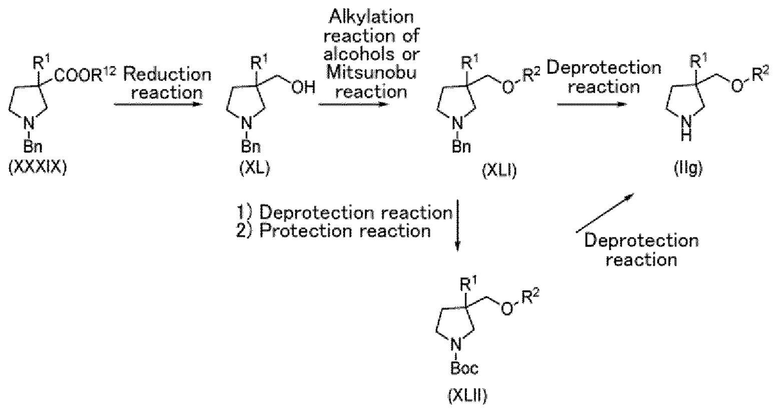

(XXXIX) (XL) (XLI) (IIg) (XLII)

In the reaction scheme, Bn represents a benzyl group, and Boc represents a tert-butoxycarbonyl group.

[Production method B-5]

Among the compounds (II), compound (IIh) can be produced from compound (XLIII) by the following method:

[Formula 11]

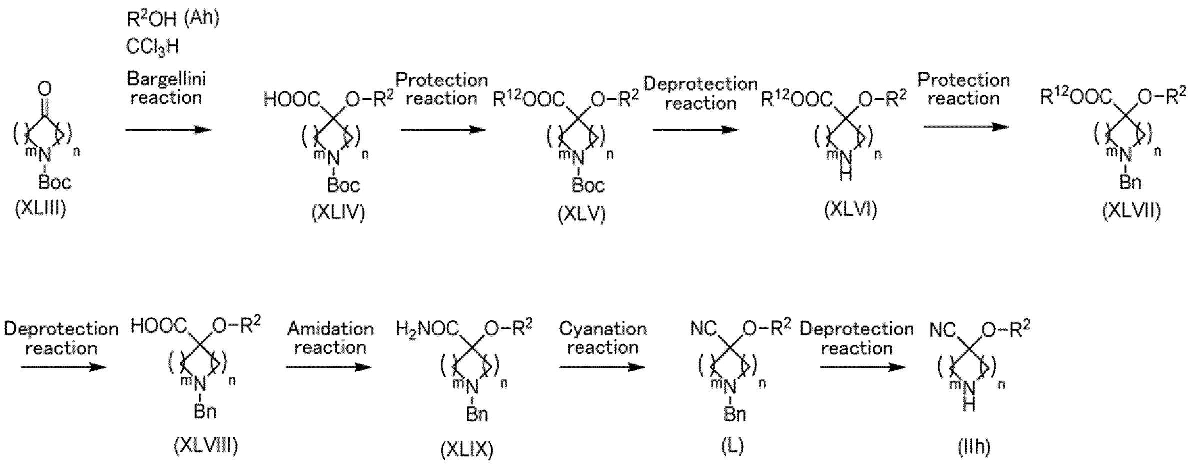

(XLIII) (XLIV) (XLV) (XLVI) (XLVII) (XLVIII) (XLIX) (L) (IIh)

Compound (XLIV) can be produced through the Bargellini reaction of compound (XLIII). This reaction can be appropriately carried out according to a method known per se in the art [e.g., Tetrahedron Letters, 50 (21), 2497-2500 (2009), WO2013/010453] or a method equivalent thereto.

Compound (L) can be produced through the cyanation reaction of compound (XLIX). This reaction can be appropriately carried out according to a method known per se in the art [e.g., WO2013/010453, WO2006/0084808, WO2000/076514, Journal of Medicinal Chemistry, 46 (25), 5512-5532 (2003)] or a method equivalent thereto.

[Production method B-6]

Among the compounds (II), compound (IIi) can be produced from compound (LI) by the following method:

[Formula 12]

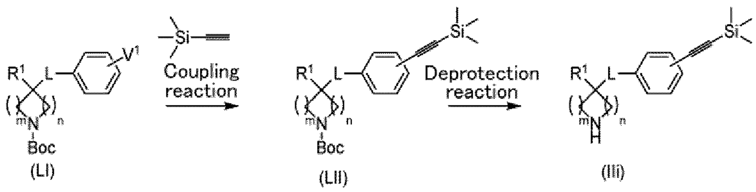

(LI) (LII) (IIi)

[Production method B-7]

Among the compounds (II), compound (IIj) can be produced from compound (LIII) by the following method:

[Formula 13]

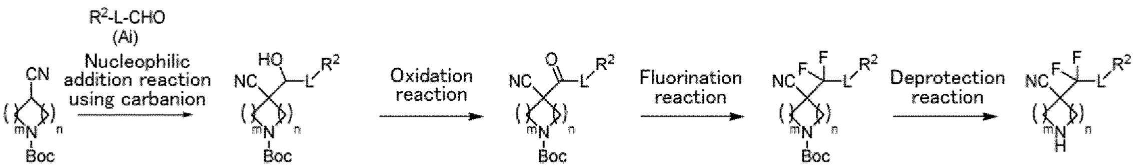

(LIII) (LIV) (LV) (LVI) (IIj)

[Production method B-8]

Among the compounds (II), compound (IIk) can be produced from compound (LVII) by the following method:

[Formula 14]

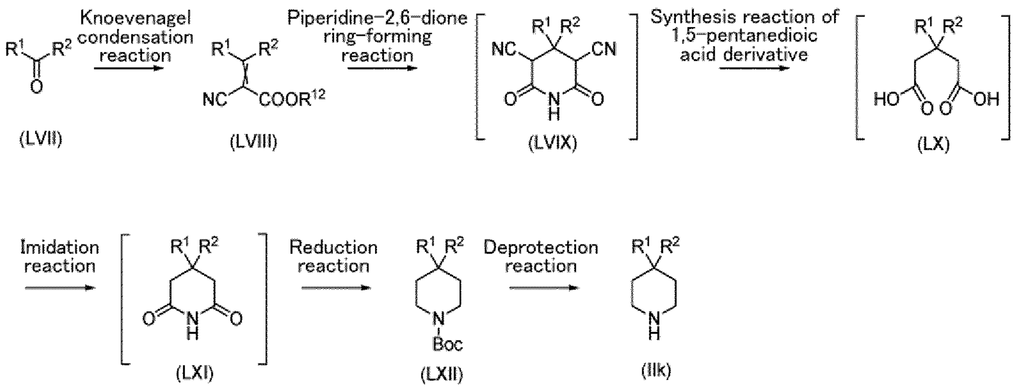

(LVII) (LVIII) (LVIX) (LX) (LXI) (LXII) (IIk)

Compound (LVIX) can be produced through the piperidine-2,6-dione ring-forming reaction between compound (LVIII) and 2-cyanoacetamide. In this respect, examples of the reagent used include organic acid salts (e.g., sodium acetate).

Compound (LX) can be produced through the synthesis reaction of a 1,5-pentanedioic acid derivative of compound (LVIX). This reaction can be appropriately carried out according to a method known per se in the art [e.g., WO2010/130424, WO2007/0123591, WO2008/099019, Journal of Organic Chemistry, 47 (8), 1445-51 (1982), Journal of the American Pharmaceutical Association (1912-1977), 39, 451-4 (1950), Journal of the American Chemical Society, 115 (1), 77-81 (1993), Journal of the American Chemical Society, 80, 3915-23 (1958), Journal of Organic Chemistry, 52 (24), 5480-2 (1987), Organic Syntheses, 39, 54-5 (1959), Organic Syntheses, 36, 28-30 (1956)] or a method equivalent thereto.

The compound (I) can also be further subjected to substituent conversion, i.e., the introduction of a substituent or functional group conversion, by the application of an approach known per se in the art to produce a compound included in the scope of the present invention.

A general method known in the art is used as a method for the substituent conversion. Examples thereof include conversion to a carboxy group by the hydrolysis of ester, conversion to a carbamoyl group by the amidation of a carboxy group, conversion to a hydroxymethyl group by the reduction of a carboxy group, conversion to an alcohol form by the reduction or alkylation of a carbonyl group, reductive amination of a carbonyl group, oximation of a carbonyl group, acylation of an amino group, ureation of an amino group, sulfonylation of an amino group, alkylation of an amino group, substitution or amination of active halogen using amine, alkylation of a hydroxy group and substitution or amination of a hydroxy group.

For this introduction of a substituent or functional group conversion, a protective group is introduced beforehand to a reactive site, if present, at which unintended reaction occurs, by an approach known per se in the art according to the need, and after the reaction of interest, the protective group can be removed again by an approach known per se in the art to produce a compound included in the scope of the present invention.

When a starting compound or an intermediate has, for example, an amino group, a carboxyl group or a hydroxy group as a substituent, the group may be protected with a protective group as generally used in peptide chemistry or the like. In this case, after the reaction, the protective group can be removed according to the need to obtain the compound of interest.

When the compound (I) has isomers such as optical isomers, stereoisomers, positional isomers, rotational isomers or the like, either of the isomers and a mixture thereof are both included in the compound (I). When the compound (I) has, for example, optical isomers, the optical isomers resolved from a racemate are also included in the compound (I). These isomers can each be obtained as a single product by a synthesis approach and a separation approach known per se in the art (e.g., concentration, solvent extraction, column chromatography, recrystallization).

The compound (I) may be crystalline. A single crystal form and a mixture of crystal forms are both included in the compound (I). The crystals can be produced by crystallization by the application of a crystallization method known per se in the art.

Also, the compound (I) may be a pharmaceutically acceptable cocrystal or cocrystal salt. In this context, the cocrystal or the cocrystal salt means a crystalline substance constituted by two or more unique solids at room temperature, each having distinctive physical properties (e.g., structure, melting point, heat of melting, hygroscopicity, stability). The cocrystal and the cocrystal salt can be produced according to a cocrystallization method known per se in the art.

The compound (I) may be a hydrate, a non-hydrate, a solvate or a non-solvate. All of them are included in the compound (I).

A compound labeled with an isotope (e.g., $^{2}H$, $^{3}H$, $^{11}C$, $^{14}C$, $^{18}F$, $^{35}S$, $^{125}I$) or the like is also included in the compound (I). The compound (I) labeled or substituted with an isotope can be used as, for example, a tracer (PET tracer), in positron emission tomography (PET) and is useful in fields of medical diagnosis and the like.

The compound (I) may be a prodrug.

The prodrug of the compound (I) refers to a compound that is converted to the compound (I) through a reaction caused by an enzyme, gastric acid or the like under physiological conditions *in vivo,* i.e., a compound that is converted to the compound (I) by enzymatic oxidation, reduction, hydrolysis, etc., or a compound that is converted to the compound (I) by hydrolysis, etc., caused by gastric acid or the like.

Examples of the prodrug of the compound (I) include:

(1) a compound in which amino of the compound (I) is acylated, alkylated or phosphorylated (e.g., a compound in which amino of the compound (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated, ethoxycarbonylated, tert-butoxycarbonylated, acetylated, cyclopropylcarbonylated, etc.);

(2) a compound in which hydroxy of the compound (I) is acylated, alkylated, phosphorylated or borated (e.g., a compound in which hydroxy of the compound (I) is acetylated, palmitoylated, propanoylated, pivaloylated, succinylated, fumarylated, alanylated, dimethylaminomethylcarbonylated, etc.); and

(3) a compound in which carboxy of the compound (I) is esterified or amidated (e.g., a compound in which carboxy of the compound (I) is ethyl-esterified, phenyl-esterified, carboxymethyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolen-4-yl)methyl-esterified, cyclohexyloxycarbonylethyl-esterified, methylamidated, etc.). These compounds can be produced from the compound (I) by a method known per se in the art.

The prodrug of the compound (I) may be converted to the compound (I) under physiological conditions as described in Iyakuhin No Kaihatsu (Development of Pharmaceuticals in English), Vol. 7, Molecular Design, p. 163-198, Hirokawa Shoten Ltd. (1990).

The compound (I) or the prodrug thereof (in the present specification, these are also collectively referred to as the "compound of the present invention") has SCD (particularly, SCD1) inhibitory activity and is useful as a prophylactic or therapeutic agent for cancer, a cancer growth inhibitor and a cancer metastasis inhibitor.

The compound of the present invention exhibits selective inhibitory activity against SCD1. In addition, the compound of the present invention is also excellent in efficacy development, pharmacokinetics (e.g., absorbability, distribution, metabolism, excretion), solubility (e.g., water solubility), interaction with other medicaments (e.g., drug-metabolizing enzyme inhibitory effect), safety (e.g., acute toxicity, chronic toxicity, genotoxicity, reproductive toxicity, cardiotoxicity, carcinogenicity, central toxicity) and stability (e.g., chemical stability, stability against enzymes) and is therefore useful as a medicament.

The compound of the present invention has low inhibitory activity against subtypes of the SCD family except for SCD1 and is therefore useful as a prophylactic or therapeutic agent for cancer with reduced toxicity to normal cells.

Thus, the compound of the present invention can be used for inhibiting excessive (abnormal) SCD1 effects in a mammal (e.g., a mouse, a rat, a hamster, a rabbit, a cat, a dog, cattle, sheep, a monkey, a human).

The compound of the present invention is used as a medicament such as a prophylactic or therapeutic agent for diseases that are probably influenced by SCD1 (in the present specification, also referred to as "SCD1-related diseases"), for example, cancer [e.g., large intestine cancer (e.g., colon cancer, rectal cancer, anus cancer, familial colorectal cancer, hereditary non-polyposis colorectal cancer, gastrointestinal stromal tumor), lung cancer (e.g., non-small cell lung cancer, small-cell lung cancer, malignant mesothelioma), mesothelioma, pancreatic cancer (e.g., ductal pancreatic cancer, pancreatic endocrine tumor), throat cancer, voice box cancer, esophageal cancer, stomach cancer (e.g., papillary adenocarcinoma, mucous adenocarcinoma, adenosquamous carcinoma), duodenal cancer, small intestine cancer, breast cancer (e.g., invasive ductal breast cancer, noninvasive ductal breast cancer, inflammatory breast cancer), ovarian cancer (e.g., epithelial ovarian cancer, extragonadal germ cell tumor, ovarian germ cell tumor, ovarian tumor of low malignant potential), testicular tumor, prostate cancer (e.g., hormone-dependent prostate cancer, hormone-independent prostate cancer, castration-resistant prostate cancer), liver cancer (e.g., hepatocellular cancer, primary liver cancer, extrahepatic bile duct cancer), thyroid cancer (e.g., medullary thyroid cancer), kidney cancer (e.g., renal cell cancer (e.g., clear cell renal cell carcinoma), transitional cell cancer of the renal pelvis and ureter), uterine cancer (e.g., uterine cervical cancer, uterine body cancer, uterine sarcoma), gestational choriocarcinoma, brain tumor (e.g., medulloblastoma, glioma, pineal astrocytoma, pilocytic astrocytoma, diffuse astrocytoma, anaplastic astrocytoma, pituitary adenoma), retinoblastoma, skin cancer (e.g., basalioma, malignant melanoma), sarcoma (e.g., rhabdomyosarcoma, leiomyosarcoma, soft tissue sarcoma, spindle cell sarcoma), malignant bone tumor, bladder cancer, blood cancer (e.g., multiple myeloma, leukemia, malignant lymphoma, Hodgkin disease, chronic myeloproliferative disease), primary unknown cancer], a cancer growth inhibitor, a cancer metastasis inhibitor, an apoptosis promoter, a therapeutic agent for premalignant lesions (e.g., myelodysplastic syndrome) or the like.

The compound of the present invention can be orally or parenterally administered as a medicament containing the compound of the present invention alone or as a mixture with a pharmacologically acceptable carrier to a mammal (preferably a human).

Hereinafter, the medicament comprising the compound of the present invention (also referred to as the "medicament of the present invention") will be described in detail. Examples of the dosage form of the medicament of the present invention include an oral preparation such as tablets (including sugar-coated tablets, film-coated tablets, sublingual tablets, buccal tablets and rapidly orally disintegrating tablets), pills, granules, powders, capsules (including soft capsules and microcapsules), syrups, emulsions, suspensions, films (e.g., orally disintegrating films and patch films for application to the oral mucosa) and the like. Other examples of the dosage form of the medicament of the present invention include a parenteral preparation such as injections, transfusions, transdermal preparations (e.g., iontophoresis dermal preparations), suppositories, ointments, transnasal preparations, transpulmonary preparations, eye drops and the like. Alternatively, the medicament of the present invention may be a controlled-release preparation such as a rapid-release preparation, a sustained-release preparation (including a sustained-release microcapsule) or the like.

The medicament of the present invention can be produced by a production method known in the art (e.g., a method described in Japanese Pharmacopoeia) generally used in the field of pharmaceutical technology. If necessary, the medicament of the present invention can appropriately contain an appropriate amount of an additive usually used in the pharmaceutical field, such as an excipient, a binder, a disintegrant, a lubricant, a sweetener, a surfactant, a suspending agent, an emulsifier, a colorant, a preservative, a fragrance, a corrigent, a stabilizer, a viscosity modifier and the like.

Examples of the pharmacologically acceptable carrier described above include these additives.

For example, the tablets can be produced using an excipient, a binder, a disintegrant, a lubricant and the like. The pills and the granules can be produced using an excipient, a binder and a disintegrant. The powders and the capsules can be produced using an excipient and the like. The syrups can be produced using a sweetener and the like. The emulsions or the suspensions can be produced using a suspending agent, a surfactant, an emulsifier and the like.

Examples of the excipient include lactose, saccharose, glucose, starch, sucrose, microcrystalline cellulose, licorice powder, mannitol, sodium bicarbonate, calcium phosphate and calcium sulfate.

Examples of the binder include a solution containing 5 to 10% by weight of starch paste, a solution containing 10 to 20% by weight of gum arabic or gelatin, a solution containing 1 to 5% by weight of tragacanth, a carboxymethylcellulose solution, a sodium alginate solution and glycerin.

Examples of the disintegrant include starch and calcium carbonate.

Examples of the lubricant include magnesium stearate, stearic acid, calcium stearate and purified talc.

Examples of the sweetener include glucose, fructose, invert sugar, sorbitol, xylitol, glycerin and simple syrup.

Examples of the surfactant include sodium lauryl sulfate, polysorbate 80, sorbitan monofatty acid ester and polyoxyl 40 stearate.

Examples of the suspending agent include gum arabic, sodium alginate, carboxymethylcellulose sodium, methylcellulose and bentonite.

Examples of the emulsifier include gum arabic, tragacanth, gelatin and polysorbate 80.

When the medicament of the present invention is, for example, tablets, the tablets can be produced according to a method known per se in the art by adding, for example, an excipient (e.g., lactose, saccharose, starch), a disintegrant (e.g., starch, calcium carbonate), a binder (e.g., starch, gum arabic, carboxymethylcellulose, polyvinylpyrrolidone, hydroxypropylcellulose) or a lubricant (e.g., talc, magnesium stearate, polyethylene glycol 6000) to the compound of the present invention and molding the mixture by compression, followed by coating, if necessary, by a method known per se in the art for the purpose of taste masking, enteric properties or durability. For example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit (manufactured by Rohm GmbH, Germany, methacrylic acid-acrylic acid copolymer) and a dye (e.g., iron red, titanium dioxide) are used as coating agents for the coating.

The injections include intravenous injections as well as subcutaneous injections, intracutaneous injections, intramuscular injections, intraperitoneal injections, drip injections and the like.

Such injections are prepared by a method known per se in the art, i.e., by dissolving, suspending or emulsifying the compound of the present invention in a sterile aqueous solution or oily solution. Examples of the aqueous solution include saline, an isotonic solution containing glucose or an additional adjuvant (e.g., D-sorbitol, D-mannitol, sodium chloride) and the like. The aqueous solution may contain an appropriate solubilizing agent, for example, an alcohol (e.g., ethanol), a polyalcohol (e.g., propylene glycol, polyethylene glycol) or a nonionic surfactant (e.g., polysorbate 80, HCO-50). Examples of the oily solution include sesame oil, soybean oil and the like. The oily solution may contain an appropriate solubilizing agent. Examples of the solubilizing agent include benzyl benzoate, benzyl alcohol and the like. The injections may be further supplemented with a buffer (e.g., a phosphate buffer solution, a sodium acetate buffer solution), a soothing agent (e.g., benzalkonium chloride, procaine hydrochloride), a stabilizer (e.g., human serum albumin, polyethylene glycol), a preservative (e.g., benzyl alcohol, phenol) or the like. Ampules are usually filled with the prepared injection solutions.

The content of the compound of the present invention in the medicament of the present invention differs depending on the form of the preparation and is usually approximately 0.01 to approximately 100% by weight, preferably approximately 2 to approximately 85% by weight, more preferably approximately 5 to approximately 70% by weight, with respect to the whole preparation.

The content of the additive in the medicament of the present invention differs depending on the form of the preparation and is usually approximately 1 to approximately 99.9% by weight, preferably approximately 10 to approximately 90% by weight, with respect to the whole preparation.

The compound of the present invention can be used stably, low toxically and safely. The daily dose of the compound of the present invention differs depending on the status and body weight of a patient, the type of the compound, an administration route, etc. In the case of, for example, oral administration to a patient for the purpose of treating cancer, the daily dose in adult (body weight: approximately 60 kg) is approximately 1 to approximately 1000 mg, preferably approximately 3 to approximately 300 mg, more preferably approximately 10 to approximately 200 mg, of the compound of the present invention, which can be administered in one portion or in two or three portions.

In the case of parenterally administering the compound of the present invention, the compound of the present invention is usually administered in the form of a solution (e.g., an injection). The single dose of the compound of the present invention also differs depending on a recipient, a target organ, symptoms, an administration method, etc. For example, usually approximately 0.01 to approximately 100 mg, preferably approximately 0.01 to approximately 50 mg,

more preferably approximately 0.01 to approximately 20 mg, of the compound of the present invention per kg of body weight is preferably administered by intravenous injection.

The compound of the present invention can be used in combination with an additional drug. Specifically, the compound of the present invention can be used in combination with a drug such as a hormone therapeutic, a chemotherapeutic, an immunotherapeutic, an agent inhibiting the effects of a cell growth factor and its receptor, or the like. Hereinafter, the drug that may be used in combination with the compound of the present invention is referred to as a concomitant drug.

Examples of the "hormone therapeutic" that may be used include fosfestrol, diethylstilbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogen (e.g., tamoxifen citrate, toremifene citrate), contraceptive pills, mepitiostane, testololactone, aminoglutethimide, LH-RH agonists

(e.g., goserelin acetate, buserelin, leuprorelin acetate), droloxifene, epitiostanol, ethinyl estradiol sulfonate, aromatase inhibitors (e.g., fadrozole hydrochloride, anastrozole, letrozole, exemestane, vorozole, formestane), anti-androgen (e.g., flutamide, bicalutamide, nilutamide, enzalutamide), 5α-reductase inhibitors (e.g., finasteride, epristeride, dutasteride), adrenal corticosteroid agents (e.g., dexamethasone, prednisolone, betamethasone, triamcinolone), androgen synthesis inhibitors (e.g., abiraterone), retinoid and agents delaying retinoid metabolism (e.g., liarozole), thyroid hormones and DDS (drug delivery system) preparations thereof.

Examples of the "chemotherapeutic" that may be used include an alkylating agent, an antimetabolite, an anticancer antibiotic and a plant-derived anticancer agent.

Examples of the "alkylating agent" that may be used include nitrogen mustard, nitrogen mustard-N-oxide hydrochloride, chlorambucil, cyclophosphamide, ifosfamide, thiotepa, carboquone, improsulfan tosilate, busulfan, nimustine hydrochloride, mitobronitol, melphalan, dacarbazine, ranimustine, estramustine sodium phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, Ribomustin, temozolomide, treosulfan, trofosfamide, zinostatin stimalamer, adozelesin, cystemustine, bizelesin and DDS preparations thereof.

Examples of the "antimetabolite" that may be used include mercaptopurine, 6-mercaptopurine riboside, thioinosine, methotrexate, pemetrexed, enocitabine, cytarabine, cytarabine ocfosfate, ancitabine hydrochloride, 5-FU drugs (e.g., fluorouracil, tegafur, UFT, doxifluridine, carmofur, galocitabine, emitefur, capecitabine), aminopterin, nelarabine, leucovorin calcium, Tabloid, butocine, calcium folinate, calcium levofolinate, cladribine, emitefur, fludarabine, gemcitabine, hydroxycarbamide, pentostatin, piritrexim, idoxuridine, mitoguazone, tiazofurin, ambamustine, bendamustine and DDS preparations thereof.

Examples of the "anticancer antibiotic" that may be used include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane, zorubicin hydrochloride, mitoxantrone hydrochloride, idarubicin hydrochloride and DDS preparations (e.g., PEG liposomal doxorubicin) thereof.

Examples of the "plant-derived anticancer agent" that may be used include etoposide, etoposide phosphate, vinblastine sulfate, vincristine sulfate, vindesine sulfate, teniposide, paclitaxel, docetaxel, cabazitaxel, vinorelbine and DDS preparations thereof.

Examples of the "immunotherapeutic" that may be used include picibanil, Krestin, schizophyllan, lentinan, ubenimex, interferon, interleukin, macrophage colony-stimulating factor, granulocyte colony-stimulating factor, erythropoietin, lymphotoxin, BCG vaccines, *Corynebacterium parvum,* levamisole, polysaccharide K, procodazol, anti-CTLA4 antibodies (e.g., ipilimumab, tremelimumab), anti-PD-1 antibodies (e.g., nivolumab, pembrolizumab) and anti-PD-L1 antibodies.

The "cell growth factor" in the "agent inhibiting the effects of a cell growth factor and its receptor" can be any substance that promotes the growth of cells. Typical examples thereof include a factor that is a peptide having a molecular weight of 20,000 or smaller and exerts its effects at a low concentration through binding to its receptor. Specific examples of the cell growth factor that may be used include (1) EGF (epidermal growth factor) or a substance having activity substantially identical thereto [e.g., TGFα], (2) insulin or a substance having activity substantially identical thereto [e.g., insulin, IGF (insulin-like growth factor)-1, IGF-2], (3) FGF (fibroblast growth factor) or a substance having activity substantially identical thereto [e.g., acidic FGF, basic FGF, KGF (keratinocyte growth factor), FGF-10], and (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), heregulin, angiopoietin].

The "receptor of the cell growth factor" can be any receptor having the ability to bind to any of the cell growth factor described above. Specific examples of the receptor that may be used include EGF receptor, heregulin receptor (e.g., HER3), insulin receptor, IGF receptor-1, IGF receptor-2, FGF receptor-1 or FGF receptor-2, VEGF receptor,

angiopoietin receptor (e.g., Tie2), PDGF receptor and the like.

Examples of the "agent inhibiting the effects of a cell growth factor and its receptor" that may be used include EGF inhibitors, TGFα inhibitors, heregulin inhibitors, insulin inhibitors, IGF inhibitors, FGF inhibitors, KGF inhibitors, CSF inhibitors, EPO inhibitors, IL-2 inhibitors, NGF inhibitors, PDGF inhibitors, TGFβ inhibitors, HGF inhibitors, VEGF inhibitors, angiopoietin inhibitors, EGF receptor inhibitors, HER2 inhibitors, HER4 inhibitors, insulin receptor inhibitors, IGF-1 receptor inhibitors, IGF-2 receptor inhibitors, FGF receptor-1 inhibitors, FGF receptor-2 inhibitors, FGF receptor-3 inhibitors, FGF receptor-4 inhibitors, VEGF receptor inhibitors, Tie-2 inhibitors, PDGF receptor inhibitors, Abl inhibitors, Raf inhibitors, FLT3 inhibitors, c-Kit inhibitors, Src inhibitors, PKC inhibitors, Smo inhibitors, ALK inhibitors, ROR1 inhibitors, Trk inhibitors, Ret inhibitors, mTOR inhibitors, Aurora inhibitors, PLK inhibitors, MEK (MEK1/2) inhibitors, MET inhibitors, CDK inhibitors, Akt inhibitors, ERK inhibitors, PI3K inhibitors and the like. More specific examples of the agent that may be used include anti-VEGF antibodies (e.g., bevacizumab, ramucirumab), anti-HER2 antibodies (e.g., trastuzumab, pertuzumab), anti-EGFR antibodies (e.g., cetuximab, panitumumab, matuzumab, nimotuzumab), anti-HGF antibodies, imatinib, erlotinib, gefitinib, sorafenib, sunitinib, dasatinib, lapatinib, vatalanib, ibrutinib, bosutinib, cabozantinib, crizotinib, alectinib, vismodegib, cediranib, tivantinib, quizartinib, dovitinib, axitinib, motesanib, nilotinib, 6-[4-(4-ethylpiperazin-1-ylmethyl)phenyl]-N-[1(R)-phenylethyl]-7H-pyrrolo[2,3-d]pyrimidin-4-amine (AEE-788), vandetanib, temsirolimus, everolimus, enzastaurin, tozasertib, phosphoric acid 2-[N-[3-[4-[5-[N-(3-fluorophenyl)carbamoylmethyl]-1H-pyrazol-3-ylamino] quinazolin-7-yloxy]propyl]-N-ethylamino]ethyl ester (AZD-1152), 4-[9-chloro-7-(2,6-difluorophenyl)-5H-pyrimido[5,4-d][2]benzazapin-2-ylamino]benzoic acid, N-[2-methoxy-5-[(E)-2-(2,4,6-trimethoxyphenyl)vinylsulfonylmethyl]phenyl]glycine sodium salt (ON-1910Na), volasertib, selumetinib, trametinib, N-[2(R),3-dihydroxypropoxy]-3,4-difluoro-2-(2-fluoro-4-iodophenylamino)benzamide (PD-0325901), bosutinib, regorafenib, afatinib, idelalisib, ceritinib, dabrafenib and the like.

In addition to the drugs described above, L-asparaginase, L-arginase, arginine deiminase, aceglatone, procarbazine hydrochloride, protoporphyrin-cobalt complex salt, mercury hematoporphyrin-sodium, topoisomerase I inhibitors (e.g., irinotecan, topotecan, indotecan, indimitecan), topoisomerase II inhibitors (e.g., sobuzoxane), differentiation inducers (e.g., retinoid, vitamins D), other angiogenesis inhibitors (e.g., fumagillin, shark extracts, COX-2 inhibitors), α-blockers (e.g., tamsulosin hydrochloride), bisphosphonic acids (e.g., pamidronate, zoledronate), thalidomide, lenalidomide, pomalidomide, 5-azacytidine, decitabine, proteasome inhibitors (e.g., bortezomib, carfilzomib, ixazomib), NEDD8 inhibitors (e.g., pevonedistat), UAE inhibitors, PARP inhibitors (e.g., olaparib, niraparib, veliparib), antitumor antibodies such as anti-CD20 antibodies (e.g., rituximab, obinutuzumab), anti-CCR4 antibodies (e.g., mogamulizumab) and the like, antibody-drug conjugates (e.g., trastuzumab emtansine, brentuximab vedotin) or the like can also be used as the concomitant drug.

The combination of the compound of the present invention and the concomitant drug can produce excellent effects such as: (1) the dose of the compound of the present invention or the concomitant drug can be reduced as compared with the administration of the compound of the present invention or the concomitant drug alone; (2) the concomitant drug can be selected for combined use with the compound of the present invention according to the symptoms (mild, serious, etc.) of a patient; (3) the period of treatment can be set longer; (4) a sustained therapeutic effect can be achieved; (5) a synergistic effect can be obtained by the combined use of the compound of the present invention and the concomitant drug; and the like.

Hereinafter, the combined use of the compound of the present invention and the concomitant drug is referred to as the "combination drug of the present invention".

For use of the combination drug of the present invention, the time of administration of the compound of the present invention and the time of administration of the concomitant drug are not limited, and the compound of the present invention and the concomitant drug may be administered simultaneously or in a staggered manner to a recipient. In the case of administration in a staggered manner, the staggered manner differs depending on active ingredients to be administered, a dosage form and an administration method. In the case of first administering, for example, the concomitant drug, the compound of the present invention can be administered within 1 minute to 3 days, preferably within 10 minutes to 1 day, more preferably within 15 minutes to 1 hour, after the administration of the concomitant drug. In the case of first administering the compound of the present invention, the concomitant drug can be administered within 1 minute to 1 day, preferably within 10 minutes to 6 hours, more preferably within 15 minutes to 1 hour, after the administration of the compound of the present invention. The dose of the concomitant drug can abide by a dose clinically used and can be appropriately selected according to a recipient, an administration route, a disease, a combination, etc.

Examples of the administration mode of the compound of the present invention and the concomitant drug used in combination include (1) the administration of a single preparation obtained by simultaneously formulating the compound of the present invention and the concomitant drug, (2) the simultaneous administration through the same administration route of two preparations obtained by separately formulating the compound of the present invention and the concomitant drug, (3) the administration through the same administration route in a staggered manner of two preparations obtained by separately formulating the compound of the present invention and the concomitant drug, (4) the simultaneous administration through different administration routes of two preparations obtained by separately formulating the compound

of the present invention and the concomitant drug, and (5) the administration through different administration routes in a staggered manner of two preparations obtained by separately formulating the compound of the present invention and the concomitant drug (e.g., administration in the order of the compound of the present invention and then the concomitant drug, or in the reverse order).

The dose of the concomitant drug can be appropriately selected on the basis of a dose clinically used. The mixing ratio between the compound of the present invention and the concomitant drug can be appropriately selected according to a recipient, an administration route, a target disease, symptoms, a combination, etc. When the recipient is, for example, a human, 0.01 to 100 parts by weight of the concomitant drug can be used with respect to 1 part by weight of the compound of the present invention.

The compound of the present invention or the combination drug of the present invention can be further used in combination with a non-drug therapy. Specifically, the compound of the present invention or the combination drug of the present invention may be combined with a non-drug therapy, for example, (1) surgery, (2) induced hypertension chemotherapy using angiotensin II or the like, (3) gene therapy, (4) thermotherapy, (5) cryotherapy, (6) laser cauterization or (7) radiotherapy.

The compound of the present invention or the combination drug of the present invention is used, for example, before or after the surgery or the like or before or after treatment involving two or three of these therapies in combination to produce effects such as prevention of development of resistance, prolonged disease-free survival, inhibition of cancer metastasis or recurrence, life prolongation and the like.

Also, the treatment with the compound of the present invention or the combination drug of the present invention may be combined with supportive care [(i) the administration of an antibiotic (e.g., a β-lactam antibiotic such as Pansporin and the like, a macrolide antibiotic such as clarithromycin and the like) against various intercurrent infections, (ii) the administration of a high-calorie infusion, an amino acid preparation or multivitamin for the improvement of malnutrition, (iii) the administration of morphine for pain relief, (iv) the administration of a drug improving adverse reactions such as nausea, vomiting, anorexia, diarrhea, leukopenia, thrombocytopenia, decreased hemoglobin concentration, alopecia, liver damage, kidney damage, DIC, fever and the like and (v) the administration of a drug for inhibiting multidrug resistance of cancer, etc.].

The present invention will be described further specifically with reference to Examples, Formulation Examples and Test Examples given below. However, the present invention is not intended to be limited by them, and various changes or modifications may be made therein without departing from the scope of the present invention.

Examples

In Examples below, the term "room temperature" usually means approximately 10°C to approximately 35°C. A ratio used for a mixed solvent represents a volume ratio unless otherwise specified. % represents % by weight unless otherwise specified.

The term "NH" in silica gel column chromatography represents that an aminopropylsilane-bound silica gel was used. The term "Diol" therein represents that a 3-(2,3-dihydroxypropoxy)propylsilane-bound silica gel was used. The term "DiNH" therein represents that a N-(2-aminoethyl)-3-aminopropylsilane-bound silica gel was used. The term "C18" in HPLC (high-performance liquid chromatography) represents that an octadecyl-bound silica gel was used. A ratio used for elution solvents represents a volume ratio unless otherwise specified.

In Examples below, the following abbreviations are used:

| | |
|---|---|
| mp: | melting point |
| MS: | mass spectrum |
| $[M+H]^+$, $[M-H]^-$: | molecular ion peak |
| M: | molar concentration |
| N: | normal |
| $CDCl_3$: | deuterated chloroform |
| DMSO-$d_6$: | deuterated dimethyl sulfoxide |
| $^1H$ NMR: | proton nuclear magnetic resonance |
| LC/MS: | liquid chromatograph-mass spectrometer |
| ESI: | electrospray ionization |
| APCI: | atmospheric pressure chemical ionization |
| THF: | tetrahydrofuran |
| DME: | 1,2-dimethoxyethane |
| DMF: | N,N-dimethylformamide |
| DMA: | N,N-dimethylacetamide |
| HATU: | 2-(7-azabenzotriazol-1-yl)-1,1,3,3-tetramethyluronium hexafluorophosphate |

| | |
|---|---|
| HOBt: | 1-hydroxybenzotriazole |
| CDI: | 1,1'-carbonyldiimidazole |
| Py: | pyridine |
| IPE: | diisopropyl ether |
| DIPEA: | N,N'-diisopropylethylamine |
| IPA: | isopropanol |
| KHMDS: | potassium bis(trimethylsilyl)amide |
| DIPA: | diisopropylamine |
| NMO: | N-methylmorpholine N-oxide |
| TBAF: | tetrabutylammonium fluoride |
| pTsCl: | p-toluenesulfonyl chloride |
| TFA: | trifluoroacetic acid |
| DIAD: | diisopropyl azodicarboxylate |
| CPME: | cyclopentyl methyl ether |
| EDC: | 1-ethyl-3-(3-dimethylaminopropyl) carbodiimide |
| TLC: | thin-layer chromatography |
| IBX: | 2-iodoxybenzoic acid |
| TBAI: | tetrabutylammonium iodide |
| DIBAL: | diisobutyl aluminum hydride |
| AIBN: | 2,2'-(E)-diazene-1,2-diyl bis(2-methylpropanenitrile) |

$^1$H NMR was measured by Fourier transform NMR. ACD/SpecManager (trade name) or the like was used in analysis. No mention was made about the very broad peaks of protons of a hydroxy group, an amino group and the like.

MS was measured by LC/MS. ESI or APCI was used as an ionization method. Data was indicated by actually measured values (found). In general, molecular ion peaks are observed. In the case of a compound having a tert-butoxycarbonyl group, a fragment ion peak derived from the elimination of the tert-butoxycarbonyl group or the tert-butyl group may be observed. In the case of a compound having a hydroxy group, a fragment ion peak derived from the elimination of $H_2O$ may be observed. In the case of a salt, a molecular ion peak or fragment ion peak of a free form is usually observed.

The unit of a sample concentration (c) in optical rotation ($[\alpha]_D$) was g/100 mL.

Element analysis values (Anal.) were indicated by calculated values (Calcd) and actually measured values (Found).

Example 1

(5-(6-(4-(2-Chlorobenzyl)-4-methoxypiperidin-1-yl)pyridazin-3-yl)-1,2,4-oxadiazol-3-yl)methyl benzoate

A) tert-Butyl 4-(2-chlorobenzyl)-4-hydroxypiperidine-1-carboxylate

To a mixed solution of magnesium (2.74 g), iodine (0.955 g) and diethyl ether (50 mL), a solution of 2-chlorobenzyl chloride (14.75 mL) in diethyl ether (50 mL) was added at room temperature, and the mixture was stirred at 40°C for 1 hour. A solution of tert-butyl 4-oxopiperidine-1-carboxylate (15 g) in diethyl ether (100 mL) was added to the reaction solution. In a nitrogen atmosphere, the mixture was stirred overnight at room temperature. The mixture was poured to a saturated aqueous solution of ammonium chloride, followed by extraction with ethyl acetate. The organic layer was washed with water and saturated brine, dried over magnesium sulfate and then concentrated. The residue was purified by column chromatography (ethyl acetate/hexane) to obtain the title compound (19.22 g). $^1$H NMR (300 MHz, CDCl3) $\delta$ 1.45 (9H, s), 1.48-1.56 (2H, m), 1.75 (3H, s), 2.97 (2 H, s), 3.01-3.18 (2H, m), 3.77-4.02 (2H, m), 7.09-7.24 (2H, m), 7.26-7.32 (1H, m), 7.34-7.41 (1H, m).

B) tert-Butyl 4-(2-chlorobenzyl)-4-methoxypiperidine-1-carboxylate

To a solution of tert-butyl 4-(2-chlorobenzyl)-4-hydroxypiperidine-1-carboxylate (3.5 g) and iodomethane (1.337 mL) in DMA (30 mL), 60% (w/w) sodium hydride in oil (0.516 g) was added at room temperature. In a nitrogen atmosphere, the mixture was stirred at room temperature for 3 hours. The mixture was poured to a saturated aqueous solution of ammonium chloride, followed by extraction with ethyl acetate. The organic layer was washed with water and saturated brine, dried over magnesium sulfate and then concentrated. The residue was purified by column chromatography (ethyl acetate/hexane) to obtain the title compound (3.16 g). $^1$H NMR (300 MHz, CDCl3) $\delta$ 1.36-1.58 (11H, m), 1.65-1.81 (2H, m), 2.87-3.06 (4H, m), 3.35 (3H, s), 3.71-3.97 (2H, m), 7.08-7.28 (3H, m), 7.31-7.40 (1H, m).

C) 4-(2-Chlorobenzyl)-4-methoxypiperidine hydrochloride

A mixture of tert-butyl 4-(2-chlorobenzyl)-4-methoxypiperidine-1-carboxylate (3.0 g), hydrogen chloride (4 N solution in ethyl acetate, 10 mL) and ethyl acetate (20 mL) was stirred overnight at room temperature. The deposit was collected by filtration to obtain the title compound (2.1 g).
MS, found: 239.9.

D) (5-(6-(4-(2-Chlorobenzyl)-4-methoxypiperidin-1-yl)pyridazin-3-yl)-1,2,4-oxadiazol-3-yl)methyl benzoate

A mixture of 4-(2-chlorobenzyl)-4-methoxypiperidine hydrochloride (218 mg), (5-(6-chloropyridazin-3-yl)-1,2,4-oxadiazol-3-yl)methyl benzoate (250 mg), DIPEA (0.687 mL) and IPA (3 mL) was stirred at 180°C for 2 hours under irradiation with microwave. The reaction solution was concentrated under reduced pressure, and the residue was purified by column chromatography (ethyl acetate/hexane) to obtain the title compound (280 mg). $^1$H NMR (300 MHz, CDCl3) $\delta$ 1.60-1.75 (2H, m), 1.87-2.00 (2H, m), 3.05 (2H, s), 3.26-3.39 (2H, m), 3.44 (3H, s), 4.25-4.46 (2H, m), 5.54 (2H, s), 6.82-6.96 (1H, m), 7.11-7.25 (3H, m), 7.33-7.38 (1H, m), 7.41-7.49 (2H, m), 7.54-7.64 (1H, m), 7.88-7.96 (1H, m), 8.07-8.15 (2H, m).

Example 2

(5-(6-(4-(2-Chlorobenzyl)-4-methoxypiperidin-1-yl)pyridazin-3-yl)-1,2,4-oxadiazol-3-yl)methanol

In a nitrogen atmosphere, a mixture of (5-(6-(4-(2-chlorobenzyl)-4-methoxypiperidin-1-yl)pyridazin-3-yl)-1,2,4-oxadiazol-3-yl)methyl benzoate (280 mg), sodium hydroxide (2 N aqueous solution, 2 mL) and THF (10 mL) was stirred at room temperature for 1 hour. The reaction mixture was neutralized with acetic acid (0.247 mL)/water at 0°C, followed by extraction with ethyl acetate. The organic layer was washed with water and saturated brine, dried over magnesium sulfate and then concentrated. The residue was purified by column chromatography (ethyl acetate/hexane) to obtain the title compound (190 mg). $^1$H NMR (300 MHz, DMSO-d6) $\delta$ 1.46-1.64 (2H, m), 1.77-1.91 (2H, m), 3.00 (2H, s), 3.11-3.27 (2H, m), 3.37 (3H, s), 4.29-4.46 (2H, m), 4.56-4.69 (2H, m), 5.69-5.82 (1H, m), 7.18-7.53 (5H, m), 7.87-8.04 (1H, m).

Example 16

4-(2-Chlorobenzyl)-1-(6-(3-(hydroxymethyl)-1,2,4-oxadiazol-5-yl)pyridazin-3-yl)piperidine-4-carbonitrile

A) tert-Butyl 4-(2-chlorobenzyl)-4-cyanopiperidine-1-carboxylate

To a solution of tert-butyl 4-cyanopiperidine-1-carboxylate (5.1 g) in dehydrated THF (200 mL), KHMDS (1 N solution in THF, 48.5 mL) was added at 0°C. The mixture was stirred at 0°C for 1 hour. 1-(Bromomethyl)-2-chlorobenzene (5.48 g) was added to the reaction solution. In a nitrogen atmosphere, the mixture was stirred at 0°C for 2 hours. The mixture was poured to a saturated aqueous solution of ammonium chloride, followed by extraction with ethyl acetate. The organic layer was washed with water and saturated brine, dried over magnesium sulfate and then concentrated. The residue was purified by column chromatography (ethyl acetate/hexane) to obtain the title compound (3.5 g). $^1$H NMR (300 MHz, CDCl3) $\delta$ 1.46 (9H, s), 1.58-1.73 (2H, m), 1.79-1.94 (2H, m), 2.90-3.07 (2H, m), 3.10 (2H, s), 3.96-4.33 (2H, m), 7.19-7.32 (2H, m), 7.36-7.43 (1H, m), 7.45-7.52 (1H, m).

B) 4-(2-Chlorobenzyl)piperidine-4-carbonitrile hydrochloride

In a nitrogen atmosphere, a mixture of tert-butyl 4-(2-chlorobenzyl)-4-cyanopiperidine-1-carboxylate (2.3 g), hydrogen chloride (4 N solution in ethyl acetate, 1.717 mL) and ethyl acetate (50 mL) was stirred overnight at 50°C. The deposit was collected by filtration to obtain the title compound (1.85 g).
MS, found: 234.9.

C) 4-(2-Chlorobenzyl)-1-(6-(3-(hydroxymethyl)-1,2,4-oxadiazol-5-yl)pyridazin-3-yl)piperidine-4-carbonitrile

A mixture of 4-(2-chlorobenzyl)piperidine-4-carbonitrile hydrochloride (193 mg), (5-(6-chloropyridazin-3-yl)-1,2,4-oxadiazol-3-yl)methyl benzoate (150 mg), DIPEA (0.247 mL) and DMA (3 mL) was stirred at 150°C for 1 hour under irradiation with microwave. The reaction solution was poured to a saturated aqueous solution of sodium bicarbonate at room temperature, followed by extraction with ethyl acetate. The organic layer was washed with water and saturated

brine, dried over magnesium sulfate and then concentrated. The residue was purified by column chromatography (ethyl acetate/hexane) to obtain (5-(6-(4-(2-chlorobenzyl)-4-cyanopiperidin-1-yl)pyridazin-3-yl)-1,2,4-oxadiazol-3-yl)methyl benzoate (69 mg).

In a nitrogen atmosphere, a mixture of the obtained (5-(6-(4-(2-chlorobenzyl)-4-cyanopiperidin-1-yl)pyridazin-3-yl)-1,2,4-oxadiazol-3-yl)methyl benzoate (60 mg), sodium hydroxide (1 N aqueous solution, 500 μL), methanol (5 mL) and THF (10 mL) was stirred at room temperature for 1 hour. The reaction solution was poured to a saturated aqueous solution of ammonium chloride, followed by extraction with ethyl acetate. The organic layer was washed with water and saturated brine, dried over magnesium sulfate and then concentrated. The residue was crystallized from ethyl acetate to obtain the title compound (15 mg).
$^1$H NMR (300 MHz, DMSO-d6) δ 1.77-1.92 (2H, m), 1.94-2.07 (2H, m), 3.03-3.24 (4H, m), 4.59-4.77 (4H, m), 5.71-5.83 (1H, m), 7.30-7.43 (2H, m), 7.45-7.57 (3H, m), 7.98-8.09 (1H, m).

Example 17

4-(3-Chlorobenzyl)-1-(6-(3-(hydroxymethyl)-1,2,4-oxadiazol-5-yl)pyridazin-3-yl)piperidine-4-carbonitrile

A) tert-Butyl 4-(3-chlorobenzyl)-4-cyanopiperidine-1-carboxylate

To a solution of DIPA (4.17 mL) in dehydrated THF (100 mL), n-butyllithium (1.6 N solution in hexane, 17.83 mL) was added at 0°C. After stirring at 0°C for 30 minutes, a solution of tert-butyl 4-cyanopiperidine-1-carboxylate (5 g) in dehydrated THF (50 mL) was added to the reaction solution. In a nitrogen atmosphere, the mixture was stirred at 0°C for 1 hour. 1-(Bromomethyl)-3-chlorobenzene (5.37 g) was added to the reaction solution at -78°C. In a nitrogen atmosphere, the reaction solution was stirred overnight while the temperature was gradually raised to room temperature. The mixture was poured to a saturated aqueous solution of ammonium chloride at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (7.0 g).
$^1$H NMR (300 MHz, CDCl3) δ 1.36-1.57 (11H, m), 1.74-1.93 (2H, m), 2.84 (2H, s), 2.91-3.08 (2H, m), 3.94-4.35 (2H, m), 7.13-7.37 (4H, m).

B) 4-(3-Chlorobenzyl)piperidine-4-carbonitrile hydrochloride

In a nitrogen atmosphere, a mixture of tert-butyl 4-(3-chlorobenzyl)-4-cyanopiperidine-1-carboxylate (7.0 g), hydrogen chloride (4 N solution in ethyl acetate, 20 mL) and ethyl acetate (100 mL) was stirred overnight at 50°C. The deposit was collected by filtration to obtain the title compound (5.55 g).

MS, found: 234.9.

C) (5-(6-(4-(3-Chlorobenzyl)-4-cyanopiperidin-1-yl)pyridazin-3-yl)-1,2,4-oxadiazol-3-yl)methyl benzoate

A mixture of 4-(3-chlorobenzyl)piperidine-4-carbonitrile hydrochloride (193 mg), (5-(6-chloropyridazin-3-yl)-1,2,4-oxadiazol-3-yl)methyl benzoate (150 mg), DIPEA (0.247 mL) and DMA (3 mL) was stirred at 150°C for 1 hour under irradiation with microwave. The reaction solution was poured to a saturated aqueous solution of sodium bicarbonate at room temperature, followed by extraction with ethyl acetate. The organic layer was washed with water and saturated brine, dried over magnesium sulfate and then concentrated. The residue was crystallized from ethyl acetate to obtain the title compound (190 mg).

D) 4-(3-Chlorobenzyl)-1-(6-(3-(hydroxymethyl)-1,2,4-oxadiazol-5-yl)pyridazin-3-yl)piperidine-4-carbonitrile

In a nitrogen atmosphere, a mixture of (5-(6-(4-(3-chlorobenzyl)-4-cyanopiperidin-1-yl)pyridazin-3-yl)-1,2,4-oxadiazol-3-yl)methyl benzoate (180 mg), sodium hydroxide (IN aqueous solution, 1.1 mL), methanol (5 mL) and THF (10 mL) was stirred at room temperature for 1 hour. The reaction solution was poured to a saturated aqueous solution of ammonium chloride, followed by extraction with ethyl acetate. The organic layer was washed with water and saturated brine, dried over magnesium sulfate and then concentrated. The residue was crystallized from ethyl acetate to obtain the title compound (100 mg).
$^1$H NMR (300 MHz, DMSO-d6) δ 1.67-1.83 (2H, m), 1.87-1.97 (2H, m), 3.00 (2H, s), 3.08-3.26 (2H, m), 4.55-4.76 (4H, m), 5.68-5.87 (1H, m), 7.20-7.58 (5H, m), 7.94-8.13 (1H, m).

Example 19

4-(4-Chlorobenzyl)-1-(6-(3-(hydroxymethyl)-1,2,4-oxadiazol-5-yl)pyridazin-3-yl)piperidine-4-carbonitrile

A) (5-(6-(4-(4-Chlorobenzyl)-4-cyanopiperidin-1-yl)pyridazin-3-yl)-1,2,4-oxadiazol-3-yl)methyl benzoate

A mixture of 4-(4-chlorobenzyl)piperidine-4-carbonitrile hydrochloride (141 mg), (5-(6-chloropyridazin-3-yl)-1,2,4-oxadiazol-3-yl)methyl benzoate (110 mg), DIPEA (500 μL) and IPA (4 mL) was stirred at 150°C for 1 hour under irradiation with microwave. After cooling to room temperature, the deposit was collected by filtration to obtain the title compound (150 mg).

B) 4-(4-Chlorobenzyl)-1-(6-(3-(hydroxymethyl)-1,2,4-oxadiazol-5-yl)pyridazin-3-yl)piperidine-4-carbonitrile

In a nitrogen atmosphere, a mixture of (5-(6-(4-(4-chlorobenzyl)-4-cyanopiperidin-1-yl)pyridazin-3-yl)-1,2,4-oxadiazol-3-yl)methyl benzoate (150 mg), sodium hydroxide (IN aqueous solution, 1 mL), methanol (5 mL) and THF (10 mL) was stirred at room temperature for 1 hour. The reaction solution was poured to a saturated aqueous solution of ammonium chloride, followed by extraction with ethyl acetate. The organic layer was washed with water and saturated brine, dried over magnesium sulfate and then concentrated. The residue was crystallized from ethyl acetate to obtain the title compound (15 mg). $^1$H NMR (300 MHz, DMSO-d6) δ 1.66-1.82 (2H, m), 1.86-1.97 (2H, m), 2.98 (2H, s), 3.08-3.22 (2H, m), 4.56-4.75 (4H, m), 5.78 (1H, s), 7.31-7.38 (2H, m), 7.41-7.53 (3H, m), 7.96-8.12 (1H, m).

Example 22

4-(4-Fluorophenyl)-1-(6-(3-(hydroxymethyl)-1,2,4-oxadiazol-5-yl)pyridazin-3-yl)piperidine-4-carbonitrile

A mixture of 4-(4-fluorophenyl)piperidine-4-carbonitrile hydrochloride (137 mg), (5-(6-chloropyridazin-3-yl)-1,2,4-oxadiazol-3-yl)methyl benzoate (150 mg), DIPEA (0.412 mL) and IPA (5 mL) was stirred at 150°C for 1 hour under irradiation with microwave. The deposit was crystallized using IPA to obtain (5-(6-(4-cyano-4-(4-fluorophenyl)piperidin-1-yl)pyridazin-3-yl)-1,2,4-oxadiazol-3-yl)methyl benzoate (182 mg).

In a nitrogen atmosphere, a mixture of the obtained (5-(6-(4-cyano-4-(4-fluorophenyl)piperidin-1-yl)pyridazin-3-yl)-1,2,4-oxadiazol-3-yl)methyl benzoate (182 mg), sodium hydroxide (2 N aqueous solution, 1 mL), methanol (5 mL) and THF (10 mL) was stirred at room temperature for 1 hour. The reaction solution was poured to water, followed by extraction with ethyl acetate. The organic layer was washed with water and saturated brine, dried over magnesium sulfate and then concentrated. The residue was crystallized from ethyl acetate/hexane to obtain the title compound (120 mg).

$^1$H NMR (300 MHz, DMSO-d6) δ 2.05-2.20 (2H, m), 2.27-2.39 (2H, m), 3.25-3.42 (2H, m), 4.61-4.69 (2H, m), 4.74-4.91 (2H, m), 5.73-5.82 (1H, m), 7.22-7.37 (2H, m), 7.51-7.57 (1H, m), 7.59-7.72 (2H, m), 8.00-8.13 (1H, m).

Example 23

(5-(6-(3-((2,5-Dichlorophenoxy)methyl)-3-methoxypyrrolidin-1-yl)pyridazin-3-yl)-1,2,4-oxadiazo 1-3 -yl)methanol

A) tert-Butyl 3-hydroxy-3-(((triisopropylsilyl)oxy)methyl)pyrrolidine-1-carboxylate

In a nitrogen atmosphere, a mixture of tert-butyl 3-methylenepyrrolidine-1-carboxylate (4.0 g), NMO (5.11 g), osmium tetroxide (7% in a fixation catalyst, 2.378 g), acetonitrile (25 mL), acetone (25 mL) and water (25 mL) was stirred overnight at room temperature. The solid was filtered off, and then, the filtrate was concentrated under reduced pressure. The residue was purified by column chromatography (ethyl acetate/hexane) to obtain tert-butyl 3-hydroxy-3-(hydroxymethyl)pyrrolidine-1-carboxylate (4.89 g). In a nitrogen atmosphere, a mixture of tert-butyl 3-hydroxy-3-(hydroxymethyl)pyrrolidine-1-carboxylate (4.74 g), triisopropylsilyl chloride (4.67 mL), imidazole (4.46 g) and dehydrated DMF (50 mL) was stirred overnight at 70°C. The reaction solution was poured to a saturated aqueous solution of sodium bicarbonate, followed by extraction with ethyl acetate. The organic layer was washed with water and saturated brine, dried over magnesium sulfate and then concentrated. The residue was purified by column chromatography (ethyl acetate/hexane) to obtain the title compound (6.60 g).

$^1$HNMR (300 MHz, CDCl3) δ 1.03-1.11 (21H, m), 1.46 (9H, s), 1.79-1.98 (2H, m), 2.65-2.82 (1H, m), 3.22-3.59 (4H, m), 3.65-3.75 (2H, m).

B) tert-Butyl 3-methoxy-3-(((triisopropylsilyl)oxy)methyl)pyrrolidine-1-carboxylate

To a solution of tert-butyl 3-hydroxy-3-(((triisopropylsilyl)oxy)methyl)pyrrolidine-1-carboxylate (2.25 g) and iodomethane (0.750 mL) in dehydrated DMF (50 mL), 60% (w/w) sodium hydride in oil (0.361 g) was added at 0°C. In a nitrogen atmosphere, the mixture was stirred overnight at room temperature. The reaction solution was poured to water, followed by extraction with ethyl acetate. The organic layer was washed with water and saturated brine, dried over magnesium sulfate and then concentrated. The residue was purified by column chromatography (ethyl acetate/hexane) to obtain the title compound (2.3 g).
$^1$H NMR (300 MHz, CDCl3) δ 1.00-1.16 (21H, m), 1.46 (9H, s), 1.87-2.11 (2H, m), 3.28-3.60 (7H, m), 3.68-3.85 (2H, m).

C) tert-Butyl 3-(hydroxymethyl)-3-methoxypyrrolidine-1-carboxylate

A mixture of tert-butyl 3-methoxy-3-(((triisopropylsilyl)oxy)methyl)pyrrolidine-1-carboxylate (2.3 g), TBAF (1 N solution in THF, 11.87 mL) and dehydrated THF (30 mL) was stirred at 50°C for 2 hours. The reaction mixture was concentrated under reduced pressure. The residue was purified by column chromatography (ethyl acetate/hexane) to obtain the title compound (1.289 g).
$^1$H NMR (300 MHz, CDCl3) δ 1.46 (9H, s), 1.69-2.20 (3H, m), 3.12-3.88 (9H, m).

D) tert-Butyl 3-((2,5-dichlorophenoxy)methyl)-3-methoxypyrrolidine-1-carboxylate

To a solution of tert-butyl 3-(hydroxymethyl)-3-methoxypyrrolidine-1-carboxylate (1.29 g), 2,5-dichlorophenol (1.091 g) and triphenylphosphine (2.194 g) in toluene (20 mL), bis(2-methoxyethyl) azodicarboxylate (1.959 g) was added at room temperature. In a nitrogen atmosphere, the mixture was stirred at room temperature for 1 hour. The reaction solution was poured to water, followed by extraction with ethyl acetate. The organic layer was washed with water and saturated brine, dried over magnesium sulfate and then concentrated. The residue was purified by column chromatography (ethyl acetate/hexane) to obtain the title compound (760 mg).
$^1$H NMR (300 MHz, CDCl3) δ 1.47 (9H, s), 1.97-2.26 (2H, m), 3.40 (3H, s), 3.42-3.76 (4H, m), 3.99-4.19 (2H, m), 6.88-6.96 (2H, m), 7.26-7.33 (1H, m).

E) 3-((2,5-Dichlorophenoxy)methyl)-3-methoxypyrrolidine

In a nitrogen atmosphere, a mixture of tert-butyl 3-((2,5-dichlorophenoxy)methyl)-3-methoxypyrrolidine-1-carboxylate (760 mg), hydrogen chloride (4 N solution in ethyl acetate, 2 mL) and ethyl acetate (20 mL) was stirred overnight at 50°C. The reaction solution was poured to sodium hydroxide (1 N aqueous solution), followed by extraction with ethyl acetate. The organic layer was washed with water and saturated brine, dried over magnesium sulfate and then concentrated. The residue was purified by column chromatography (NH, ethyl acetate/hexane) to obtain the title compound (320 mg). MS: $[M+H]^+$ 277.8.

F) (5-(6-(3-((2,5-Dichlorophenoxy)methyl)-3-methoxypyrrolidin-1-yl)pyridazin-3-yl)-1,2,4-oxadiazol-3-yl)methanol

A mixture of 3-((2,5-dichlorophenoxy)methyl)-3-methoxypyrrolidine (131 mg), (5-(6-chloropyridazin-3-yl)-1,2,4-oxadiazol-3-yl)methyl benzoate (150 mg), DIPEA (0.412 mL) and IPA (5 mL) was stirred at 150°C for 1 hour under irradiation with microwave. The deposit was crystallized from ethyl acetate-hexane to obtain (5-(6-(3-((2,5-dichlorophenoxy)methyl)-3-methoxypyrrolidin-1-yl)pyridazin-3-yl)-1,2,4-oxadiazol-3-yl)methyl benzoate (175 mg).

In a nitrogen atmosphere, a mixture of the obtained (5-(6-(3-((2,5-dichlorophenoxy)methyl)-3-methoxypyrrolidin-1-yl)pyridazin-3-yl)-1,2,4-oxadiazol-3-yl)methyl benzoate (175 mg), sodium hydroxide (2 N aqueous solution, 1 mL), THF (5 mL) and methanol (5 mL) was stirred at room temperature for 1 hour. The reaction solution was poured to water, followed by extraction with ethyl acetate. The organic layer was washed with water and saturated brine, dried over magnesium sulfate and then concentrated. The residue was crystallized from ethyl acetate-hexane to obtain the title compound (85 mg). $^1$H NMR (300 MHz, DMSO-d6) δ 2.11-2.44 (2H, m), 3.32 (3H, s), 3.53-4.14 (4H, m), 4.30-4.50 (2H, m), 4.56-4.73 (2H, m), 5.72-5.83 (1H, m), 6.98-7.14 (2H, m), 7.35-7.40 (1H, m), 7.44-7.53 (1H, m), 7.95-8.09 (1H, m).

Example 24

(5-(6-(4-(2-Chlorobenzyl)-4-(difluoromethyl)piperidin-1-yl)pyridazin-3-yl)-1,2,4-oxadiazol-3-yl)methanol

A) tert-Butyl 4-(2-chlorobenzyl)-4-(difluoromethyl)piperidine-1-carboxylate

In a nitrogen atmosphere, to a solution of tert-butyl 4-(2-chlorobenzyl)-4-cyanopiperidine-1-carboxylate (8.15 g) in dehydrated THF (200 mL), DIBAL (1.5 N solution in toluene, 40.6 mL) was added at -78°C. The mixture was stirred at -78°C for 1 hour and then stirred at 0°C for 1 hour. DIBAL (1.5 M solution in toluene, 20 mL) was further added thereto at 0°C. In a nitrogen atmosphere, the mixture was stirred overnight at 0°C. The reaction solution was poured to a saturated aqueous solution of ammonium chloride, followed by extraction with ethyl acetate. The organic layer was washed with water and saturated brine, dried over magnesium sulfate and then concentrated. The residue was purified by column chromatography (ethyl acetate/hexane) to obtain tert-butyl 4-(2-chlorobenzyl)-4-formylpiperidine-1-carboxylate (1.20 g). To a solution of tert-butyl 4-(2-chlorobenzyl)-4-formylpiperidine-1-carboxylate (340 mg) in toluene (10 mL), bis(2-methoxyethyl)aminosulfur trifluoride (0.557 mL) was added at 0°C. In a nitrogen atmosphere, the mixture was stirred at 80°C for 2 hours. The reaction solution was poured to a saturated aqueous solution of sodium bicarbonate at 0°C, followed by extraction with ethyl acetate. The organic layer was washed with water and saturated brine, dried over magnesium sulfate and then concentrated. The residue was purified by column chromatography (ethyl acetate/hexane) to obtain the title compound (190 mg).
$^1$H NMR (300 MHz, CDCl3) $\delta$ 1.27-1.35 (2H, m), 1.41-1.46 (9H, m), 1.67-1.88 (2H, m), 3.01 (2H, s), 3.45-3.58 (4H, m), 5.49-6.02 (1H, m), 7.16-7.24 (3H, m), 7.34-7.43 (1H, m).

B) 4-(2-Chlorobenzyl)-4-(difluoromethyl)piperidine hydrochloride

In a nitrogen atmosphere, a mixture of tert-butyl 4-(2-chlorobenzyl)-4-(difluoromethyl)piperidine-1-carboxylate (190 mg), hydrogen chloride (4 N solution in ethyl acetate, 5 mL) and ethyl acetate (5 mL) was stirred overnight at 50°C. The reaction mixture was concentrated under reduced pressure to obtain the title compound (156 mg). MS, found: 259.9.

C) (5-(6-(4-(2-Chlorobenzyl)-4-(difluoromethyl)piperidin-1-yl)pyridazin-3-yl)-1,2,4-oxadiazol-3-yl)methyl benzoate

A mixture of 4-(2-chlorobenzyl)-4-(difluoromethyl)piperidine hydrochloride (139 mg), (5-(6-chloropyridazin-3-yl)-1,2,4-oxadiazol-3-yl)methyl benzoate (130 mg), DIPEA (0.5 mL) and IPA (4 mL) was stirred at 150°C for 1 hour under irradiation with microwave. After cooling to room temperature, the reaction mixture was concentrated under reduced pressure. The residue was purified by column chromatography (ethyl acetate/hexane) to obtain the title compound (65 mg).
MS: $[M+H]^+$ 540.1.

D) (5-(6-(4-(2-Chlorobenzyl)-4-(difluoromethyl)piperidin-1-yl)pyridazin-3-yl)-1,2,4-oxadiazol-3-yl)methanol

In a nitrogen atmosphere, a mixture of (5-(6-(4-(2-chlorobenzyl)-4-(difluoromethyl)piperidin-1-yl)pyridazin-3-yl)-1,2,4-oxadiazol-3-yl)methyl benzoate (250 mg), sodium hydroxide (2 N aqueous solution, 2 mL),methanol (5 mL) and THF (5 mL) was stirred at room temperature for 1 hour. The reaction solution was neutralized with hydrogen chloride (1 N aqueous solution, 3 mL). The reaction solution was poured to a saturated aqueous solution of sodium bicarbonate at room temperature, followed by extraction with ethyl acetate. The organic layer was washed with water and saturated brine, dried over magnesium sulfate and then concentrated. The residue was crystallized from ethyl acetate-hexane to obtain the title compound (100 mg).
$^1$H NMR (300 MHz, DMSO-d6) $\delta$ 1.56-1.71 (2H, m), 1.76-1.92 (2H, m), 3.07 (2H, s), 3.72-3.88 (2H, m), 3.90-4.04 (2H, m), 4.60-4.68 (2H, m), 5.76 (1H, s), 6.03-6.50 (1H, m), 7.20-7.49 (5H, m), 7.93-8.02 (1H, m).

Example 25

4-(2-Chlorophenyl)-1-(6-(3-(hydroxymethyl)-1,2,4-oxadiazol-5-yl)pyridazin-3-yl)piperidine-4-carbonitrile

A) (5-(6-(4-(2-Chlorophenyl)-4-cyanopiperidin-1-yl)pyridazin-3-yl)-1,2,4-oxadiazol-3-yl)methyl benzoate

A mixture of 4-(2-chlorophenyl)piperidine-4-carbonitrile hydrochloride (97 mg), (5-(6-chloropyridazin-3-yl)-1,2,4-oxadiazol-3-yl)methyl benzoate (100 mg), DIPEA (0.275 mL) and IPA (5 mL) was stirred at 150°C for 1 hour under irradiation with microwave. The deposit was crystallized from IPA to obtain the title compound (120 mg).

B) 4-(2-Chlorophenyl)-1-(6-(3-(hydroxymethyl)-1,2,4-oxadiazol-5-yl)pyridazin-3-yl)piperidine-4-carbonitrile

In a nitrogen atmosphere, a mixture of (5-(6-(4-(2-chlorophenyl)-4-cyanopiperidin-1-yl)pyridazin-3-yl)-1,2,4-oxadiazol-3-yl)methyl benzoate (182 mg), sodium hydroxide (2 N aqueous solution, 1 mL), methanol (5 mL) and THF (5 mL) was stirred at room temperature for 1 hour. The reaction solution was poured to water, followed by extraction with ethyl acetate. The organic layer was washed with water and saturated brine, dried over magnesium sulfate and then concentrated. The residue was crystallized from ethyl acetate-hexane to obtain the title compound (120 mg).
$^1$H NMR (300 MHz, DMSO-d6) δ 2.04-2.25 (2H, m), 2.57-2.75 (2H, m), 3.34-3.50 (2H, m), 4.56-4.69 (2H, m), 4.75-4.90 (2H, m), 5.71-5.88 (1H, m), 7.41-7.67 (5H, m), 8.01-8.14 (1H, m).

Example 26

4-(3-Chlorophenyl)-1-(6-(3-(hydroxymethyl)-1,2,4-oxadiazol-5-yl)pyridazin-3-yl)piperidine-4-carbonitrile

A) (5-(6-(4-(3-Chlorophenyl)-4-cyanopiperidin-1-yl)pyridazin-3-yl)-1,2,4-oxadiazol-3-yl)methyl benzoate

A mixture of 4-(3-chlorophenyl)piperidine-4-carbonitrile hydrochloride (97 mg), (5-(6-chloropyridazin-3-yl)-1,2,4-oxadiazol-3-yl)methyl benzoate (100 mg), DIPEA (0.275 mL) and IPA (5 mL) was stirred at 150°C for 1 hour under irradiation with microwave. The deposit was crystallized from IPA to obtain the title compound (140 mg).
$^1$H NMR (300 MHz, DMSO-d6) δ 2.07-2.24 (2H, m), 2.28-2.41 (2H, m), 3.26-3.44 (2H, m), 4.66-4.95 (2H, m), 5.63 (2H, s), 7.37-7.81 (8H, m), 7.98-8.16 (3H, m).

B) 4-(3-Chlorophenyl)-1-(6-(3-(hydroxymethyl)-1,2,4-oxadiazol-5-yl)pyridazin-3-yl)piperidine-4-carbonitrile

In a nitrogen atmosphere, a mixture of (5-(6-(4-(3-chlorophenyl)-4-cyanopiperidin-1-yl)pyridazin-3-yl)-1,2,4-oxadiazol-3-yl)methyl benzoate (182 mg), sodium hydroxide (2 N aqueous solution, 1 mL), methanol (5 mL) and THF (5 mL) was stirred at room temperature for 1 hour. The reaction solution was poured to water, followed by extraction with ethyl acetate. The organic layer was washed with water and saturated brine, dried over magnesium sulfate and then concentrated. The residue was crystallized from ethyl acetate-hexane to obtain the title compound (120 mg).
$^1$H NMR (300 MHz, DMSO-d6) δ 2.09-2.25 (2H, m), 2.28-2.39 (2H, m), 3.27-3.41 (2 H, m), 4.57-4.70 (2H, m), 4.76-4.89 (2H, m), 5.66-5.89 (1H, m), 7.44-7.62 (4H, m), 7.64-7.70 (1H, m), 8.00-8.14 (1H, m).

Example 27

4-(4-Chlorophenyl)-1-(6-(3-(hydroxymethyl)-1,2,4-oxadiazol-5-yl)pyridazin-3-yl)piperidine-4-carbonitrile

A) (5-(6-(4-(4-Chlorophenyl)-4-cyanopiperidin-1-yl)pyridazin-3-yl)-1,2,4-oxadiazol-3-yl)methyl benzoate

A mixture of 4-(4-chlorophenyl)piperidine-4-carbonitrile hydrochloride (97 mg), (5-(6-chloropyridazin-3-yl)-1,2,4-oxadiazol-3-yl)methyl benzoate (100 mg), DIPEA (0.275 mL) and IPA (5 mL) was stirred at 150°C for 1 hour under irradiation with microwave. The deposit was crystallized from IPA to obtain the title compound (145 mg).

B) 4-(4-Chlorophenyl)-1-(6-(3-(hydroxymethyl)-1,2,4-oxadiazol-5-yl)pyridazin-3-yl)piperidine-4-carbonitrile

In a nitrogen atmosphere, a mixture of (5-(6-(4-(4-chlorophenyl)-4-cyanopiperidin-1-yl)pyridazin-3-yl)-1,2,4-oxadiazol-3-yl)methyl benzoate (182 mg), sodium hydroxide (2 N aqueous solution, 1 mL), methanol (5 mL) and THF (5 mL) was stirred at room temperature for 1 hour. The reaction solution was poured to water, followed by extraction with ethyl acetate. The organic layer was washed with water and saturated brine, dried over magnesium sulfate and then concentrated. The residue was crystallized from ethyl acetate-hexane to obtain the title compound (120 mg).
$^1$H NMR (300 MHz, DMSO-d6) δ 2.05-2.22 (2H, m), 2.25-2.38 (2H, m), 3.22-3.46 (2H, m), 4.59-4.69 (2H, m), 4.74-4.88 (2H, m), 5.71-5.85 (1H, m), 7.48-7.57 (3H, m), 7.58-7.66 (2H, m), 8.00-8.12 (1H, m).

Example 33

(5-(6-(4-(2-Chlorobenzyl)-4-cyanopiperidin-1-yl)pyridazin-3-yl)-1,3,4-oxadiazol-2-yl)methyl acetate

A) (5-(6-Oxo-1,6-dihydropyridazin-3-yl)-1,3,4-oxadiazol-2-yl)methyl acetate

To a solution of 6-oxo-1,6-dihydropyridazine-3-carbohydrazide (1.0 g) in Py (20 mL), 2-acetoxyacetyl chloride (0.699 mL) was added at 0°C. After stirring at room temperature for 30 minutes, pTsCl (1.299 g) was added to the reaction mixture. In a nitrogen atmosphere, the mixture was stirred overnight at 80°C. The reaction mixture was concentrated under reduced pressure. The residue was purified by column chromatography (ethyl acetate/hexane) to obtain the title compound (230 mg).
MS: $[M+H]^+$ 236.9.

B) (5-(6-Chloropyridazin-3-yl)-1,3,4-oxadiazol-2-yl)methyl acetate

A solution of (5-(6-oxo-1,6-dihydropyridazin-3-yl)-1,3,4-oxadiazol-2-yl)methyl acetate (200 mg) in phosphoryl chloride (3.53 mL) was refluxed for 1 hour. The reaction mixture was concentrated under reduced pressure. The residue was neutralized with a saturated aqueous solution of sodium bicarbonate, followed by extraction with ethyl acetate. The organic layer was washed with water and saturated brine, dried over magnesium sulfate and then concentrated. The residue was purified by column chromatography (ethyl acetate/hexane) to obtain the title compound (180 mg).
MS: $[M+H]^+$ 254.8.

C) (5-(6-(4-(2-Chlorobenzyl)-4-cyanopiperidin-1-yl)pyridazin-3-yl)-1,3,4-oxadiazol-2-yl)methyl acetate

A mixture of 4-(2-chlorobenzyl)piperidine-4-carbonitrile hydrochloride (120 mg), (5-(6-chloropyridazin-3-yl)-1,3,4-oxadiazol-2-yl)methyl acetate (100 mg), DIPEA (300 $\mu$L) and IPA (4 mL) was stirred at 150°C for 1 hour under irradiation with microwave. After cooling to room temperature, the deposit was collected by filtration to obtain the title compound (135 mg). $^1$H NMR (300 MHz, DMSO-d6) $\delta$ 1.74-1.91 (2H, m), 1.97-2.05 (2H, m), 2.15 (3H, s), 3.06-3.22 (4H, m), 4.58-4.73 (2H, m), 5.42 (2H, s), 7.30-7.43 (2H, m), 7.47-7.55 (3H, m), 7.98-8.08 (1H, m).

Example 34

(5-(6-(4-(2-Chlorobenzyl)-4-(difluoromethyl)piperidin-1-yl)pyridazin-3-yl)-1,3,4-oxadiazol-2-yl)methyl acetate

A mixture of 4-(2-chlorobenzyl)-4-(difluoromethyl)piperidine (120 mg), (5-(6-chloropyridazin-3-yl)-1,3,4-oxadiazol-2-yl)methyl acetate (100 mg), DIPEA (300 $\mu$L) and IPA (4 mL) was stirred at 150°C for 1 hour under irradiation with microwave. After cooling to room temperature, the deposit was collected by filtration to obtain the title compound (71 mg). $^1$H NMR (300 MHz, DMSO-d6) $\delta$ 1.50-1.71 (2H, m), 1.76-1.96 (2H, m), 2.14 (3H, s), 3.06 (2H, s), 3.66-4.02 (4H, m), 5.41 (2H, s), 6.04-6.53 (1H, m), 7.25-7.33 (2H, m), 7.35-7.49 (3H, m), 7.94-8.01 (1H, m).

Example 35

4-(2-Chlorobenzyl)-1-(6-(5-(hydroxymethyl)-1,3,4-oxadiazol-2-yl)pyridazin-3-yl)piperidine-4-carbonitrile

In a nitrogen atmosphere, a mixture of (5-(6-(4-(2-chlorobenzyl)-4-cyanopiperidin-1-yl)pyridazin-3-yl)-1,3,4-oxadiazol-2-yl)methyl acetate (130 mg), sodium hydroxide (1 N aqueous solution, 1 mL), THF (4 mL) and methanol (4 mL) was stirred at room temperature for 1 hour. The reaction solution was poured to water, followed by extraction with ethyl acetate. The organic layer was washed with water and saturated brine, dried over magnesium sulfate and then concentrated. The residue was suspended in IPE, and the insoluble matter was collected by filtration to obtain the title compound (90 mg).
$^1$H NMR (300 MHz, DMSO-d6) $\delta$ 1.74-1.92 (2H, m), 1.95-2.08 (2H, m), 3.04-3.20 (4H, m), 4.57-4.68 (2H, m), 4.70-4.79 (2H, m), 5.96-6.05 (1H, m), 7.30-7.43 (2H, m), 7.46-7.55 (3H, m), 8.00-8.07 (1H, m).

Example 36

(5-(6-(4-(2-Chlorobenzyl)-4-(difluoromethyl)piperidin-1-yl)pyridazin-3-yl)-1,3,4-oxadiazol-2-yl)methanol

In a nitrogen atmosphere, a mixture of (5-(6-(4-(2-chlorobenzyl)-4-(difluoromethyl)piperidin-1-yl)pyridazin-3-yl)-1,3,4-oxadiazol-2-yl)methyl acetate (65 mg), sodium hydroxide (1 N aqueous solution, 500 μL), THF (4 mL) and methanol (4 mL) was stirred at room temperature for 1 hour. The reaction solution was poured to hydrogen chloride (IN aqueous solution), followed by extraction with ethyl acetate. The organic layer was washed with water and saturated brine, dried over magnesium sulfate and then concentrated. The residue was suspended in IPE, and the insoluble matter was collected by filtration to obtain the title compound (40 mg).
$^1$H NMR (300 MHz, DMSO-d6) δ 1.52-1.70 (2H, m), 1.75-1.91 (2H, m), 3.06 (2H, s), 3.72-3.84 (2H, m), 3.87-4.02 (2H, m), 4.68-4.78 (2H, m), 5.95-6.03 (1H, m), 6.07-6.49 (1H, m), 7.24-7.33 (2H, m), 7.35-7.50 (3H, m), 7.93-8.01 (1H, m).

Example 37

1-(6-(3-(Hydroxymethyl)-1,2,4-oxadiazol-5-yl)pyridazin-3-yl)-4-(3-(trifluoromethyl)phenyl)piperidine-4-carbonitrile

A) (5-(6-(4-Cyano-4-(3-(trifluoromethyl)phenyl)piperidin-1-yl)pyridazin-3-yl)-1,2,4-oxadiazol-3-yl)methyl benzoate

A mixture of 4-(3-(trifluoromethyl)phenyl)piperidine-4-carbonitrile hydrochloride (120 mg), (5-(6-chloropyridazin-3-yl)-1,2,4-oxadiazol-3-yl)methyl benzoate (100 mg), DIPEA (300 μL) and IPA (4 mL) was stirred at 150°C for 1 hour under irradiation with microwave. After cooling to room temperature, the deposit was collected by filtration to obtain the title compound (130 mg).
MS: $[M+H]^+$ 535.1.

B) 1-(6-(3-(Hydroxymethyl)-1,2,4-oxadiazol-5-yl)pyridazin-3-yl)-4-(3-(trifluoromethyl)phenyl)piperidine-4-carbonitrile

In a nitrogen atmosphere, a mixture of (5-(6-(4-cyano-4-(3-(trifluoromethyl)phenyl)piperidin-1-yl)pyridazin-3-yl)-1,2,4-oxadiazol-3-yl)methyl benzoate (130 mg), sodium hydroxide (1 N aqueous solution, 1 mL), methanol (5 mL) and THF (5 mL) was stirred at room temperature for 1 hour. The mixture was neutralized by the addition of hydrogen chloride (IN aqueous solution) at 0°C, followed by extraction with ethyl acetate. The organic layer was washed with water and saturated brine, dried over magnesium sulfate and then concentrated. The residue was crystallized from IPE to obtain the title compound (80 mg). $^1$H NMR (300 MHz, DMSO-d6) δ 2.15-2.44 (4H, m), 3.28-3.43 (2H, m), 4.65 (2H, d, J = 6.1 Hz), 4.76 - 4.96 (2 H, m), 5.78(1 H, t, J = 6.2 Hz), 7.55 (1H, d, J = 9.7 Hz), 7.66 - 7.83 (2H, m), 7.88 - 8.00 (2H, m), 8.07 (1H, d, J = 9.7 Hz).

Example 38

1-(6-(3-(Hydroxymethyl)-1,2,4-oxadiazol-5-yl)pyridazin-3-yl)-4-(4-(trifluoromethyl)phenyl)piperidine-4-carbonitrile

A) (5-(6-(4-Cyano-4-(4-(trifluoromethyl)phenyl)piperidin-1-yl)pyridazin-3-yl)-1,2,4-oxadiazol-3-yl)methyl benzoate

A mixture of 4-(4-(trifluoromethyl)phenyl)piperidine-4-carbonitrile hydrochloride (120 mg), (5-(6-chloropyridazin-3-yl)-1,2,4-oxadiazol-3-yl)methyl benzoate (100 mg), DIPEA (300 μL) and IPA (4 mL) was stirred at 150°C for 1 hour under irradiation with microwave. After cooling to room temperature, the deposit was collected by filtration to obtain the title compound (150 mg).
MS: $[M+H]^+$ 535.1.

B) 1-(6-(3-(Hydroxymethyl)-1,2,4-oxadiazol-5-yl)pyridazin-3-yl)-4-(4-(trifluoromethyl)phenyl)piperidine-4-carbonitrile

In a nitrogen atmosphere, a mixture of (5-(6-(4-cyano-4-(4-(trifluoromethyl)phenyl)piperidin-1-yl)pyridazin-3-yl)-1,2,4-oxadiazol-3-yl)methyl benzoate (150 mg), sodium hydroxide (1 N aqueous solution, 1 mL), methanol (5 mL) and THF (5 mL) was stirred at room temperature for 1 hour. The mixture was neutralized by the addition of hydrogen chloride (IN aqueous solution) at 0°C, followed by extraction with ethyl acetate. The organic layer was washed with water and saturated brine, dried over magnesium sulfate and then concentrated. The residue was crystallized from IPE to obtain the title compound (110 mg).
$^1$H NMR (300 MHz, DMSO-d6) δ 2.10-2.43 (4H, m), 3.26-3.47 (2H, m), 4.65 (2H, d, J = 6.2 Hz), 4.77-4.94 (2H, m), 5.79 (1H, t, J = 6.2 Hz), 7.56(1H, d, J = 9.7 Hz), 7.85 (4 H, s), 8.08 (1 H, d, J = 9.7 Hz).

Example 39

3-((2,5-Dichlorophenoxy)methyl)-1-(6-(3-(hydroxymethyl)-1,2,4-oxadiazol-5-yl)pyridazin-3-yl)pyrrolidine-3-carbonitrile

A) 1-Benzyl-3-(hydroxymethyl)pyrrolidine-3-carbonitrile

To a solution of ethyl 1-benzyl-3-cyanopyrrolidine-3-carboxylate (1.9 g) in methanol (30 mL), sodium borohydride (2.78 g) was added in small portions over 30 minutes. After the completion of the addition, in a nitrogen atmosphere, the mixture was stirred at room temperature for 3 hours. The reaction was terminated by the addition of an aqueous acetic acid solution at 0°C, and then, the mixture was subjected to extraction with ethyl acetate. The organic layer was washed with water and saturated brine, dried over magnesium sulfate and then concentrated. The residue was purified by column chromatography (ethyl acetate/hexane) to obtain the title compound (1.39 g).
MS: $[M+H]^+$ 217.0.

B) 1-Benzyl-3-((2,5-dichlorophenoxy)methyl)pyrrolidine-3-carbonitrile

To a solution of 1-benzyl-3-(hydroxymethyl)pyrrolidine-3-carbonitrile (1.05 g), 2,5-dichlorophenol (1.583 g) and triphenylphosphine (3.82 g) in toluene (50 mL), DIAD (1.9 M solution in toluene, 7.67 mL) was added at room temperature. In a nitrogen atmosphere, the mixture was stirred at 50°C for 2 hours. The reaction mixture was concentrated under reduced pressure. The residue was purified by column chromatography (ethyl acetate/hexane) to obtain the title compound (1.62 g).
MS: $[M+H]^+$ 362.9.

C) tert-Butyl 3-cyano-3-((2,5-dichlorophenoxy)methyl)pyrrolidine-1-carboxylate

To a solution of 1-benzyl-3-((2,5-dichlorophenoxy)methyl)pyrrolidine-3-carbonitrile (1.62 g) in acetonitrile (25 mL), 1-chloroethyl chloroformate (0.732 mL) was added at room temperature. In a nitrogen atmosphere, the mixture was stirred overnight at room temperature. Methanol (25 mL) was added to the reaction mixture. In a nitrogen atmosphere, the mixture was stirred at 70°C for 2 hours. The reaction mixture was concentrated under reduced pressure to obtain the reaction intermediate of interest. In a nitrogen atmosphere, a mixture of the obtained reaction intermediate, di-tert-butyl dicarbonate (1.957 g), DIPEA (3.91 mL) and dehydrated DMF (25.00 mL) was stirred at room temperature for 2 hours. The mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (1.05 g). $^1H$ NMR (300 MHz, CDCl3) $\delta$ 1.48 (9H, s), 2.22-2.57 (2H, m), 3.50-3.72 (3H, m), 3.79-3.99 (1H, m), 4.03-4.18 (2H, m), 6.90-6.94 (1H, m), 6.95-7.02 (1H, m), 7.29-7.35 (1H, m).

D) 3-((2,5-Dichlorophenoxy)methyl)pyrrolidine-3-carbonitrile hydrochloride

In a nitrogen atmosphere, a mixture of tert-butyl 3-cyano-3-((2,5-dichlorophenoxy)methyl)pyrrolidine-1-carboxylate (1.05 g), hydrogen chloride (4 N solution in ethyl acetate, 4 mL) and ethyl acetate (20 mL) was stirred at 50°C over weekend. The deposit was collected by filtration to obtain the title compound (740 mg).
MS, found: 272.8.

E) (5-(6-(3-Cyano-3-((2,5-dichlorophenoxy)methyl)pyrrolidin-1-yl)pyridazin-3-yl)-1,2,4-oxadiazol-3-yl)methyl benzoate

A mixture of 3-((2,5-dichlorophenoxy)methyl)pyrrolidine-3-carbonitrile hydrochloride (82 mg), (5-(6-chloropyridazin-3-yl)-1,2,4-oxadiazol-3-yl)methyl benzoate (70 mg), DIPEA (0.192 mL) and IPA (4 mL) was stirred at 150°C for 1 hour under irradiation with microwave. After cooling to room temperature, the deposit was collected by filtration to obtain the title compound (50 mg).
MS: $[M+H]^+$ 551.1.

F) 3-((2,5-Dichlorophenoxy)methyl)-1-(6-(3-(hydroxymethyl)-1,2,4-oxadiazol-5-yl)pyridazin-3-yl)pyrrolidine-3-carbonitrile

In a nitrogen atmosphere, a mixture of (5-(6-(3-cyano-3-((2,5-dichlorophenoxy)methyl)pyrrolidin-1-yl)pyridazin-3-yl)-1,2,4-oxadiazol-3-yl)methyl benzoate (50 mg), sodium hydroxide (1 N aqueous solution, 0.091 mL), methanol (4 mL) and THF (4 mL) was stirred at room temperature for 1 hour. The mixture was neutralized by the addition of hydrogen

chloride (IN aqueous solution) at 0°C, followed by extraction with ethyl acetate. The organic layer was washed with water and saturated brine, dried over magnesium sulfate and then concentrated. The residue was crystallized from IPE to obtain the title compound.
$^1$H NMR (300 MHz, DMSO-d6) δ 2.45-2.53 (1H, m), 2.58-2.73 (1H, m), 3.75-3.91 (2H, m), 3.94-4.08 (1H, m), 4.16-4.27 (1H, m), 4.45-4.55 (2H, m), 4.60-4.70 (2H, m), 5.66-5.84 (1H, m), 7.11 (1H, dd, J = 8.5, 2.4 Hz), 7.17 (1H, d, J = 9.5 Hz), 7.35 (1H, d, J = 2.4 Hz), 7.52 (1H, d, J = 8.5 Hz), 8.09 (1H, d, J = 9.5 Hz).

Example 40

(5-(6-(3-((2,5-Dichlorophenoxy)methyl)-3-(trifluoromethyl)pyrrolidin-1-yl)pyridazin-3-yl)-1,2,4-oxadiazol-3-yl)methanol

A) 1-Benzyl-3-((2,5-dichlorophenoxy)methyl)-3-(trifluoromethyl)pyrrolidine

To a solution of (1-benzyl-3-(trifluoromethyl)pyrrolidin-3-yl)methanol (5.0 g), 2,5-dichlorophenol (3.77 g) and triphenylphosphine (5.56 g) in toluene (50 mL), DIAD (1.9 N solution in toluene, 11.17 mL) was added at room temperature. In a nitrogen atmosphere, the mixture was stirred at 50°C for 2 hours. The reaction mixture was concentrated under reduced pressure. The residue was purified by column chromatography (ethyl acetate/hexane) to obtain the title compound (3.0 g).
MS: $[M+H]^+$ 405.7.

B) tert-Butyl 3-((2,5-dichlorophenoxy)methyl)-3-(trifluoromethyl)pyrrolidine-1-carboxylate

To a solution of 1-benzyl-3-((2,5-dichlorophenoxy)methyl)-3-(trifluoromethyl)pyrrolidine (3.0 g) in acetonitrile (25 mL), 1-chloroethyl chloroformate (1.211 mL) was added at room temperature. In a nitrogen atmosphere, the mixture was stirred overnight at room temperature. Methanol (25 mL) was added to the reaction mixture. In a nitrogen atmosphere, the mixture was stirred at 70°C for 2 hours. The reaction mixture was concentrated under reduced pressure to obtain the reaction intermediate of interest. In a nitrogen atmosphere, a mixture of the obtained reaction intermediate, di-tert-butyl dicarbonate (3.24 g), DIPEA (6.46 mL) and dehydrated DMF (25.00 mL) was stirred at room temperature for 2 hours. The mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (3.0 g).
$^1$H NMR (300 MHz, DMSO-d6) δ 1.40 (9H, s), 2.06-2.35 (2H, m), 3.42-3.63 (4H, m), 4.22-4.39 (2H, m), 7.08 (1H, dd, J = 8.5, 2.4 Hz), 7.38 (1H, d, J = 2.4 Hz), 7.48 (1H, d, J = 8.5 Hz).

C) 3-((2,5-Dichlorophenoxy)methyl)-3-(trifluoromethyl)pyrrolidine hydrochloride

In a nitrogen atmosphere, a mixture of tert-butyl 3-((2,5-dichlorophenoxy)methyl)-3-(trifluoromethyl)pyrrolidine-1-carboxylate (3.0 g), hydrogen chloride (4 N solution in CPME, 20 mL) and CPME (25 mL) was stirred at 50°C over weekend. The deposit was collected by filtration to obtain the title compound (2.1 g).
MS, found: 315.9.

D) (5-(6-(3-((2,5-Dichlorophenoxy)methyl)-3-(trifluoromethyl)pyrrolidin-1-yl)pyridazin-3-yl)-1,2,4-oxadiazol-3-yl)methyl benzoate

A mixture of 3-((2,5-dichlorophenoxy)methyl)-3-(trifluoromethyl)pyrrolidine hydrochloride (106 mg), (5-(6-chloropyridazin-3-yl)-1,2,4-oxadiazol-3-yl)methyl benzoate (80 mg), DIPEA (0.220 mL) and IPA (4 mL) was stirred at 150°C for 1 hour under irradiation with microwave. After cooling to room temperature, the deposit was collected by filtration to obtain the title compound (130 mg).
MS: $[M+H]^+$ 595.9.

E) (5-(6-(3-((2,5-Dichlorophenoxy)methyl)-3-(trifluoromethyl)pyrrolidin-1-yl)pyridazin-3-yl)-1,2,4-oxadiazol-3-yl)methanol

In a nitrogen atmosphere, a mixture of (5-(6-(3-((2,5-dichlorophenoxy)methyl)-3-(trifluoromethyl)pyrrolidin-1-yl)pyridazin-3-yl)-1,2,4-oxadiazol-3-yl)methyl benzoate (130 mg), sodium hydroxide (IN aqueous solution, 0.219 mL), methanol (4 mL) and THF (4 mL) was stirred at room temperature for 1 hour. The mixture was neutralized by the addition of hydrogen chloride (IN aqueous solution) at 0°C, followed by extraction with ethyl acetate. The organic layer was

washed with water and saturated brine, dried over magnesium sulfate and then concentrated. The residue was crystallized from IPE to obtain the title compound (30 mg). $^1$H NMR (300 MHz, DMSO-d6) δ 2.35-2.58 (2H, m), 3.69-4.14 (4H, m), 4.44 (2H, s), 4.64 (2H, d, J = 6.1 Hz), 5.77 (1H, t, J = 6.1 Hz), 7.07 (1H, dd, J = 8.5, 2.3 Hz), 7.17 (1H, d, J = 9.5 Hz), 7.38 (1H, d, J = 2.4 Hz), 7.47 (1H, d, J = 8.5 Hz), 8.07 (1H, d, J = 9.5 Hz).

Example 42

4-(2-Chloro-5-fluorophenoxy)-1-(6-(3-(hydroxymethyl)-1,2,4-oxadiazol-5-yl)pyridazin-3-yl)piperidine-4-carbonitrile

A) 1-tert-Butyl 4-methyl 4-(2-chloro-5-fluorophenoxy)piperidine-1,4-dicarboxylate

To a solution of sodium hydroxide (8.87 g), 2-chloro-5-fluorophenol (6.5 g) and tert-butyl 4-oxopiperidine-1-carboxylate (26.5 g) in dehydrated THF (296 mL), chloroform (17.74 mL) was added at 0°C. In a nitrogen atmosphere, the mixture was stirred at room temperature over weekend. The reaction mixture was concentrated under reduced pressure. The residue was poured to water at room temperature, followed by extraction with diethyl ether. The aqueous layer was washed with diethyl ether and then poured to hydrogen chloride (IN aqueous solution) at 0°C, followed by extraction with ethyl acetate. The organic layer was washed with water and saturated brine, dried over magnesium sulfate and then concentrated. The residue 1-(tert-butoxycarbonyl)-4-(2-chloro-5-fluorophenoxy)piperidine-4-carboxylic acid was used in the next reaction without being further purified.

To a solution of thionyl chloride (6.47 mL) in methanol (300 mL), a solution of the 1-(tert-butoxycarbonyl)-4-(2-chloro-5-fluorophenoxy)piperidine-4-carboxylic acid (16.58 g) obtained in the preceding reaction in methanol (50 mL) was added at 0°C. In a nitrogen atmosphere, the mixture was stirred overnight at 70°C. The reaction mixture was concentrated under reduced pressure. The residue was poured to water at 0°C and washed with diethyl ether. The aqueous layer was poured to sodium hydroxide (IN aqueous solution) at room temperature, followed by extraction with ethyl acetate. The organic layer was washed with water and saturated brine, dried over magnesium sulfate and then concentrated to obtain the intermediate of interest. To a solution of the obtained intermediate and DIPEA (38.6 mL) in dehydrated DMF (300 mL), di-tert-butyl dicarbonate (19.36 g) was added at room temperature. In a nitrogen atmosphere, the mixture was stirred at 50°C for 2 hours. The mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (11.5 g).
$^1$H NMR (300 MHz, CDCl3) δ 1.46 (9H, s), 1.97-2.12 (2H, m), 2.14-2.27 (2H, m), 3.03-3.32 (2H, m), 3.83 (3H, s), 3.85-4.01 (2H, m), 6.34-6.45 (1H, m), 6.62-6.75 (1H, m), 7.30-7.38 (1H, m).

B) 1-(tert-Butoxycarbonyl)-4-(2-chloro-5-fluorophenoxy)piperidine-4-carboxylic acid

A solution of 1-tert-butyl 4-methyl 4-(2-chloro-5-fluorophenoxy)piperidine-1,4-dicarboxylate (1.05 g) and lithium hydroxide (4 N aqueous solution, 2.5 mL) in THF (10 mL) and methanol (5 mL) was heated to reflux for 2 hours. The reaction mixture was concentrated under reduced pressure. The residue was neutralized by the addition of hydrogen chloride (1 N aqueous solution) at 0°C, followed by extraction with ethyl acetate. The organic layer was washed with water and saturated brine, dried over magnesium sulfate and then concentrated to obtain the title compound (1.01 g).
$^1$H NMR (300 MHz, CDCl3) δ 1.46 (9H, s), 2.01-2.16 (2H, m), 2.19-2.30 (2H, m), 3.01-3.31 (2H, m), 3.86-4.04 (2H, m), 6.51-6.61 (1H, m), 6.64-6.74 (1H, m), 7.29-7.40 (1H, m), 8.50-9.52 (1H, m).

C) tert-Butyl 4-carbamoyl-4-(2-chloro-5-fluorophenoxy)piperidine-1-carboxylate

To a solution of 1-(tert-butoxycarbonyl)-4-(2-chloro-5-fluorophenoxy)piperidine-4-carboxylic acid (1.05 g) and HOBt (1.822 g) in THF (10 mL), EDC (2.387 mL) was added at room temperature. After stirring at room temperature for 1 hour, ammonia water (28% by weight, 3 mL) was added to the reaction mixture. In a nitrogen atmosphere, the mixture was stirred overnight at room temperature. The reaction mixture was concentrated under reduced pressure. The residue was poured to water at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (900 mg). $^1$H NMR (300 MHz, CDCl3) δ 1.45 (9H, s), 2.05-2.24 (4H, m), 2.99-3.19 (2H, m), 3.83-4.06 (2H, m), 5.50-5.75 (1H, m), 6.24-6.43 (1H, m), 6.59-6.82 (2H, m), 7.30-7.43 (1H, m).

D) tert-Butyl 4-(2-chloro-5-fluorophenoxy)-4-cyanopiperidine-1-carboxylate

To a solution of tert-butyl 4-carbamoyl-4-(2-chloro-5-fluorophenoxy)piperidine-1-carboxylate (900 mg) and triethylamine (1.342 mL) in THF (10 mL), trifluoroacetic anhydride (1.007 mL) was added at 0°C. In a nitrogen atmosphere, the mixture was stirred at 0°C for 2 hours. The mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (840 mg).
$^1$H NMR (300 MHz, CDCl3) δ 1.47 (9H, s), 2.06-2.29 (4H, m), 3.31-3.49 (2H, m), 3.74-3.96 (2H, m), 6.79-6.91 (1H, m), 7.26-7.32 (1H, m), 7.35-7.44 (1H, m).

E) 4-(2-Chloro-5-fluorophenoxy)piperidine-4-carbonitrile hydrochloride

To a solution of tert-butyl 4-(2-chloro-5-fluorophenoxy)-4-cyanopiperidine-1-carboxylate (830 mg) in ethyl acetate (10 mL), hydrogen chloride (4 N solution in ethyl acetate, 0.585 mL) was added at room temperature. In a nitrogen atmosphere, the mixture was stirred overnight at 50°C. The reaction mixture was concentrated to obtain the title compound (650 mg).
MS, found: 254.9.

F) 4-(2-Chloro-5-fluorophenoxy)-1-(6-(3-(hydroxymethyl)-1,2,4-oxadiazol-5-yl)pyridazin-3-yl)piperidine-4-carbonitrile

A mixture of (5-(6-chloropyridazin-3-yl)-1,2,4-oxadiazol-3-yl)methyl benzoate (100 mg), (5-(6-chloropyridazin-3-yl)-1,2,4-oxadiazol-3-yl)methyl benzoate (110 mg), DIPEA (0.5 mL) and IPA (4 mL) was stirred at 150°C for 1 hour under irradiation with microwave. After cooling to room temperature, the deposit was collected by filtration and washed with IPA to obtain (5-(6-(4-(2-chloro-5-fluorophenoxy)-4-cyanopiperidin-1-yl)pyridazin-3-yl)-1,2,4-oxadiazol-3-yl)methyl benzoate (150 mg).
Sodium hydroxide (1 N aqueous solution, 0.280 mL) was added to a solution of (5-(6-(4-(2-chloro-5-fluorophenoxy)-4-cyanopiperidin-1-yl)pyridazin-3-yl)-1,2,4-oxadiazol-3-yl)methyl benzoate (150 mg) in methanol (10 mL) and THF (10 mL). The mixture was neutralized by the addition of hydrogen chloride (1 N aqueous solution) at 0°C, followed by extraction with ethyl acetate. The organic layer was washed with water and saturated brine, dried over magnesium sulfate and then concentrated. The residue was crystallized from ethyl acetate-hexane to obtain the title compound (80 mg).
$^1$H NMR (300 MHz, DMSO-d6) δ 2.25-2.38 (2H, m), 2.42-2.56 (2H, m), 3.73-3.89 (2H, m), 4.09-4.26 (2H, m), 4.60-4.72 (2H, m), 5.74-5.84 (1H, m), 7.12-7.23 (1H, m), 7.43-7.59 (2H, m), 7.62-7.73 (1H, m), 8.02-8.13 (1H, m).

Example 43

(5-(6-(4-(2-Chloro-5-fluorophenoxy)-4-cyanopiperidin-1-yl)pyridazin-3-yl)-1,3,4-oxadiazol-2-yl)methyl acetate

A mixture of (5-(6-chloropyridazin-3-yl)-1,3,4-oxadiazol-2-yl)methyl acetate (100 mg), 4-(2-chloro-5-fluorophenoxy)piperidine-4-carbonitrile hydrochloride (130 mg), DIPEA (0.5 mL) and IPA(4 mL) was stirred at 150°C for 1 hour under irradiation with microwave. After cooling to room temperature, the deposit was collected by filtration and washed with IPA to obtain the title compound (160 mg).
$^1$H NMR (300 MHz, DMSO-d6) δ 2.15 (3H, s), 2.23-2.37 (2H, m), 2.40-2.48 (2H, m), 3.70-3.87 (2H, m), 4.07-4.24 (2H, m), 5.43 (2H, s), 7.10-7.22 (1H, m), 7.43-7.51 (1H, m), 7.54 (1H, d, J = 9.6 Hz), 7.60-7.72 (1H, m), 8.07 (1H, d, J = 9.6 Hz).

Example 44

4-(2-Chloro-5-fluorophenoxy)-1-(6-(5-(hydroxymethyl)-1,3,4-oxadiazol-2-yl)pyridazin-3-yl)piperidine-4-carbonitrile

To a solution of (5-(6-(4-(2-Chloro-5-fluorophenoxy)-4-cyanopiperidin-1-yl)pyridazin-3-yl)-1,3,4-oxadiazol-2-yl)methyl acetate (90 mg) in methanol (10 mL) and THF (10 mL), sodium hydroxide (1 N aqueous solution, 0.190 mL) was added at room temperature. The mixture was neutralized by the addition of hydrogen chloride (IN aqueous solution) at room temperature, followed by extraction with ethyl acetate. The organic layer was washed with water and saturated brine, dried over magnesium sulfate and then concentrated. The residue was crystallized from ethyl acetate-hexane to obtain the title compound (70 mg). $^1$H NMR (300 MHz, DMSO-d6) δ2.21-2.37 (2H, m), 2.41-2.55 (2H, m), 3.65-3.85 (2H, m), 4.07-4.24 (2H, m), 4.71-4.78 (2H, m), 5.97-6.04 (1H, m), 7.11-7.22 (1H, m), 7.44-7.50 (1H, m), 7.52-7.57 (1H, m), 7.62-7.70 (1H, m), 8.02-8.12 (1H, m).

Example 45

4-(2-Chlorobenzyl)-1-(6-(1H-imidazol-1-yl)pyridazin-3-yl)piperidine-4-carbonitrile

A mixed solution of 4-(2-chlorobenzyl)piperidine-4-carbonitrile hydrochloride (200 mg), 3-chloro-6-(1H-imidazol-1-yl)pyridazine (173 mg) and DIPEA (0.664 mL) in NMP (3 mL) was heated at 150°C for 60 minutes under irradiation with microwave. The reaction mixture was poured to water, followed by extraction with ethyl acetate. The organic layer was washed with water and saturated brine, then dried over anhydrous magnesium sulfate and concentrated under reduced pressure. The residue was purified by silica gel column chromatography (ethyl acetate/hexane) and then recrystallized from ethyl acetate/heptane to obtain the title compound (58.1 mg).
$^1$H NMR (300 MHz, CDCl3) δ 1.79-1.94 (2H, m), 1.99-2.11 (2H, m), 3.15 (2H, s), 3.23-3.38 (2H, m), 4.38-4.51 (2H, m), 7.12 (1H, d, J = 9.7 Hz), 7.21-7.34 (3H, m), 7.36-7.44 (2H, m), 7.51 (1H, dd, J = 7.4, 2.0 Hz), 7.64 (1H, dd, J = 1.4 Hz), 8.25 (1H, s).

Example 46

4-(3,4-Dichlorophenyl)-1-(6-(3-(hydroxymethyl)-1,2,4-oxadiazol-5-yl)pyridazin-3-yl)piperidine-4-carbonitrile

A) tert-Butyl 4-cyano-4-(3,4-dichlorophenyl)piperidine-1-carboxylate

To a solution of tert-butyl bis(2-chloroethyl)carbamate (5.08 g) and 2-(3,4-dichlorophenyl)acetonitrile (3.00 g) in anhydrous DMF (50 mL), 60% (w/w) sodium hydride in oil (1.935 g) was added at 0°C, and the mixture was stirred overnight at 70°C in a nitrogen atmosphere. The reaction mixture was diluted with a saturated aqueous solution of ammonium chloride, followed by extraction with ethyl acetate. The extract was washed with water and saturated brine, dried over magnesium sulfate and concentrated under reduced pressure. The residue was purified by column chromatography (ethyl acetate/hexane) to obtain the title compound (3.41 g).
MS, found: 254.9.

B) 4-(3,4-Dichlorophenyl)piperidine-4-carbonitrile hydrochloride

To a mixture of tert-butyl 4-cyano-4-(3,4-dichlorophenyl)piperidine-1-carboxylate (3.41 g) and ethyl acetate (15 mL), hydrogen chloride (4 N solution in ethyl acetate, 12 mL) was added, and the resulting mixture was stirred overnight at room temperature in a nitrogen atmosphere,. The insoluble matter was collected by filtration and washed with ethyl acetate to obtain the title compound (2.75 g).
MS, found: 254.8.

C) (5-(6-(4-Cyano-4-(3,4-dichlorophenyl)piperidin-1-yl)pyridazin-3-yl)-1,2,4-oxadiazol-3-yl)methyl benzoate

A mixture of 4-(3,4-dichlorophenyl)piperidine-4-carbonitrile hydrochloride (110 mg), (5-(6-chloropyridazin-3-yl)-1,2,4-oxadiazol-3-yl)methyl benzoate (100 mg), DIPEA (0.275 mL) and IPA (5 mL) was stirred at 150°C for 1 hour under irradiation with microwave. IPA was added to the reaction mixture, and the resulting crystals were collected by filtration to obtain the title compound (148 mg).
MS: $[M+H]^+$ 535.0.

D) 4-(3,4-Dichlorophenyl)-1-(6-(3-(hydroxymethyl)-1,2,4-oxadiazol-5-yl)pyridazin-3-yl)piperidine-4-carbonitrile

To a mixture of (5-(6-(4-cyano-4-(3,4-dichlorophenyl)piperidin-1-yl)pyridazin-3-yl)-1,2,4-oxadiazol-3-yl)methyl benzoate (148 mg), THF (1 mL) and ethanol (1 mL), sodium hydroxide (2 N aqueous solution, 0.276 mL) was added, and the resulting mixture was stirred overnight at room temperature. The reaction mixture was diluted with a saturated aqueous solution of sodium bicarbonate, followed by extraction with acetone/ethyl acetate. The organic layer was washed with water and saturated brine, dried over anhydrous sodium sulfate and concentrated under reduced pressure. The residue was recrystallized from acetone/hexane to obtain the title compound (64.4 mg).
$^1$H NMR (300 MHz, CDCl3) δ 2.03-2.35 (5H, m), 3.57 (2H, t, J = 12.3 Hz), 4.74-4.95 (4H, m), 7.06 (1H, d, J = 9.6 Hz), 7.35 (1H, dd, J = 8.5, 2.3 Hz), 7.49-7.60 (2H, m), 8.05 (1H, d, J = 9.6 Hz).

Example 49

1-(6-(3-(Hydroxymethyl)-1,2,4-oxadiazol-5-yl)pyridazin-3-yl)-4-(1-naphthyl)piperidine-4-carbonitrile

A) 4-(1-Naphthyl)piperidine-4-carbonitrile hydrochloride

In a nitrogen atmosphere, tert-butyl 4-cyano-4-(1-naphthyl)piperidine-1-carboxylate (2.53 g), hydrogen chloride (4 N solution in CPME, 8 mL) and ethyl acetate (30 mL) were stirred overnight at 50°C. The deposit was collected by filtration to obtain the title compound (1.95 g).
MS, found: 236.9.

B) (5-(6-(4-Cyano-4-(1-naphthyl)piperidin-1-yl)pyridazin-3-yl)-1,2,4-oxadiazol-3-yl)methyl benzoate

A mixture of (5-(6-chloropyridazin-3-yl)-1,2,4-oxadiazol-3-yl)methyl benzoate (100 mg), 4-(1-naphthyl)piperidine-4-carbonitrile hydrochloride (110 mg), DIPEA(0.5 mL) and IPA (4 mL) was stirred at 150°C for 1 hour under irradiation with microwave. After cooling to room temperature, the deposit was collected by filtration and washed with IPA to obtain the title compound (160 mg).
$^1$H NMR (300 MHz, DMSO-d6) $\delta$ 2.11-2.34 (2H, m), 2.67-2.91 (2H, m), 3.46-3.66 (2H, m), 4.77-4.96 (2H, m), 5.62 (2H, s), 7.48-7.80 (8H, m), 7.95-8.17 (5H, m), 8.51-8.61 (1H, m).

C) 1-(6-(3-(Hydroxymethyl)-1,2,4-oxadiazol-5-yl)pyridazin-3-yl)-4-(1-naphthyl)piperidine-4-carbonitrile

To a solution of (5-(6-(4-cyano-4-(1-naphthyl)piperidin-1-yl)pyridazin-3-yl)-1,2,4-oxadiazol-3-yl)methyl benzoate (160 mg) in THF (4 mL) and methanol (4 mL), sodium hydroxide (1 N aqueous solution, 0.310 mL) was added at room temperature. In a nitrogen atmosphere, the mixture was stirred at room temperature for 30 minutes. The mixture was neutralized by the addition of hydrogen chloride (1 N aqueous solution) at 0°C, followed by extraction with ethyl acetate. The organic layer was washed with water and saturated brine, dried over magnesium sulfate and then concentrated. The residue was crystallized from IPE to obtain the title compound (100 mg).
$^1$H NMR (300 MHz, DMSO-d6) $\delta$ 2.10-2.36 (2H, m), 2.66-2.90 (2H, m), 3.45-3.66 (2H, m), 4.59-4.71 (2H, m), 4.79-4.94 (2H, m), 5.71-5.88 (1H, m), 7.45-7.78 (5H, m), 7.94-8.18 (3H, m), 8.51-8.61 (1H, m).

Example 52

4-(4-Bromophenyl)-1-(6-(3-(hydroxymethyl)-1,2,4-oxadiazol-5-yl)pyridazin-3-yl)piperidine-4-carbonitrile

A) 4-(4-Bromophenyl)piperidine-4-carbonitrile hydrochloride

To a solution of tert-butyl 4-(4-bromophenyl)-4-cyanopiperidine-1-carboxylate (1.98 g) in ethyl acetate (50 mL), hydrogen chloride (4 N solution in CPME, 8 mL) was added at room temperature. In a nitrogen atmosphere, the mixture was stirred overnight at 50°C. The deposit was collected by filtration to obtain the title compound (1.30 g).
MS, found: 264.8.

B) (5-(6-(4-(4-Bromophenyl)-4-cyanopiperidin-1-yl)pyridazin-3-yl)-1,2,4-oxadiazol-3-yl)methyl benzoate

A mixture of (5-(6-chloropyridazin-3-yl)-1,2,4-oxadiazol-3-yl)methyl benzoate (100 mg), 4-(4-bromophenyl)piperidine-4-carbonitrile hydrochloride (120 mg), DIPEA(0.5 mL) and IPA (4 mL) was stirred at 150°C for 1 hour under irradiation with microwave. The reaction solution was cooled to room temperature, and then, the deposit was collected by filtration and washed with IPA to obtain the title compound (160 mg).
MS: $[M+H]^+$ 546.9.

C) 4-(4-Bromophenyl)-1-(6-(3-(hydroxymethyl)-1,2,4-oxadiazol-5-yl)pyridazin-3-yl)piperidine-4-carbonitrile

To a solution of (5-(6-(4-(4-bromophenyl)-4-cyanopiperidin-1-yl)pyridazin-3-yl)-1,2,4-oxadiazol-3-yl)methyl benzoate (160 mg) in THF (10 mL) and methanol (10 mL), sodium hydroxide (1 N aqueous solution, 0.293 mL) was added at room temperature. In a nitrogen atmosphere, the mixture was stirred at room temperature for 30 minutes. The mixture was neutralized by the addition of hydrogen chloride (1 N aqueous solution) at 0°C, followed by extraction with ethyl acetate. The organic layer was washed with water and saturated brine, dried over magnesium sulfate and then concentrated. The residue was crystallized from ethyl acetate-hexane to obtain the title compound (100 mg).

$^1$H NMR (300 MHz, DMSO-d6) δ 2.06-2.21 (2H, m), 2.24-2.37 (2H, m), 3.23-3.41 (2H, m), 4.65 (2H, d, J = 6.1 Hz), 4.74-4.91 (2H, m), 5.78 (1H, t, J = 6.2 Hz), 7.50-7.60 (3H, m), 7.62-7.71 (2H, m), 8.01-8.12 (1H, m).

Example 53

1-(6-(3-(Hydroxymethyl)-1,2,4-oxadiazol-5-yl)pyridazin-3-yl)-4-(4-iodophenyl)piperidine-4-carbonitrile

A) 4-(4-Iodophenyl)piperidine-4-carbonitrile hydrochloride

To a solution of tert-butyl 4-cyano-4-(4-iodophenyl)piperidine-1-carboxylate (1.58 g) in ethyl acetate (50 mL), hydrogen chloride (4 N solution in CPME, 8 mL) was added at room temperature. In a nitrogen atmosphere, the mixture was stirred overnight at 50°C. The deposit was collected by filtration to obtain the title compound (1.290 g). MS, found: 312.8.

B) (5-(6-(4-Cyano-4-(4-iodophenyl)piperidin-1-yl)pyridazin-3-yl)-1,2,4-oxadiazol-3-yl)methyl benzoate

A mixture of 4-(4-iodophenyl)piperidine-4-carbonitrile hydrochloride (120 mg), (5-(6-chloropyridazin-3-yl)-1,2,4-oxadiazol-3-yl)methyl benzoate (100 mg), DIPEA (0.5 mL) and IPA (4 mL) was stirred at 150°C for 1 hour under irradiation with microwave. After cooling to room temperature, the deposit was collected by filtration and washed with IPA to obtain the title compound (180 mg).
MS: $[M+H]^+$ 593.1.

C) 1-(6-(3-(Hydroxymethyl)-1,2,4-oxadiazol-5-yl)pyridazin-3-yl)-4-(4-iodophenyl)piperidine-4-carbonitrile

To a solution of (5-(6-(4-cyano-4-(4-iodophenyl)piperidin-1-yl)pyridazin-3-yl)-1,2,4-oxadiazol-3-yl)methyl benzoate (180 mg) in THF (10 mL) and methanol (10 mL), sodium hydroxide (IN aqueous solution, 0.304 mL) was added at room temperature. The mixture was stirred at room temperature for 30 minutes. The mixture was neutralized by the addition of hydrogen chloride (IN aqueous solution) at 0°C, followed by extraction with ethyl acetate. The organic layer was washed with water and saturated brine, dried over magnesium sulfate and then concentrated. The residue was crystallized from ethyl acetate-hexane to obtain the title compound (80 mg).
$^1$H NMR (300 MHz, DMSO-d6) δ 2.04-2.21 (2H, m), 2.24-2.36 (2H, m), 3.24-3.42 (2H, m), 4.65 (2H, d, J = 6.2 Hz), 4.74-4.88 (2H, m), 5.78 (1H, t, J = 6.2 Hz), 7.37-7.44 (2H, m), 7.50-7.57 (1H, m), 7.77-7.88 (2H, m), 8.01-8.12 (1H, m).

Example 54

4-(4-Ethynylphenyl)-1-(6-(3-(hydroxymethyl)-1,2,4-oxadiazol-5-yl)pyridazin-3-yl)piperidine-4-carbonitrile

A) tert-Butyl 4-cyano-4-(4-((trimethylsilyl)ethynyl)phenyl)piperidine-1-carboxylate

In a nitrogen atmosphere, a solution of tert-butyl 4-(4-bromophenyl)-4-cyanopiperidine-1-carboxylate (1.25 g), trimethylsilylacetylene (0.709 mL), copper(I) iodide (0.130 g), dichlorobis(triphenylphosphine)palladium(II) (0.240 g) and triethylamine (10 mL) in dehydrated DMF (10 mL) was stirred overnight at 70°C. The mixture was poured to water at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (1.30 g). Experimental item corrected, note corrected
$^1$H NMR (300 MHz, CDCl3) δ 0.21-0.28 (9H, m), 1.48 (9H, s), 1.82-1.98 (2H, m), 2.01-2.14 (2H, m), 3.00-3.36 (2H, m), 4.05-4.53 (2H, m), 7.33-7.44 (2H, m), 7.46-7.56 (2H, m).

B) 4-(4-((Trimethylsilyl)ethynyl)phenyl)piperidine-4-carbonitrile hydrochloride

To a solution of tert-butyl 4-cyano-4-(4-((trimethylsilyl)ethynyl)phenyl)piperidine-1-carboxylate (1.30 g) in ethyl acetate (50 mL), hydrogen chloride (4 N solution in CPME, 8 mL) was added at room temperature. In a nitrogen atmosphere, the mixture was stirred overnight at 50°C. The deposit was collected by filtration to obtain the title compound (1.02 g).
$^1$H NMR (300 MHz, DMSO-d6) δ 0.23 (9H, s), 2.27-2.49 (4H, m), 2.97-3.21 (2H, m), 3.40-3.59 (2H, m), 7.46-7.65 (4H, m), 9.19-9.74 (2H, m).

C) (5-(6-(4-Cyano-4-(4-((trimethylsilyl)ethynyl)phenyl)piperidin-1-yl)pyridazin-3-yl)-1,2,4-oxadiazol-3-yl)methyl benzoate

A mixture of 4-(4-((trimethylsilyl)ethynyl)phenyl)piperidine-4-carbonitrile hydrochloride (120 mg), (5-(6-chloropyridazin-3-yl)-1,2,4-oxadiazol-3-yl)methyl benzoate (100 mg), DIPEA (0.5 mL) and IPA (4 mL) was stirred at 150°C for 1 hour under irradiation with microwave. After cooling to room temperature, the deposit was collected by filtration and washed with IPA to obtain the title compound (170 mg).
MS: $[M+H]^+$ 563.2.

D) 4-(4-Ethynylphenyl)-1-(6-(3-(hydroxymethyl)-1,2,4-oxadiazol-5-yl)pyridazin-3-yl)piperidine-4-carbonitrile

In a nitrogen atmosphere, a mixture of (5-(6-(4-cyano-4-(4-((trimethylsilyl)ethynyl)phenyl)piperidin-1-yl)pyridazin-3-yl)-1,2,4-oxadiazol-3-yl)methyl benzoate (170 mg, 0.30 mmol), sodium hydroxide (1 N aqueous solution, 0.302 mL), THF (10 mL) and methanol (10 mL) was stirred at room temperature for 30 minutes. The mixture was neutralized by the addition of hydrogen chloride (1 N aqueous solution) at 0°C, followed by extraction with ethyl acetate. The organic layer was washed with water and saturated brine, dried over magnesium sulfate and then concentrated. The residue was crystallized from ethyl acetate-hexane to obtain the title compound (101 mg).
$^1$H NMR (300 MHz, DMSO-d6) $\delta$ 2.07-2.21 (2H, m), 2.26-2.37 (2H, m), 3.27-3.42 (2H, m), 4.26 (1H, s), 4.60-4.70 (2H, m), 4.74-4.88 (2H, m), 5.73-5.81 (1H, m), 7.49-7.66 (5H, m), 8.02-8.11 (1H, m).

Example 55

1-(6-(3-(Hydroxymethyl)-1,2,4-oxadiazol-5-yl)pyridazin-3-yl)-4-(4-(trifluoromethoxy)phenyl)piperidine-4-carbonitrile

A) 4-(4-(Trifluoromethoxy)phenyl)piperidine-4-carbonitrile hydrochloride

In a nitrogen atmosphere, a mixture of tert-butyl 4-cyano-4-(4-(trifluoromethoxy)phenyl)piperidine-1-carboxylate (2.95 g), hydrogen chloride (4 N solution in CPME, 1.991 mL) and ethyl acetate (20 mL) was stirred overnight at 50°C. The deposit was collected by filtration to obtain the title compound (2.11 g).
$^1$H NMR (300 MHz, DMSO-d6) $\delta$ 2.32-2.47 (4H, m), 2.99-3.19 (2H, m), 3.42-3.59 (2 H, m), 7.46-7.58 (2H, m), 7.63-7.76 (2H, m), 9.28 (2H, brs).

B) (5-(6-(4-Cyano-4-(4-(trifluoromethoxy)phenyl)piperidin-1-yl)pyridazin-3-yl)-1,2,4-oxadiazol-3-yl)methyl benzoate

A mixture of 4-(4-(trifluoromethoxy)phenyl)piperidine-4-carbonitrile hydrochloride (120 mg), (5-(6-chloropyridazin-3-yl)-1,2,4-oxadiazol-3-yl)methyl benzoate (100 mg), DIPEA (0.5 mL) and IPA (4 mL) was stirred at 150°C for 1 hour under irradiation with microwave. After cooling to room temperature, the deposit was collected by filtration and washed with IPA to obtain the title compound (170 mg).
MS: $[M+H]^+$ 551.1.

C) 1-(6-(3-(Hydroxymethyl)-1,2,4-oxadiazol-5-yl)pyridazin-3-yl)-4-(4-(trifluoromethoxy)phenyl)piperidine-4-carbonitrile

In a nitrogen atmosphere, a mixture of (5-(6-(4-cyano-4-(4-(trifluoromethoxy)phenyl)piperidin-1-yl)pyridazin-3-yl)-1,2,4-oxadiazol-3-yl)methyl benzoate (170 mg), sodium hydroxide (1 N aqueous solution, 0.309 mL), THF (10 mL) and methanol (10 mL) was stirred at room temperature for 30 minutes. The mixture was neutralized by the addition of hydrogen chloride (1 N aqueous solution) at 0°C, followed by extraction with ethyl acetate. The organic layer was washed with water and saturated brine, dried over magnesium sulfate and then concentrated. The residue was crystallized from ethyl acetate-hexane to obtain the title compound (110 mg).
$^1$H NMR (300 MHz, DMSO-d6) $\delta$ 2.08-2.23 (2H, m), 2.29-2.41 (2H, m), 3.26-3.44 (2H, m), 4.60-4.69 (2H, m), 4.76-4.91 (2H, m), 5.72-5.82 (1H, m), 7.42-7.50 (2H, m), 7.51-7.59 (1H, m), 7.70-7.79 (2H, m), 8.03-8.11 (1H, m).

Example 57

1-(6-(3-(Hydroxymethyl)-1,2,4-oxadiazol-5-yl)pyridazin-3-yl)-4-(4-(pentafluorosulfanyl)phenyl)piperidine-4-carbonitrile

A) tert-Butyl 4-cyano-4-(4-(pentafluorosulfanyl)phenyl)piperidine-1-carboxylate

To a solution of tert-butyl bis(2-chloroethyl)carbamate (1.1 g) and 4-(pentafluorothio)phenylacetonitrile (834

mg) in dehydrated DMF (300 mL), 60% (w/w) sodium hydride in oil (0.411 g) was added at 0°C. In a nitrogen atmosphere, the mixture was stirred overnight at 70°C. The reaction solution was poured to a saturated aqueous solution of ammonium chloride at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (293 mg).
$^1$H NMR (300 MHz, CDCl3) δ 1.49 (9H, s), 1.84-2.18 (4H, m), 3.01-3.40 (2H, m), 4.11-4.57 (2H, m), 7.53-7.64 (2H, m), 7.78-7.87 (2H, m).

B) 4-(4-(Pentafluorosulfanyl)phenyl)piperidine-4-carbonitrile hydrochloride

In a nitrogen atmosphere, a mixture of tert-butyl 4-cyano-4-(4-(pentafluorosulfanyl)phenyl)piperidine-1-carboxylate (293 mg), hydrogen chloride (4 N solution in CPME, 0.5 mL) and ethyl acetate (5 mL) was stirred overnight at 50°C. After cooling to room temperature, the deposit was collected by filtration to obtain the title compound (220 mg). $^1$H NMR (300 MHz, DMSO-d6) δ 2.26-2.47 (4H, m), 3.01-3.21 (2H, m), 3.45-3.62 (2H, m), 7.72-7.82 (2H, m), 8.03-8.13 (2H, m), 8.87-9.16 (2H, m).

C) (5-(6-(4-Cyano-4-(4-(pentafluorosulfanyl)phenyl)piperidin-1-yl)pyridazin-3-yl)-1,2,4-oxadiazol-3-yl)methyl benzoate

A mixture of 4-(4-(pentafluorosulfanyl)phenyl)piperidine-4-carbonitrile hydrochloride (96 mg), (5-(6-chloropyridazin-3-yl)-1,2,4-oxadiazol-3-yl)methyl benzoate (70 mg), DIPEA (0.3 mL) and IPA (4 mL) was stirred at 150°C for 1 hour under irradiation with microwave. After cooling to room temperature, the deposit was collected by filtration and washed with IPA to obtain the title compound (84 mg).
$^1$H NMR (300 MHz, DMSO-d6) δ 2.11-2.41 (4H, m), 3.23-3.45 (2H, m), 4.74-4.96 (2H, m), 5.62 (2H, s), 7.47-7.64 (3H, m), 7.68-7.76 (1H, m), 7.79-7.89 (2H, m), 7.95-8.15 (5H, m).

D) 1-(6-(3-(Hydroxymethyl)-1,2,4-oxadiazol-5-yl)pyridazin-3-yl)-4-(4-(pentafluorosulfanyl)phenyl)piperidine-4-carbonitrile

In a nitrogen atmosphere, a mixture of (5-(6-(4-cyano-4-(4-(pentafluorosulfanyl)phenyl)piperidin-1-yl)pyridazin-3-yl)-1,2,4-oxadiazol-3-yl)methyl benzoate (84 mg), sodium hydroxide (1 N aqueous solution, 0.142 mL), THF (10 mL) and methanol (10 mL) was stirred at room temperature for 30 minutes. The mixture was neutralized by the addition of hydrogen chloride (1 N aqueous solution) at 0°C, followed by extraction with ethyl acetate. The organic layer was washed with water and saturated brine, dried over magnesium sulfate and then concentrated. The residue was crystallized from ethyl acetate-hexane to obtain the title compound (62 mg).
$^1$H NMR (300 MHz, DMSO-d6) δ 2.14-2.28 (2H, m), 2.30-2.39 (2H, m), 3.27-3.45 (2H, m), 4.61-4.70 (2H, m), 4.78-4.92 (2H, m), 5.73-5.83 (1H, m), 7.51-7.61 (1H, m), 7.80-7.91 (2H, m), 7.97-8.12 (3H, m).

Example 58

1-(6-(5-(Hydroxymethyl)-1,3,4-oxadiazol-2-yl)pyridazin-3-yl)-4-(4-(pentafluorosulfanyl)phenyl)piperidine-4-carbonitrile

A) 6-Chloro-N'-(((4-methoxybenzyl)oxy)acetyl)pyridazine-3-carbohydrazide

To a suspension of 6-oxo-1,6-dihydropyridazine-3-carboxylic acid (3.44 g) and thionyl chloride (35 mL), DMF (0.317 mL) was added at room temperature, and the mixture was stirred at 80°C for 1 hour. The reaction mixture thus stirred was diluted with toluene and concentrated under reduced pressure. The obtained residue was diluted with THF (40 mL). A solution of pyridine (3.30 mL) and 2-((4-methoxybenzyl)oxy)acetohydrazide (4.30 g) in THF (40 mL) was added thereto at 0°C, and the mixture was stirred overnight at room temperature in a dry atmosphere. The reaction mixture thus stirred overnight was diluted with a saturated aqueous solution of sodium bicarbonate, followed by extraction with THF-ethyl acetate. The obtained organic layer was washed with saturated brine, dried over anhydrous sodium sulfate and concentrated under reduced pressure. The residue was washed with IPE to obtain the title compound (4.99 g). MS, found: 372.9.

B) 3-Chloro-6-(5-(((4-methoxybenzyl)oxy)methyl)-1,3,4-oxadiazol-2-yl)pyridazine

To a suspension of 6-chloro-N'-(((4-methoxybenzyl)oxy)acetyl)pyridazine-3-carbohydrazide (70.0 g) and acetonitrile (600 mL), trimethylamine hydrochloride (5.72 g), triethylamine (83.4 mL), and pTsCl (57.0 g) were added at room temperature, and the mixture was stirred at 50°C for 1 hour in a nitrogen atmosphere. The reaction mixture thus

stirred was diluted with THF (300 mL), and the insoluble matter was filtered off. The filtrate was poured to water, followed by extraction with ethyl acetate. The obtained organic layer was washed with water and saturated brine, dried over anhydrous sodium sulfate and concentrated under reduced pressure. The obtained residue was purified by column chromatography (ethyl acetate/hexane) to obtain the title compound (60 g).
MS: $[M+H]^+$ 333.0.

C) 1-(6-(5-(((4-Methoxybenzyl)oxy)methyl)-1,3,4-oxadiazol-2-yl)pyridazin-3-yl)-4-(4-(pentafluorosulfanyl)phenyl)piperidine-4-carbonitrile

A mixture of 4-(4-(pentafluorosulfanyl)phenyl)piperidine-4-carbonitrile hydrochloride (4.0 g), 3-chloro-6-(5-(((4-methoxybenzyl)oxy)methyl)-1,3,4-oxadiazol-2-yl)pyridazine (3.6 g), DIPEA (7.54 mL) and IPA (10 mL) was stirred at 150°C for 1 hour under irradiation with microwave. The reaction mixture was concentrated under reduced pressure. The residue was purified by column chromatography (ethyl acetate/hexane) to obtain the title compound (6.23 g).
$^1$H NMR (300 MHz, DMSO-d6) $\delta$ 2.08-2.43 (4H, m), 3.25-3.43 (2H, m), 3.74 (3H, s), 4.58 (2H, s), 4.74-4.88 (4H, m), 6.88-6.97 (2H, m), 7.25-7.35 (2H, m), 7.52-7.62 (1H, m), 7.79-7.90 (2H, m), 7.95-8.11 (3H, m).

D) 1-(6-(5-(Hydroxymethyl)-1,3,4-oxadiazol-2-yl)pyridazin-3-yl)-4-(4-(pentafluorosulfanyl)phenyl)piperidine-4-carbonitrile

In a nitrogen atmosphere, a mixture of l-(6-(5-(((4-methoxybenzyl)oxy)methyl)-1,3,4-oxadiazol-2-yl)pyridazin-3-yl)-4-(4-(pentafluorosulfanyl)phenyl)piperidine-4-carbonitrile (6.23 g), TFA (30 mL) and anisole (3 mL) was stirred at room temperature for 1 hour. The reaction mixture was concentrated under reduced pressure. The residue was poured to a saturated aqueous solution of sodium bicarbonate at room temperature, followed by extraction with ethyl acetate. The organic layer was washed with water and saturated brine, dried over magnesium sulfate and then concentrated. The residue was suspended in IPE, and the insoluble matter was collected by filtration to obtain crude crystals (4.7 g).
The obtained crude crystals (4.5 g) were recrystallized twice from 5% aqueous ethanol (350 mL)-water (2000 mL) and further recrystallized from 5% aqueous ethanol (300 mL)-water (2000 mL) to obtain the title compound (3.43 g).
$^1$H NMR (300 MHz, DMSO-d6) $\delta$ 2.12-2.44 (4H, m), 3.16-3.47 (2H, m), 4.62-4.93 (4H, m), 5.91-6.09 (1H, m), 7.48-7.63 (1H, m), 7.80-7.92 (2H, m), 7.95-8.15 (3H, m).

Example 59

1-(6-(5-(Hydroxymethyl)-1,3,4-oxadiazol-2-yl)pyridazin-3-yl)-4-(4-(trifluoromethyl)phenyl)piperidine-4-carbonitrile

In a nitrogen atmosphere, a mixture of (5-(6-(4-cyano-4-(4-(trifluoromethyl)phenyl)piperidin-1-yl)pyridazin-3-yl)-1,3,4-oxadiazol-2-yl)methyl acetate (220 mg), sodium hydroxide (1 N aqueous solution, 0.466 mL), THF (10 mL) and methanol (10 mL) was stirred at room temperature for 30 minutes. The mixture was neutralized by the addition of hydrogen chloride (IN aqueous solution) at 0°C, followed by extraction with ethyl acetate. The organic layer was washed with water and saturated brine, dried over magnesium sulfate and then concentrated. The residue was crystallized from ethyl acetate-hexane to obtain the title compound (148 mg).
$^1$H NMR (300 MHz, DMSO-d6) $\delta$ 2.13-2.26 (2H, m), 2.28-2.39 (2H, m), 3.24-3.45 (2H, m), 4.71-4.90 (4H, m), 5.96-6.06 (1H, m), 7.56 (1H, d, J = 9.7 Hz), 7.84 (4H, s), 8.06 (1H, d, J = 9.7 Hz).

Example 60

(5-(6-(4-Cyano-4-(4-(pentafluorosulfanyl)phenyl)piperidin-1-yl)pyridazin-3-yl)-1,3,4-oxadiazol-2-yl)methyl acetate

A mixture of 4-(4-(pentafluorosulfanyl)phenyl)piperidine-4-carbonitrile hydrochloride (64 mg), (5-(6-chloropyridazin-3-yl)-1,3,4-oxadiazol-2-yl)methyl acetate (40 mg), DIPEA (0.2 mL) and IPA (1.5 mL) was stirred at 150°C for 1 hour under irradiation with microwave. After cooling to room temperature, the deposit was collected by filtration and washed with IPA to obtain the title compound (30 mg).
$^1$H NMR (300 MHz, DMSO-d6) $\delta$ 2.14 (3H, s), 2.17-2.26 (2H, m), 2.29-2.40 (2H, m), 3.25-3.41 (2H, m), 4.70-4.93 (2H, m), 5.43 (2H, s), 7.50-7.62 (1H, m), 7.79-7.90 (2H, m), 7.99 (3H, s).

Example 61

(5-(6-(4-Cyano-4-(4-(trifluoromethyl)phenyl)piperidin-1-yl)pyridazin-3-yl)-1,3,4-oxadiazol-2-yl)methyl acetate

A mixture of 4-(4-(trifluoromethyl)phenyl)piperidine-4-carbonitrile hydrochloride (200 mg), (5-(6-chloropyridazin-3-yl)-1,3,4-oxadiazol-2-yl)methyl acetate (150 mg), DIPEA (0.5 mL) and IPA (4 mL) was stirred at 150°C for 1 hour under irradiation with microwave. After cooling to room temperature, the deposit was collected by filtration and washed with IPA to obtain the title compound (220 mg).
$^1$H NMR (300 MHz, DMSO-d6) δ 2.14 (3H, s), 2.17-2.27 (2H, m), 2.29-2.40 (2H, m), 3.26-3.44 (2H, m), 4.73-4.88 (2H, m), 5.43 (2H, s), 7.57 (1H, d, J = 9.7 Hz), 7.84 (4H, s), 8.07 (1H, d, J = 9.7 Hz).

Example 62

4-(2-Chlorobenzyl)-1-(6-(1-methyl-1H-pyrazol-4-yl)pyridazin-3-yl)piperidine-4-carbonitrile

A) 1-(6-Bromopyridazin-3-yl)-4-(2-chlorobenzyl)piperidine-4-carbonitrile

To a solution of 4-(2-chlorobenzyl)piperidine-4-carbonitrile hydrochloride (1.5 g) in DMA (12 mL), 3,6-dibromopyridazine (1.49 g) and DIPEA (3.98 mL) were added, and the mixture was heated at 150°C for 60 minutes under irradiation with microwave. The reaction mixture was poured to water, followed by extraction with ethyl acetate/THF. The organic layer was washed with water and saturated brine, dried over anhydrous magnesium sulfate and then concentrated under reduced pressure. The residue was recrystallized from ethyl acetate/heptane to obtain the title compound (1.5243 g).
MS: $[M+H]^+$ 390.9.

B) 4-(2-Chlorobenzyl)-1-(6-(1-methyl-1H-pyrazol-4-yl)pyridazin-3-yl)piperidine-4-carbonitrile

To a solution of 1-(6-bromopyridazin-3-yl)-4-(2-chlorobenzyl)piperidine-4-carbonitrile (200 mg) in DME (5 mL), 1-methyl-4-(4,4,5,5-tetramethyl-1,3,2-dioxaborolan-2-yl)-1H-pyrazole (133 mg), tetrakis(triphenylphosphine)palladium (59 mg) and sodium carbonate (2.0 N aqueous solution, 0.511 mL) were added, and the mixture was stirred overnight at 85°C in an Ar atmosphere. The reaction mixture was poured to water, followed by extraction with ethyl acetate. The organic layer was washed with saturated brine, dried over anhydrous magnesium sulfate and then concentrated under reduced pressure. The residue was purified by silica gel column chromatography (ethyl acetate/hexane) and then recrystallized from ethyl acetate/heptane to obtain the title compound (91 mg).
$^1$H NMR (300 MHz, CDCl3) δ 1.77-1.92 (2H, m), 1.96-2.07 (2H, m), 3.13 (2H, s), 3.19-3.33 (2H, m), 3.97 (3H, s), 4.38-4.50 (2H, m), 6.96 (1H, d, J = 9.5 Hz), 7.21-7.33 (2H, m), 7.36-7.45 (2H, m), 7.51 (1H, dd, J = 7.3, 1.9 Hz), 7.90 (1H, s), 7.95 (1H, s).

Example 63

4-(2-Chlorobenzyl)-1-(6-(1H-pyrazol-4-yl)pyridazin-3-yl)piperidine-4-carbonitrile

To a solution of 1-(6-bromopyridazin-3-yl)-4-(2-chlorobenzyl)piperidine-4-carbonitrile (200 mg) in DME (5 mL), 4-(4,4,5,5-tetramethyl-1,3,2-dioxaborolan-2-yl)-1H-pyrazole (124 mg), tetrakis(triphenylphosphine)palladium (59 mg) and sodium carbonate (2.0 N aqueous solution, 0.766 mL) were added, and the mixture was stirred overnight at 85°C in an Ar atmosphere. The reaction mixture was poured to water, followed by extraction with ethyl acetate. The organic layer was washed with saturated brine, dried over anhydrous magnesium sulfate and then concentrated under reduced pressure. The residue was purified by silica gel column chromatography (ethyl acetate/hexane) and then recrystallized from ethyl acetate/heptane to obtain the title compound (27.5 mg).
$^1$H NMR (300 MHz, CDCl3) δ 1.78-1.93 (2H, m), 1.96-2.09 (2H, m), 3.14 (2H, s), 3.20-3.35 (2H, m), 4.39-4.53 (2H, m), 6.98 (1H, d, J = 9.4 Hz), 7.21-7.33 (2H, m), 7.37-7.43 (1H, m), 7.45 (1H, d, J = 9.4 Hz), 7.51 (1H, dd, J = 7.3, 1.9 Hz), 8.13 (2H, s).

Example 64

4-(2-Chloro-4-fluorobenzyl)-1-(6-(3-(hydroxymethyl)-1,2,4-oxadiazol-5-yl)pyridazin-3-yl)piperidine-4-carbonitrile

A) (5-(6-(4-(2-Chloro-4-fluorobenzyl)-4-cyanopiperidin-1-yl)pyridazin-3-yl)-1,2,4-oxadiazol-3-yl)methyl benzoate

A mixture of DIPEA (0.550 mL), (5-(6-chloropyridazin-3-yl)-1,2,4-oxadiazol-3-yl)methyl benzoate (200 mg), 4-(2-chloro-4-fluorobenzyl)piperidine-4-carbonitrile hydrochloride (219 mg) and IPA (4.0 mL) was stirred at 150°C for 1 hour under irradiation with microwave. The reaction mixture was cooled to room temperature, and the deposit was collected by filtration to obtain the title compound (273 mg).
MS: $[M+H]^+$ 533.1.

B) 4-(2-Chloro-4-fluorobenzyl)-1-(6-(3-(hydroxymethyl)-1,2,4-oxadiazol-5-yl)pyridazin-3-yl)piperidine-4-carbonitrile

To a mixture of (5-(6-(4-(2-chloro-4-fluorobenzyl)-4-cyanopiperidin-1-yl)pyridazin-3-yl)-1,2,4-oxadiazol-3-yl)methyl benzoate (273 mg), THF (3 mL) and methanol (3 mL), sodium hydroxide (IN aqueous solution, 1.024 mL) was added, and the resulting mixture was stirred overnight at room temperature. The reaction mixture was diluted with water, followed by extraction with ethyl acetate. The extract was washed with sodium hydroxide (0.1 N aqueous solution), hydrogen chloride (0.1 N aqueous solution) and saturated brine, dried over anhydrous sodium sulfate and concentrated under reduced pressure. The residue was recrystallized from DMSO/ethanol to obtain the title compound (63.7 mg). $^1$H NMR (300 MHz, DMSO-d6) $\delta$ 1.71-2.09 (4H, m), 3.06-3.25 (4H, m), 4.57-4.77 (4H, m), 5.77 (1H, t, J = 6.0 Hz), 7.29 (1H, td, J = 8.4, 2.6 Hz), 7.45-7.60 (3H, m), 8.04 (1H, d, J = 9.6 Hz).

Example 65

4-(2-Chloro-4-fluorobenzyl)-1-(6-(5-(hydroxymethyl)-1,3,4-oxadiazol-2-yl)pyridazin-3-yl)piperidine-4-carbonitrile

A) tert-Butyl 4-(2-chloro-4-fluorobenzyl)-4-cyanopiperidine-1-carboxylate

To a solution of DIPA (4 mL) in dehydrated THF (100 mL), n-butyllithium (1.6 N solution in hexane, 17 mL) was added at 0°C. After stirring at 0°C for 30 minutes, a solution of tert-butyl 4-cyanopiperidine-1-carboxylate (5 g) in dehydrated THF (30 mL) was added to the reaction solution. In an argon atmosphere, the mixture was stirred at 0°C for 1 hour. 1-(Bromomethyl)-2-chloro-4-fluorobenzene (5.31 g) was added to the reaction solution at -78°C. The temperature of the mixture was gradually raised to room temperature, and the mixture was stirred overnight at room temperature. The completion of the reaction was confirmed by TLC. The mixture was poured to a saturated aqueous solution of ammonium chloride (100 mL) at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (7.50 g).
$^1$H NMR (300 MHz, CDCl3) $\delta$ 1.46 (9H, s), 1.55-1.71 (2H, m), 1.80-1.94 (2H, m), 2.82-3.15 (4H, m), 3.96-4.42 (2H, m), 6.96-7.07 (1H, m), 7.12-7.21 (1H, m), 7.39-7.51 (1H, m).

B) 4-(2-Chloro-4-fluorobenzyl)piperidine-4-carbonitrile hydrochloride

In a nitrogen atmosphere, a mixture of tert-butyl 4-(2-chloro-4-fluorobenzyl)-4-cyanopiperidine-1-carboxylate (7.5 g), hydrogen chloride (4 N solution in CPME, 15 mL) and CPME (50 mL) was stirred overnight at 50°C. The deposit was collected by filtration to obtain the title compound (6.12 g).
MS, found: 253.2.

C) 4-(2-Chloro-4-fluorobenzyl)-1-(6-(5-(((4-methoxybenzyl)oxy)methyl)-1,3,4-oxadiazol-2-yl)pyridazin-3-yl)piperidine-4-carbonitrile

A mixture of DIPEA (0.785 mL), 3-chloro-6-(5-(((4-methoxybenzyl)oxy)methyl)-1,3,4-oxadiazol-2-yl)pyridazine (300 mg), 4-(2-chloro-4-fluorobenzyl)piperidine-4-carbonitrile hydrochloride (330 mg) and DMA (4 mL) was stirred at 150°C for 1 hour under irradiation with microwave. The mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (450 mg).
MS: $[M+H]^+$ 549.1.

D) 4-(2-Chloro-4-fluorobenzyl)-1-(6-(5-(hydroxymethyl)-1,3,4-oxadiazol-2-yl)pyridazin-3-yl)piperidine-4-carbonitrile

In a nitrogen atmosphere, a mixture of 4-(2-chloro-4-fluorobenzyl)-1-(6-(5-(((4-methoxybenzyl)oxy)methyl)-1,3,4-oxadiazol-2-yl)pyridazin-3-yl)piperidine-4-carbonitrile (450 mg), anisole (0.5 mL) and TFA (5 mL) was stirred at room temperature for 1 hour. The reaction mixture was concentrated under reduced pressure. The residue was poured to a saturated aqueous solution of sodium bicarbonate at 0°C, followed by extraction with ethyl acetate. The organic layer was washed with water and saturated brine, dried over magnesium sulfate and then concentrated. The residue was crystallized from ethyl acetate-hexane to obtain crude crystals (330 mg). The obtained crude crystals (330 mg) were purified by column chromatography (ethyl acetate/hexane) and then recrystallized from ethyl acetate-hexane to obtain the title compound (100 mg).
$^1$H NMR (300 MHz, DMSO-d6) δ 1.73-1.91 (2H, m), 1.93-2.06 (2H, m), 3.04-3.22 (4H, m), 4.59-4.70 (2H, m), 4.70-4.79 (2H, m), 5.95-6.05 (1H, m), 7.24-7.35 (1H, m), 7.45-7.62 (3H, m), 7.98-8.07 (1H, m).

Example 67

4-(2-Chloro-5-fluorobenzyl)-1-(6-(5-(hydroxymethyl)-1,3,4-oxadiazol-2-yl)pyridazin-3-yl)piperidine-4-carbonitrile

A) tert-Butyl 4-(2-chloro-5-fluorobenzyl)-4-cyanopiperidine-1-carboxylate

To a solution of DIPA (3.4 mL) in dehydrated THF (100 mL), n-butyllithium (1.6 M solution in hexane, 13.38 mL) was added at 0°C. After stirring at 0°C for 30 minutes, a solution of tert-butyl 4-cyanopiperidine-1-carboxylate (4.5 g) in dehydrated THF (30 mL) was added to the reaction solution. In an argon atmosphere, the mixture was stirred at 0°C for 1 hour. 1-(Bromomethyl)-2-chloro-5-fluorobenzene (5 g) was added to the reaction solution at-78°C. The temperature of the mixture was raised to room temperature, and the mixture was stirred overnight at room temperature. The completion of the reaction was confirmed by TLC. The mixture was poured to a saturated aqueous solution of ammonium chloride (100 mL) at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (7.50 g).
$^1$H NMR (300 MHz, CDCl3) δ 1.46 (9H, s), 1.56-1.74 (2H, m), 1.80-1.98 (2H, m), 3.07 (4H, s), 4.00-4.34 (2H, m), 6.93-7.03 (1H, m), 7.18-7.24 (1H, m), 7.33-7.42 (1H, m).

B) 4-(2-Chloro-5-fluorobenzyl)piperidine-4-carbonitrile hydrochloride

In a nitrogen atmosphere, a mixture of tert-butyl 4-(2-chloro-5-fluorobenzyl)-4-cyanopiperidine-1-carboxylate (7.5 g), hydrogen chloride (4 N solution in CPME, 15 mL) and CPME (50 mL) was stirred overnight at 50°C. The deposit was collected by filtration to obtain the title compound (5.8 g).
MS, found: 252.9.

C) (5-(6-(4-(2-Chloro-5-fluorobenzyl)-4-cyanopiperidin-1-yl)pyridazin-3-yl)-1,3,4-oxadiazol-2-yl)methyl acetate

A mixture of 4-(2-chloro-5-fluorobenzyl)piperidine-4-carbonitrile hydrochloride (200 mg), (5-(6-chloropyridazin-3-yl)-1,3,4-oxadiazol-2-yl)methyl acetate (211 mg), DIPEA (0.489 mL) and DMA (5 mL) was heated at 150°C for 60 minutes under irradiation with microwave. The reaction mixture was cooled to room temperature, and then, water was added thereto, followed by extraction with ethyl acetate. The organic layer was washed with water and saturated brine, dried over anhydrous magnesium sulfate and concentrated under reduced pressure. The residue was recrystallized from ethyl acetate/heptane to obtain the title compound (262 mg).
MS: $[M+H]^+$ 471.0.

D) 4-(2-Chloro-5-fluorobenzyl)-1-(6-(5-(hydroxymethyl)-1,3,4-oxadiazol-2-yl)pyridazin-3-yl)piperidine-4-carbonitrile

To a solution of (5-(6-(4-(2-chloro-5-fluorobenzyl)-4-cyanopiperidin-1-yl)pyridazin-3-yl)-1,3,4-oxadiazol-2-yl)methyl acetate (250 mg) in THF (5 mL) and methanol (5 mL), sodium hydroxide (2 N aqueous solution, 1.327 mL) was added, and the mixture was stirred at room temperature for 30 minutes. The reaction mixture was neutralized by the addition of hydrogen chloride (2 N aqueous solution, 1.30 mL) and a saturated aqueous solution of ammonium chloride (50 mL) at room temperature, and then, the reaction mixture was subjected to extraction with ethyl acetate. The organic layer was washed with saturated brine, dried over anhydrous magnesium sulfate and concentrated under reduced pressure. The residue was recrystallized from ethyl acetate/heptane to obtain the title compound (192 mg).
$^1$H NMR (300 MHz, DMSO-d6) δ 1.76-1.92 (2H, m), 1.95-2.07 (2H, m), 3.06-3.21 (4H, m), 4.59-4.70 (2H, m, J = 14.1

Hz), 4.75 (2H, d, J = 6.3 Hz), 5.99 (1H, t, J = 6.3 Hz), 7.25 (1H, ddd, J = 8.9, 8.1, 3.1 Hz), 7.38 (1H, dd, J = 9.6, 3.1 Hz), 7.49 (1H, d, J = 9.7 Hz), 7.57 (1H, dd, J = 8.9, 5.3 Hz), 8.03 (1H, d, J = 9.7 Hz).

Example 68

4-(2-Chloro-5-fluorobenzyl)-1-(6-(3-(hydroxymethyl)-1,2,4-oxadiazol-5-yl)pyridazin-3-yl)piperidine-4-carbonitrile

A) (5-(6-(4-(2-Chloro-5-fluorobenzyl)-4-cyanopiperidin-1-yl)pyridazin-3-yl)-1,2,4-oxadiazol-3-yl)methyl benzoate

A mixture of 4-(2-chloro-5-fluorobenzyl)piperidine-4-carbonitrile hydrochloride (200 mg), (5-(6-chloropyridazin-3-yl)-1,2,4-oxadiazol-3-yl)methyl benzoate (263 mg), DIPEA (0.489 mL) and DMA (5 mL) was heated at 150°C for 60 minutes under irradiation with microwave. The reaction mixture was poured to water, followed by extraction with ethyl acetate. The organic layer was washed with water and saturated brine, dried over anhydrous magnesium sulfate and then concentrated under reduced pressure. The residue was recrystallized from ethyl acetate/heptane to obtain the title compound (318 mg).
MS: $[M+H]^+$ 533.0.

B) 4-(2-Chloro-5-fluorobenzyl)-1-(6-(3-(hydroxymethyl)-1,2,4-oxadiazol-5-yl)pyridazin-3-yl)piperidine-4-carbonitrile

To a solution of (5-(6-(4-(2-chloro-5-fluorobenzyl)-4-cyanopiperidin-1-yl)pyridazin-3-yl)-1,2,4-oxadiazol-3-yl)methyl benzoate (290 mg) in THF (5 mL) and methanol (5 mL), sodium hydroxide (2.0 N aqueous solution, 2.72 mL) was added, and the mixture was stirred at room temperature for 5 hours. The reaction mixture was neutralized by the addition of hydrogen chloride (2.0 N aqueous solution, 2.50 mL) and a saturated aqueous solution of ammonium chloride (50 mL) at room temperature, and then, the reaction mixture was subjected to extraction with ethyl acetate/THF. The organic layer was washed with saturated brine, dried over anhydrous magnesium sulfate and then concentrated under reduced pressure. The residue was recrystallized from ethyl acetate/heptane to obtain the title compound (199 mg). $^1$H NMR (300 MHz, DMSO-d6) δ 1.79-1.93 (2H, m), 1.95-2.08 (2H, m), 3.08-3.24 (4H, m), 4.60-4.75 (4H, m), 5.77 (1H, t, J = 6.2 Hz), 7.25 (1H, ddd, J = 8.9, 8.1, 3.1 Hz), 7.38 (1H, dd, J = 9.5, 3.1 Hz), 7.48 (1H, d, J = 9.7 Hz), 7.57 (1H, dd, J = 8.9, 5.3 Hz), 8.05 (1H, d, J = 9.7 Hz).

Example 69

3-(2-Chlorobenzyl)-1-(6-(3-(hydroxymethyl)-1,2,4-oxadiazol-5-yl)pyridazin-3-yl)azetidine-3-carbonitrile

A) tert-Butyl 3-(2-chlorobenzyl)-3-cyanoazetidine-1-carboxylate

To a solution of DIPA (2.366 mL) in THF (40 mL), n-butyllithium (1.6 M solution in hexane, 9.98 mL) was added, and the mixture was stirred at 0°C for 30 minutes. In an Ar atmosphere, a solution of tert-butyl 3-cyanoazetidine-1-carboxylate (2.5 g) in THF (10 mL) was added to the reaction mixture, and the mixture was stirred for 1 hour under ice cooling. The reaction mixture was cooled to -78°C. Then, 1-(bromomethyl)-2-chlorobenzene (3.07 g) was added thereto, and the mixture was stirred overnight in an Ar atmosphere while the temperature was gradually raised to room temperature. The reaction solution was poured to a saturated aqueous solution of ammonium chloride, followed by extraction with ethyl acetate. The organic layer was washed with saturated brine, dried over anhydrous magnesium sulfate and concentrated under reduced pressure. The residue was purified by silica gel column chromatography (ethyl acetate/hexane) to obtain the title compound (457 mg).
$^1$H NMR (300 MHz, CDCl3) δ 1.44 (9H, s), 3.39 (2H, s), 4.04-4.11 (2H, m), 4.21-4.32 (2H, m), 7.24-7.33 (2H, m), 7.34-7.40 (1H, m), 7.41-7.47 (1H, m).

B) (5-(6-(3-(2-Chlorobenzyl)-3-cyanoazetidin-1-yl)pyridazin-3-yl)-1,2,4-oxadiazol-3-yl)methyl benzoate

To a solution of tert-butyl 3-(2-chlorobenzyl)-3-cyanoazetidine-1-carboxylate (150 mg) in toluene (2 mL), TFA (2 mL) was added, and the mixture was stirred at room temperature for 3 hours in an Ar atmosphere. The reaction mixture was concentrated under reduced pressure and then subjected to azeotropy with toluene. A mixture of the residue, (5-(6-chloropyridazin-3-yl)-1,2,4-oxadiazol-3-yl)methyl benzoate (186 mg), DIPEA(0.346 mL) and DMA (5 mL) was heated at 150°C for 60 minutes under irradiation with microwave. The reaction mixture was poured to water, followed by extraction with ethyl acetate. The organic layer was washed with water and saturated brine, dried over anhydrous magnesium sulfate and concentrated under reduced pressure. The residue was purified by silica gel column chromatography (ethyl acetate/hexane) to obtain the title compound (169 mg).

MS: $[M+H]^+$ 487.0.

C) 3-(2-Chlorobenzyl)-1-(6-(3-(hydroxymethyl)-1,2,4-oxadiazol-5-yl)pyridazin-3-yl)azetidine-3-carbonitrile

To a solution of (5-(6-(3-(2-chlorobenzyl)-3-cyanoazetidin-1-yl)pyridazin-3-yl)-1,2,4-oxadiazol-3-yl)methyl benzoate (165 mg) in THF (5 mL) and methanol (5 mL), sodium hydroxide (2.0 N aqueous solution, 0.847 mL) was added, and the mixture was stirred at room temperature for 5 hours. Hydrogen chloride (2.0 N aqueous solution, 0.80 mL) and a saturated aqueous solution of ammonium chloride (50 mL) were added to the reaction mixture at room temperature, and then, the reaction mixture was subjected to extraction with ethyl acetate. The organic layer was washed with saturated brine, dried over anhydrous magnesium sulfate and concentrated under reduced pressure. The residue was purified by silica gel column chromatography (ethyl acetate/hexane) and then recrystallized from ethyl acetate/heptane to obtain the title compound (44.3 mg).
$^1$H NMR (300 MHz, DMSO-d6) δ 3.32 (2H, s), 4.51 (2H, d, J = 9.1 Hz), 4.61 (2H, d, J = 9.1 Hz), 4.65 (2H, d, J = 6.0 Hz), 5.78 (1H, t, J = 6.0 Hz), 7.08 (1H, d, J = 9.4 Hz), 7.33-7.44 (2H, m), 7.48-7.57 (2H, m), 8.11 (1H, d, J = 9.4 Hz).

Example 70 1-(6-(3-(Hydroxymethyl)-1,2,4-oxadiazol-5-yl)pyridazin-3-yl)-4-(3-(pentafluorosulfanyl)phenyl)piperidine-4-carbonitrile

A) tert-Butyl 4-cyano-4-(3-(pentafluorosulfanyl)phenyl)piperidine-1-carboxylate

To a solution of tert-butyl bis(2-chloroethyl)carbamate (4 g) and 3-(pentafluorothio)phenylacetonitrile (2.75 g) in dehydrated DMF (300 mL), 60% (w/w) sodium hydride in oil (1.357 g) was added at 0°C. In a nitrogen atmosphere, the mixture was stirred overnight at 70°C. The reaction solution was poured to a saturated aqueous solution of ammonium chloride at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (910 mg).
$^1$H NMR (300 MHz, CDCl3) δ 1.49 (9H, s), 1.88-2.02 (2H, m), 2.07-2.21 (2H, m), 3.07-3.33 (2H, m), 4.19-4.50 (2H, m), 7.50-7.61 (1H, m), 7.65-7.71 (1H, m), 7.74-7.79 (1H, m), 7.80-7.85 (1H, m).

B) 4-(3-(Pentafluorosulfanyl)phenyl)piperidine-4-carbonitrile hydrochloride

In a nitrogen atmosphere, a mixture of tert-butyl 4-cyano-4-(3-(pentafluorosulfanyl)phenyl)piperidine-1-carboxylate (910 mg), hydrogen chloride (4 N solution in CPME, 0.552 mL) and CPME (10 mL) was stirred overnight at 50°C. The deposit was collected by filtration to obtain the title compound (760 mg).
$^1$H NMR (300 MHz, DMSO-d6) δ 2.42-2.63 (4H, m), 3.00-3.22 (2H, m), 3.43-3.63 (2H, m), 7.73-7.84 (1H, m), 7.85-7.92 (1H, m), 7.95-8.05 (2H, m), 9.50 (2H, brs).

C) (5-(6-(4-Cyano-4-(3-(pentafluorosulfanyl)phenyl)piperidin-1-yl)pyridazin-3-yl)-1,2,4-oxadiazol-3-yl)methyl benzoate

A mixture of 4-(3-(pentafluorosulfanyl)phenyl)piperidine-4-carbonitrile hydrochloride (140 mg), (5-(6-chloropyridazin-3-yl)-1,2,4-oxadiazol-3-yl)methyl benzoate (100 mg), DIPEA (0.3 mL) and IPA (4 mL) was stirred at 150°C for 1 hour under irradiation with microwave. After cooling to room temperature, the deposit was collected by filtration and washed with IPA to obtain the title compound (160 mg).
$^1$H NMR (300 MHz, DMSO-d6) δ 2.17-2.45 (4H, m), 3.28-3.43 (2H, m), 4.77-4.93 (2H, m), 5.62 (2H, s), 7.48-7.63 (3H, m), 7.67-7.77 (2H, m), 7.91-8.14 (6H, m).

D) 1-(6-(3-(Hydroxymethyl)-1,2,4-oxadiazol-5-yl)pyridazin-3-yl)-4-(3-(pentafluorosulfanyl)phenyl)piperidine-4-carbonitrile

In a nitrogen atmosphere, a mixture of (5-(6-(4-cyano-4-(3-(pentafluorosulfanyl)phenyl)piperidin-1-yl)pyridazin-3-yl)-1,2,4-oxadiazol-3-yl)methyl benzoate (160 mg), sodium hydroxide (1 N aqueous solution, 0.270 mL), methanol (10 mL) and THF (10 mL) was stirred at room temperature for 20 minutes. Hydrogen chloride (1 N aqueous solution, 3 mL) was added thereto at 0°C to adjust the pH to 2 to 3. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (120 mg).
$^1$H NMR (300 MHz, DMSO-d6) δ 2.17-2.44 (4H, m), 3.28-3.43 (2H, m), 4.59-4.69 (2H, m), 4.79-4.93 (2H, m), 5.70-5.85 (1H, m), 7.49-7.61 (1H, m), 7.69-7.80 (1H, m), 7.92-8.00 (2H, m), 8.03-8.11 (2H, m).

Example 71

(5-(6-(4-Phenyl-4-(trifluoromethyl)piperidin-1-yl)pyridazin-3-yl)-1,2,4-oxadiazol-3-yl)methanol

A) (5-(6-(4-Phenyl-4-(trifluoromethyl)piperidin-1-yl)pyridazin-3-yl)-1,2,4-oxadiazol-3-yl)methyl benzoate

A mixture of 4-phenyl-4-(trifluoromethyl)piperidine (130 mg), (5-(6-chloropyridazin-3-yl)-1,2,4-oxadiazol-3-yl)methyl benzoate (100 mg), DIPEA(0.3 mL) and IPA (4 mL) was stirred at 150°C for 1 hour under irradiation with microwave. After cooling to room temperature, the deposit was collected by filtration and washed with IPA to obtain the title compound (130 mg).
$^1$H NMR (300 MHz, DMSO-d6) $\delta$ 1.99-2.19 (2H, m), 2.69-3.00 (4H, m), 4.45-4.65 (2H, m), 5.60 (2H, s), 7.37-7.61 (6H, m), 7.64-7.77 (3H, m), 7.95-8.10 (3H, m).

B) (5-(6-(4-Phenyl-4-(trifluoromethyl)piperidin-1-yl)pyridazin-3-yl)-1,2,4-oxadiazol-3-yl)methanol

In a nitrogen atmosphere, a mixture of (5-(6-(4-phenyl-4-(trifluoromethyl)piperidin-1-yl)pyridazin-3-yl)-1,2,4-oxadiazol-3-yl)methyl benzoate (117 mg), sodium hydroxide (1 N aqueous solution, 0.23 mL), methanol (10 mL) and THF (10 mL) was stirred at room temperature for 20 minutes. Hydrogen chloride (IN aqueous solution, 3 mL) was added thereto at 0°C to adjust the pH to 2 to 3. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (90 mg).
$^1$H NMR (300 MHz, DMSO-d6) $\delta$ 2.00-2.17 (2H, m), 2.69-2.81 (2H, m), 2.84-2.99 (2H, m), 4.47-4.58 (2H, m), 4.60-4.68 (2H, m), 5.69-5.83 (1H, m), 7.38-7.56 (4H, m), 7.66-7.73 (2H, m), 7.96-8.06 (1H, m).

Example 72

4-((2-Chlorophenyl)(difluoro)methyl)-1-(6-(3-(hydroxymethyl)-1,2,4-oxadiazol-5-yl)pyridazin-3-yl)piperidine-4-carbonitrile

A) tert-Butyl 4-((2-chlorophenyl)(hydroxy)methyl)-4-cyanopiperidine-1-carboxylate

To a solution of DIPA (4 mL) in dehydrated THF (100 mL), n-butyllithium (1.6 M solution in hexane, 17 mL) was added at 0°C. After stirring at 0°C for 30 minutes, a solution of tert-butyl 4-cyanopiperidine-1-carboxylate (5 g) in dehydrated THF (30 mL) was added to the reaction solution. In an argon atmosphere, the mixture was stirred at 0°C for 1 hour. 2-Chlorobenzaldehyde (3.34 g) was added to the reaction solution at -78°C. In an argon atmosphere, the temperature of the reaction mixture was gradually raised to room temperature, and the reaction mixture was stirred overnight. After the completion of the reaction, the mixture was poured to a saturated aqueous solution of ammonium chloride (100 mL) at room temperature, followed by extraction with ethyl acetate (100 mL). The organic layer was washed with water and saturated brine, dried over magnesium sulfate and then concentrated. The residue was purified by column chromatography (ethyl acetate/hexane) to obtain the title compound (8.00 g).
$^1$H NMR (300 MHz, DMSO-d6) $\delta$ 1.26-1.45 (10H, m), 1.50-1.71 (2H, m), 2.17-2.31 (1H, m), 2.58-3.03 (2H, m), 3.85-4.19 (2H, m), 4.92-5.02 (1H, m), 6.31-6.41 (1H, m), 7.29-7.51 (3H, m), 7.68-7.82 (1H, m).

B) tert-Butyl 4-(2-chlorobenzoyl)-4-cyanopiperidine-1-carboxylate

A mixture of tert-butyl 4-((2-chlorophenyl)(hydroxy)methyl)-4-cyanopiperidine-1-carboxylate (8 g), IBX (13.41 g) and ethyl acetate (200 mL) was heated to reflux overnight. The solid was filtered off, and the filtrate was concentrated under reduced pressure. The residue was purified by column chromatography (NH, 0% - 30% ethyl acetate/hexane) to obtain the title compound (7.4 g).
$^1$H NMR (300 MHz, CDCl3) $\delta$ 1.47 (9H, s), 1.90-2.29 (4H, m), 2.86-3.33 (2H, m), 4.04-4.40 (2H, m), 7.32-7.54 (4H, m).

C) tert-Butyl4-((2-chlorophenyl)(difluoro)methyl)-4-cyanopiperidine-1-carboxylate

To a solution of tert-butyl 4-(2-chlorobenzoyl)-4-cyanopiperidine-1-carboxylate (1.60 g) in toluene (30 mL), bis(2-methoxyethyl)aminosulfur trifluoride (5 mL) was added at room temperature. In a nitrogen atmosphere, the mixture was stirred overnight at 80°C. The mixture was poured to sodium hydroxide (IN aqueous solution) at room temperature, followed by extraction with ethyl acetate. The organic layer was washed with water and saturated brine, dried over

magnesium sulfate and then concentrated. The residue was purified by column chromatography (ethyl acetate/hexane) to obtain the title compound (1.39 g). $^1$H NMR (300 MHz, CDCl3) δ 1.46 (9H, s), 1.81-2.09 (4H, m), 2.82-3.15 (2H, m), 4.07-4.40 (2H, m), 7.34-7.54 (3H, m), 7.60-7.69 (1H, m).

D) 4-((2-Chlorophenyl)(difluoro)methyl)piperidine-4-carbonitrile hydrochloride

In a nitrogen atmosphere, a mixture of tert-butyl 4-((2-chlorophenyl)(difluoro)methyl)-4-cyanopiperidine-1-carboxylate (1.00 g), hydrogen chloride (4 N solution in CPME, 0.674 mL) and ethyl acetate (20 mL) was stirred overnight at 50°C. The deposit was collected by filtration to obtain the title compound (810 mg). MS, found: 270.9.

E) (5-(6-(4-((2-Chlorophenyl)(difluoro)methyl)-4-cyanopiperidin-1-yl)pyridazin-3-yl)-1,2,4-oxadiazol-3-yl)methyl benzoate

A mixture of 4-((2-chlorophenyl)(difluoro)methyl)piperidine-4-carbonitrile hydrochloride (140 mg), (5-(6-chloropyridazin-3-yl)-1,2,4-oxadiazol-3-yl)methyl benzoate (100 mg), DIPEA (0.5 mL) and IPA (4 mL) was stirred at 150°C for 1 hour under irradiation with microwave. The mixture was concentrated under reduced pressure. The residue was purified by column chromatography (ethyl acetate/hexane) to obtain the title compound (150 mg). $^1$H NMR (300 MHz, DMSO-d6) δ 1.81-2.01 (2H, m), 2.16-2.30 (2H, m), 3.09-3.24 (2H, m), 4.72-4.89 (2H, m), 5.61 (2H, s), 7.42-7.79 (8H, m), 7.97 -8.13 (3H, m).

F) 4-((2-Chlorophenyl)(difluoro)methyl)-1-(6-(3-(hydroxymethyl)-1,2,4-oxadiazol-5-yl)pyridazin-3-yl)piperidine-4-carbonitrile

In a nitrogen atmosphere, a mixture of (5-(6-(4-((2-chlorophenyl)(difluoro)methyl)-4-cyanopiperidin-1-yl)pyridazin-3-yl)-1,2,4-oxadiazol-3-yl)methyl benzoate (140 mg), sodium hydroxide (1 N aqueous solution, 0.254 mL), methanol (10 mL) and THF (10 mL) was stirred at room temperature for 20 minutes. Hydrogen chloride (1 N aqueous solution, 3 mL) was added thereto at 0°C to adjust the pH to 2 to 3. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (98 mg).
$^1$H NMR (300 MHz, DMSO-d6) δ 1.81-2.02 (2H, m), 2.16-2.30 (2H, m), 3.07-3.24 (2H, m), 4.60-4.68 (2H, m), 4.73-4.91 (2H, m), 5.70-5.84 (1H, m), 7.39-7.76 (5H, m), 7.98-8.13 (1H, m).

Example 75

4-((2-Chlorophenyl)(difluoro)methyl)-1-(6-(5-(hydroxymethyl)-1,3,4-oxadiazol-2-yl)pyridazin-3-yl)piperidine-4-carbonitrile

A) 4-((2-Chlorophenyl)(difluoro)methyl)-1-(6-(5-(((4-methoxybenzyl)oxy)methyl)-1,3,4-oxadiazol-2-yl)pyridazin-3-yl)piperidine-4-carbonitrile

A mixture of 4-((2-chlorophenyl)(difluoro)methyl)piperidine-4-carbonitrile hydrochloride (140 mg), 3-chloro-6-(5-(((4-methoxybenzyl)oxy)methyl)-1,3,4-oxadiazol-2-yl)pyridazine (100 mg), DIPEA (0.5 mL) and IPA (4 mL) was stirred at 150°C for 1 hour under irradiation with microwave. The reaction mixture was concentrated under reduced pressure. The residue was purified by column chromatography (ethyl acetate/hexane) to obtain the title compound (150 mg).
$^1$H NMR (300 MHz, DMSO-d6) δ 1.80-2.01 (2H, m), 2.12-2.31 (2H, m), 3.06-3.22 (2H, m), 3.74 (3H, s), 4.52-4.63 (2H, m), 4.67-4.94 (4H, m), 6.84-7.00 (2H, m), 7.24-7.36 (2 H, m), 7.40-7.79 (5H, m), 7.95-8.10 (1H, m).

B) 4-((2-Chlorophenyl)(difluoro)methyl)-1-(6-(5-(hydroxymethyl)-1,3,4-oxadiazol-2-yl)pyridazin-3-yl)piperidine-4-carbonitrile

In a nitrogen atmosphere, a mixture of 4-((2-chlorophenyl)(difluoro)methyl)-1-(6-(5-(((4-methoxybenzyl)oxy)methyl)-1,3,4-oxadiazol-2-yl)pyridazin-3-yl)piperidine-4-carbonitrile (85 mg), TFA (3 mL) and anisole (1 mL) was stirred at room temperature for 1 hour. The reaction mixture was concentrated under reduced pressure. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (60 mg).

$^1H$ NMR (300 MHz, DMSO-d6) δ 1.80-2.04 (2H, m), 2.13-2.31 (2H, m), 3.07-3.20 (2H, m), 4.65-4.86 (4H, m), 5.92-6.05 (1H, m), 7.47-7.75 (5H, m), 7.99-8.07 (1H, m).

Example 82

(5-(6-(4-(Trifluoromethyl)-4-(4-(trifluoromethyl)phenyl)piperidin-1-yl)pyridazin-3-yl)-1,2,4-oxadiazol-3-yl)methanol

A) tert-Butyl4-(trifluoromethyl)-4-(4-(trifluoromethyl)phenyl)piperidine-1-carboxylate

In a nitrogen atmosphere, a mixture of 2,2,2-trifluoro-1-(4-(trifluoromethyl)phenyl)ethanone (10 g), ethyl cyanoacetate (4.67 g), ammonium acetate (1.592 g), acetic acid (4.73 mL) and toluene (50 mL) was stirred overnight at 130°C. The reaction mixture thus stirred overnight was poured to a saturated aqueous solution of sodium bicarbonate at room temperature, followed by extraction with ethyl acetate. The obtained organic layer was washed with water and saturated brine, dried over magnesium sulfate and then concentrated. The obtained residue was purified by column chromatography (ethyl acetate/hexane) to obtain ethyl 2-cyano-4,4,4-trifluoro-3-(4-(trifluoromethyl)phenyl)but-2-enoate (3.5 g).

In a nitrogen atmosphere, a mixture of ethyl 2-cyano-4,4,4-trifluoro-3-(4-(trifluoromethyl)phenyl)but-2-enoate (3.5 g), 2-cyanoacetamide (1.527 g), sodium acetate (2.98 g) and ethanol was stirred at room temperature over weekend. The solvent was distilled off under reduced pressure from the solution thus stirred, and the obtained residue was neutralized by the addition of water (30 mL) and further the addition of hydrogen chloride (aqueous solution) to adjust the pH to 3. The obtained solution was subjected to extraction with ethyl acetate, drying over magnesium sulfate and concentration to obtain a crude product. A mixture of the obtained crude product, sulfuric acid (13.59 mL), water (25 mL) and acetic acid (25 mL) was heated to reflux overnight. The reaction mixture thus heated to reflux was cooled to 0°C, and water (30 mL) was added thereto. The obtained deposit was collected by filtration to obtain a crude product. A mixture of the obtained crude product and potassium hydroxide (20% aqueous solution, 40 mL) was heated to reflux for 3 hours. The reaction mixture thus heated to reflux was cooled to 0°C, and a mixture of sulfuric acid (6 mL) and water (25 mL) was added dropwise thereto. The obtained mixture was heated to reflux for 2 hours. The mixture thus heated to reflux was poured to water at 0°C, followed by extraction with ethyl acetate. The obtained organic layer was washed with saturated brine, dried over magnesium sulfate and then concentrated to obtain a crude product. The obtained crude product was dissolved in acetic anhydride (50 mL), and the solution was heated to reflux for 2 hours. Excessive acetic anhydride was distilled off from the solution thus heated to reflux, and urea (0.447 g) was added to the residue at room temperature. The obtained mixture was stirred at 190°C for 20 minutes in a nitrogen atmosphere to obtain a crude product. A borane-THF complex (1 M solution in THF, 50 mL) was added to a solution of the obtained crude product in dehydrated THF (30 mL) at 0°C. The obtained mixture was stirred overnight at 70°C, and then, hydrogen chloride (6 N aqueous solution, 50 mL) was added to the reaction mixture. The obtained mixture was heated to reflux for 1 hour. The mixture thus heated to reflux was poured to sodium hydroxide (6 N aqueous solution, 50 mL) at 0°C, followed by extraction with ethyl acetate. The obtained organic layer was washed with water and saturated brine, dried over magnesium sulfate and then concentrated to obtain an intermediate. Di-tert-butyl dicarbonate (1.624 g) was added to a solution of the obtained intermediate and DIPEA (1.296 mL) in dehydrated DMF (100 mL) at room temperature. The obtained mixture was poured to a saturated aqueous solution of sodium bicarbonate at room temperature, followed by extraction with ethyl acetate. The obtained organic layer was washed with water and saturated brine, dried over magnesium sulfate and then concentrated. The obtained residue was purified by column chromatography (ethyl acetate/hexane) to obtain the title compound (1.10 g).
$^1H$ NMR (300 MHz, CDCl3) δ 1.44 (9H, s), 2.00-2.20 (2H, m), 2.40-2.54 (2H, m), 2.60-2.85 (2H, m), 3.84-4.34 (2H, m), 7.55-7.62 (2H, m), 7.66-7.73 (2H, m).

B) 4-(Trifluoromethyl)-4-(4-(trifluoromethyl)phenyl)piperidine hydrochloride

In a nitrogen atmosphere, a mixture of tert-butyl4-(trifluoromethyl)-4-(4-(trifluoromethyl)phenyl)piperidine-1-carboxylate (1.10 g), hydrogen chloride (4 N solution in CPME, 0.692 mL) and ethyl acetate (10 mL) was stirred overnight at 50°C. The obtained deposit was collected by filtration to obtain the title compound (890 mg). $^1H$ NMR (300 MHz, DMSO-d6) δ 2.29-2.45 (2H, m), 2.47-2.67 (2H, m), 2.73-2.89 (2H, m), 3.19-3.41 (2H, m), 7.87 (4H, s), 9.29 (2H, brs).

C) (5-(6-(4-(Trifluoromethyl)-4-(4-(trifluoromethyl)phenyl)piperidin-1-yl)pyridazin-3-yl)-1,2,4-oxadiazol-3-yl)methyl benzoate

A mixture of 4-(trifluoromethyl)-4-(4-(trifluoromethyl)phenyl)piperidine hydrochloride (140 mg), (5-(6-chloropyridazin-3-yl)-1,2,4-oxadiazol-3-yl)methyl benzoate (100 mg), DIPEA (0.3 mL) and IPA (4 mL) was stirred at 150°C for 1

hour under irradiation with microwave. After cooling to room temperature, the deposit was collected by filtration and washed with IPA to obtain the title compound (164 mg).
$^1$H NMR (300 MHz, DMSO-d6) δ 2.03-2.25 (2H, m), 2.74-3.02 (4H, m), 4.45-4.65 (2H, m), 5.60 (2H, s), 7.38-7.46 (1H, m), 7.52-7.62 (2H, m), 7.66-7.76 (1H, m), 7.82-7.90 (2H, m), 7.92-7.99 (2H, m), 8.00-8.08 (3H, m).

D) (5-(6-(4-(Trifluoromethyl)-4-(4-(trifluoromethyl)phenyl)piperidin-1-yl)pyridazin-3-yl)-1,2,4-oxadiazol-3-yl)methanol

In a nitrogen atmosphere, a mixture of (5-(6-(4-(trifluoromethyl)-4-(4-(trifluoromethyl)phenyl)piperidin-1-yl)pyridazin-3-yl)-1,2,4-oxadiazol-3-yl)methyl benzoate (164 mg), sodium hydroxide (1 N aqueous solution, 0.284 mL), methanol (10 mL) and THF (10 mL) was stirred at room temperature for 20 minutes. Hydrogen chloride (IN aqueous solution, 3 mL) was added thereto at 0°C to adjust the pH to 2 to 3. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (120 mg).
$^1$H NMR (300 MHz, DMSO-d6) δ 2.05-2.25 (2H, m), 2.71-3.01 (4H, m), 4.48-4.60 (2H, m), 4.61-4.66 (2H, m), 5.72-5.81 (1H, m), 7.39-7.49 (1H, m), 7.83-7.90 (2H, m), 7.92-8.07 (3H, m).

Example 83

4-(4-tert-Butylphenyl)-1-(6-(3-(hydroxymethyl)-1,2,4-oxadiazol-5-yl)pyridazin-3-yl)piperidine-4-carbonitrile

A) tert-Butyl4-(4-tert-butylphenyl)-4-cyanopiperidine-1-carboxylate

To a solution of tert-butyl bis(2-chloroethyl)carbamate (10 g) and 2-(4-(tert-butyl)phenyl)acetonitrile (5 g) in dehydrated DMF (300 mL), 60% (w/w) sodium hydride in oil (3.46 g) was added at 70°C. In a nitrogen atmosphere, the mixture was stirred overnight at 70°C. The reaction solution was poured to a saturated aqueous solution of ammonium chloride at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (3.50 g).
$^1$H NMR (300 MHz, CDCl3) δ 1.32 (9H, s), 1.43-1.52 (9H, m), 1.85-2.19 (4H, m), 3.12-3.35 (2H, m), 4.17-4.41 (2H, m), 7.35-7.46 (4H, m).

B) 4-(4-tert-Butylphenyl)piperidine-4-carbonitrile hydrochloride

In a nitrogen atmosphere, a mixture of tert-butyl 4-(4-tert-butylphenyl)-4-cyanopiperidine-1-carboxylate (3.50 g), hydrogen chloride (4 N solution in CPME, 2.55 mL) and ethyl acetate (10 mL) was stirred overnight at 50°C. The deposit was collected by filtration to obtain the title compound (2.68 g).
$^1$H NMR (300 MHz, DMSO-d6) δ 1.29 (9H, s), 2.29-2.46 (4H, m), 3.00-3.18 (2H, m), 3.42-3.55 (2H, m), 7.38-7.56 (4H, m), 9.31 (2H, brs).

C) (5-(6-(4-(4-tert-Butylphenyl)-4-cyanopiperidin-1-yl)pyridazin-3-yl)-1,2,4-oxadiazol-3-yl)methyl benzoate

A mixture of 4-(4-tert-butylphenyl)piperidine-4-carbonitrile hydrochloride (140 mg), (5-(6-chloropyridazin-3-yl)-1,2,4-oxadiazol-3-yl)methyl benzoate (100 mg), DIPEA (0.3 mL) and IPA (4 mL) was stirred at 150°C for 1 hour under irradiation with microwave. After cooling to room temperature, the deposit was collected by filtration and washed with IPA to obtain the title compound (157 mg).
$^1$H NMR (300 MHz, DMSO-d6) δ 1.28 (9H, s), 2.05-2.19 (2H, m), 2.23-2.35 (2H, m), 3.33-3.44 (2H, m), 4.73-4.90 (2H, m), 5.61 (2H, s), 7.39-7.63 (7H, m), 7.67-7.77 (1H, m), 7.97-8.13 (3H, m).

D) 4-(4-tert-Butylphenyl)-1-(6-(3-(hydroxymethyl)-1,2,4-oxadiazol-5-yl)pyridazin-3-yl)piperidine-4-carbonitrile

In a nitrogen atmosphere, a mixture of (5-(6-(4-(4-tert-butylphenyl)-4-cyanopiperidin-1-yl)pyridazin-3-yl)-1,2,4-oxadiazol-3-yl)methyl benzoate (157 mg), sodium hydroxide (IN aqueous solution, 0.30 mL), methanol (10 mL) and THF (10 mL) was stirred at room temperature for 20 minutes. Hydrogen chloride (IN aqueous solution, 3 mL) was added thereto at 0°C to adjust the pH to 2 to 3. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (110 mg).

$^{1}$H NMR (300 MHz, DMSO-d6) δ 1.28 (9H, s), 2.06-2.20 (2H, m), 2.24-2.37 (2H, m), 3.25-3.44 (2H, m), 4.60-4.69 (2H, m), 4.74-4.89 (2H, m), 5.72-5.83 (1H, m), 7.41-7.59 (5H, m), 8.01-8.13 (1H, m).

Example 84

(5-(6-(4-(Trifluoromethyl)-4-(4-(trifluoromethyl)phenyl)piperidin-1-yl)pyridazin-3-yl)-1,3,4-oxadiazol-2-yl)methanol

A) 3-(5-(((4-Methoxybenzyl)oxy)methyl)-1,3,4-oxadiazol-2-yl)-6-(4-(trifluoromethyl)-4-(4-(trifluoromethyl)phenyl)piperidin-1-yl)pyridazine

A mixture of 4-(trifluoromethyl)-4-(4-(trifluoromethyl)phenyl)piperidine hydrochloride (140 mg), 3-chloro-6-(5-(((4-methoxybenzyl)oxy)methyl)-1,3,4-oxadiazol-2-yl)pyridazine (100 mg), DIPEA (0.3 mL) and IPA (4 mL) was stirred at 150°C for 1 hour under irradiation with microwave. The solution thus stirred was cooled to room temperature, and then, the deposit was collected by filtration and washed with IPA to obtain the title compound (145 mg).
$^{1}$H NMR (300 MHz, DMSO-d6) δ 2.02-2.25 (2H, m), 2.72-3.01 (4 H, m), 3.74 (3H, s), 4.45-4.60 (4H, m), 4.81 (2H, s), 6.84-6.98 (2H, m), 7.24-7.35 (2H, m), 7.39 - 7.49 (1H, m), 7.80-8.06 (5H, m).

B) (5-(6-(4-(Trifluoromethyl)-4-(4-(trifluoromethyl)phenyl)piperidin-1-yl)pyridazin-3-yl)-1,3,4-oxadiazol-2-yl)methanol

In a nitrogen atmosphere, a mixture of 3-(5-(((4-methoxybenzyl)oxy)methyl)-1,3,4-oxadiazol-2-yl)-6-(4-(trifluoromethyl)-4-(4-(trifluoromethyl)phenyl)piperidin-1-yl)pyridazine (145 mg), TFA (3 mL) and anisole (1 mL) was stirred at room temperature for 1 hour. The reaction mixture thus stirred was concentrated under reduced pressure. The obtained residue was poured to a saturated aqueous solution of sodium bicarbonate at room temperature, followed by extraction with ethyl acetate. The obtained organic layer was washed with water and saturated brine, dried over magnesium sulfate and then concentrated. The obtained residue was crystallized from IPE to obtain the title compound (100 mg). $^{1}$H NMR (300 MHz, DMSO-d6) δ 2.06-2.28 (2H, m), 2.70-3.04 (4H, m), 4.43-4.59 (2H, m), 4.70-4.81 (2H, m), 5.88-6.10 (1H, m), 7.35-7.52 (1H, m), 7.78-8.10 (5H, m).

Example 85

4-(4-tert-Butylphenyl)-1-(6-(5-(hydroxymethyl)-1,3,4-oxadiazol-2-yl)pyridazin-3-yl)piperidine-4-carbonitrile

A) 4-(4-tert-Butylphenyl)-1-(6-(5-(((4-methoxybenzyl)oxy)methyl)-1,3,4-oxadiazol-2-yl)pyridazin-3-yl)piperidine-4-carbonitrile

A mixture of 4-(4-tert-butylphenyl)piperidine-4-carbonitrile hydrochloride (140 mg), 3-chloro-6-(5-(((4-methoxybenzyl)oxy)methyl)-1,3,4-oxadiazol-2-yl)pyridazine (100 mg), DIPEA (0.3 mL) and IPA (4 mL) was stirred at 150°C for 1 hour under irradiation with microwave. After cooling to room temperature, the deposit was collected by filtration and washed with IPA to obtain the title compound (143 mg).
$^{1}$H NMR (300 MHz, DMSO-d6) δ 1.28 (9H, s), 2.01-2.38 (4H, m), 3.21-3.41 (2H, m), 3.74 (3H, s), 4.57 (2H, s), 4.70-4.89 (4H, m), 6.88-6.97 (2H, m), 7.27-7.35 (2H, m), 7.43-7.59 (5H, m), 8.00-8.10 (1H, m).

B) 4-(4-tert-Butylphenyl)-1-(6-(5-(hydroxymethyl)-1,3,4-oxadiazol-2-yl)pyridazin-3-yl)piperidine-4-carbonitrile

In a nitrogen atmosphere, a mixture of 4-(4-tert-butylphenyl)-1-(6-(5-(((4-methoxybenzyl)oxy)methyl)-1,3,4-oxadiazol-2-yl)pyridazin-3-yl)piperidine-4-carbonitrile (143 mg), TFA (3 mL) and anisole (1 mL) was stirred at room temperature for 1 hour. The reaction mixture was concentrated under reduced pressure. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (90 mg).
$^{1}$H NMR (300 MHz, DMSO-d6) δ 1.28 (9H, s), 2.05-2.20 (2H, m), 2.25-2.36 (2H, m), 3.24-3.40 (2H, m), 4.66-4.86 (4H, m), 5.95-6.05 (1H, m), 7.41-7.61 (5H, m), 7.98-8.09 (1H, m).

Example 90

4-(2-Chlorobenzyl)-1-(6-(5-(hydroxymethyl)-1,3,4-thiadiazol-2-yl)pyridazin-3-yl)piperidine-4-carbonitrile

A) Ethyl ((4-methoxybenzyl)oxy)acetate

To a suspension of 60% (w/w) sodium hydride in oil (8.07 g) in dehydrated THF (200 mL), ethyl 2-hydroxyacetate (20.00 g) was added at 0°C. After stirring for 30 minutes, 1-(chloromethyl)-4-methoxybenzene (26.2 mL) and TBAI (7.10 g) were added thereto at 0°C. In a dry atmosphere, the mixture was stirred overnight at room temperature. The mixture was poured to a saturated aqueous solution of ammonium chloride at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (34.1 g). MS, found: 247.

B) 2-((4-Methoxybenzyl)oxy)acetohydrazide

To a solution of ethyl ((4-methoxybenzyl)oxy)acetate (34.1 g, 152.06 mmol) in ethanol (200 mL), hydrazine monohydrate (37.0 mL) was added at room temperature. In a nitrogen atmosphere, the mixture was heated at 80°C for 2 hours. The mixture was poured to water, followed by extraction with THF-ethyl acetate. The organic layer was washed with water and saturated brine, dried over magnesium sulfate and then concentrated to obtain the title compound (24.90 g). MS, found: 232.9.

C) Methyl 6-(4-(2-chlorobenzyl)-4-cyanopiperidin-1-yl)pyridazine-3-carboxylate

A mixture of 4-(2-chlorobenzyl)piperidine-4-carbonitrile hydrochloride (2.16 g), methyl 6-chloropyridazine-3-carboxylate (1.649 g), potassium carbonate (5.53 g), TBAI (0.600 g) and THF (40 mL) was stirred overnight at 80°C. The mixture was poured to water (100 mL) at room temperature, followed by extraction with ethyl acetate-THF (200 mL). The organic layer was washed with saturated brine, dried over magnesium sulfate and then concentrated. The residue was crystallized from ethyl acetate-heptane to obtain the title compound (1.929 g, 5.20 mmol).
$^1$H NMR (300 MHz, DMSO-d6) $\delta$ 1.74-1.88 (2H, m), 1.93-2.04 (2H, m), 3.05-3.17 (4H, m), 3.88 (3H, s), 4.58-4.71 (2H, m), 7.30-7.41 (3H, m), 7.46-7.54 (2H, m), 7.87 (1H, d, J = 9.6 Hz).

D) 6-(4-(2-Chlorobenzyl)-4-cyanopiperidin-1-yl)pyridazine-3-carboxylic acid

To a solution of methyl 6-(4-(2-chlorobenzyl)-4-cyanopiperidin-1-yl)pyridazine-3-carboxylate (1.89 g) in THF (15 mL) and methanol (15 mL), sodium hydroxide (2 N aqueous solution, 7.64 mL) was added at room temperature. The mixture was stirred at room temperature for 2 hours. The mixture was neutralized by the addition of hydrogen chloride (2 N aqueous solution, 7.5 mL) and a saturated aqueous solution of ammonium chloride (100 mL). The deposit was collected by filtration and then washed with ethyl acetate to obtain the title compound (1.780 g).
$^1$H NMR (300 MHz, DMSO-d6) $\delta$ 1.73-1.89 (2H, m), 1.92-2.07 (2H, m), 3.03-3.20 (4H, m), 4.56-4.72 (2H, m), 7.26-7.59 (5H, m), 7.85 (1H, d, J = 9.6 Hz), 13.11 (1H, brs).

E) 4-(2-Chlorobenzyl)-1-(6-(5-(((4-methoxybenzyl)oxy)methyl)-1,3,4-thiadiazol-2-yl)pyridazin-3-yl)piperidine-4-carbonitrile

To a solution of 6-(4-(2-chlorobenzyl)-4-cyanopiperidin-1-yl)pyridazine-3-carboxylic acid (150 mg) and DMF (3.25 $\mu$L) in dehydrated THF (10 mL), oxalyl chloride (0.073 mL) was added at room temperature. In a nitrogen atmosphere, the mixture was stirred at 50°C for 2 hours. The reaction mixture was concentrated under reduced pressure to obtain 6-(4-(2-chlorobenzyl)-4-cyanopiperidin-1-yl)pyridazine-3-carbonyl chloride (158 mg).
To a solution of 6-(4-(2-chlorobenzyl)-4-cyanopiperidin-1-yl)pyridazine-3-carbonyl chloride (158 mg) in DMA (5 mL), a solution of 2-((4-methoxybenzyl)oxy)acetohydrazide (132 mg, 0.63 mmol) in DMA (5 mL) and DIPEA (0.219 mL) was added at 0°C. In a nitrogen atmosphere, the mixture was stirred at room temperature for 1 hour. The mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (ethyl acetate/hexane) to obtain 6-(4-(2-chlorobenzyl)-4-cyanopiperidin-1-yl)-N'-(((4-methoxybenzyl)oxy)acetyl)pyridazine-3-carbohydrazide (220 mg).
In a nitrogen atmosphere, a mixture of 6-(4-(2-chlorobenzyl)-4-cyanopiperidin-1-yl)-N'-(((4-methoxybenzyl)oxy)acetyl)pyridazine-3-carbohydrazide (220 mg), a Lawesson's reagent (243 mg) and dehydrated THF (5 mL) was

stirred at 55°C over weekend. The reaction mixture was concentrated under reduced pressure. The residue was purified by column chromatography (NH, ethyl acetate/hexane) to obtain the title compound (150 mg). $^1$H NMR (300 MHz, CDCl3) δ 1.73-1.94 (2H, m), 1.99-2.14 (2H, m), 3.15 (2H, s), 3.26-3.44 (2H, m), 3.81 (3H, s), 4.54-4.71 (4H, m), 4.94 (2H, s), 6.86-6.96 (2H, m), 7.00-7.08 (1H, m), 7.23-7.36 (4H, m), 7.38-7.45 (1H, m), 7.47-7.55 (1H, m), 8.15-8.25 (1H, m).

F) 4-(2-Chlorobenzyl)-1-(6-(5-(hydroxymethyl)-1,3,4-thiadiazol-2-yl)pyridazin-3-yl)piperidine-4-carbonitrile

In a nitrogen atmosphere, a mixture of 4-(2-chlorobenzyl)-1-(6-(5-(((4-methoxybenzyl)oxy)methyl)-1,3,4-thiadiazol-2-yl)pyridazin-3-yl)piperidine-4-carbonitrile (150 mg), TFA (21.12 μL) and anisole (29.8 μL) was stirred at room temperature for 30 minutes. The reaction mixture was concentrated under reduced pressure. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (88 mg).
$^1$H NMR (300 MHz, DMSO-d6) δ 1.61-2.14 (4H, m), 2.94-3.25 (4H, m), 4.49-4.70 (2H, m), 4.79-5.01 (2H, m), 6.17-6.42 (1H, m), 7.30-7.42 (2H, m), 7.46-7.58 (3H, m), 8.06-8.19 (1H, m).

Example 92

(5-(6-(4-Methoxy-4-(4-(pentafluorosulfanyl)phenyl)piperidin-1-yl)pyridazin-3-yl)-1,2,4-oxadiazol-3-yl)methanol

A) tert-Butyl4-hydroxy-4-(4-(pentafluorosulfanyl)phenyl)piperidine-1-carboxylate

To a solution of 1-bromo-4-(pentafluorosulfanyl)benzene (3.6 g) in dehydrated THF (30 mL), an isopropyl magnesium chloride-lithium chloride complex (1 M solution in THF, 17.34 mL) was added at -15°C. After stirring at room temperature for 30 minutes, a solution of tert-butyl 4-oxopiperidine-1-carboxylate (2.304 g) in dehydrated THF (20 mL) was added thereto. In a nitrogen atmosphere, the mixture was stirred at 0°C for 1 hour. The mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (NH, ethyl acetate/hexane) to obtain the title compound (670 mg).
$^1$H NMR (300 MHz, CDCl3) δ 1.47 (9H, s), 1.66-1.78 (2H, m), 1.87-2.10 (3H, m), 3.02-3.41 (2H, m), 3.96-4.17 (2H, m), 7.51-7.63 (2H, m), 7.67-7.81 (2H, m).

B) tert-Butyl4-methoxy-4-(4-(pentafluorosulfanyl)phenyl)piperidine-1-carboxylate

To a solution of tert-butyl 4-hydroxy-4-(4-(pentafluorosulfanyl)phenyl)piperidine-1-carboxylate (400 mg) in dehydrated DMF (3 mL), 60% (w/w) sodium hydride in oil (59.5 mg) was added at 0°C. After stirring at room temperature for 10 minutes, methyl iodide (123 μL) was added to the mixture. In a nitrogen atmosphere, the mixture was stirred at room temperature for 30 minutes. The mixture was poured to a saturated aqueous solution of ammonium chloride at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (400 mg).
$^1$H NMR (300 MHz, CDCl3) δ 1.47 (9H, s), 1.72-1.90 (2H, m), 1.93-2.03 (2H, m), 3.01 (3H, s), 3.08-3.26 (2H, m), 3.87-4.13 (2H, m), 7.40-7.54 (2H, m), 7.70-7.82 (2H, m).

C) 4-Methoxy-4-(4-(pentafluorosulfanyl)phenyl)piperidine hydrochloride

In a nitrogen atmosphere, a mixture of tert-butyl 4-methoxy-4-(4-(pentafluorosulfanyl)phenyl)piperidine-1-carboxylate (400 mg), hydrogen chloride (4 N solution in CPME, 0.5 mL) and ethyl acetate (3 mL) was stirred overnight at 50°C. The deposit was collected by filtration to obtain the title compound (300 mg).
$^1$H NMR (300 MHz, DMSO-d6) δ 2.06-2.25 (4H, m), 2.94 (3H, s), 3.00-3.14 (2H, m), 3.17-3.29 (2H, m), 7.54-7.66 (2H, m), 7.89-8.04 (2H, m), 9.10 (2H, brs).

D) (5-(6-(4-Methoxy-4-(4-(pentafluorosulfanyl)phenyl)piperidin-1-yl)pyridazin-3-yl)-1,2,4-oxadiazol-3-yl)methyl benzoate

A mixture of 4-methoxy-4-(4-(pentafluorosulfanyl)phenyl)piperidine hydrochloride (110 mg), (5-(6-chloropyridazin-3-yl)-1,2,4-oxadiazol-3-yl)methyl benzoate (80 mg), DIPEA (0.2 mL) and IPA (4 mL) was stirred at 150°C for 1 hour under irradiation with microwave. After cooling to room temperature, the deposit was collected by filtration and

washed with IPA to obtain the title compound (138 mg).
$^1$H NMR (300 MHz, DMSO-d6) δ 1.91-2.07 (2H, m), 2.09-2.20 (2H, m), 3.02 (3H, s), 3.33-3.47 (2H, m), 4.43-4.61 (2H, m), 5.61 (2H, s), 7.42-7.50 (1H, m), 7.54-7.61 (2H, m), 7.63-7.76 (3H, m), 7.89-7.95 (2H, m), 7.99-8.07 (3H, m).

E) (5-(6-(4-Methoxy-4-(4-(pentafluorosulfanyl)phenyl)piperidin-1-yl)pyridazin-3-yl)-1,2,4-oxadiazol-3-yl)methanol

In a nitrogen atmosphere, a mixture of (5-(6-(4-methoxy-4-(4-(pentafluorosulfanyl)phenyl)piperidin-1-yl)pyridazin-3-yl)-1,2,4-oxadiazol-3-yl)methyl benzoate (138 mg), sodium hydroxide (1 N aqueous solution, 0.231 mL), methanol (5 mL) and THF (5 mL) was stirred at room temperature for 20 minutes. Hydrogen chloride (IN aqueous solution, 3 mL) was added thereto at 0°C to adjust the pH to 2 to 3. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (93 mg).
$^1$H NMR (300 MHz, DMSO-d6) δ 1.92-2.07 (2H, m), 2.09-2.22 (2H, m), 3.02 (3H, s), 3.33-3.46 (2H, m), 4.43-4.58 (2H, m), 4.60-4.68 (2H, m), 5.73-5.81 (1H, m), 7.48 (1H, d, J = 9.8 Hz), 7.66 (2H, d, J = 9.0 Hz), 7.92 (2H, d, J = 9.0 Hz), 8.02 (1H, d, J = 9.8 Hz).

Example 93

(5-(6-(4-Methoxy-4-(4-(pentafluorosulfanyl)phenyl)piperidin-1-yl)pyridazin-3-yl)-1,3,4-oxadiazol-2-yl)methanol

A mixture of 4-methoxy-4-(4-(pentafluorosulfanyl)phenyl)piperidine hydrochloride (110 mg), 3-chloro-6-(5-(((4-methoxybenzyl)oxy)methyl)-1,3,4-oxadiazol-2-yl)pyridazine (80 mg), DIPEA (0.3 mL) and IPA (4 mL) was stirred at 150°C for 1 hour under irradiation with microwave. After cooling to room temperature, the deposit was collected by filtration and washed with IPA to obtain 3-(5-(((4-methoxybenzyl)oxy)methyl)-1,3,4-oxadiazol-2-yl)-6-(4-methoxy-4-(4-(pentafluorosulfanyl)phenyl)piperidin-1-yl)pyridazine (131 mg).

In a nitrogen atmosphere, a mixture of 3-(5-(((4-methoxybenzyl)oxy)methyl)-1,3,4-oxadiazol-2-yl)-6-(4-methoxy-4-(4-(pentafluorosulfanyl)phenyl)piperidin-1-yl)pyridazine (131 mg), TFA (3 mL) and anisole (1 mL) was stirred at room temperature for 1 hour. The reaction mixture was concentrated under reduced pressure. The residue was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (48.5 mg).
$^1$H NMR (300 MHz, DMSO-d6) δ 1.89-2.24 (4H, m), 3.02 (3H, s), 3.19-3.50 (2H, m), 4.41-4.60 (2H, m), 4.74 (2H, s), 5.88-6.11 (1H, m), 7.49 (1H, d, J = 9.7 Hz), 7.66 (2H, d, J = 8.7 Hz), 7.92 (2H, d, J = 8.7 Hz), 8.00 (1H, d, J = 9.7 Hz).

Example 94

4-(2-Chlorobenzyl)-1-(6-(5-(hydroxymethyl)-4-methyl-1,3-thiazol-2-yl)pyridazin-3-yl)piperidine-4-carbonitrile

A) 6-(4-(2-Chlorobenzyl)-4-cyanopiperidin-1-yl)pyridazine-3-carboxamide

To a solution of 6-(4-(2-chlorobenzyl)-4-cyanopiperidin-1-yl)pyridazine-3-carboxylic acid (250 mg) and DMF (5.42 μL) in dehydrated THF (10 mL), oxalyl chloride (0.122 mL) was added at room temperature. In a nitrogen atmosphere, the mixture was stirred at 50°C for 2 hours. The reaction mixture was concentrated under reduced pressure to obtain 6-(4-(2-chlorobenzyl)-4-cyanopiperidin-1-yl)pyridazine-3-carbonyl chloride (263 mg).

To a solution of ammonia (26% aqueous solution, 0.3 mL) in DMA (3.00 mL), a solution of 6-(4-(2-chlorobenzyl)-4-cyanopiperidin-1-yl)pyridazine-3-carbonyl chloride (263 mg) in DMA (3 mL) was added at 0°C. In a nitrogen atmosphere, the mixture was stirred at room temperature for 1 hour. The reaction mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (235 mg). $^1$H NMR (300 MHz, DMSO-d6) δ 1.70-2.07 (4H, m), 3.00-3.19 (4H, m), 4.52-4.67 (2H, m), 7.30-7.60 (6H, m), 7.81-7.91 (1H, m), 8.14 (1H, brs).

B) 6-(4-(2-Chlorobenzyl)-4-cyanopiperidin-1-yl)pyridazine-3-carbothioamide

A solution of 6-(4-(2-chlorobenzyl)-4-cyanopiperidin-1-yl)pyridazine-3-carboxamide (235 mg) and a Lawesson's reagent (160 mg) in dehydrated THF (3 mL) was heated to reflux overnight. The reaction mixture was concentrated under reduced pressure. The residue was purified by column chromatography (NH, ethyl acetate/hexane) to obtain the

title compound (200 mg).
$^1$H NMR (300 MHz, CDCl3) δ 1.77-1.90 (2H, m), 2.03-2.12 (2H, m), 3.15 (2H, s), 3.26-3.44 (2H, m), 4.55-4.68 (2H, m), 6.92-7.02 (1H, m), 7.22-7.36 (2H, m), 7.38-7.57 (3H, m), 8.47-8.60 (1H, m), 9.17-9.37 (1H, m).

C) Ethyl 2-(6-(4-(2-chlorobenzyl)-4-cyanopiperidin-1-yl)pyridazin-3-yl)-4-methyl-1,3-thiazole-5-carboxylate

A solution of 6-(4-(2-chlorobenzyl)-4-cyanopiperidin-1-yl)pyridazine-3-carbothioamide (200 mg) and ethyl 2-chloroacetoacetate (0.090 mL) in dehydrated THF (3 mL) was heated to reflux over weekend. The deposit was collected by filtration to obtain the title compound (120 mg).
MS: $[M+H]^+$ 482.0.

D) 4-(2-Chlorobenzyl)-1-(6-(5-(hydroxymethyl)-4-methyl-1,3-thiazol-2-yl)pyridazin-3-yl)piperidine-4-carbonitrile

To a solution of ethyl 2-(6-(4-(2-chlorobenzyl)-4-cyanopiperidin-1-yl)pyridazin-3-yl)-4-methyl-1,3-thiazole-5-carboxylate(120 mg) in dehydrated THF (30 mL), lithium aluminum hydride (20 mg) was added at 0°C. In a nitrogen atmosphere, the mixture was stirred at room temperature for 1 hour. The reaction was terminated by the addition of water (50 μL). The deposit was filtered off, and the filtrate was concentrated under reduced pressure. The residue was purified by column chromatography (NH, ethyl acetate/hexane) to obtain the title compound (25 mg).
$^1$H NMR (300 MHz, DMSO-d6) δ 1.75-1.90 (2H, m), 1.92-2.03 (2H, m), 2.35 (3H, s), 3.00-3.21 (4H, m), 4.50-4.72 (4H, m), 5.49-5.59 (1H, m), 7.31-7.55 (5H, m), 7.93-8.00 (1H, m).

Example 95

4-(2-Chlorobenzyl)-1-(6-(5-(hydroxymethyl)-1,2,4-oxadiazol-3-yl)pyridazin-3-yl)piperidine-4-carbonitrile

In a nitrogen atmosphere, to a solution of (3-(6-(4-(2-chlorobenzyl)-4-cyanopiperidin-1-yl)pyridazin-3-yl)-1,2,4-oxadiazol-5-yl)methyl acetate (221 mg) in methanol (3 mL) and THF (3 mL), sodium hydroxide (IN aqueous solution, 1 mL) was added at room temperature. The mixture was stirred at room temperature for 10 minutes. The reaction solution was neutralized by the addition of hydrogen chloride (IN aqueous solution) at 0°C, followed by extraction with ethyl acetate. The organic layer was washed with water and saturated brine, dried over magnesium sulfate and then concentrated. The residue was crystallized from IPE to obtain the title compound (180 mg).
$^1$H NMR (300 MHz, DMSO-d6) δ 1.77-1.90 (2H, m), 1.94-2.07 (2H, m), 3.02-3.22 (4H, m), 4.55-4.70 (2H, m), 4.76-4.90 (2H, m), 6.00-6.17 (1H, m), 7.31-7.56 (5H, m), 7.85-8.02 (1H, m).

Example 96

(3-(6-(4-(2-Chlorobenzyl)-4-cyanopiperidin-1-yl)pyridazin-3-yl)-1,2,4-oxadiazol-5-yl)methyl acetate

A) 6-(4-(2-Chlorobenzyl)-4-cyanopiperidin-1-yl)pyridazine-3-carbonitrile

In a nitrogen atmosphere, a mixture of 6-chloropyridazine-3-carbonitrile (300 mg), 4-(2-chlorobenzyl)piperidine-4-carbonitrile hydrochloride (700 mg), potassium carbonate (743 mg), TBAI (39.7 mg) and dehydrated THF (15 mL) was stirred overnight at room temperature. The mixture was poured to water at room temperature, followed by extraction with ethyl acetate. The organic layer was washed with water and saturated brine, dried over magnesium sulfate and then concentrated. The residue was purified by column chromatography (ethyl acetate/hexane) to obtain 6-(4-(2-chlorobenzyl)-4-cyanopiperidin-1-yl)pyridazine-3-carbonitrile (420 mg).
$^1$H NMR (300 MHz, DMSO-d6) δ 1.71-2.10 (4H, m), 3.03-3.22 (4H, m), 4.56-4.72 (2H, m), 7.30-7.57 (5H, m), 7.85-7.93 (1H, m).

B) 6-(4-(2-Chlorobenzyl)-4-cyanopiperidin-1-yl)-N-hydroxypyridazine-3-carboximidamide

A solution of 6-(4-(2-chlorobenzyl)-4-cyanopiperidin-1-yl)pyridazine-3-carbonitrile (420 mg) and hydroxylamine (50% aqueous solution, 50 μL) in ethanol (20 mL) was heated to reflux for 3 hours. After cooling to room temperature, the deposit was collected by filtration to obtain the title compound (340 mg).
$^1$HNMR (300 MHz, DMSO-d6) δ 1.72-2.05 (4H, m), 2.96-3.11 (2H, m), 3.15 (2H, s), 4.41-4.58 (2H, m), 5.88 (2H, s), 7.28-7.43 (3H, m), 7.44-7.56 (2H, m), 7.68-7.78 (1H, m), 9.90 (1H, s).

C) (3-(6-(4-(2-Chlorobenzyl)-4-cyanopiperidin-1-yl)pyridazin-3-yl)-1,2,4-oxadiazol-5-yl)methyl acetate

To a solution of 6-(4-(2-chlorobenzyl)-4-cyanopiperidin-1-yl)-N-hydroxypyridazine-3-carboximidamide (340 mg) in Py (10 mL), 2-acetoxyacetyl chloride (0.119 mL) was added at room temperature. In a nitrogen atmosphere, the mixture was stirred at room temperature for 2 hours and then stirred overnight at 80°C. The reaction mixture was concentrated under reduced pressure. The residue was purified by column chromatography (ethyl acetate/hexane) to obtain the title compound (221 mg).
$^1$H NMR (300 MHz, DMSO-d6) $\delta$ 1.75-1.92 (2H, m), 1.94-2.07 (2H, m), 2.17 (3H, s), 3.16 (4H, s), 4.54-4.72 (2H, m), 5.48 (2H, s), 7.28-7.58 (5H, m), 7.87-7.99 (1H, m).

Example 97

4-(2-Bromobenzyl)-1-(6-(5-(hydroxymethyl)-1,3,4-oxadiazol-2-yl)pyridazin-3-yl)piperidine-4-carbonitrile

A) tert-Butyl 4-(2-bromobenzyl)-4-cyanopiperidine-1-carboxylate

To a solution of DIPA (8 mL) in dehydrated THF (100 mL), n-butyllithium (1.6 M solution in hexane, 34.2 mL) was added at 0°C. After stirring at 0°C for 30 minutes, a solution of tert-butyl 4-cyanopiperidine-1-carboxylate (10 g) in dehydrated THF (30 mL) was added to the reaction solution. In an argon atmosphere, the mixture was stirred at 0°C for 1 hour. 1-Bromo-2-(bromoethyl)benzene (13.07 g) was added to the reaction solution at -78°C. The temperature of the mixture was raised to room temperature, and the mixture was stirred at room temperature. After the completion of the reaction, the mixture was poured to a saturated aqueous solution of ammonium chloride (100 mL) at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (18.0 g).
$^1$H NMR (300 MHz, CDCl3) $\delta$ 1.46 (9H, s), 1.60-1.76 (2H, m), 1.81-1.93 (2H, m), 2.85-3.07 (2H, m), 3.13 (2H, s), 3.96-4.33 (2H, m), 7.12-7.20 (1H, m), 7.28-7.36 (1H, m), 7.47-7.53 (1H, m), 7.57-7.62 (1H, m).

B) 4-(2-Bromobenzyl)piperidine-4-carbonitrile hydrochloride

In a nitrogen atmosphere, a mixture of tert-butyl 4-(2-bromobenzyl)-4-cyanopiperidine-1-carboxylate (18.0 g), hydrogen chloride (4 N solution in CPME, 27 mL) and ethyl acetate was stirred overnight at 50°C. The deposit was collected by filtration to obtain the title compound (14.0 g).
$^1$H NMR (300 MHz, DMSO-d6) $\delta$ 1.95-2.18 (4H, m), 2.72-2.98 (2H, m), 3.18 (2H, s), 3.28-3.46 (2H, m), 7.21-7.33 (1H, m), 7.36-7.57 (2H, m), 7.61-7.75 (1H, m), 9.40 (2H, brs).

C) 4-(2-Bromobenzyl)-1-(6-(5-(((4-methoxybenzyl)oxy)methyl)-1,3,4-oxadiazol-2-yl)pyridazin-3-yl)piperidine-4-carbonitrile

A mixture of 4-(2-bromobenzyl)piperidine-4-carbonitrile hydrochloride (200 mg), 3-chloro-6-(5-(((4-methoxybenzyl)oxy)methyl)-1,3,4-oxadiazol-2-yl)pyridazine (150 mg), DIPEA (0.314 mL) and IPA (2 mL) was stirred at 150°C for 1 hour under irradiation with microwave. The reaction mixture was concentrated under reduced pressure. The residue was purified by column chromatography (ethyl acetate/hexane) to obtain the title compound (255 mg).
MS: $[M+H]^+$ 577.0.

D) 4-(2-Bromobenzyl)-1-(6-(5-(hydroxymethyl)-1,3,4-oxadiazol-2-yl)pyridazin-3-yl)piperidine-4-carbonitrile

In a nitrogen atmosphere, a mixture of 4-(2-bromobenzyl)-1-(6-(5-(((4-methoxybenzyl)oxy)methyl)-1,3,4-oxadiazol-2-yl)pyridazin-3-yl)piperidine-4-carbonitrile (255 mg), TFA (5 mL) and anisole (200 mg) was stirred at room temperature for 1 hour. The reaction mixture was concentrated under reduced pressure. The residue was poured to a saturated aqueous solution of sodium bicarbonate at room temperature for neutralization, followed by extraction with ethyl acetate. The organic layer was washed with water and saturated brine, dried over magnesium sulfate and then concentrated. The residue was purified by column chromatography (ethyl acetate/hexane) to obtain the title compound (100 mg). $^1$H NMR (300 MHz, DMSO-d6) $\delta$ 1.77-1.93 (2H, m), 1.95-2.08 (2H, m), 3.01-3.23 (4H, m), 4.58-4.70 (2H, m), 4.71-4.80 (2H, m), 5.94-6.04 (1H, m), 7.21-7.32 (1H, m), 7.36-7.55 (3H, m), 7.63-7.73 (1H, m), 7.98-8.07 (1H, m).

Example 98

1-(6-(5-(Hydroxymethyl)-1,3,4-oxadiazol-2-yl)pyridazin-3-yl)-4-(2-(trifluoromethyl)benzyl)piperidine-4-carbonitrile

A) 4-(2-(Trifluoromethyl)benzyl)piperidine-4-carbonitrile hydrochloride

To a solution of DIPA (8 mL) in dehydrated THF (100 mL), n-butyllithium (1.6 M solution in hexane, 34.2 mL) was added at 0°C. After stirring at 0°C for 30 minutes, a solution of tert-butyl 4-cyanopiperidine-1-carboxylate (10 g) in dehydrated THF (30 mL) was added to the reaction solution. In an argon atmosphere, the mixture was stirred at 0°C for 1 hour. 1-(Bromomethyl)-2-(trifluoromethyl)benzene (12.50 g) was added to the reaction solution at - 78°C. The temperature of the mixture was raised to room temperature, and the mixture was stirred at room temperature. The completion of the reaction was confirmed by TLC. The mixture was poured to a saturated aqueous solution of ammonium chloride (100 mL) at room temperature, followed by extraction with ethyl acetate. The organic layer was washed with water and saturated brine, dried over magnesium sulfate and then concentrated. The residue was purified by column chromatography (ethyl acetate/hexane) to obtain tert-butyl 4-cyano-4-(2-(trifluoromethyl)benzyl)piperidine-1-carboxylate (17.2 g).
In a nitrogen atmosphere, a mixture of tert-butyl 4-cyano-4-(2-(trifluoromethyl)benzyl)piperidine-1-carboxylate (17.2 g), hydrogen chloride (4 N solution in CPME, 11.67 mL) and ethyl acetate was stirred overnight at 50°C. The deposit was collected by filtration to obtain the title compound (13.5 g).
MS, found: 268.9.

B) 1-(6-(5-(((4-Methoxybenzyl)oxy)methyl)-1,3,4-oxadiazol-2-yl)pyridazin-3-yl)-4-(2-(trifluoromethyl)benzyl)piperidine-4-carbonitrile

A mixture of 4-(2-(trifluoromethyl)benzyl)piperidine-4-carbonitrile hydrochloride (200 mg), 3-chloro-6-(5-(((4-methoxybenzyl)oxy)methyl)-1,3,4-oxadiazol-2-yl)pyridazine (150 mg), DIPEA (0.314 mL) and IPA (2 mL) was stirred at 150°C for 1 hour under irradiation with microwave. The reaction mixture was concentrated under reduced pressure. The residue was purified by column chromatography (ethyl acetate/hexane) to obtain the title compound (250 mg).
MS: $[M+H]^+$ 565.2.

C) 1-(6-(5-(Hydroxymethyl)-1,3,4-oxadiazol-2-yl)pyridazin-3-yl)-4-(2-(trifluoromethyl)benzyl)piperidine-4-carbonitrile

In a nitrogen atmosphere, a mixture of 1-(6-(5-(((4-methoxybenzyl)oxy)methyl)-1,3,4-oxadiazol-2-yl)pyridazin-3-yl)-4-(2-(trifluoromethyl)benzyl)piperidine-4-carbonitrile (250 mg), TFA (5 mL) and anisole (200 mg) was stirred at room temperature for 1 hour. The reaction mixture was concentrated under reduced pressure. The residue was poured to a saturated aqueous solution of sodium bicarbonate at room temperature for neutralization, followed by extraction with ethyl acetate. The organic layer was washed with water and saturated brine, dried over magnesium sulfate and then concentrated. The residue was purified by column chromatography (ethyl acetate/hexane) to obtain the title compound (85 mg). $^1$HNMR (300 MHz, DMSO-d6) $\delta$ 1.72-1.92 (2H, m), 1.96-2.11 (2H, m), 3.05-3.23 (4H, m), 4.58-4.70 (2H, m), 4.72-4.77 (2H, m), 5.93-6.05 (1H, m), 7.44-7.61 (2H, m), 7.66-7.84 (3H, m), 8.00-8.08 (1H, m).

Example 99

4-(2-Bromobenzyl)-1-(6-(3-(hydroxymethyl)-1,2,4-oxadiazol-5-yl)pyridazin-3-yl)piperidine-4-carbonitrile

A) (5-(6-(4-(2-Bromobenzyl)-4-cyanopiperidin-1-yl)pyridazin-3-yl)-1,2,4-oxadiazol-3-yl)methyl benzoate

A mixture of 4-(2-bromobenzyl)piperidine-4-carbonitrile hydrochloride (250 mg), (5-(6-chloropyridazin-3-yl)-1,2,4-oxadiazol-3-yl)methyl benzoate (200 mg), DIPEA (0.330 mL) and IPA (3 mL) was stirred at 150°C for 1 hour under irradiation with microwave. After cooling to room temperature, the deposit was collected by filtration to obtain the title compound (320 mg).
$^1$HNMR (300 MHz, DMSO-d6) $\delta$ 1.77-1.93 (2H, m), 1.96-2.10 (2H, m), 3.07-3.25 (4H, m), 4.58-4.79 (2H, m), 5.61 (2H, s), 7.21-7.33 (1H, m), 7.37-7.61 (5H, m), 7.64-7.78 (2H, m), 7.98-8.13 (3H, m).

B) 4-(2-Bromobenzyl)-1-(6-(3-(hydroxymethyl)-1,2,4-oxadiazol-5-yl)pyridazin-3-yl)piperidine-4-carbonitrile

In a nitrogen atmosphere, a mixture of (5-(6-(4-(2-bromobenzyl)-4-cyanopiperidin-1-yl)pyridazin-3-yl)-1,2,4-oxadiazol-3-yl)methyl benzoate (320 mg), sodium hydroxide (1 N aqueous solution, 0.572 mL), methanol (10 mL) and THF (10 mL) was stirred at room temperature for 20 minutes. Hydrogen chloride (IN aqueous solution, 3 mL) was added

thereto at 0°C to adjust the pH to 2 to 3. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (120 mg).
$^1$H NMR (300 MHz, DMSO-d6) δ 1.78-1.94 (2H, m), 1.97-2.09 (2H, m), 3.07-3.24 (4H, m), 4.59-4.76 (4H, m), 5.70-5.83 (1H, m), 7.21-7.33 (1H, m), 7.38-7.57 (3H, m), 7.63-7.73 (1H, m), 7.98-8.11 (1H, m).

Example 100

1-(6-(3-(Hydroxymethyl)-1,2,4-oxadiazol-5-yl)pyridazin-3-yl)-4-(2-(trifluoromethyl)benzyl)piperidine-4-carbonitrile

A) (5-(6-(4-Cyano-4-(2-(trifluoromethyl)benzyl)piperidin-1-yl)pyridazin-3-yl)-1,2,4-oxadiazol-3-yl)methyl benzoate

A mixture of 4-(2-(trifluoromethyl)benzyl)piperidine-4-carbonitrile hydrochloride (250 mg), (5-(6-chloropyridazin-3-yl)-1,2,4-oxadiazol-3-yl)methyl benzoate (200 mg), DIPEA (0.330 mL) and IPA (3 mL) was stirred at 150°C for 1 hour under irradiation with microwave. After cooling to room temperature, the deposit was collected by filtration to obtain the title compound (345 mg).
$^1$H NMR (300 MHz, DMSO-d6) δ 1.74-1.92 (2H, m), 1.98-2.10 (2H, m), 3.08-3.24 (4H, m), 4.61-4.77 (2H, m), 5.61 (2H, s), 7.44-7.62 (4H, m), 7.66-7.82 (4H, m), 8.00-8.11 (3H, m).

B) 1-(6-(3-(Hydroxymethyl)-1,2,4-oxadiazol-5-yl)pyridazin-3-yl)-4-(2-(trifluoromethyl)benzyl)piperidine-4-carbonitrile

In a nitrogen atmosphere, a mixture of (5-(6-(4-cyano-4-(2-(trifluoromethyl)benzyl)piperidin-1-yl)pyridazin-3-yl)-1,2,4-oxadiazol-3-yl)methyl benzoate (345 mg), sodium hydroxide (1 N aqueous solution, 0.629 mL), methanol (10 mL) and THF (10 mL) was stirred at room temperature for 20 minutes. Hydrogen chloride (1 N aqueous solution, 3 mL) was added thereto at 0°C to adjust the pH to 2 to 3. The mixture was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (200 mg).
$^1$H NMR (300 MHz, DMSO-d6) δ 1.76-1.93 (2H, m), 1.99-2.10 (2H, m), 3.09-3.24 (4H, m), 4.59-4.78 (4H, m), 5.73-5.82 (1H, m), 7.45-7.62 (2H, m), 7.67-7.83 (3H, m), 8.01-8.10 (1H, m).

Example 101

1-(6-(5-(Hydroxymethyl)-1,3,4-oxadiazol-2-yl)pyridazin-3-yl)-4-(2-(pentafluorosulfanyl)benzyl)piperidine-4-carbonitrile

A) tert-Butyl 4-cyano-4-(2-(pentafluorosulfanyl)benzyl)piperidine-1-carboxylate

To a solution of DIPA (0.467 mL) in dehydrated THF (100 mL), n-butyllithium (1.6 M solution in hexane, 1.783 mL) was added at 0°C. After stirring at 0°C for 30 minutes, a solution of tert-butyl 4-cyanopiperidine-1-carboxylate (500 mg) in dehydrated THF (30 mL) was added to the reaction solution. In an argon atmosphere, the mixture was stirred at 0°C for 1 hour. 2-(Pentafluorosulfur)benzyl bromide (706 mg) was added to the reaction solution at-78°C. The temperature of the mixture was raised to room temperature, and the mixture was stirred at room temperature. The completion of the reaction was confirmed by TLC. The mixture was poured to a saturated aqueous solution of ammonium chloride (100 mL) at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (350 mg).
$^1$H NMR (300 MHz, CDCl3) δ 1.41-1.54 (11H, m), 1.76-1.96 (2H, m), 2.92 (4H, s), 3.97-4.39 (2H, m), 7.43-7.52 (2H, m), 7.61-7.66 (1H, m), 7.69-7.76 (1H, m).

B) 4-(2-(Pentafluorosulfanyl)benzyl)piperidine-4-carbonitrile hydrochloride

In a nitrogen atmosphere, a mixture of tert-butyl 4-cyano-4-(2-(pentafluorosulfanyl)benzyl)piperidine-1-carboxylate (350 mg), hydrogen chloride (4 N solution in CPME, 205 μL) and ethyl acetate was stirred overnight at 50°C. The reaction mixture was concentrated to obtain the title compound (298 mg).
MS, found: 326.9.

C) 1-(6-(5-(((4-Methoxybenzyl)oxy)methyl)-1,3,4-oxadiazol-2-yl)pyridazin-3-yl)-4-(2-(pentafluorosulfanyl)benzyl)piperidine-4-carbonitrile

A mixture of 4-(2-(pentafluorosulfanyl)benzyl)piperidine-4-carbonitrile hydrochloride (218 mg), 3-chloro-6-(5-(((4-methoxybenzyl)oxy)methyl)-1,3,4-oxadiazol-2-yl)pyridazine (200 mg), DIPEA (0.419 mL) and IPA(2 mL) was stirred at 150°C for 1 hour under irradiation with microwave. The reaction mixture was concentrated under reduced pressure. The residue was purified by column chromatography (ethyl acetate/hexane) to obtain the title compound (250 mg).
MS: $[M+H]^+$ 623.1.

D) 1-(6-(5-(Hydroxymethyl)-1,3,4-oxadiazol-2-yl)pyridazin-3-yl)-4-(2-(pentafluorosulfanyl)benzyl)piperidine-4-carbonitrile

In a nitrogen atmosphere, a mixture of 1-(6-(5-(((4-methoxybenzyl)oxy)methyl)-1,3,4-oxadiazol-2-yl)pyridazin-3-yl)-4-(2-(pentafluorosulfanyl)benzyl)piperidine-4-carbonitrile (250 mg), TFA (5 mL) and anisole (200 mg) was stirred at room temperature for 1 hour. The reaction mixture was concentrated under reduced pressure. The residue was poured to a saturated aqueous solution of sodium bicarbonate at room temperature for neutralization, followed by extraction with ethyl acetate. The organic layer was washed with water and saturated brine, dried over magnesium sulfate and then concentrated. The residue was purified by column chromatography (ethyl acetate/hexane) to obtain the title compound (170 mg).
$^1$H NMR (300 MHz, DMSO-d6) δ 1.70-2.00 (4H, m), 3.05-3.24 (4H, m), 4.56-4.69 (2H, m), 4.70-4.79 (2H, m), 5.93-6.06 (1H, m), 7.50 (1H, d, J = 9.7 Hz), 7.58-7.70 (2H, m), 7.79-7.93 (2H, m), 8.04 (1H, d, J = 9.6 Hz).

Example 102

1-(6-(3-Methyl-1,2,4-thiadiazol-5-yl)pyridazin-3-yl)-4-(4-(pentafluorosulfanyl)phenyl)piperidine-4-carbonitrile

To a solution of 3-chloro-6-(3-methyl-1,2,4-thiadiazol-5-yl)pyridazine (150 mg) and 4-(4-(pentafluorosulfanyl)phenyl)piperidine-4-carbonitrile hydrochloride (271 mg) in DMA (1.0 mL), DIPEA (0.370 mL) was added. In a dry atmosphere, the mixture was stirred overnight at 80°C. The residue was poured to water at room temperature, followed by extraction with ethyl acetate. The organic layer was washed with water and saturated brine, dried over sodium sulfate and then concentrated. The residue was crystallized from IPE to obtain the title compound (270 mg).
$^1$H NMR (300 MHz, DMSO-d6) δ 2.13-2.27 (2H, m), 2.29-2.38 (2H, m), 2.66 (3H, s), 3.25-3.42 (2H, m), 4.70-4.90 (2H, m), 7.52-7.61 (1H, m), 7.80-7.90 (2H, m), 7.96-8.08 (3H, m).

Example 103

3-(4-Ethynyl-4-(4-(trifluoromethyl)phenyl)piperidin-1-yl)-6-(5-(((4-methoxybenzyl)oxy)methyl)-1,3,4-oxadiazol-2-yl)pyridazine

A) 1-tert-Butyl4-methyl4-(4-(trifluoromethyl)phenyl)piperidine-1,4-dicarboxylate

To a solution of tert-butyl bis(2-chloroethyl)carbamate (32.61 g) and methyl (4-(trifluoromethyl)phenyl)acetate (26.01 g) in DMF (200 mL), 60% (w/w) sodium hydride in oil (14.30 g) was added under ice cooling. The reaction mixture was stirred overnight at 70°C and cooled, and then, a saturated aqueous solution of ammonium chloride was added thereto, followed by extraction with ethyl acetate. The obtained organic layer was washed with water and saturated brine, dried over anhydrous magnesium sulfate and then concentrated under reduced pressure. The obtained residue was purified by silica gel column chromatography (hexane/ethyl acetate) to obtain the title compound (15.56 g).
MS, found: 331.9

B) tert-Butyl4-(hydroxymethyl)-4-(4-(trifluoromethyl)phenyl)piperidine-1-carboxylate

To a suspension of lithium aluminum hydride (1.862 g) in dehydrated THF (100 mL), a solution of 1-tert-butyl 4-methyl 4-(4-(trifluoromethyl)phenyl)piperidine-1,4-dicarboxylate (9.05 g) in anhydrous THF (50 mL) was added dropwise under ice cooling. The reaction mixture was stirred at room temperature for 30 minutes in a nitrogen atmosphere, and then, water (2.0 mL), sodium hydroxide (4 N aqueous solution, 2.0 mL) and water (6.0 mL) were added thereto. The obtained mixture was stirred at room temperature for 30 minutes. Then, the precipitate was filtered off, and the filtrate was concentrated under reduced pressure to obtain the title compound (8.31 g).

MS, found: 303.9

C) tert-Butyl4-formyl-4-(4-(trifluoromethyl)phenyl)piperidine-1-carboxylate

A mixture of tert-butyl 4-(hydroxymethyl)-4-(4-(trifluoromethyl)phenyl)piperidine-1-carboxylate (5.67 g), triethylamine (21.93 mL), a sulfur trioxide-pyridine complex (7.53 g) and DMSO (100 mL) was stirred overnight at room temperature in a nitrogen atmosphere. The reaction mixture was added to a saturated aqueous solution of sodium bicarbonate, followed by extraction with ethyl acetate. The obtained organic layer was washed with water and saturated brine, dried over anhydrous magnesium sulfate and then concentrated under reduced pressure. The obtained residue was purified by silica gel column chromatography (hexane/ethyl acetate) to obtain the title compound (4.61 g). MS, found: 301.9

D) tert-Butyl4-ethynyl-4-(4-(trifluoromethyl)phenyl)piperidine-1-carboxylate

To a mixture of tert-butyl 4-formyl-4-(4-(trifluoromethyl)phenyl)piperidine-1-carboxylate (0.35 g), dimethyl (1-diazo-2-oxopropyl)phosphonate (0.193 mL) and methanol (5 mL), potassium carbonate (0.271 g) was added, and the resulting mixture was stirred overnight at room temperature. The reaction mixture was poured to water, followed by extraction with ethyl acetate. The organic layer was washed with saturated brine, dried over anhydrous sodium sulfate and then concentrated under reduced pressure. The obtained residue was purified by silica gel column chromatography (hexane/ethyl acetate) to obtain the title compound (0.34 g). MS, found: 297.

E) 4-Ethynyl-4-(4-(trifluoromethyl)phenyl)piperidine hydrochloride

A mixture of tert-butyl4-ethynyl-4-(4-(trifluoromethyl)phenyl)piperidine-1-carboxylate (0.34 g) and hydrogen chloride (4 N solution in ethyl acetate, 5 mL) was stirred overnight at room temperature. The resulting deposit was collected by filtration and dried under reduced pressure to obtain the title compound (0.18 g).
MS, found: 253.9

F) 3-(4-Ethynyl-4-(4-(trifluoromethyl)phenyl)piperidin-1-yl)-6-(5-(((4-methoxybenzyl)oxy)methyl)-1,3,4-oxadiazol-2-yl)pyridazine

A mixture of 3-chloro-6-(5-(((4-methoxybenzyl)oxy)methyl)-1,3,4-oxadiazol-2-yl)pyridazine (89.1 mg), 4-ethynyl-4-(4-(trifluoromethyl)phenyl)piperidine hydrochloride (85.2 mg), DIPEA (0.2 mL) and IPA (2 mL) was heated at 150°C for 1 hour under irradiation with microwave. The reaction mixture was cooled to room temperature, and then, the resulting deposit was collected by filtration and washed with IPA to obtain the title compound (127.0 mg).
MS: $[M+H]^+$ 550.1

G) (5-(6-(4-Ethynyl-4-(4-(trifluoromethyl)phenyl)piperidin-1-yl)pyridazin-3-yl)-1,3,4-oxadiazol-2-yl)methanol

A mixture of 3-(4-ethynyl-4-(4-(trifluoromethyl)phenyl)piperidin-1-yl)-6-(5-(((4-methoxybenzyl)oxy)methyl)-1,3,4-oxadiazol-2-yl)pyridazine (127.0 mg), anisole (0.126 mL) and TFA (5 mL) was stirred at room temperature for 30 minutes. The reaction mixture was concentrated under reduced pressure, and the residue was diluted with ethyl acetate and then poured to a saturated aqueous solution of sodium bicarbonate, followed by extraction with ethyl acetate. The organic layer was washed with water and saturated brine, dried over anhydrous magnesium sulfate and then concentrated under reduced pressure. The obtained residue was crystallized from ethyl acetate and IPE to obtain the title compound (64.7 mg). $^1H$ NMR (300 MHz, DMSO-d6) $\delta$ 1.87-2.17 (4H, m), 3.35-3.53 (2H, m), 3.67 (1H, s), 4.54-4.83 (4H, m), 5.99 (1H, t, J = 6.2 Hz), 7.52 (1H, d, J = 9.7 Hz), 7.66-7.91 (4H, m), 8.03 (1H, d, J = 9.7 Hz).

Example 104

(5-(6-(4-Ethynyl-4-(4-(trifluoromethyl)phenyl)piperidin-1-yl)pyridazin-3-yl)-1,2,4-oxadiazol-3-yl)methanol

A) (5-(6-(4-Ethynyl-4-(4-(trifluoromethyl)phenyl)piperidin-1-yl)pyridazin-3-yl)-1,2,4-oxadiazol-3-yl)methyl benzoate

A mixture of (5-(6-chloropyridazin-3-yl)-1,2,4-oxadiazol-3-yl)methyl benzoate (84.0 mg), 4-ethynyl-4-(4-(trifluoromethyl)phenyl)piperidine hydrochloride (85.0 mg), DIPEA (0.2 mL) and IPA (2 mL) was heated at 150°C for 1 hour under irradiation with microwave. The reaction mixture was cooled to room temperature, and then, the resulting precipitate was collected by filtration, washed with IPA and dried under reduced pressure to obtain the title compound (132.0 mg).
MS: $[M+H]^+$ 534.1

B) (5-(6-(4-Ethynyl-4-(4-(trifluoromethyl)phenyl)piperidin-1-yl)pyridazin-3-yl)-1,2,4-oxadiazol-3-yl)methanol

To a solution of (5-(6-(4-ethynyl-4-(4-(trifluoromethyl)phenyl)piperidin-1-yl)pyridazin-3-yl)-1,2,4-oxadiazol-3-yl)methyl benzoate (132.0 mg) in methanol (10 mL) and THF (10 mL), sodium hydroxide (1 N aqueous solution, 0.247 mL) was added, and the mixture was stirred at room temperature for 1 hour. Hydrogen chloride (IN aqueous solution, 1 mL) was added to the reaction mixture, and then, the mixture was poured to a saturated aqueous solution of sodium bicarbonate, followed by extraction with ethyl acetate. The obtained organic layer was washed with water and saturated brine, dried over anhydrous magnesium sulfate and then concentrated under reduced pressure. The obtained residue was crystallized from ethyl acetate/IPE to obtain the title compound (67.8 mg).
$^1$H NMR (300 MHz, DMSO-d6) $\delta$ 1.86-2.20 (4H, m), 3.36-3.54 (2H, m), 3.68 (1H, s), 4.64 (2H, d, J = 6.2 Hz), 4.72 (2H, d, J = 13.7 Hz), 5.77 (1H, t, J = 6.2 Hz), 7.50 (1H, d, J = 9.8 Hz), 7.68-7.90 (4H, m), 8.04 (1H, d, J = 9.8 Hz).

Example 105

(5-(6-(4-(2,2,2-Trifluoroethyl)-4-(4-(trifluoromethyl)phenyl)piperidin-1-yl)pyridazin-3-yl)-1,3,4-oxadiazol-2-yl)methanol

A) tert-Butyl4-(4-(trifluoromethyl)phenyl)-4-(2,2,2-trifluoro-1-(((methylsulfanyl)carbonothioyl)oxy)ethyl)piperidine-1-carboxylate

To a solution of trimethyl(trifluoromethyl)silane (1.66 mL) and tert-butyl 4-formyl-4-(4-(trifluoromethyl)phenyl)piperidine-1-carboxylate (2.01 g) in THF (30 mL), TBAF (IN solution in THF, 0.560 mL) was added under ice cooling, and the mixture was stirred overnight at room temperature. The reaction mixture was poured to water, followed by extraction with ethyl acetate. The organic layer was washed with water and saturated brine, dried over anhydrous magnesium sulfate and then concentrated under reduced pressure. 60% (w/w) sodium hydride in oil (0.449 g) was added to a solution of the residue in dehydrated THF (10 mL) under ice cooling. After stirring for 10 minutes under ice cooling, methanedithione (0.678 mL) was added thereto. After further stirring for 10 minutes under ice cooling, iodomethane (1.053 mL) was added thereto, and the mixture was stirred at room temperature for 3 hours. The reaction mixture was added to water, followed by extraction with ethyl acetate. The obtained organic layer was washed with water and saturated brine, dried over anhydrous magnesium sulfate and then concentrated under reduced pressure. The obtained residue was purified by silica gel column chromatography (hexane/ethyl acetate) to obtain the title compound (2.84 g).
MS, found: 461.9

B) tert-Butyl 4-(2,2,2-trifluoroethyl)-4-(4-(trifluoromethyl)phenyl)piperidine-1-carboxylate

To a solution of tert-butyl4-(4-(trifluoromethyl)phenyl)-4-(2,2,2-trifluoro-1-(((methylsulfanyl)carbonothioyl)oxy)ethyl)piperidine-1-carboxylate (2.42 g) and tributylstannane (3.77 mL) in toluene (50 mL), AIBN (0.38 g) was added, and the mixture was stirred at 100°C for 5 hours. The reaction mixture was added to water, followed by extraction with ethyl acetate. The organic layer was washed with water and saturated brine, dried over anhydrous sodium sulfate and then concentrated under reduced pressure. The obtained residue was purified by silica gel column chromatography (hexane/ethyl acetate) to obtain the title compound (1.140 g).
MS, found: 355.9

C) 4-(2,2,2-Trifluoroethyl)-4-(4-(trifluoromethyl)phenyl)piperidine hydrochloride

A mixture of tert-butyl 4-(2,2,2-trifluoroethyl)-4-(4-(trifluoromethyl)phenyl)piperidine-1-carboxylate (1.14 g) and hydrogen chloride (4 N solution in ethyl acetate, 15 mL) was stirred overnight at room temperature. The resulting precipitate was collected by filtration and dried under reduced pressure to obtain the title compound (0.86 g). MS, found: 311.9

D) 3-(5-(((4-Methoxybenzyl)oxy)methyl)-1,3,4-oxadiazol-2-yl)-6-(4-(2,2,2-trifluoroethyl)-4-(4-(trifluoromethyl)phenyl)piperidin-1-yl)pyridazine

A mixture of 3-chloro-6-(5-(((4-methoxybenzyl)oxy)methyl)-1,3,4-oxadiazol-2-yl)pyridazine (131.1 mg), 4-(2,2,2-trifluoroethyl)-4-(4-(trifluoromethyl)phenyl)piperidine hydrochloride (150.2 mg), DIPEA (0.3 mL) and IPA (3 mL) was heated at 150°C for 1 hour under irradiation with microwave. The reaction mixture was cooled to room temperature, and then, the resulting deposit was collected by filtration and washed with IPA to obtain the title compound (153.0 mg).
MS: $[M+H]^+$ 608.1

E) (5-(6-(4-(2,2,2-Trifluoroethyl)-4-(4-(trifluoromethyl)phenyl)piperidin-1-yl)pyridazin-3-yl)-1,3,4-oxadiazol-2-yl)methanol

A mixture of 3-(5-(((4-methoxybenzyl)oxy)methyl)-1,3,4-oxadiazol-2-yl)-6-(4-(2,2,2-trifluoroethyl)-4-(4-(trifluoromethyl)phenyl)piperidin-1-yl)pyridazine (153.0 mg), anisole (0.137 mL) and TFA (5 mL) was stirred at room temperature for 30 minutes. The reaction mixture was concentrated under reduced pressure, and the residue was diluted with ethyl acetate and then added to a saturated aqueous solution of sodium bicarbonate, followed by extraction with ethyl acetate. The obtained organic layer was washed with water and saturated brine, dried over anhydrous magnesium sulfate and then concentrated under reduced pressure. The obtained residue was recrystallized from ethyl acetate and hexane to obtain the title compound (91.3 mg).
$^1$H NMR (300 MHz, DMSO-d6) δ2.01-2.19 (2H, m), 2.22-2.41 (2H, m), 2.89 (2H, q, J = 12.0 Hz), 3.36-3.59 (2H, m), 3.95-4.19 (2H, m), 4.73 (2H, d, J = 6.3 Hz), 5.98 (1H, t, J = 6.3 Hz), 7.43 (1H, d, J = 9.7 Hz), 7.65-7.87 (4H, m), 7.98 (1H, d, J = 9.7 Hz).

Example 106

(5-(6-(4-(2,2,2-Trifluoroethyl)-4-(4-(trifluoromethyl)phenyl)piperidin-1-yl)pyridazin-3-yl)-1,2,4-oxadiazol-3-yl)methanol

A) (5-(6-(4-(2,2,2-Trifluoroethyl)-4-(4-(trifluoromethyl)phenyl)piperidin-1-yl)pyridazin-3-yl)-1,2,4-oxadiazol-3-yl)methyl benzoate

A mixture of (5-(6-chloropyridazin-3-yl)-1,2,4-oxadiazol-3-yl)methyl benzoate (124.2 mg), 4-(2,2,2-trifluoroethyl)-4-(4-(trifluoromethyl)phenyl)piperidine hydrochloride (150.0 mg), DIPEA (0.2 mL) and IPA (3 mL) was heated at 150°C for 1 hour under irradiation with microwave. The reaction mixture was cooled to room temperature, and then, the resulting deposit was collected by filtration and washed with IPA to obtain the title compound (211.6 mg). MS: $[M+H]^+$ 592.1

B) (5-(6-(4-(2,2,2-Trifluoroethyl)-4-(4-(trifluoromethyl)phenyl)piperidin-1-yl)pyridazin-3-yl)-1,2,4-oxadiazol-3-yl)methanol

To a mixed solution of (5-(6-(4-(2,2,2-trifluoroethyl)-4-(4-(trifluoromethyl)phenyl)piperidin-1-yl)pyridazin-3-yl)-1,2,4-oxadiazo1-3-yl)methyl benzoate (211.6 mg) in methanol (5 mL) and THF (5 mL), sodium hydroxide (IN aqueous solution, 0.358 mL) was added, and the mixture was stirred at room temperature for 20 minutes. Hydrogen chloride (IN aqueous solution, 1 mL) was added to the reaction mixture, and then, the mixture was added to a saturated aqueous solution of sodium bicarbonate, followed by extraction with ethyl acetate. The obtained organic layer was washed with water and saturated brine, dried over anhydrous magnesium sulfate and then concentrated under reduced pressure. The obtained residue was recrystallized from ethyl acetate/hexane to obtain the title compound (123.8 mg).
$^1$H NMR (300 MHz, DMSO-d6) δ1.99-2.20 (2H, m), 2.22-2.42 (2H, m), 2.90 (2H, q, J = 12.0 Hz), 3.38-3.67 (2H, m), 3.94-4.26 (2H, m), 4.63 (2H, d, J = 6.1 Hz), 5.66-5.84 (1H, m), 7.42 (1H, d, J = 9.7 Hz), 7.63-7.87 (4H, m), 8.00 (1H, d, J = 9.7 Hz).

Example 107

4-(2-Chlorobenzyl)-1-(6-(1H-1,2,3-triazol-4-yl)pyridazin-3-yl)piperidine-4-carbonitrile

A) 4-(2-Chlorobenzyl)-1-(6-iodopyridazin-3-yl)piperidine-4-carbonitrile

In an argon atmosphere, a mixture of 3,6-diiodopyridazine (9.3 g), 4-(2-chlorobenzyl)piperidine-4-carbonitrile hydrochloride (8.36 g), potassium bicarbonate (8.42 g) and dehydrated DMF (40 mL) was stirred at 90°C over weekend. The mixture was poured to water, followed by extraction with ethyl acetate. The organic layer was washed with water and saturated brine, dried over magnesium sulfate and then concentrated. The residue was crystallized from IPE-ethyl acetate to obtain the title compound (9.45 g). $^1$H NMR (300 MHz, DMSO-d6) δ 1.65-2.01 (4H, m), 2.92-3.07 (2H, m), 3.13 (2H, s), 4.28-4.53 (2H, m), 7.14 (1H, d, J = 9.5 Hz), 7.29-7.42 (2H, m), 7.46-7.54 (2H, m), 7.72 (1H, d, J = 9.4 Hz).

B) 4-(2-Chlorobenzyl)-1-(6-ethynylpyridazin-3-yl)piperidine-4-carbonitrile

In a nitrogen atmosphere, a mixture of 4-(2-chlorobenzyl)-1-(6-iodopyridazin-3-yl)piperidine-4-carbonitrile (1.0 g), trimethylsilylacetylene (0.235 g), dichlorobis(triphenylphosphine)palladium(II) (0.480 g), copper(I) iodide (0.130 g), triethylamine (0.951 mL) and dehydrated DMF (30 mL) was stirred at room temperature for 3 hours. The mixture was

poured to water, followed by extraction with ethyl acetate. The organic layer was washed with water and saturated brine, dried over magnesium sulfate and then concentrated. The residue was purified by column chromatography (NH, ethyl acetate/hexane) to obtain the title compound (680 mg).
$^1$H NMR (300 MHz, DMSO-d6) δ 1.69-1.86 (2H, m), 1.88-2.03 (2H, m), 2.97-3.11 (2H, m), 3.14 (2H, s), 4.41 (1H, s), 4.47-4.59 (2H, m), 7.25-7.42 (3H, m), 7.46-7.55 (3H, m).

C) 4-(2-Chlorobenzyl)-1-(6-(1H-1,2,3-triazol-4-yl)pyridazin-3-yl)piperidine-4-carbonitrile

To a solution of 4-(2-chlorobenzyl)-1-(6-ethynylpyridazin-3-yl)piperidine-4-carbonitrile (100 mg) and trimethylsilylazide (0.043 mL) in dehydrated DMF (2 mL) and methanol (0.2 mL), copper (I) iodide (2.83 mg) was added at room temperature. The mixture was stirred at 100°C for hours. The mixture was poured to hydrogen chloride (IN aqueous solution) at room temperature, followed by extraction with ethyl acetate. The organic layer was washed with a saturated aqueous solution of sodium bicarbonate, water and saturated brine, dried over magnesium sulfate and then concentrated. The residue was purified by column chromatography (ethyl acetate/hexane) to obtain the title compound (47 mg). $^1$H NMR (300 MHz, DMSO-d6) δ 1.72-2.08 (4H, m), 2.95-3.21 (4H, m), 4.36-4.68 (2H, m), 7.26-7.57 (5H, m), 7.84-8.01 (1H, m), 8.36 (1H, s), 14.93-15.76 (1H, m).

Example 108

1-(6-(3-(Hydroxymethyl)-1,2,4-thiadiazol-5-yl)pyridazin-3-yl)-4-(4-(pentafluorosulfanyl)phenyl)piperidine-4-carbonitrile

A) (5-(6-Chloropyridazin-3-yl)-1,2,4-thiadiazol-3-yl)methyl benzoate

A solution of 3-chloro-6-methylpyridazine (17.35 g) in thionyl chloride (98 mL) was heated to reflux overnight. The mixture was cooled to room temperature and then concentrated under reduced pressure. The residue was suspended in THF (250 mL). 2-Amino-2-iminoethyl benzoate acetate (32 g) was added to the mixture, and the resulting mixture was stirred at 0°C. Sodium hydroxide (50% aqueous solution, 60.6 mL) was added dropwise to the mixture at 0°C, and the resulting mixture was stirred at 0°C for 1 hour. The reaction was terminated by the addition of cold water to the mixture, followed by extraction with ethyl acetate. The organic layer was filtered, washed with water and saturated brine, dried over magnesium sulfate and then concentrated. The residue was purified by column chromatography (ethyl acetate/hexane) to obtain the title compound (3.8 g). $^1$H NMR (300 MHz, DMSO-d6) δ5.74 (2H, s), 7.54-7.65 (2H, m), 7.66-7.75 (1H, m), 7.97-8.08 (2H, m), 8.20 (1H, d, J = 8.85 Hz), 8.41 (1H, d, J = 8.85 Hz).

B) (5-(6-(4-Cyano-4-(4-(pentafluorosulfanyl)phenyl)piperidin-1-yl)pyridazin-3-yl)-1,2,4-thiadiazol-3-yl)methyl benzoate

To a solution of (5-(6-chloropyridazin-3-yl)-1,2,4-thiadiazol-3-yl)methyl benzoate (300 mg), 4-(4-(pentafluorosulfanyl)phenyl)piperidine-4-carbonitrile hydrochloride (346 mg) in DMA (10 mL), DIPEA (0.630 mL) was added at room temperature. In a dry atmosphere, the mixture was stirred overnight at 80°C. The residue was poured to hydrogen chloride (IN aqueous solution), followed by extraction with ethyl acetate. The organic layer was washed with water and saturated brine, dried over sodium sulfate and then concentrated to obtain the title compound (548 mg).
MS: $[M+H]^+$ 609.2

C) 1-(6-(3-(Hydroxymethyl)-1,2,4-thiadiazol-5-yl)pyridazin-3-yl)-4-(4-(pentafluorosulfanyl)phenyl)piperidine-4-carbonitrile

To a solution of (5-(6-(4-cyano-4-(4-(pentafluorosulfanyl)phenyl)piperidin-1-yl)pyridazin-3-yl)-1,2,4-thiadiazol-3-yl)methyl benzoate (548 mg) in THF (5 mL) and methanol (5 mL), sodium hydroxide (1 N aqueous solution, 2.70 mL) was added, and the mixture was stirred at room temperature for 1 hour in a nitrogen atmosphere. The mixture was poured to a saturated aqueous solution of ammonium chloride, followed by extraction with ethyl acetate. The organic layer was washed with water and saturated brine, dried over magnesium sulfate and then concentrated. The residue was purified by column chromatography (ethyl acetate/hexane) to obtain the title compound (220 mg).
$^1$H NMR (300 MHz, DMSO-d6) δ2.08-2.45 (4H, m), 3.25-3.44 (2H, m), 4.63-4.94 (4H, m), 5.60-5.76 (1H, m), 7.53-7.64 (1H, m), 7.79-7.92 (2H, m), 7.95-8.08 (3H, m).

Example 109

1-(6-(5-(Hydroxymethyl)-1,3,4-thiadiazol-2-yl)pyridazin-3-yl)-4-(4-(pentafluorosulfanyl)phenyl)piperidine-4-carbonitrile

A) Methyl 6-(4-cyano-4-(4-(pentafluorosulfanyl)phenyl)piperidin-1-yl)pyridazine-3-carboxylate

In a nitrogen atmosphere, a mixture of methyl 6-chloropyridazine-3-carboxylate (1.0 g), 4-(4-(pentafluorosulfanyl)phenyl)piperidine-4-carbonitrile hydrochloride (2.223 g), potassium carbonate (4.00 g), TBAI (0.428 g) and dehydrated THF (80 mL) was stirred overnight at 80°C. The mixture was poured to water at room temperature, followed by extraction with ethyl acetate. The organic layer was washed with water and saturated brine, dried over magnesium sulfate and then concentrated. The obtained residue was crystallized from ethyl acetate and hexane to obtain the title compound (2.59 g).
MS: $[M+H]^+$ 449.0

B) 6-(4-Cyano-4-(4-(pentafluorosulfanyl)phenyl)piperidin-1-yl)pyridazine-3-carboxylic acid

To a solution of methyl 6-(4-cyano-4-(4-(pentafluorosulfanyl)phenyl)piperidin-1-yl)pyridazine-3-carboxylate (2.59 g) in methanol (20 mL) and THF (20 mL), sodium hydroxide (2 N aqueous solution, 10 mL) was added, and the mixture was stirred at room temperature for 2 hours. The reaction solution was poured to a mixed solution of hydrogen chloride (2 N aqueous solution) and a saturated aqueous solution of ammonium chloride at room temperature, followed by extraction with ethyl acetate. The organic layer was washed with water and saturated brine, dried over magnesium sulfate and then concentrated. The residue was crystallized from IPE to obtain the title compound (2.25 g).
MS: $[M+H]^+$ 435.0

C) 6-(4-Cyano-4-(4-(pentafluorosulfanyl)phenyl)piperidin-1-yl)-N'-(((4-methoxybenzyl)oxy)acetyl)pyridazine-3-carbohydrazide

To a solution of 6-(4-cyano-4-(4-(pentafluorosulfanyl)phenyl)piperidin-1-yl)pyridazine-3-carboxylic acid (350 mg) and oxalyl chloride (0.141 mL) in dehydrated THF (10 mL), DMF (10 μL) was added at room temperature. In a nitrogen atmosphere, the mixture was stirred at 50°C for 2 hours. The reaction mixture was concentrated under reduced pressure to obtain 6-(4-cyano-4-(4-(pentafluorosulfanyl)phenyl)piperidin-1-yl)pyridazine-3-carbonyl chloride (365 mg).

To a solution of 6-(4-cyano-4-(4-(pentafluorosulfanyl)phenyl)piperidin-1-yl)pyridazine-3-carbonyl chloride (365 mg) in DMA (5 mL), a solution of 2-((4-methoxybenzyl)oxy)acetohydrazide (254 mg) and DIPEA (0.421 mL) in DMA (5 mL) was added at 0°C. In a nitrogen atmosphere, the mixture was stirred at room temperature for 1 hour. The reaction mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (505 mg).
MS: $[M+H]^+$ 627.2

D) 1-(6-(5-(((4-Methoxybenzyl)oxy)methyl)-1,3,4-thiadiazol-2-yl)pyridazin-3-yl)-4-(4-(pentafluorosulfanyl)phenyl)piperidine-4-carbonitrile

In a nitrogen atmosphere, a mixture of 6-(4-cyano-4-(4-(pentafluorosulfanyl)phenyl)piperidin-1-yl)-N'-(((4-methoxybenzyl)oxy)acetyl)pyridazine-3-carbohydrazide (505 mg), a Lawesson's reagent (489 mg) and dehydrated THF (5 mL) was stirred at 60°C over weekend. The reaction mixture was concentrated under reduced pressure. The residue was purified by column chromatography (NH, ethyl acetate/hexane) and then further purified by column chromatography (ethyl acetate/hexane) to obtain the title compound (290 mg).
MS: $[M+H]^+$ 625.1

E) 1-(6-(5-(Hydroxymethyl)-1,3,4-thiadiazol-2-yl)pyridazin-3-yl)-4-(4-(pentafluorosulfanyl)phenyl)piperidine-4-carbonitrile

A mixture of 1-(6-(5-(((4-methoxybenzyl)oxy)methyl)-1,3,4-thiadiazol-2-yl)pyridazin-3-yl)-4-(4-(pentafluorosulfanyl)phenyl)piperidine-4-carbonitrile (280 mg), anisole (1 mL) and TFA (3 mL) was stirred at room temperature for 1 hour in a nitrogen atmosphere. The reaction mixture was concentrated under reduced pressure. The residue was poured to a saturated aqueous solution of sodium bicarbonate at room temperature, followed by extraction with ethyl acetate. The organic layer was washed with water and saturated brine, dried over magnesium sulfate and then concentrated.

The residue was purified by column chromatography (NH, ethyl acetate/hexane) to obtain the title compound (40 mg).
$^1$H NMR (300 MHz, DMSO-d6) δ2.10-2.39 (4H, m), 3.21-3.44 (2H, m), 4.70-4.83 (2H, m), 4.85-4.94 (2H, m), 6.21-6.35 (1H, m), 7.52-7.66 (1H, m), 7.79-7.90 (2H, m), 7.95-8.06 (2H, m), 8.10-8.21 (1H, m).

Example 110

(5-(6-(4-Methoxy-4-(4-(pentafluorosulfanyl)phenyl)piperidin-1-yl)pyridazin-3-yl)-1,2,4-thiadiazol-3-yl)methanol

A) (5-(6-(4-Methoxy-4-(4-(pentafluorosulfanyl)phenyl)piperidin-1-yl)pyridazin-3-yl)-1,2,4-thiadiazol-3-yl)methyl benzoate

To a solution of (5-(6-chloropyridazin-3-yl)-1,2,4-thiadiazol-3-yl)methyl benzoate (100 mg) and 4-methoxy-4-(4-(pentafluorosulfanyl)phenyl)piperidine hydrochloride (105 mg) in DMA (3 mL) and IPA (3 mL), DIPEA (0.2 mL) was added. The mixture was stirred overnight at 80°C. After cooling to room temperature, the mixture was poured to hydrogen chloride (0.1 N aqueous solution), followed by extraction with ethyl acetate. The organic layer was washed with a saturated aqueous solution of sodium bicarbonate and saturated brine, dried over magnesium sulfate and then concentrated to obtain the title compound (183.2 mg).
MS: $[M+H]^+$ 614.1

B) (5-(6-(4-Methoxy-4-(4-(pentafluorosulfanyl)phenyl)piperidin-1-yl)pyridazin-3-yl)-1,2,4-thiadiazol-3-yl)methanol

To a solution of (5-(6-(4-methoxy-4-(4-(pentafluorosulfanyl)phenyl)piperidin-1-yl)pyridazin-3-yl)-1,2,4-thiadiazol-3-yl)methyl benzoate (183.2 mg) in THF (5 mL) and methanol (5 mL), sodium hydroxide (2 N aqueous solution, 0.300 mL) was added at room temperature, and the mixture was stirred at room temperature for 1 hour. The mixture was poured to water, followed by extraction with ethyl acetate. The organic layer was washed with water and saturated brine, purified by column chromatography (NH, ethyl acetate) and concentrated. The obtained residue was crystallized from ethyl acetate and hexane to obtain the title compound (88 mg).
$^1$H NMR (300 MHz, DMSO-d6) δ1.76-2.23 (4H, m), 3.02 (3H, s), 3.33-3.47 (2H, m), 4.35-4.59 (2H, m), 4.72 (2H, d, J = 6.2 Hz), 5.56-5.77 (1H, m), 7.50 (1H, d, J = 9.7 Hz), 7.66 (2H, d, J = 8.5 Hz), 7.92 (2H, d, J = 8.5 Hz), 7.98 (1H, d, J = 9.7 Hz).

Example 111

(5-(6-(4-Methoxy-4-(4-(pentafluorosulfanyl)phenyl)piperidin-1-yl)pyridazin-3-yl)-1,3,4-thiadiazol-2-yl)methanol

A) Methyl 6-(4-methoxy-4-(4-(pentafluorosulfanyl)phenyl)piperidin-1-yl)pyridazine-3-carboxylate

In a nitrogen atmosphere, a mixture of methyl 6-chloropyridazine-3-carboxylate (900 mg), 4-methoxy-4-(4-(pentafluorosulfanyl)phenyl)piperidine hydrochloride (2.030 g), potassium carbonate (3604 mg), TBAI (385 mg) and dehydrated THF (80 mL) was stirred overnight at 80°C. The mixture was poured to water at room temperature, followed by extraction with ethyl acetate. The organic layer was washed with water and saturated brine, dried over magnesium sulfate and then concentrated. The obtained residue was crystallized from ethyl acetate and hexane to obtain the title compound (2.25 g).
MS: $[M+H]^+$ 454.0

B) 6-(4-Methoxy-4-(4-(pentafluorosulfanyl)phenyl)piperidin-1-yl)pyridazine-3-carboxylic acid

To a solution of methyl 6-(4-methoxy-4-(4-(pentafluorosulfanyl)phenyl)piperidin-1-yl)pyridazine-3-carboxylate (2.25 g) in methanol (30 mL) and THF (30 mL), sodium hydroxide (2 N aqueous solution, 8 mL) was added, and the mixture was stirred at room temperature for 2 hours. The reaction solution was poured to a mixed solution of hydrogen chloride (2 N aqueous solution) and a saturated aqueous solution of ammonium chloride at room temperature, followed by extraction with ethyl acetate. The organic layer was washed with water and saturated brine, dried over magnesium sulfate and then concentrated. The residue was crystallized from IPE to obtain the title compound (2.00 g).
MS: $[M+H]^+$ 440.0

C) N'-(((4-Methoxybenzyl)oxy)acetyl)-6-(4-methoxy-4-(4-(pentafluorosulfanyl)phenyl)piperidin-1-yl)pyridazine-3-carbohydrazide

To a solution of 6-(4-methoxy-4-(4-(pentafluorosulfanyl)phenyl)piperidin-1-yl)pyridazine-3-carboxylic acid (420

mg) and oxalyl chloride (167 μL) in dehydrated THF (10 mL), DMF (10 μL) was added at room temperature. In a nitrogen atmosphere, the mixture was stirred at 50°C for 2 hours. The reaction mixture was concentrated under reduced pressure. A solution of 2-((4-methoxybenzyl)oxy)acetohydrazide (303 mg) and DIPEA(0.502 mL) in DMA (5 mL) was added dropwise to a solution of the obtained residue in DMA (5 mL) at 0°C. In a nitrogen atmosphere, the mixture was stirred at room temperature for 1 hour. The reaction mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (600 mg). MS: $[M+H]^+$ 632.2

D) 3-(5-(((4-Methoxybenzyl)oxy)methyl)-1,3,4-thiadiazol-2-yl)-6-(4-methoxy-4-(4-(pentafluorosulfanyl)phenyl)piperidin-1-yl)pyridazine

In a nitrogen atmosphere, a mixture of N'-(((4-methoxybenzyl)oxy)acetyl)-6-(4-methoxy-4-(4-(pentafluorosulfanyl)phenyl)piperidin-1-yl)pyridazine-3-carbohydrazide (600 mg), a Lawesson's reagent (576 mg) and dehydrated THF (5 mL) was stirred at 60°C over weekend. The reaction mixture was concentrated under reduced pressure. The residue was purified by column chromatography (ethyl acetate/hexane) to obtain the title compound (280 mg).
MS: $[M+H]^+$ 630.3

E) (5-(6-(4-Methoxy-4-(4-(pentafluorosulfanyl)phenyl)piperidin-1-yl)pyridazin-3-yl)-1,3,4-thiadiazol-2-yl)methanol

A mixture of 3-(5-(((4-methoxybenzyl)oxy)methyl)-1,3,4-thiadiazol-2-yl)-6-(4-methoxy-4-(4-(pentafluorosulfanyl)phenyl)piperidin-1-yl)pyridazine (280 mg), anisole (1 mL) and TFA (3 mL) was stirred at room temperature for 1 hour in a nitrogen atmosphere. The reaction mixture was concentrated under reduced pressure. The residue was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (150 mg).
$^1H$ NMR (300 MHz, DMSO-d6) δ1.81-2.28 (4H, m), 3.02 (3H, s), 3.29-3.47 (2H, m), 4.35-4.62 (2H, m), 4.79-5.00 (2H, m), 6.19-6.35 (1H, m), 7.46-7.56 (1H, m), 7.60-7.73 (2H, m), 7.86-7.99 (2H, m), 8.03-8.17 (1H, m).

Example 112

(5-(6-(4-(Trifluoromethyl)-4-(4-(trifluoromethyl)phenyl)piperidin-1-yl)pyridazin-3-yl)-1,2,4-thiadiazol-3-yl)methanol

A) (5-(6-(4-(Trifluoromethyl)-4-(4-(trifluoromethyl)phenyl)piperidin-1-yl)pyridazin-3-yl)-1,2,4-thiadiazol-3-yl)methyl benzoate

A mixture of (5-(6-chloropyridazin-3-yl)-1,2,4-thiadiazol-3-yl)methyl benzoate (93 mg), 4-(trifluoromethyl)-4-(4-(trifluoromethyl)phenyl)piperidine hydrochloride (93.5 mg), DMF (5 mL) and DIPEA (0.245 mL) was stirred at 80°C for 16 hours. After cooling to room temperature, the mixture was poured to hydrogen chloride (0.1 N aqueous solution), followed by extraction with ethyl acetate. The organic layer was washed with saturated brine, dried over magnesium sulfate and then concentrated to obtain the title compound (172.3 mg). MS: $[M+H]^+$ 594.2

B) (5-(6-(4-(Trifluoromethyl)-4-(4-(trifluoromethyl)phenyl)piperidin-1-yl)pyridazin-3-yl)-1,2,4-thiadiazol-3-yl)methanol

To a solution of (5-(6-(4-(trifluoromethyl)-4-(4-(trifluoromethyl)phenyl)piperidin-1-yl)pyridazin-3-yl)-1,2,4-thiadiazol-3-yl)methyl benzoate (172.3 mg) in THF (5 mL) and methanol (5 mL), sodium hydroxide (2 N aqueous solution, 0.2 mL) was added at room temperature, and the mixture was stirred at room temperature for 1 hour. The mixture was poured to water, followed by extraction with ethyl acetate. The organic layer was washed with a saturated aqueous solution of sodium bicarbonate and saturated brine, dried over magnesium sulfate and then concentrated. The residue was purified by column chromatography (NH, ethyl acetate/hexane). The obtained residue was crystallized from ethanol and water to obtain the title compound (51.5 mg).
$^1H$ NMR (300 MHz, DMSO-d6) δ1.99-2.24 (2H, m), 2.68-3.04 (4H, m), 4.41-4.61 (2H, m), 4.72 (2H, d, J = 6.0 Hz), 5.57-5.72 (1H, m), 7.45 (1H, d, J = 9.7 Hz), 7.80-8.04 (5H, m).

Example 113

(5-(6-(4-(Trifluoromethyl)-4-(4-(trifluoromethyl)phenyl)piperidin-1-yl)pyridazin-3-yl)-1,3,4-thiadiazol-2-yl)methanol

A) 6-(4-(Trifluoromethyl)-4-(4-(trifluoromethyl)phenyl)piperidin-1-yl)pyridazine-3-carboxylic acid

In a nitrogen atmosphere, a mixture of 3-(5-(((4-methoxybenzyl)oxy)methyl)-1,3,4-oxadiazol-2-yl)-6-(4-(trifluoromethyl)-4-(4-(trifluoromethyl)phenyl)piperidin-1-yl)pyridazine (4.05 g), trifluoroacetic acid (10 mL) and anisole (1 mL) was stirred at room temperature for 1 hour. The reaction mixture was concentrated under reduced pressure and then poured to a saturated aqueous solution of sodium bicarbonate at room temperature, followed by extraction with ethyl acetate. The organic layer was washed with water and saturated brine, dried over magnesium sulfate and then concentrated. Hydrogen chloride (4 N aqueous solution, 30 mL) was added to a solution of the obtained residue in ethanol (100 mL) at 80°C. The mixture was heated to reflux over weekend. The reaction mixture was concentrated under reduced pressure. Sodium hydroxide (2 N aqueous solution, 20 mL) was added to a solution of the obtained residue in ethanol (100 mL) at room temperature. In a nitrogen atmosphere, the mixture was stirred at room temperature for 1 hour. The mixture was neutralized with hydrogen chloride (1 N aqueous solution), followed by extraction with ethyl acetate. The organic layer was washed with water and saturated brine, dried over magnesium sulfate and then concentrated to obtain the title compound (2.46 g).
MS: $[M+H]^+$420.0

B) N'-(((4-Methoxybenzyl)oxy)acetyl)-6-(4-(trifluoromethyl)-4-(4-(trifluoromethyl)phenyl)piperidin-1-yl)pyridazine-3-carbohydrazide

To a solution of 6-(4-(trifluoromethyl)-4-(4-(trifluoromethyl)phenyl)piperidin-1-yl)pyridazine-3-carboxylic acid (200 mg) and oxalyl chloride (83 μL) in dehydrated THF (10 mL), DMF (10 μL) was added at room temperature. In a nitrogen atmosphere, the mixture was stirred at 50°C for 2 hours. The reaction mixture was concentrated under reduced pressure. A solution of 2-((4-methoxybenzyl)oxy)acetohydrazide (151 mg) and DIPEA (0.251 mL) in DMA (5 mL) was added dropwise to a solution of the obtained residue in DMA (5 mL) at 0°C. In a nitrogen atmosphere, the mixture was stirred at room temperature for 1 hour. The reaction mixture was poured to a saturated aqueous solution of sodium bicarbonate at room temperature, followed by extraction with ethyl acetate. The organic layer was washed with water and saturated brine, dried over magnesium sulfate and then concentrated. The residue was purified by column chromatography (ethyl acetate/hexane) to obtain the title compound (340 mg). MS: $[M+H]^+$ 612.3

C) 3-(5-(((4-Methoxybenzyl)oxy)methyl)-1,3,4-thiadiazol-2-yl)-6-(4-(trifluoromethyl)-4-(4-(trifluoromethyl)phenyl)piperidin-1-yl)pyridazine

In a nitrogen atmosphere, a mixture of N'-(((4-methoxybenzyl)oxy)acetyl)-6-(4-(trifluoromethyl)-4-(4-(trifluoromethyl)phenyl)piperidin-1-yl)pyridazine-3-carbohydrazide (340 mg), a Lawesson's reagent (337 mg) and dehydrated THF (5 mL) was stirred at 60°C over weekend. The reaction mixture was concentrated under reduced pressure. The residue was purified by column chromatography (NH, ethyl acetate/hexane) and then further purified by column chromatography (ethyl acetate/hexane) to obtain the title compound (95 mg).
MS: $[M+H]^+$ 610.1

D) (5-(6-(4-(Trifluoromethyl)-4-(4-(trifluoromethyl)phenyl)piperidin-1-yl)pyridazin-3-yl)-1,3,4-thiadiazol-2-yl)methanol

A mixture of 3-(5-(((4-methoxybenzyl)oxy)methyl)-1,3,4-thiadiazol-2-yl)-6-(4-(trifluoromethyl)-4-(4-(trifluoromethyl)phenyl)piperidin-1-yl)pyridazine (95 mg), anisole (1 mL) and TFA (3 mL) was stirred at room temperature for 1 hour in a nitrogen atmosphere. The reaction mixture was concentrated under reduced pressure. The residue was poured to a saturated aqueous solution of sodium bicarbonate at room temperature, followed by extraction with ethyl acetate. The organic layer was washed with water and saturated brine, dried over magnesium sulfate and then concentrated. The residue was crystallized from IPE to obtain the title compound (50 mg).
$^1H$ NMR (300 MHz, DMSO-d6) δ1.97-2.24 (2H, m), 2.74-2.96 (4H, m), 4.42-4.56 (2H, m), 4.82-4.94 (2H, m), 6.22-6.33 (1H, m), 7.42-7.51 (1H, m), 7.82-7.90 (2H, m), 7.93-8.00 (2H, m), 8.07-8.14 (1H, m).

Example 114

(5-(6-(3-((3,4-Dichlorophenyl)ethynyl)-3-methoxypyrrolidin-1-yl)pyridazin-3-yl)-1,2,4-thiadiazol-3-yl)methanol

To a mixture of ethynyltrimethylsilane (6.17 g) and anhydrous THF (60 mL), n-butyllithium (1.6 M solution in hexane, 39.2 mL) was added at -78°C. After stirring at -78°C for 30 minutes, a mixture of 1-benzylpyrrolidin-3-one (10 g) and THF (5 mL) was added thereto. In a nitrogen atmosphere, the mixture was stirred at -40°C for 1 hour. An aqueous ammonium chloride solution was added to the mixture, followed by extraction with ethyl acetate. The combined organic layer was washed with saturated brine, dried over magnesium sulfate and then concentrated. The residue was purified by column chromatography (ethyl acetate/hexane) to obtain 1-benzyl-3-((trimethylsilyl)ethynyl)pyrrolidin-3-ol (11.8 g). To a mixture of 1-benzyl-3-((trimethylsilyl)ethynyl)pyrrolidin-3-ol (4.45 g) and anhydrous DMF (80 mL), 60% (w/w) sodium hydride in oil (2.20 g) was added at 0°C. After stirring at room temperature for 10 minutes, methyl iodide (1.123 mL) was added to the mixture. The mixture was stirred at room temperature for 1 hour. The mixture was poured to a saturated aqueous solution of ammonium chloride, followed by extraction with ethyl acetate. The organic layer was washed with water and saturated brine and purified by column chromatography (NH, ethyl acetate) to obtain 1-benzyl-3-ethynyl-3-methoxypyrrolidine (1.650 g). To a solution of 1-benzyl-3-ethynyl-3-methoxypyrrolidine (1.65 g) in acetonitrile (5 mL), 1-chloroethyl chloroformate (2.00 mL) was added at room temperature, and the mixture was stirred overnight at room temperature. The mixture was concentrated under reduced pressure, and methanol (20 mL) was added to the residue. The mixture was stirred at 70°C for 1 hour. The reaction mixture was concentrated under reduced pressure, and the obtained solid was washed with ethyl acetate to obtain 3-ethynyl-3-methoxypyrrolidine hydrochloride (1.07 g). A mixture of (5-(6-chloropyridazin-3-yl)-1,2,4-thiadiazol-3-yl)methyl benzoate (1.80 g), 3-ethynyl-3-methoxypyrrolidine hydrochloride (0.92 g), DIPEA (2.84 mL) and DMA (2 mL) was stirred at 150°C for 1 hour under irradiation with microwave. The mixture was poured to water, followed by extraction with ethyl acetate/THF. The organic layer was washed with water and saturated brine and then purified by column chromatography (NH, ethyl acetate) to obtain (5-(6-(3-ethynyl-3-methoxypyrrolidin-1-yl)pyridazin-3-yl)-1,2,4-thiadiazol-3-yl)methyl benzoate (1.84 g). A mixture of (5-(6-(3-ethynyl-3-methoxypyrrolidin-1-yl)pyridazin-3-yl)-1,2,4-thiadiazol-3-yl)methyl benzoate (1.50 g), sodium hydroxide (2 N aqueous solution, 3.56 mL), methanol (10 mL) and THF (10 mL) was stirred at room temperature for 1 hour. The reaction solution was poured to water, followed by extraction with ethyl acetate. The organic layer was washed with water and saturated brine and then purified by column chromatography (NH, ethyl acetate). The residue was crystallized from ethyl acetate-hexane to obtain (5-(6-(3-ethynyl-3-methoxypyrrolidin-1-yl)pyridazin-3-yl)-1,2,4-thiadiazol-3-yl)methanol (1.06 g). In a nitrogen atmosphere, a mixture of (5-(6-(3-ethynyl-3-methoxypyrrolidin-1-yl)pyridazin-3-yl)-1,2,4-thiadiazol-3-yl)methanol (150 mg), 3,4-dichloroiodobenzene (143.2 mg), dichlorobis(triphenylphosphine)palladium (II) (30.4 mg), copper (I) iodide (11.2 mg), triethylamine (0.70 mL) and DMF (3 mL) was stirred at room temperature for 1 hour. The mixture was poured to water, followed by extraction with ethyl acetate. The organic layer was washed with water and saturated brine, dried over magnesium sulfate and then concentrated. The residue was purified by column chromatography (ethyl acetate/hexane). The residue was crystallized from ethanol-water to obtain the title compound (160 mg).
$^1$H NMR (300 MHz, DMSO-$d_6$) $\delta$ 2.31-2.46 (1H, m), 2.53-2.68 (1H, m), 3.42 (3H, s), 3.50-3.72 (1H, m), 3.73-3.96 (2H, m), 3.98-4.26 (1H, m), 4.72 (2H, d, J = 6.0 Hz), 5.65 (1H, t, J = 6.0 Hz), 7.14 (1H, d, J = 9.5 Hz), 7.47-7.57 (1H, m), 7.64-7.71 (1H, m), 7.85 (1H, d, J = 1.9 Hz), 8.01 (1H, d, J = 9.5 Hz).

Example 115

(5-(6-(4-(4-(Pentafluorosulfanyl)phenyl)-4-(trifluoromethyl)piperidin-1-yl)pyridazin-3-yl)-1,2,4-thiadiazol-3-yl)methanol

A) tert-Butyl4-(4-(pentafluorosulfanyl)phenyl)-4-(trifluoromethyl)piperidine-1-carboxylate

To a mixture of 1-bromo-4-(pentafluorosulfanyl)benzene (39.5 g) and anhydrous THF (400 mL), an isopropyl magnesium chloride-lithium chloride complex (1.3 M solution in hexane, 215 mL) was added dropwise at -15°C. The mixture was stirred at -15°C for 30 minutes, and then, the temperature was raised to room temperature. A mixture of ethyl trifluoroacetate (43.7 g) and THF (80 mL) was added to the reaction mixture at -78°C. The mixture was stirred at -78°C for 30 minutes, and then, the temperature was raised to room temperature. The reaction mixture was poured to saturated brine, followed by extraction with ethyl acetate (200 mL) three times. The combined organic layer was dried, then kept at 30°C or lower and concentrated under reduced pressure. The organic layer was dried over magnesium sulfate and then concentrated under reduced pressure at 25°C. The residue was purified by column chromatography (ethyl acetate/petroleum ether) to obtain 2,2,2-trifluoro-1-(4-(pentafluorosulfanyl)phenyl)ethanone (35 g). In a nitrogen atmosphere, titanium tetrachloride (27.8 g) was added dropwise to a mixture of 2,2,2-trifluoro-1-(4-(pentafluorosulfanyl)phenyl)ethanone (21 g), ethyl cyanoacetate (8.7 g) and dichloromethane (400 mL) at 0°C. The mixture was stirred at 0°C for 20 minutes, and then, pyridine (6 mL) was added dropwise thereto at 5°C or lower. After the completion of

the dropwise addition, the reaction mixture was stirred at room temperature for 30 minutes. Pyridine (20 mL) was added thereto, and the mixture was stirred overnight at room temperature. The reaction mixture was diluted with dichloromethane (1000 mL) and washed with hydrochloric acid (2 N, 300 mL), water (100 mL) and saturated brine (100 mL) in this order. The obtained organic layer was dried over magnesium sulfate and then concentrated at 25°C. The obtained residue was purified by column chromatography (ethyl acetate/petroleum ether) to obtain ethyl 2-cyano-4,4,4-trifluoro-3-(4-(pentafluorosulfanyl)phenyl)but-2-enoate (25 g). In a nitrogen atmosphere, a mixture of ethyl 2-cyano-4,4,4-trifluoro-3-(4-(pentafluorosulfanyl)phenyl)but-2-enoate (23.7 g), 2-cyanoacetamide (8.8 g), sodium acetate (17.2 g) and ethanol (300 mL) was stirred overnight at 30°C. The solvent was distilled off under reduced pressure, and the pH of the obtained residue was adjusted to 3 by the addition of water (300 mL) and further the addition of hydrochloric acid. The mixture was subjected to extraction with ethyl acetate (200 mL) three times. The combined organic layer was dried over sodium sulfate and concentrated under reduced pressure to obtain 2,6-dioxo-4-(4-(pentafluorosulfanyl)phenyl)-4-(trifluoromethyl)piperidine-3,5-dicarbonitrile (27.0 g). Amixture of 2,6-dioxo-4-(4-(pentafluorosulfanyl)phenyl)-4-(trifluoromethyl)piperidine-3,5-dicarbonitrile (27 g), sulfuric acid (88.2 g), water (200 mL) and acetic acid (200 mL) was stirred overnight at 140°C. The reaction mixture was cooled, and water (200 mL) was added thereto, followed by extraction with ethyl acetate (200 mL) three times. The combined organic layer was washed with saturated brine, dried over magnesium sulfate and concentrated under reduced pressure to obtain 9-(4-(pentafluorosulfanyl)phenyl)-9-(trifluoromethyl)-3,7-diazabicyclo[3.3.1]nonane-2,4,6,8-tetraone (25 g). A mixture of 9-(4-(pentafluorosulfanyl)phenyl)-9-(trifluoromethyl)-3,7-diazabicyclo[3.3.1]nonane-2,4,6,8-tetraone (25 g) and potassium hydroxide (20% aqueous solution, 130 g) was heated to reflux for 4 hours. The reaction mixture was cooled to 0°C, and a mixture of sulfuric acid (52.1 g) and water (200 mL) was added dropwise thereto. The obtained mixture was heated to reflux for 3 hours. The reaction mixture was cooled, followed by extraction with ethyl acetate (200 mL) three times. The combined organic layer was concentrated under reduced pressure to obtain 3-(4-(pentafluorosulfanyl)phenyl)-3-(trifluoromethyl)pentanedioic acid (25 g). 3-(4-(Pentafluorosulfanyl)phenyl)-3-(trifluoromethyl)pentanedioic acid (25 g) was dissolved in acetic anhydride (131.6 g), and the solution was heated to reflux for 3 hours. Excessive acetic anhydride was distilled off from the solution thus heated to reflux, and urea (14.4 g) was added to the residue at room temperature. In a nitrogen atmosphere, the mixture was stirred at 190°C for 30 minutes and cooled to room temperature. The reaction mixture was dissolved in dichloromethane (300 mL), and the insoluble matter was filtered off. The filtrate was concentrated under reduced pressure. The obtained residue was purified by column chromatography (dichloromethane/methanol) to obtain 4-(4-(pentafluorosulfanyl)phenyl)-4-(trifluoromethyl)piperidine-2,6-dione (11.5 g). In a nitrogen atmosphere, a borane-dimethyl sulfide complex (2 M solution in dimethyl sulfide, 100 mL) was added dropwise to 4-(4-(pentafluorosulfanyl)phenyl)-4-(trifluoromethyl)piperidine-2,6-dione (15.3 g) at 0°C. After the completion of the dropwise addition, the mixture was heated to reflux overnight and then cooled to 0°C. Hydrogen chloride (6 N aqueous solution, 70 mL) was added to the reaction mixture. The obtained mixture was heated to reflux for 1 hour. The mixture thus heated to reflux was cooled in ice, and sodium hydroxide (8 N aqueous solution, 60 mL) was added thereto, followed by extraction with ethyl acetate (150 mL) three times. The combined organic layer was dried and concentrated under reduced pressure to obtain partially purified 4-(4-(pentafluorosulfanyl)phenyl)-4-(trifluoromethyl)piperidine. The obtained 4-(4-(pentafluorosulfanyl)phenyl)-4-(trifluoromethyl)piperidine was dissolved in dehydrated THF (150 mL), and DIPEA (6.2 g) and di-tert-butyl dicarbonate (9.6 g) were added to the solution at room temperature. The mixture was stirred at room temperature for 2 hours. The reaction mixture was poured to a saturated aqueous solution of sodium bicarbonate, followed by extraction with ethyl acetate (150 mL) three times. The combined organic layer was dried and then concentrated under reduced pressure. The obtained residue was purified by column chromatography (ethyl acetate/petroleum ether) to obtain the title compound (13.1 g).
$^1$H NMR (400 MHz, CDCl3) δ 1.44 (9H, s), 2.06-2.14 (2H, m), 2.45 (2H, d, J = 13.6 Hz), 2.70 (2H, brs), 4.06 (2H, brs), 7.55 (2H, d, J = 8.4 Hz), 7.81 (2H, d, J = 9.2 Hz).

B) 4-(4-(Pentafluorosulfanyl)phenyl)-4-(trifluoromethyl)piperidine hydrochloride

A mixture of tert-butyl 4-(4-(pentafluorosulfanyl)phenyl)-4-(trifluoromethyl)piperidine-1-carboxylate (4 g), hydrogen chloride (4 M solution in isopropanol, 12 mL) and ethyl acetate (20 mL) was heated to reflux for 5 hours. The reaction mixture was concentrated under reduced pressure. Diethyl ether (100 mL) was added to the obtained residue, and the mixture was stirred for 30 minutes. The deposit was collected by filtration to obtain the title compound (3 g).
$^1$H NMR (400 MHz, DMSO-d6) δ 2.33-2.39 (2H, m), 2.51-2.58 (2H, m), 2.78-2.80 (2H, m), 3.28-3.36 (2H, m), 7.88 (2H, d, J = 8.4 Hz), 8.02 (2H, d, J = 8.8 Hz), 9.13 (1H, brs), 9.36 (1H, brs).

C) (5-(6-(4-(4-(Pentafluorosulfanyl)phenyl)-4-(trifluoromethyl)piperidin-1-yl)pyridazin-3-yl)-1,2,4-thiadiazol-3-yl)methanol

A mixture of 4-(4-(pentafluorosulfanyl)phenyl)-4-(trifluoromethyl)piperidine hydrochloride (400 mg), (5-(6-chloropyridazin-3-yl)-1,2,4-thiadiazol-3-yl)methyl benzoate (340 mg), DIPEA (395 mg) and DMSO (10 mL) was stirred overnight at 90°C. The mixture was poured to water (60 mL), followed by extraction with ethyl acetate (50 mL) three times. The combined organic layer was dried and then concentrated under reduced pressure. The residue was purified by column chromatography (ethyl acetate/petroleum ether) to obtain (5-(6-(4-(4-(pentafluorosulfanyl)phenyl)-4-(trifluoromethyl)piperidin-1-yl)pyridazin-3-yl)-1,2,4-thiadiazol-3-yl)methyl benzoate (230 mg). A mixture of (5-(6-(4-(4-(pentafluorosulfanyl)phenyl)-4-(trifluoromethyl)piperidin-1-yl)pyridazin-3-yl)-1,2,4-thiadiazol-3-yl)methyl benzoate (520 mg), potassium hydroxide (4.5 M aqueous solution, 1 mL) and acetonitrile (20 mL) was heated to reflux for 1 hour. The reaction mixture was concentrated, and the residue was purified by HPLC to obtain the title compound (250 mg).
$^1$H NMR (300 MHz, DMSO-d6) $\delta$ 2.05-2.23 (2H, m), 2.69-3.01 (4H, m), 4.39-4.60 (2H, m), 4.72 (2H, s), 5.70 (1H, brs), 7.38-7.52 (1H, m), 7.83-8.14 (5H, m).

Example 116

(5-(6-(4-(4-(Pentafluorosulfanyl)phenyl)-4-(trifluoromethyl)piperidin-1-yl)pyridazin-3-yl)-1,3,4-oxadiazol-2-yl)methano

In a nitrogen atmosphere, a mixture of 3-chloro-6-(5-(((4-methoxybenzyl)oxy)methyl)-1,3,4-oxadiazol-2-yl)pyridazine (50 g), TFA(150 mL) and anisole (50 mL) was stirred at room temperature for 1 hour. The reaction mixture was concentrated under reduced pressure. The residue was poured to a saturated aqueous solution of sodium bicarbonate, followed by extraction with ethyl acetate. The organic layer was washed with water and saturated brine, then dried over magnesium sulfate and concentrated. The residue was crushed in IPE to obtain (5-(6-chloropyridazin-3-yl)-1,3,4-oxadiazol-2-yl)methanol (27.56 g). A mixture of 4-(4-(pentafluorosulfanyl)phenyl)-4-(trifluoromethyl)piperidine hydrochloride (290 mg), (5-(6-chloropyridazin-3-yl)-1,3,4-oxadiazol-2-yl)methanol (150 mg), DIPEA (0.650 mL) and IPA (4 mL) was stirred at 150°C for 1 hour under irradiation with microwave. The reaction mixture was cooled to room temperature and stirred overnight at room temperature. The deposit was collected by filtration and washed with IPA. The obtained solid was recrystallized from ethanol-water to obtain the title compound (345 mg).
$^1$H NMR (300 MHz, DMSO-$d_6$) $\delta$ 2.01-2.28 (2H, m), 2.67-2.99 (4H, m), 4.41-4.60 (2H, m), 4.68-4.84 (2H, m), 5.92-6.08 (1H, m), 7.35-7.53 (1H, m), 7.87-8.14 (5H, m).

Example 117

(5-(6-(4-(4-(Pentafluorosulfanyl)phenyl)-4-(trifluoromethyl)piperidin-1-yl)pyridazin-3-yl)-1,3,4-thiadiazol-2-yl)methanol

In a nitrogen atmosphere, a mixture of methyl 6-chloropyridazine-3-carboxylate (40 g), sodium methoxide (62.5 g) and methanol (600 mL) was heated to reflux overnight. The reaction mixture was cooled to room temperature. Hydrochloric acid was added to the reaction mixture at 0°C to adjust the pH to 3. The solvent was distilled off, and then, the residue was dissolved in water (200 mL), followed by extraction with ethyl acetate (300 mL) three times. The combined organic layer was washed with saturated brine, then dried over sodium sulfate and concentrated. The residue was washed with methyl t-butyl ether/petroleum ether (1:5) to obtain 6-methoxypyridazine-3-carboxylic acid (29.0 g). A mixture of 6-methoxypyridazine-3-carboxylic acid (29.0 g), sulfuric acid (2 mL) and methanol (500 mL) was stirred at room temperature for 3 days. A saturated aqueous solution of sodium bicarbonate was added to the reaction mixture to adjust the pH to 7. The solvent was distilled off, and then, the residue was dissolved in water (200 mL), followed by extraction with ethyl acetate (200 mL) three times. The combined organic layer was washed with saturated brine, then dried over sodium sulfate and concentrated. The obtained residue was purified by column chromatography (ethyl acetate/petroleum ether) to obtain methyl 6-methoxypyridazine-3-carboxylate (22.0 g). A mixture of methyl 6-methoxypyridazine-3-carboxylate (4.5 g), hydrazine hydrate (80%, 13.4 g) and methanol (50 mL) was stirred overnight at room temperature. Water (10 mL) was added to the reaction mixture, and the mixture was concentrated. Methanol was distilled off from the mixture, and the residue was freeze-dried. The residue was washed with petroleum ether to obtain 6-methoxypyridazine-3-carbohydrazide (4.2 g). 2-Chloro-2-oxoethyl acetate (16.3 g) was added dropwise to a mixture of 6-methoxypyridazine-3-carbohydrazide (20 g), dichloromethane (250 mL) and water (10 mL) at 0°C. The mixture was stirred at 0°C for 30 minutes and then stirred at room temperature for 1.5 hours. The reaction mixture was concentrated under reduced pressure. Water (20 mL) was added to the obtained residue, and the mixture was freeze-dried. The residue was washed with petroleum ether to obtain 2-(2-((6-methoxypyridazin-3-yl)carbonyl)hydrazino)-2-oxoethyl acetate (25.0 g). A mixture of 2-(2-((6-methoxypyridazin-3-yl)carbonyl)hydrazino)-2-oxoethyl acetate (25.0 g), diphosphorus pentasulfide (21.4 g) and THF (600 mL) was stirred overnight at 50°C. The reaction mixture was cooled to room temperature, and the reaction was terminated by the addition of a saturated aqueous solution of sodium bicarbonate. The mixture was subjected to extraction with ethyl acetate (500 mL) three times. The combined organic layer was washed with saturated brine, then dried over sodium sulfate and concentrated. The obtained residue was purified by column chromatography (dichloromethane/methanol) to obtain (5-(6-hydroxypyridazin-3-yl)-1,3,4-thiadiazol-2-yl)methyl acetate (7.9 g). Amixture of (5-(6-hydroxypyridazin-3-yl)-1,3,4-thiadiazol-2-yl)methyl acetate (7.9 g), phosphoryl chloride (9.6 g) and acetonitrile (200

mL) was stirred at 70°C for 2 hours. The reaction mixture was cooled to room temperature. The reaction was terminated by the addition of water under ice cooling, and the mixture was subjected to extraction with ethyl acetate (200 mL) three times. The combined organic layer was washed with saturated brine, then dried over sodium sulfate and concentrated. The obtained residue was purified by column chromatography (ethyl acetate/petroleum ether) to obtain (5-(6-chloropyridazin-3-yl)-1,3,4-thiadiazol-2-yl)methyl acetate (2.9 g). To a mixture of (5-(6-chloropyridazin-3-yl)-1,3,4-thiadiazol-2-yl)methyl acetate (1.6 g) and THF (50 mL), lithium hydroxide (1 M aqueous solution, 14.8 mL) was added at 0°C. The mixture was stirred at 0°C for 1 hour and then stirred at room temperature for 1 hour. The solvent was distilled off, and then, the residue was subjected to extraction with ethyl acetate (50 mL) three times. The combined organic layer was washed with saturated brine, then dried over sodium sulfate and concentrated. The obtained residue was recrystallized from dichloromethane-methanol to obtain (5-(6-chloropyridazin-3-yl)-1,3,4-thiadiazol-2-yl)methanol (1.16 g). A mixture of 4-(4-(pentafluorosulfanyl)phenyl)-4-(trifluoromethyl)piperidine hydrochloride (200 mg), (5-(6-chloropyridazin-3-yl)-1,3,4-thiadiazol-2-yl)methanol (100 mg), DIPEA (0.650 mL) and IPA (4 mL) was stirred at 150°C for 1 hour under irradiation with microwave. The reaction mixture was concentrated under reduced pressure. The obtained residue was purified by column chromatography (ethyl acetate/hexane) to obtain the title compound (120 mg).
$^1$H NMR (300 MHz, DMSO-$d_6$) $\delta$ 2.05-2.25 (2H, m), 2.70-2.97 (4H, m), 4.37-4.56 (2H, m), 4.80-4.94 (2H, m), 6.18-6.34 (1H, m), 7.35-7.57 (1H, m), 7.90-8.19 (5H, m).

Compounds of Examples 3 to 15, 18, 20, 21, 28 to 32, 41, 47, 48, 50, 51, 56, 66, 73, 74, 76 to 81, 86 to 89, 91, 118 to 157, and 160 to 164 were produced according to the methods described above or methods equivalent thereto.

The compounds of Examples are shown in tables below. In the tables, MS is indicated by actually measured values.

[Table 1-1]

| Example No. | IUPAC name | Structural Formula | Salt | MS |
|---|---|---|---|---|
| 1 | (5-(6-(4-(2-Chlorobenzyl)-4-methoxypiperidin-1-yl)pyridazin-3-yl)-1,2,4-oxadiazol-3-yl)methyl benzoate | 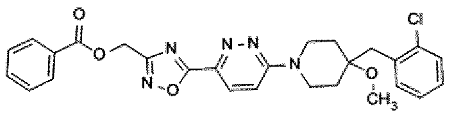 | | 520.1 |
| 2 | (5-(6-(4-(2-Chlorobenzyl)-4-methoxypiperidin-1-yl)pyridazin-3-yl)-1,2,4-oxadiazol-3-yl)methanol | 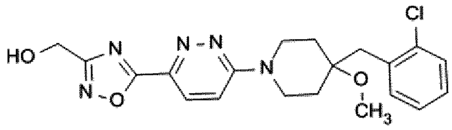 | | 416.1 |
| 3 | 4-Benzyl-1-(6-(3-methyl-1,2,4-thiadiazol -5-yl)pyridazin-3-yl)piperidine-4-carbonitrile | 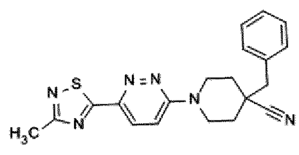 | | 377.2 |
| 4 | 3-(3-(3,4-Dichlorobenzyl)-3-ethoxyazetidin-1-yl)-6-(3-methyl-1,2,4-thiadiazol-5-yl)pyridazine | 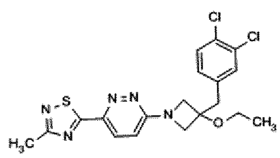 | | 436.1 |
| 5 | 3-(3-(3,4-Dichlorobenzyl)-3-methoxyazetidin-1-yl)-6-(3-methyl-1,2,4-thiadiazol-5-yl)pyridazine | 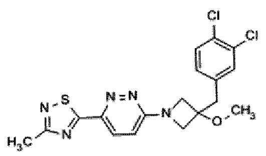 | | 422.0 |
| 6 | 4-(4-Chlorobenzyl)-1-(6-(3-methyl-1,2,4 -thiadiazol-5-yl)pyridazin-3-yl)piperidine-4-carbonitrile | 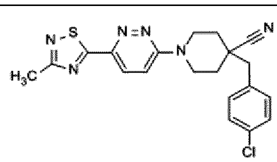 | | 411.1 |

(continued)

| Example No. | IUPAC name | Structural Formula | Salt | MS |
|---|---|---|---|---|
| 7 | 3-(4-(2-Chlorobenzyl)-4-methoxypiperidin-1-yl)-6-(3-methyl-1,2,4-thiadiazol-5-yl)pyridazine | 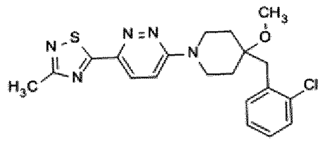 | | 416.1 |
| 8 | 3-(4-(3-Chlorobenzyl)-4-methoxypiperidin-1-yl)-6-(3-methyl-1,2,4-thiadiazol-5-yl)pyridazine | 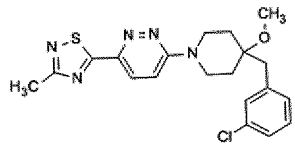 | | 416.0 |
| 9 | 3-(3-(2-Chlorobenzyl)-3-methoxypyrrolidin-1-yl)-6-(3-methyl-1,2,4-thiadiazol-5-yl)pyridazine | 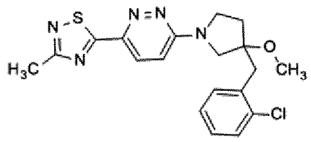 | | 402.1 |
| 10 | 3-(3-((2,5-Dichlorophenoxy)methyl)-3-methoxypyrrolidin-1-yl)-6-(3-methyl-1,2,4-thiadiazol-5-yl)pyridazine | 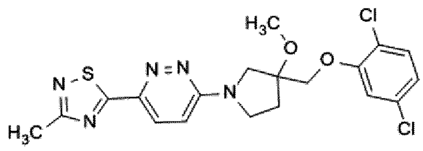 | | 452.0 |

[Table 1-2]

| Example No. | IUPAC name | Structural Formula | Salt | MS |
|---|---|---|---|---|
| 11 | (5-(6-(4-Methoxy-4-Phenylpiperidin-1-yl)pyridazin-3-yl)-1,2,4-oxadiazol-3-yl) methyl benzoate | 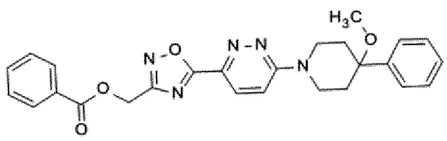 | | 472.2 |
| 12 | 3-(3-(Methoxymethyl)-1,2,4-oxadiazol-5-yl)-6-(4-methoxy-4-phenylpiperidin-1-yl) pyridazine | 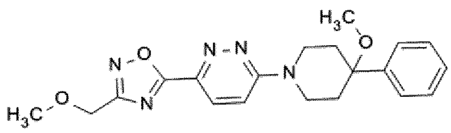 | | 382.1 |
| 13 | (5-(6-(4-Methoxy-4-phenylpiperidin-1-yl)pyridazin-3-yl)-1,2,4-oxadiazol-3-yl) methanol | 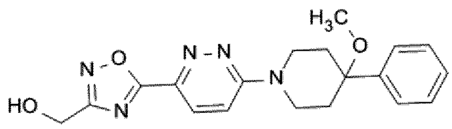 | | 368.1 |
| 14 | (5-(6-(4-(2-Chlorobenzyl)-4-cyanopiperidin-1-yl)pyridazin-3-yl)-1,2,4-oxadiazol-3-yl)methyl benzoate | 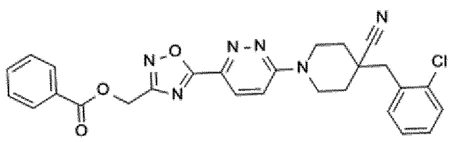 | | 515.1 |
| 15 | (5-(6-(4-(3-Chlorobenzyl)-4-cyanopiperidin-1-yl)pyridazin-3-yl)-1,2,4-oxadiazol-3-yl)methyl benzoate | 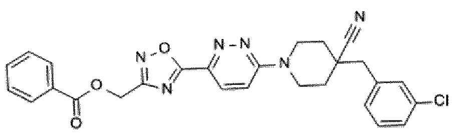 | | 515.1 |

(continued)

| Example No. | IUPAC name | Structural Formula | Salt | MS |
|---|---|---|---|---|
| **16** | 4-(2-Chlorobenzyl)-1-(6-(3-(hydroxymethyl)-1,2,4-oxadiazol-5-yl) pyridazin-3-yl)piperidine-4-carbonitrile | 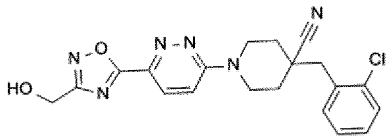 | | **411.1** |
| **17** | 4-(3-Chlorobenzyl)-1-(6-(3-(hydroxymethyl)-1,2,4-oxadiazol-5-yl) pyridazin-3-yl)piperidine-4-carbonitrile | 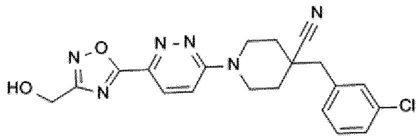 | | **411.1** |
| **18** | (5-(6-(4-(4-Chlorobenzyl)-4-cyanopiperidin-1-yl)pyridazin-3-yl)-1,2,4-oxadiazol-3-yl)methyl benzoate | 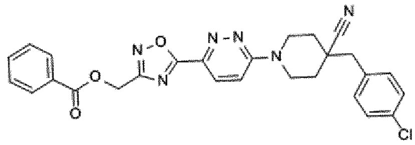 | | **515.1** |
| **19** | 4-(4-Chlorobenzyl)-1-(6-(3-(hydroxymethyl)-1,2,4-oxadiazol-5-yl) pyridazin-3-yl)piperidine-4-carbonitrile | 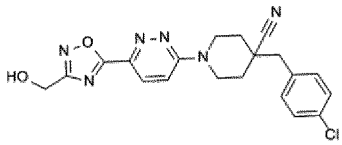 | | **411.1** |
| **20** | (5-(6-(4-Cyano-4-(4-fluorophenyl) piperidin-1-yl)pyridazin-3-yl)-1,2,4-oxadiazol-3-yl)methyl benzoate | 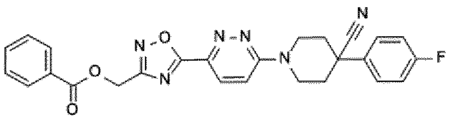 | | **485.1** |

[Table 1-3]

| Example No. | IUPAC name | Structural Formula | Salt | MS |
|---|---|---|---|---|
| 21 | (5-(6-(3-((2,5-Dichlorophenoxy)methyl)-3-methoxypyrrolidin-1-yl)pyridazin-3-yl)-1,2,4-oxadiazol-3-yl)methyl benzoate | 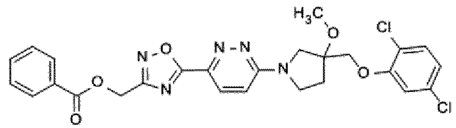 | | 556.1 |
| 22 | 4-(4-Fluorophenyl)-1-(6-(3-(hydroxymethyl)-1,2,4-oxadiazol-5-yl) pyridazin-3-yl)piperidine-4-carbonitrile | 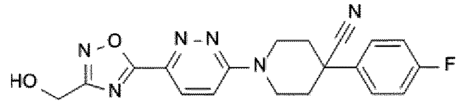 | | 381.1 |
| 23 | (5-(6-(3-((2,5-Dichlorophenoxy)methyl)-3-methoxypyrrolidin-1-yl)pyridazin-3-yl)-1,2,4-oxadiazol-3-yl)methanol | 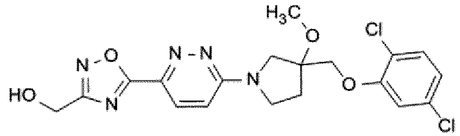 | | 452.1 |
| 24 | (5-(6-(4-(2-Chlorobenzyl)-4-(difluoromethyl)piperidin-1-yl)pyridazin-3-yl)-1,2,4-oxadiazol-3-yl)methanol | 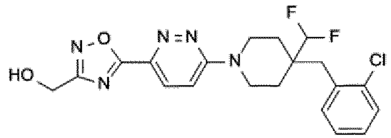 | | 436.1 |
| 25 | 4-(2-Chlorophenyl)-1-(6-(3-(hydroxymethyl)-1,2,4-oxadiazol-5-yl) pyridazin-3-yl)piperidine-4-carbonitrile | 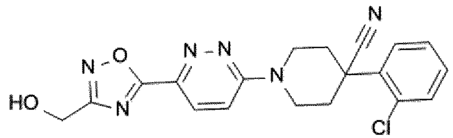 | | 397.1 |
| 26 | 4-(3-Chlorophenyl)-1-(6-(3-(hydroxymethyl)-1,2,4-oxadiazol-5-yl) pyridazin-3-yl)piperidine-4-carbonitrile | 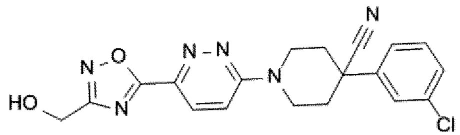 | | 397.1 |
| 27 | 4-(4-Chlorophenyl)-1-(6-(3-(hydroxymethyl)-1,2,4-oxadiazol-5-yl) pyridazin-3-yl)piperidine-4-carbonitrile | 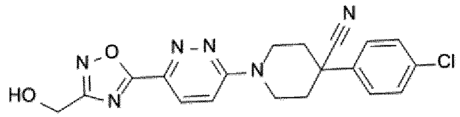 | | 397.1 |

(continued)

| Example No. | IUPAC name | Structural Formula | Salt | MS |
|---|---|---|---|---|
| 28 | (5-(6-(4-(2-Chlorophenyl)-4-cyanopiperidin-1-yl)pyridazin-3-yl)-1,2,4-oxadiazol-3-yl)methyl benzoate | 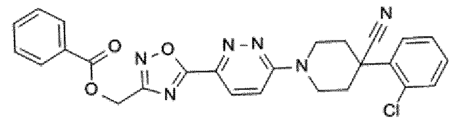 | | 501.1 |
| 29 | (5-(6-(4-(3-Chlorophenyl)-4-cyanopiperidin-1-yl)pyridazin-3-yl)-1,2,4-oxadiazol-3-yl)methyl benzoate | 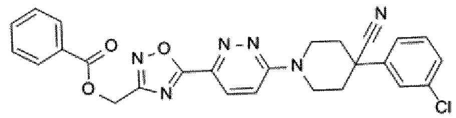 | | 501.1 |
| 30 | (5-(6-(4-(4-Chlorophenyl)-4-cyanopiperidin-1-yl)pyridazin-3-yl-1,2,4-oxadiazol-3-yl)methyl benzoate | 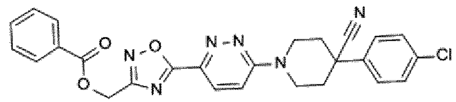 | | 501.1 |

[Table 1-4]

| Example No. | IUPAC name | Structural Formula | Salt | MS |
|---|---|---|---|---|
| 31 | 3-(4-(2-Chlorobenzyl)-4-(difluoromethyl) piperidin-1-yl)-6-(3-methyl-1,2,4-thiadiazol-5-yl)pyridazine | 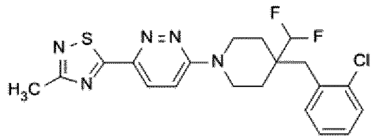 | | 436.1 |
| 32 | 4-(2-Chlorobenzyl)-1-(6-(3-methyl-1,2,4 -thiadiazol-5-yl)pyridazin-3-yl)piperidine-4-carbonitrile | 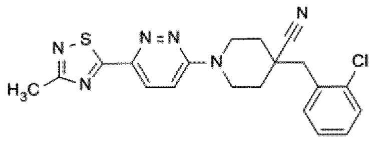 | | 411.1 |
| 33 | (5-(6-(4-(2-Chlorobenzyl)-4-cyanopiperidin-1-yl)pyridazin-3-yl)-1,3,4-oxadiazol-2-yl)methyl acetate | 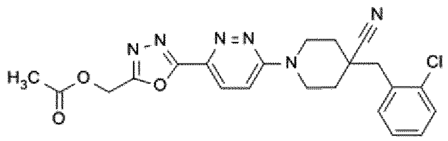 | | 453.1 |
| 34 | (5-(6-(4-(2-Chlorobenzyl)-4-(difluoromethyl)piperidin-1-yl)pyridazin-3-yl)-1,3,4-oxadiazol-2-yl)methyl acetate | 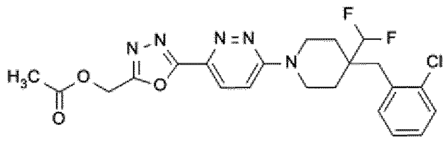 | | 478.1 |
| 35 | 4-(2-Chlorobenzyl)-1-(6-(5-(hydroxymethyl)-1,3,4-oxadiazol-2-yl) pyridazin-3-yl)piperidine-4-carbonitrile | 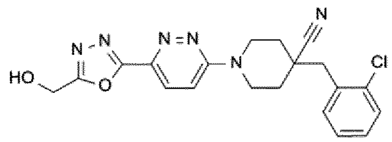 | | 411.1 |
| 36 | (5-(6-(4-(2-Chlorobenzyl)-4-(difluoromethyl)piperidin-1-yl)pyridazin-3-yl)-1,3,4-oxadiazol-2-yl)methanol | 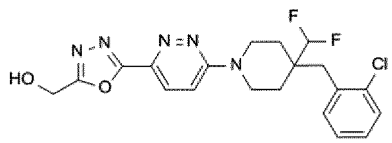 | | 436.1 |
| 37 | 1-(6-(3-(Hydroxymethyl)-1,2,4-oxadiazol-5-yl)pyridazin-3-yl)-4-(3-(trifluoromethyl) phenyl)piperidine-4-carbonitrile | 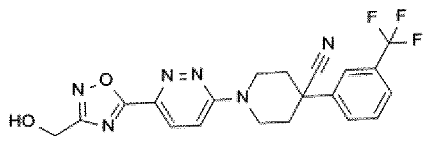 | | 431.1 |
| 38 | 1-(6-(3-(Hydroxymethyl)-1,2,4-oxadiazol-5-yl)pyridazin-3-yl)-4-(4-(trifluoromethyl) phenyl)piperidine-4-carbonitrile | 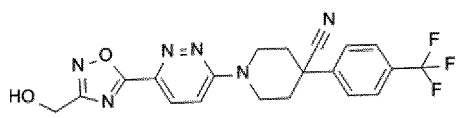 | | 431.0 |
| 39 | 3-((2,5-Dichlorophenoxy)methyl)-1-(6-(3-(hydroxymethyl)-1,2,4-oxadiazol-5-yl) pyridazin-3-yl)pyrrolidine-3-carbonitrile | 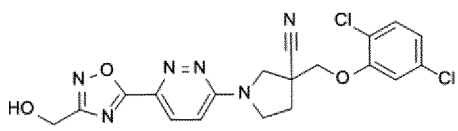 | | 446.9 |
| 40 | (5-(6-(3-((2,5-Dichlorophenoxy)methyl)-3-(trifluoromethyl)pyrrolidin-1-yl)pyridazin -3-yl)-1,2,4-oxadiazol-3-yl)methanol | 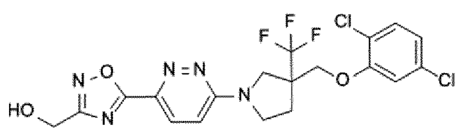 | | 490.0 |

[Table 1-5]

| Example No. | IUPAC name | Structural Formula | Salt | MS |
|---|---|---|---|---|
| **41** | (5-(6-(4-(2-Chloro-5-fluorophenoxy)-4-cyanopiperidin-1-yl)pyridazin-3-yl)-1,2,4-oxadiazol-3-yl)methyl benzoate | 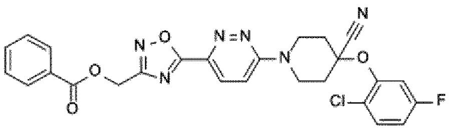 | | **535.1** |
| **42** | 4-(2-Chloro-5-fluorophenoxy)-1-(6-(3-(hydroxymethyl)-1,2,4-oxadiazol-5-yl) pyridazin-3-yl)piperidine-4-carbonitrile | 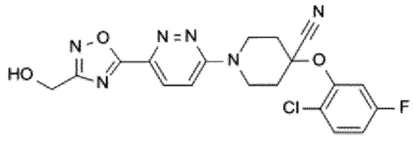 | | **431.0** |
| **43** | (5-(6-(4-(2-Chloro-5-fluorophenoxy)-4-cyanopiperidin-1-yl)pyridazin-3-yl)-1,3,4-oxadiazol-2-yl)methyl acetate | 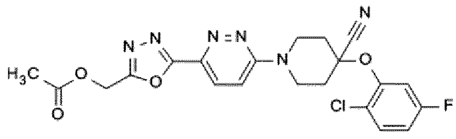 | | **473.1** |
| **44** | 4-(2-Chloro-5-fluorophenoxy)-1-(6-(5-(hydroxymethyl)-1,3,4-oxadiazol-2-yl) pyridazin-3-yl)piperidine-4-carbonitrile | 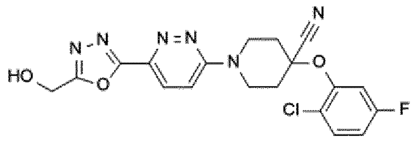 | | **431.0** |
| **45** | 4-(2-Chlorobenzyl)-1-(6-(1H-imidazol-1-yl)pyridazin-3-yl)piperidine-4-carbonitrile | 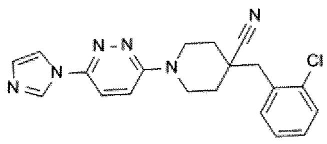 | | **379.1** |
| **46** | 4-(3,4-Dichlorophenyl)-1-(6-(3-(hydroxymethyl)-1,2,4-oxadiazol-5-yl) pyridazin-3-yl)piperidine-4-carbonitrile | 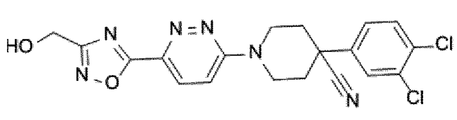 | | **431.0** |
| **47** | (5-(6-(4-Cyano-4-(1-naphthyl)piperidin-1-yl)pyridazin-3-yl)-1,2,4-oxadiazol-3-yl) methyl benzoate | 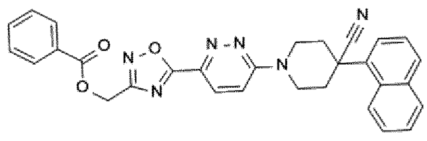 | | **517.1** |
| **48** | (5-(6-(4-Cyano-4-(2-naphthyl)piperidin-1-yl)pyridazin-3-yl)-1,2,4-oxadiazol-3-yl) methyl benzoate | 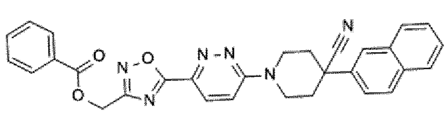 | | **517.1** |
| **49** | 1-(6-(3-(Hydroxymethyl)-1,2,4-oxadiazol-5-yl)pyridazin-3-yl)-4-(1-naphthyl) piperidine-4-carbonitrile | 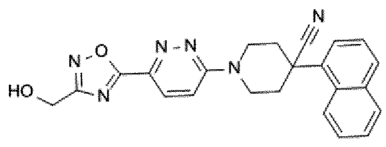 | | **413.1** |
| **50** | 4-(2-Chloro-5-fluorophenoxy)-1-(6-(3-methyl-1,2,4-thiadiazol-5-yl)pyridazin-3-yl)piperidine-4-carbonitrile | 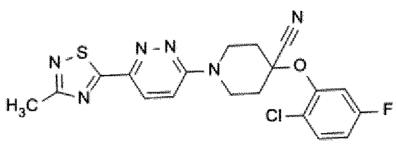 | | **431.0** |

[Table 1-6]

| Example No. | IUPAC name | Structural Formula | Salt | MS |
| --- | --- | --- | --- | --- |
| 51 | 1-(6-(3-(Hydroxymethyl)-1,2,4-oxadiazol-5-yl)pyridazin-3-yl)-4-(2-naphthyl) piperidine-4-carbonitrile | 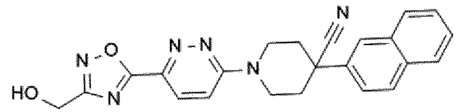 | | 413.1 |
| 52 | 4-(4-Bromophenyl)-1-(6-(3-(hydroxymethyl)-1,2,4-oxadiazol-5-yl) pyridazin-3-yl)piperidine-4-carbonitrile | 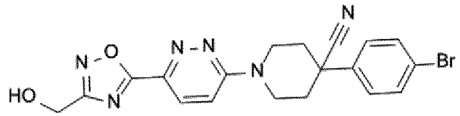 | | 441.0 |
| 53 | 1-(6-(3-(Hydroxymethyl)-1,2,4-oxadiazol-5-yl)pyridazin-3-yl)-4-(4-iodophenyl) piperidine-4-carbonitrile | 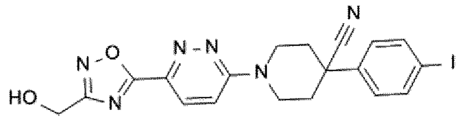 | | 488.9 |
| 54 | 4-(4-Ethynylphenyl)-1-(6-(3-(hydroxymethyl)-1,2,4-oxadiazol-5-yl) pyridazin-3-yl)piperidine-4-carbonitrile | 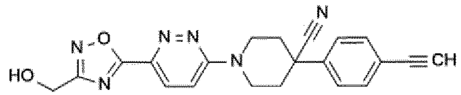 | | 387.1 |
| 55 | 1-(6-(3-(Hydroxymethyl)-1,2,4-oxadiazol-5-yl)pyridazin-3-yl)-4-(4-(trifluoromethoxy)phenyl)piperidine-4-carbonitrile | 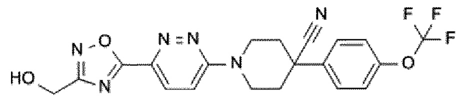 | | 447.0 |
| 56 | 1-(6-(3-(Hydroxymethyl)-1,2,4-oxadiazol-5-yl)pyridazin-3-yl)-4-(4-(methoxyphenyl)piperidine-4-carbonitrile | 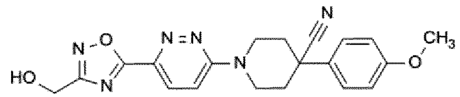 | | 393.1 |
| 57 | 1-(6-(3-(Hydroxymethyl)-1,2,4-oxadiazol-5-yl)pyridazin-3-yl)-4-(4-(pentafluorosulfanyl)phenyl)piperidine-4-carbonitrile | 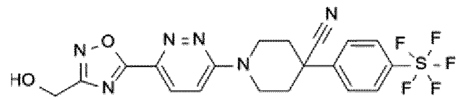 | | 489.1 |
| 58 | 1-(6-(5-(Hydroxymethyl)-1,3,4-oxadiazol-2-yl)pyridazin-3-yl)-4-(4-(pentafluorosulfanyl)phenyl)piperidine-4-carbonitrile | 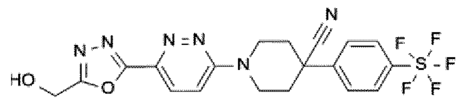 | | 489.1 |

(continued)

| Example No. | IUPAC name | Structural Formula | Salt | MS |
|---|---|---|---|---|
| 59 | 1-(6-(5-(Hydroxymethyl)-1,3,4-oxadiazol-2-yl)pyridazin-3-yl)-4-(4-(trifluoromethyl) phenyl)piperidine-4-carbonitrile | 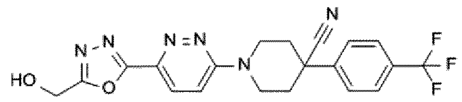 | | 431.0 |
| 60 | (5-(6-(4-Cyano-4-(4-(pentafluorosulfanyl)phenyl)piperidin-1-yl) pyridazin-3-yl)-1,3,4-oxadiazol-2-yl) methyl acetate | 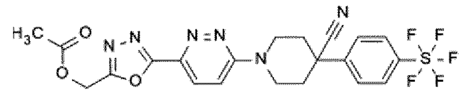 | | 531.0 |

[Table 1-7]

| Example No. | IUPAC name | Structural Formula | Salt | MS |
|---|---|---|---|---|
| 61 | (5-(6-(4-Cyano-4-(4-(trifluoromethyl) phenyl)piperidin-1-yl)pyridazin-3-yl)-1,3,4-oxadiazol-2-yl)methyl acetate | 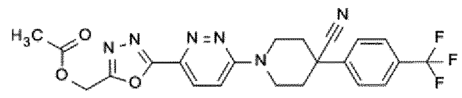 | | 473.1 |
| 62 | 4-(2-Chlorobenzyl)-1-(6-(1-methyl-1H-pyrazol-4-yl)pyridazin-3-yl)piperidine-4-carbonitrile | 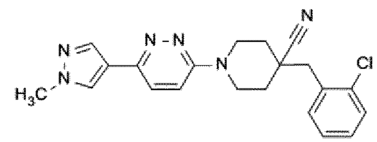 | | 393.2 |
| 63 | 4-(2-Chlorobenzyl)-1-(6-(1H-pyrazol-4-yl)pyridazin-3-yl)piperidine-4-carbonitrile | 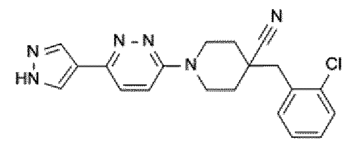 | | 379.2 |
| 64 | 4-(2-Chloro-4-fluorobenzyl)-1-(6-(3-(hydroxymethyl)-1,2,4-oxadiazol-5-yl) pyridazin-3-yl)piperidine-4-carbonitrile | 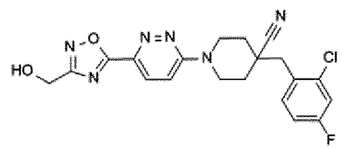 | | 429.1 |
| 65 | 4-(2-Chloro-4-fluorobenzyl)-1-(6-(5-(hydroxymethyl)-1,3,4-oxadiazol-2-yl) pyridazin-3-yl)piperidine-4-carbonitrile | 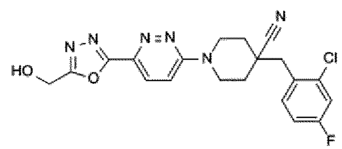 | | 429.1 |
| 66 | 4-(2-Chlorobenzyl)-1-(6-(1-methyl-1H-pyrazol-3-yl)pyridazin-3-yl)piperidine-4-carbonitrile | 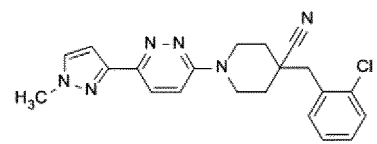 | | 393.1 |
| 67 | 4-(2-Chloro-5-fluorobenzyl)-1-(6-(5-(hydroxymethyl)-1,3,4-oxadiazol-2-yl) pyridazin-3-yl)piperidine-4-carbonitrile | 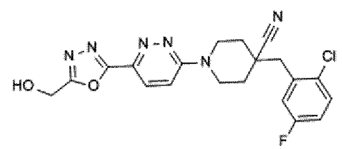 | | 429.1 |

(continued)

| Example No. | IUPAC name | Structural Formula | Salt | MS |
|---|---|---|---|---|
| 68 | 4-(2-Chloro-5-fluorobenzyl)-1-(6-(3-(hydroxymethyl)-1,2,4-oxadiazol-5-yl) pyridazin-3-yl)piperidine-4-carbonitrile | 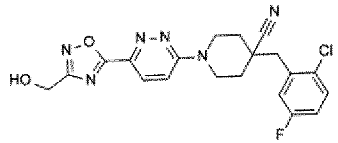 | | 429.1 |
| 69 | 3-(2-Chlorobenzyl)-1-(6-(3-(hydroxymethyl)-1,2,4-oxadiazol-5-yl) pyridazin-3-yl)azetidine-3-carbonitrile | 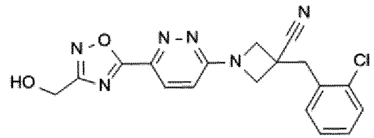 | | 383.0 |
| 70 | 1-(6-(3-(Hydroxymethyl)-1,2,4-oxadiazol-5-yl)pyridazin-3-yl)-4-(3-(pentafluorosulfanyl)phenyl)piperidine-4-carbonitrile | 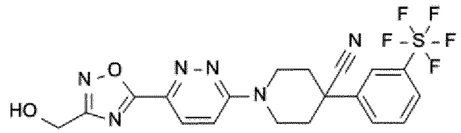 | | 489.1 |

[Table 1-8]

| Example No. | IUPAC name | Structural Formula | Salt | MS |
|---|---|---|---|---|
| 71 | (5-(6-(4-Phenyl-4-(trifluoromethyl) piperidin-1-yl)pyridazin-3-yl)-1,2,4-oxadiazol-3-yl)methanol | 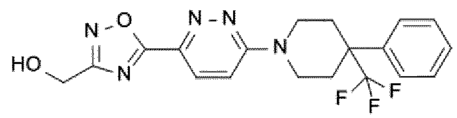 | | 406.1 |
| 72 | 4-((2-Chlorophenyl)(difluoro)methyl)-1-(6-(3-(hydroxymethyl)-1,2,4-oxadiazol-5-yl)pyridazin-3-yl)piperidine-4-carbonitrile | 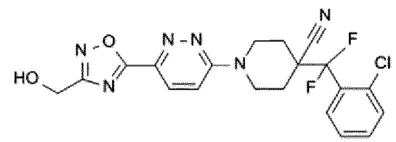 | | 447.0 |
| 73 | 1-(6-(5-(Hydroxymethyl)-1,3,4-oxadiazol-2-yl)pyridazin-3-yl)-4-(3-(pentafluorosulfanyl)phenyl)piperidine-4-carbonitrile | 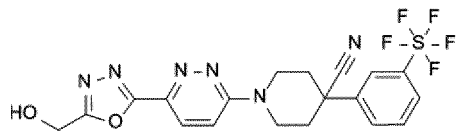 | | 489.1 |
| 74 | (5-(6-(4-Phenyl-4-(trifluoromethyl) piperidin-1-yl)pyridazin-3-yl)-1,3,4-oxadiazol-2-yl)methanol | 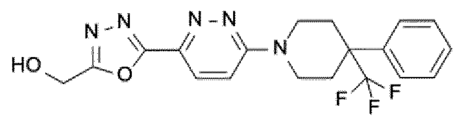 | | 406.1 |
| 75 | 4-((2-Chlorophenyl)(difluoro)methyl)-1-(6-(5-(hydroxymethyl)-1,3,4-oxadiazol-2-yl)pyridazin-3-yl)piperidine-4-carbonitrile | 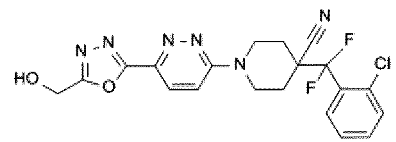 | | 447.0 |
| 76 | (5-(6-(4-Cyano-4-(3-(pentafluorosulfanyl)phenyl)piperidin-1-yl) pyridazin-3-yl)-1,2,4-oxadiazol-3-yl) methyl benzoate | 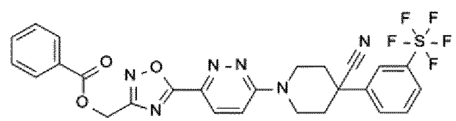 | | 593.1 |
| 77 | (5-(6-(4-Phenyl-4-(trifluoromethyl) piperidin-1-yl)pyridazin-3-yl)-1,2,4-oxadiazol-3-yl)methyl benzoate | 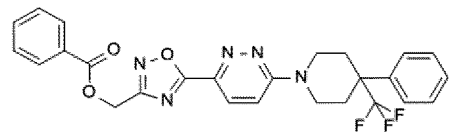 | | 510.1 |

(continued)

| Example No. | IUPAC name | Structural Formula | Salt | MS |
|---|---|---|---|---|
| 78 | (5-(6-(4-((2-Chlorophenyl)(difluoro) methyl)-4-cyanopiperidin-1-yl)pyridazin-3-yl)-1,2,4-oxadiazol-3-yl) methyl benzoate | 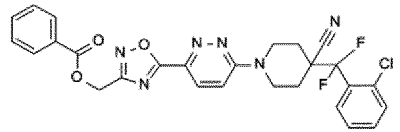 | | 551.1 |
| 79 | 1-(6-(5-(((4-Methoxybenzyl)oxy)methyl)-1,3,4-oxadiazol-2-yl)pyridazin-3-yl)-4-(3-(pentafluorosulfanyl)phenyl)piperidine-4-carbonitrile | 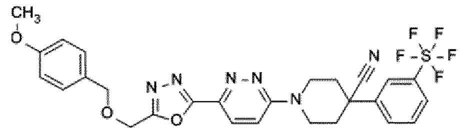 | | 609.1 |
| 80 | 3-(5-(((4-Methoxybenzyl)oxy)methyl)-1,3,4-oxadiazol-2-yl)-6-(4-phenyl-4-(trifluoromethyl)piperidin-1-yl)pyridazine | 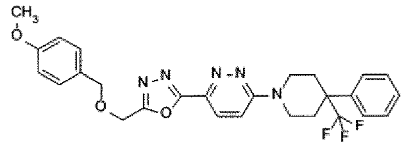 | | 526.1 |

[Table 1-9]

| Example No. | IUPAC name | Structural Formula | Salt | MS |
|---|---|---|---|---|
| 81 | 4-((2-Chlorophenyl)(difluoro)methyl)-1-(6-(5-(((4-methoxybenzyl)oxyl)methyl)-1,3,4-oxadiazol-2-yl)pyridazin-3-yl) piperidine-4-carbonitrile | 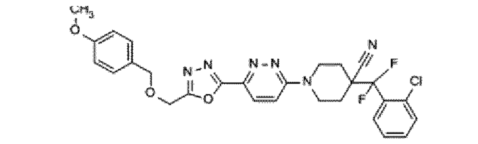 | | 567.1 |
| 82 | (5-(6-(4-(Trifluoromethyl)-4-(4-(trifluoromethyl)phenyl)piperidin-1-yl) pyridazin-3-yl)-1,2,4-oxadiazol-3-yl) methanol | 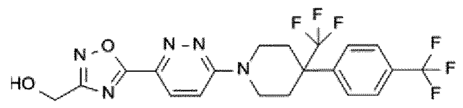 | | 474.1 |
| 83 | 4-(4-tert-Butylphenyl)-1-(6-(3-(hydroxymethyl)-1,2,4-oxadiazol-5-yl) pyridazin-3-yl)piperidine-4-carbonitrile | 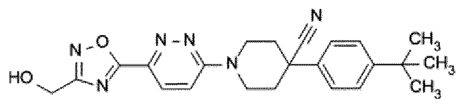 | | 419.2 |
| 84 | (5-(6-(4-(Trifluoromethyl)-4-(4-(trifluoromethyl)phenyl)piperidin-1-yl) pyridazin-3-yl)-1,3,4-oxadiazol-2-yl) methanol | 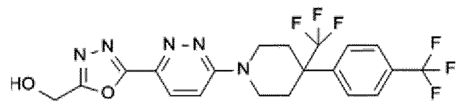 | | 474.0 |
| 85 | 4-(4-tert-Butylphenyl)-1-(6-(5-(hydroxymethyl)-1,3,4-oxadiazol-2-yl) pyridazin-3-yl)piperidine-4-carbonitrile | 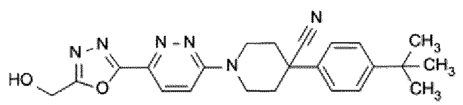 | | 419.2 |
| 86 | (5-(6-(4-(Trifluoromethyl)-4-(4-(trifluoromethyl)phenyl)piperidin-1-yl) pyridazin-3-yl)-1,2,4-oxadiazol-3-yl) methyl benzoate | 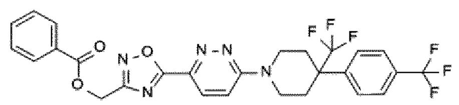 | | 578.0 |
| 87 | (5-(6-(4-(4-tert-Butylphenyl)-4-cyanopiperidin-1-yl)pyridazin-3-yl)-1,2,4-oxadiazol-3-yl)methyl benzoate | 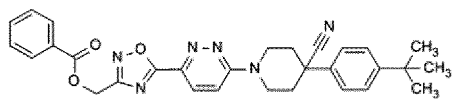 | | 523.2 |
| 88 | 3-(5-(((4-Methoxybenzyl)oxy)methyl)-1,3,4-oxadiazol-2-yl)-6-(4-(trifluoromethyl)-4-(4-(trifluoromethyl) phenyl)piperidin-1-yl)pyridazine | 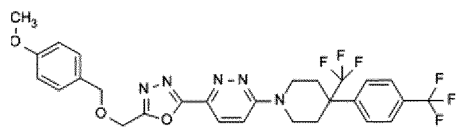 | | 594.0 |

(continued)

| Example No. | IUPAC name | Structural Formula | Salt | MS |
|---|---|---|---|---|
| 89 | 4-(4-tert-Butylphenyl)-1-(6-(5-(((4-methoxybenzyl)oxy)methyl)-1,3,4-oxadiazol-2-yl)pyridazin-3-y)piperidine-4-carbonitrile | 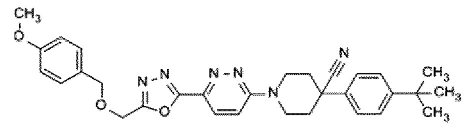 | | 539.2 |
| 90 | 4-(2-Chlorobenzyl)-1-(6-(5-(hydroxymethyl)-1,3,4-thiadiazol-2-yl) pyridazin-3-yl)piperidine-4-carbonitrile | 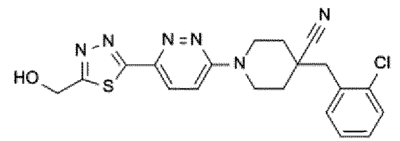 | | 427.0 |

[Table 1-10]

| Example No | IUPAC name | Structural Formula | Salt | MS |
|---|---|---|---|---|
| 91 | 4-(2-Chlorobenzyl)-1-(6-(5-(((4-methoxybenzyl)oxy)methyl)-1,3,4-thiadiazol-2-yl)pyridazin-3-yl)piperidine-4-carbonitrile | 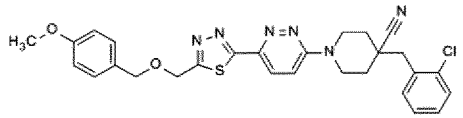 | | 547.1 |
| 92 | (5-(6-(4-Methoxy-4-(4-(pentafluorosulfanyl)phenyl)piperidin-1-yl) pyridazin-3-yl)-1,2,4-oxadiazol-3-yl) methanol | 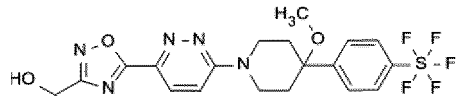 | | 494.1 |
| 93 | (5-(6-(4-Methoxy-4-(4-(pentafluorosulfanyl)phenyl)piperidin-1-yl) pyridazin-3-yl)-1,3,4-oxadiazol-2-yl) methanol | 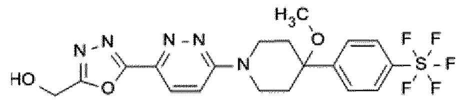 | | 494.1 |
| 94 | 4-(2-Chlorobenzyl)-1-(6-(5-(hydroxymethyl)-4-methyl-1,3-thiazol-2-yl)pyridazin-3-yl)piperidine-4-carbonitrile | 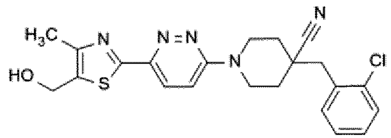 | | 440.0 |
| 95 | 4-(2-Chlorobenzyl)-1-(6-(5-(hydroxymethyl)-1,2,4-oxadiazol-3-yl) pyridazin-3-yl)piperidine-4-carbonitrile | 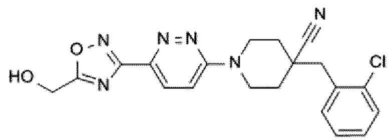 | | 411.1 |
| 96 | (3-(6-(4-(2-Chlorobenzyl)-4-cyanopiperidin-1-yl)pyridazin-3-yl)-1,2,4-oxadiazol-5-yl)methyl acetate | 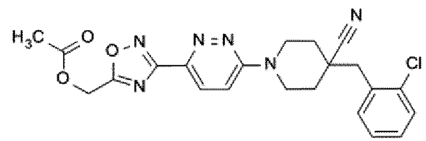 | | 453.1 |
| 97 | 4-(2-Bromobenzyl)-1-(6-(5-(hydroxymethyl)-1,3,4-oxadiazol-2-yl) pyridazin-3-yl)piperidine-4-carbonitrile | 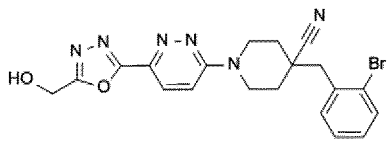 | | 455.0 |

(continued)

| Example No | IUPAC name | Structural Formula | Salt | MS |
|---|---|---|---|---|
| 98 | 1-(6-(5-(Hydroxymethyl)-1,3,4-oxadiazol-2-yl)pyridazin-3-yl)-4-(2-(trifluoromethyl) benzyl)piperidine-4-carbonitrile | 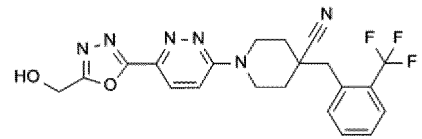 | | 445.1 |
| 99 | 4-(2-Bromobenzyl)-1-(6-(3-(hydroxymethyl)-1,2,4-oxadiazol-5-yl) pyridazin-3-yl)piperidine-4-carbonitrile | 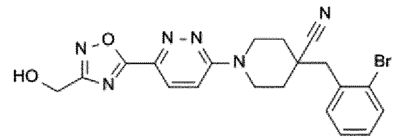 | | 455.1 |
| 100 | 1-(6-(3-(Hydroxymethyl)-1,2,4-oxadiazol-5-yl)pyridazin-3-yl)-4-(2-(trifluoromethyl) benzyl)piperidine-4-carbonitrile | 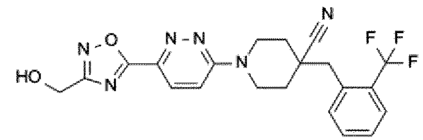 | | 445.1 |

[Table 1-11]

| Example No | IUPAC name | Structural Formula | Salt | MS |
|---|---|---|---|---|
| 101 | 1-(6-(5-(Hydroxymethyl)-1,3,4-oxadiazol-2-yl)pyridazin-3-yl)-4-(2-(pentafluorosulfanyl)benzyl)piperidine-4-carbonitrile | 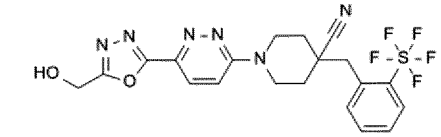 | | 503.0 |
| 102 | 1-(6-(3-Methyl-1,2,4-thiadiazol-5-yl) pyridazin-3-yl)-4-(4-(pentafluorosulfanyl) phenyl)piperidine-4-carbonitrile | 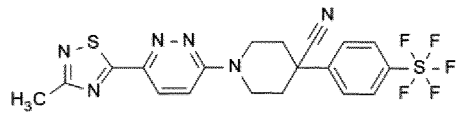 | | 489.0 |
| 103 | (5-(6-(4-Ethynyl-4-(4-(trifluoromethyl) phenyl)piperidin-1-yl)pyridazin-3-yl)-1,3,4 -oxadiazol-2-yl)methanol | 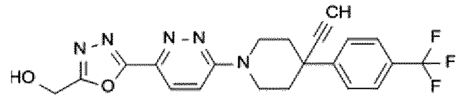 | | 430.1 |
| 104 | (5-(6-(4-Ethynyl-4-(4-(trifluoromethyl) phenyl)piperidin-1-yl)pyridazin-3-yl)-1,2,4 -oxadiazol-3-yl)methanol | 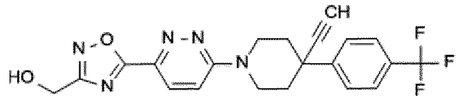 | | 430.1 |
| 105 | (5-(6-(4-(2,2,2-Trifluoroethyl)-4-(4-(trifluoromethyl)phenyl)piperidin-1-yl) pyridazin-3-yl)-1,3,4-oxadiazol-2-yl) methanol | 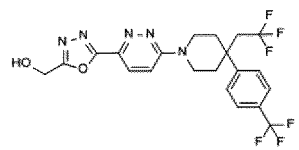 | | 488.0 |
| 106 | (5-(6-(4-(2,2,2-Trifluoroethyl)-4-(4-(trifluoromethyl)phenyl)piperidin-1-yl) pyridazin-3-yl)-1,2,4-oxadiazol-3-yl) methanol | 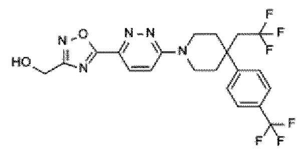 | | 488.1 |
| 107 | 4-(2-Chlorobenzyl)-1-(6-(1H-1,2,3-triazol-4-yl)pyridazin-3-yl)piperidine-4-carbonitrile | 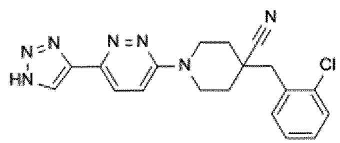 | | 380.1 |

(continued)

| Example No | IUPAC name | Structural Formula | Salt | MS |
|---|---|---|---|---|
| 108 | 1-(6-(3-(Hydroxymethyl)-1,2,4-thiadiazol -5-yl)pyridazin-3-yl)-4-(4-(pentafluorosulfanyl)phenyl)piperidine-4-carbonitrile | 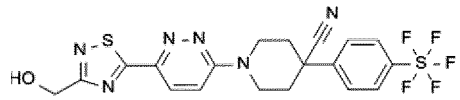 | | 505.1 |
| 109 | 1-(6-(5-(Hydroxymethyl)-1,3,4-thiadiazol -2-yl)pyridazin-3-yl)-4-(4-(pentafluorosulfanyl)phenyl)piperidine-4-carbonitrile | 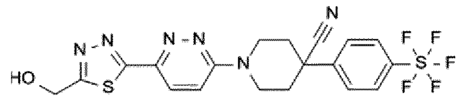 | | 505.1 |
| 110 | (5-(6-(4-Methoxy-4-(4-(pentafluorosulfanyl)phenyl)piperidin-1-yl) pyridazin-3-yl)-1,2,4-thiadiazol-3-yl) methanol | 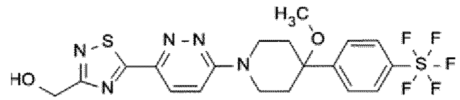 | | 510.0 |

[Table 1-12]

| Example No. | IUPAC name | Structural Formula | Salt | MS |
|---|---|---|---|---|
| 111 | (5-(6-(4-Methoxy-4-(4-(pentafluorosulfanyl)phenyl)piperidin-1-yl) pyridazin-3-yl)-1,3,4-thiadiazol-2-yl) methanol | 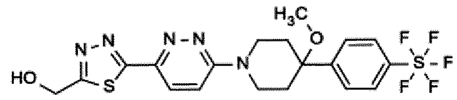 | | 510.1 |
| 112 | (5-(6-(4-(Trifluoromethyl)-4-(4-(trifluoromethyl)phenyl)piperidin-1-yl) pyridazin-3-yl)-1,2,4-thiadiazol-3-yl) methanol | 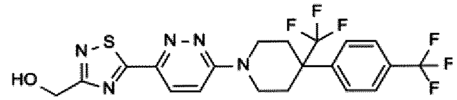 | | 490.0 |
| 113 | (5-(6-(4-(Trifluoromethyl)-4-(4-(trifluoromethyl)phenyl)piperidin-1-yl) pyridazin-3-yl)-1,3,4-thiadiazol-2-yl) methanol | 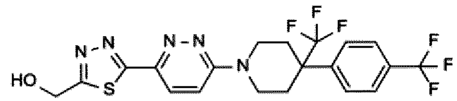 | | 489.9 |
| 114 | (5-(6-(3-((3,4-Dichlorophenyl)ethynyl)-3-methoxypyrrolidin-1-yl)pyridazin-3-yl)-1,2,4-thiadiazol-3-yl)methanol | 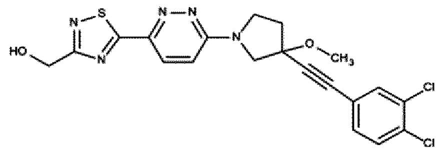 | | 462.1 |
| 115 | (5-(6-(4-(4-(Pentafluorosulfanyl)phenyl)-4-(trifluoromethyl)piperidin-1-yl)pyridazin -3-yl)-1,2,4-thiadiazol-3-yl)methanol | 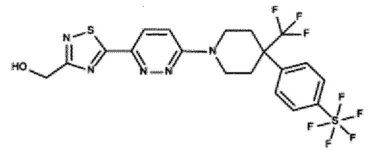 | | 548.0 |
| 116 | (5-(6-(4-(4-(Pentafluorosulfanyl)phenyl)-4-(trifluoromethyl)piperidin-1-yl)pyridazin -3-yl)-1,3,4-oxadiazol-2-yl)methanol | 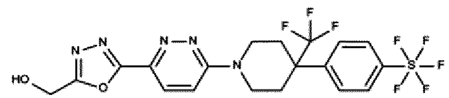 | | 532.0 |
| 117 | (5-(6-(4-(4-(Pentafluorosulfanyl)phenyl)-4-(trifluoromethyl)piperidin-1-yl)pyridazin -3-yl)-1,3,4-thiadiazol-2-yl)methanol | 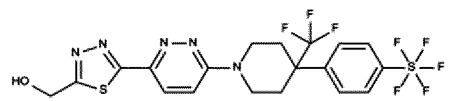 | | 548.0 |
| 118 | (5-(6-(4-(4-(Pentafluorosulfanyl)phenyl)-4-(trifluoromethyl)piperidin-1-yl)pyridazin -3-yl)-1,2,4-oxadiazol-3-yl)methanol | 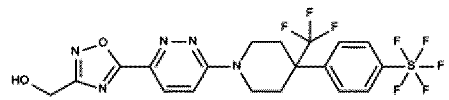 | | 531.9 |

(continued)

| Example No. | IUPAC name | Structural Formula | Salt MS | |
|---|---|---|---|---|
| 119 | (5-(6-(3-Methoxy-3-(phenylethynyl) pyrrolidin-1-yl)pyridazin-3-yl)-1,3,4-oxadiazol-2-yl)methanol | 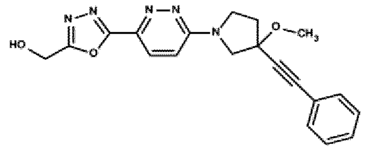 | | 378.2 |
| 120 | (5-(6-(4-Methoxy-4-(phenylethynyl) piperidin-1-yl)pyridazin-3-yl)-1,3,4-oxadiazol-2-yl)methanol | 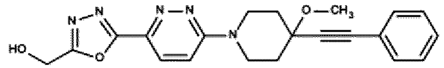 | | 392.1 |

[Table 1-13]

| Example No. | IUPAC name | Structural Formula | Salt | MS |
|---|---|---|---|---|
| **121** | (5-(6-(3-((2-Chlorophenyl)ethynyl)-3-methoxypyrrolidin-1-yl)pyridazin-3-yl)-1,2,4-thiadiazol-3-yl)methanol | 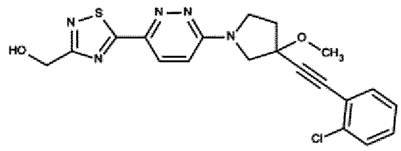 | | **428.0** |
| **122** | (5-(6-(3-((3-Chlorophenyl)ethynyl)-3-methoxypyrrolidin-1-yl)pyridazin-3-yl)-1,2,4-thiadiazol-3-yl)methanol | 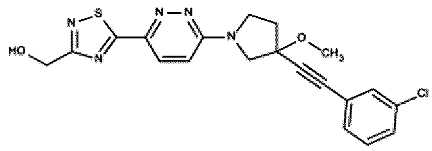 | | **428.0** |
| **123** | (5-(6-(3-Methoxy-3-((2-trifluoromethyl) phenyl)ethynyl)pyrrolidin-1-yl)pyridazin-3 -yl)-1,2,4-thiadiazol-3-yl)methanol | 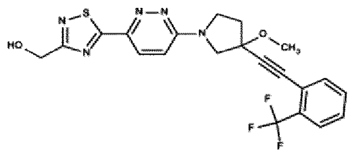 | | **462.0** |
| **124** | (5-(6-(3-Methoxy-3-((3-trifluoromethyl) phenyl)ethynyl)pyrrolidin-1-yl)pyridazin-3 -yl)-1,2,4-thiadiazol-3-yl)methanol | 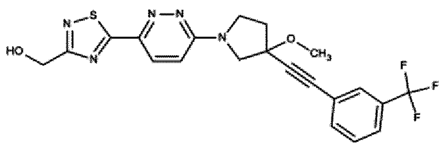 | | **462.0** |
| **125** | (5-(6-(3-Methoxy-3-((4-trifluoromethyl) phenyl)ethynyl)pyrrolidin-1-yl)pyridazin-3 -yl)-1,2,4-thiadiazol-3-yl)methanol | 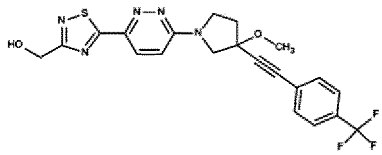 | | **462.0** |
| **126** | 2-((1-(6-(3-(Hydroxymethyl)-1,2,4-thiadiazol-5-yl)pyridazin-3-yl)-3-methoxypyrrolidin-3-yl)ethynyl) benzonitrile | 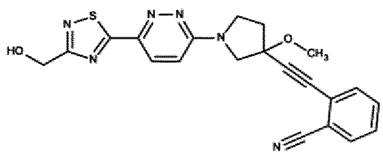 | | **419.0** |
| **127** | 3-((1-(6-(3-(Hydroxymethyl)-1,2,4-thiadiazol-5-yl)pyridazin-3-yl)-3-methoxypyrrolidin-3-yl)ethynyl) benzonitrile | 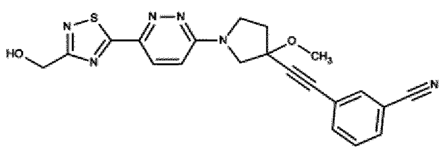 | | **419.0** |
| **128** | 4-((1-(6-(3-(Hydroxymethyl)-1,2,4-thiadiazol-5-yl)pyridazin-3-yl)-3-methoxypyrrolidin-3-yl)ethynyl) benzonitrile | 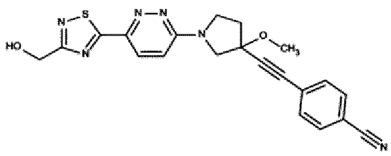 | | **419.0** |
| **129** | (5-(6-(3-Methoxy-3-((3-pentafluorosulfanyl)phenyl)ethynyl) pyrrolidin-1-yl)pyridazin-3-yl)-1,2,4-thiadiazol-3-yl)methanol | 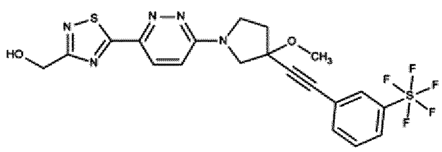 | | **520.0** |

(continued)

| Example No. | IUPAC name | Structural Formula | Salt | MS |
|---|---|---|---|---|
| **130** | (5-(6-(3-Methoxy-3-((4-pentafluorosulfanyl)phenyl)ethynyl) pyrrolidin-1-yl)pyridazin-3-yl)-1,2,4-thiadiazol-3-yl)methanol | 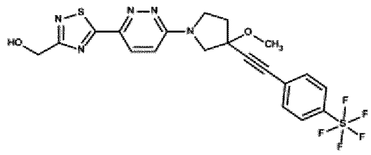 | | **520.0** |

[Table 1-14]

| Example No. | IUPAC name | Structural Formula | Salt | MS |
|---|---|---|---|---|
| 131 | (5-(6-(3-((2,4-Dichlorophenyl)ethynyl)-3-methoxypyrrolidin-1-yl)pyridazin-3-yl)-1,2,4-thiadiazol-3-yl)methanol | 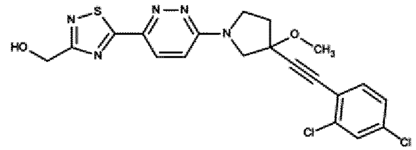 | | 462.0 |
| 132 | (5-(6-(3-((3,5-Dichlorophenyl)ethynyl)-3-methoxypyrrolidin-1-yl)pyridazin-3-yl)-1,2,4-thiadiazol-3-yl)methanol | 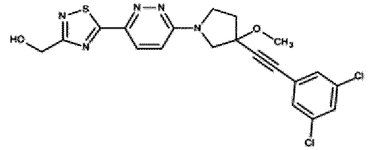 | | 461.9 |
| 133 | (5-(6-(3-((2,5-Dichlorophenyl)ethynyl)-3-methoxypyrrolidin-1-yl)pyridazin-3-yl)-1,2,4-thiadiazol-3-yl)methanol | 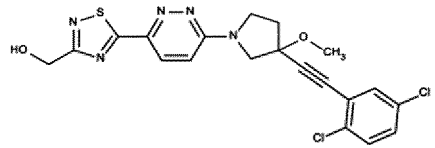 | | 462.0 |
| 134 | (5-(6-(3-((2,6-Dichlorophenyl)ethynyl)-3-methoxypyrrolidin-1-yl)pyridazin-3-yl)-1,2,4-thiadiazol-3-yl)methanol | 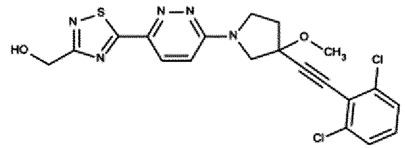 | | 461.9 |
| 135 | (5-(6-(3-((2,3-Dichlorophenyl)ethynyl)-3-methoxypyrrolidin-1-yl)pyridazin-3-yl)-1,2,4-thiadiazol-3-yl)methanol | 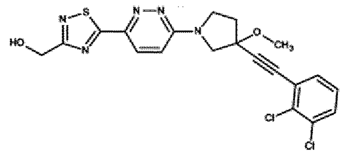 | | 462.0 |
| 136 | (5-(6-(3-Methoxy-3-((2-methoxyphenyl) ethynyl)pyrrolidin-1-yl)pyridazin-3-yl)-1,2,4-thiadiazol-3-yl)methanol | 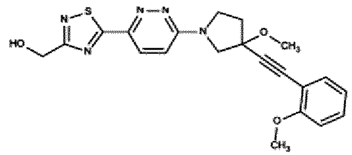 | | 424.0 |

(continued)

| Example No. | IUPAC name | Structural Formula | Salt | MS |
|---|---|---|---|---|
| 137 | (5-(6-(3-Methoxy-3-((3-methoxyphenyl) ethynyl)pyrrolidin-1-yl)pyridazin-3-yl)-1,2,4-thiadiazol-3-yl)methanol | 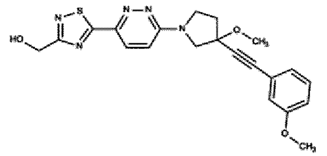 | | 424.0 |
| 138 | (5-(6-(3-Methoxy-3-((4-methoxyphenyl) ethynyl)pyrrolidin-1-yl)pyridazin-3-yl)-1,2,4-thiadiazol-3-yl)methanol | 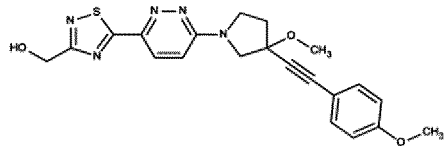 | | 424.0 |
| 139 | (5-(6-(3-Methoxy-3-((2-trifluoromethoxy) phenyl)ethynyl)pyrrolidin-1-yl)pyridazin-3 -yl)-1,2,4-thiadiazol-3-yl)methanol | 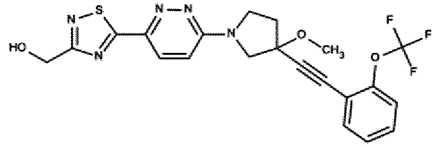 | | 478.0 |
| 140 | (5-(6-(3-Methoxy-3-((3-trifluoromethoxy) phenyl)ethynyl)pyrrolidin-1-yl)pyridazin-3 -yl)-1,2,4-thiadiazol-3-yl)methanol | 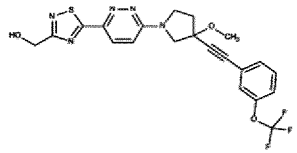 | | 478.0 |

[Table 1-15]

| Example No. | IUPAC name | Structural Formula | Salt | MS |
|---|---|---|---|---|
| 141 | (5-(6-(3-Methoxy-3-((4-(trifluoromethoxy)phenyl)ethynyl)pyrrolidin -1-yl)pyridazin-3-yl)-1,2,4-thiadiazol-3-yl)methanol | 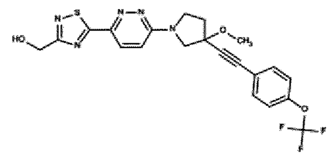 | | 478.0 |
| 142 | (5-(6-(3-((5-Chloro-2-thienyl)ethynyl)-3-methoxypyrrolidin-1-yl)pyridazin-3-yl)-1,2,4-thiadiazol-3-yl)methanol | 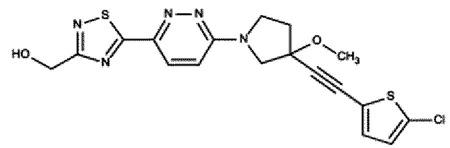 | | 434.0 |
| 143 | (5-(6-(3-Methoxy-3-(2-thienylethynyl) pyrrolidin-1-yl)pyridazin-3-yl)-1,2,4-thiadiazol-3-yl)methanol | 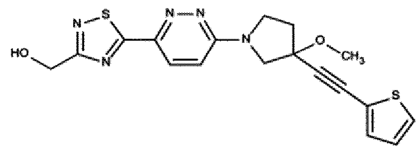 | | 400.0 |
| 144 | (5-(6-(3-Methoxy-3-(3-thienylethynyl) pyrrolidin-1-yl)pyridazin-3-yl)-1,2,4-thiadiazol-3-yl)methanol | 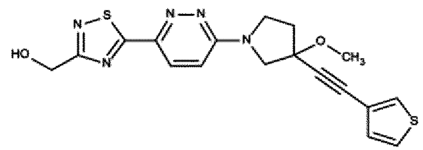 | | 400.0 |
| 145 | (5-(6-(3-Methoxy-3-(pyridin-2-ylethynyl) pyrrolidin-1-yl)pyridazin-3-yl)-1,2,4-thiadiazol-3-yl)methanol | 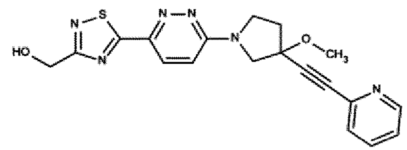 | | 395.1 |
| 146 | (5-(6-(3-Methoxy-3-(pyridin-3-ylethynyl) pyrrolidin-1-yl)pyridazin-3-yl)-1,2,4-thiadiazol-3-yl)methanol | 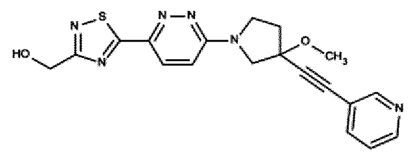 | | 395.1 |

(continued)

| Example No. | IUPAC name | Structural Formula | Salt | MS |
|---|---|---|---|---|
| 147 | (5-(6-(3-Methoxy-3-(pyridin-4-ylethynyl) pyrrolidin-1-yl)pyridazin-3-yl)-1,2,4-thiadiazol-3-yl)methanol | 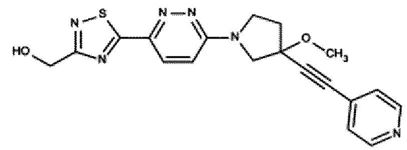 | | 395.1 |
| 148 | (5-(6-(3-Methoxy-3-(1-naphthylethynyl) pyrrolidin-1-yl)pyridazin-3-yl)-1,2,4-thiadiazol-3-yl)methanol | 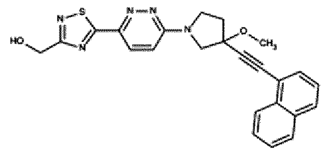 | | 444.1 |
| 149 | (5-(6-(3-Methoxy-3-(2-naphthylethynyl) pyrrolidin-1-yl)pyridazin-3-yl)-1,2,4-thiadiazol-3-yl)methanol | 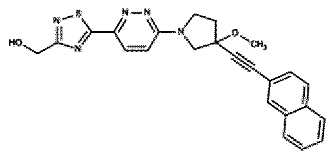 | | 444.1 |
| 150 | (5-(6-(3-Methoxy-3-(phenylethynyl) pyrrolidin-1-yl)pyridazin-3-yl)-1,2,4-thiadiazol-3-yl)methanol | 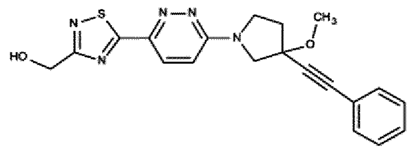 | | 394.1 |

[Table 1-16]

| Example No. | IUPAC name | Structural Formula | Salt | MS |
|---|---|---|---|---|
| 151 | (5-(6-(3-((4-Chlorophenyl)ethynyl)-3-methoxypyrrolidin-1-yl)pyridazin-3-yl)-1,2,4-thiadiazol-3-yl)methanol | 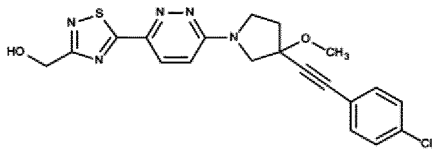 | | 428.1 |
| 152 | (5-(6-(4-(3-Fluoro-4-(trifluoromethyl) phenyl)-4-(trifluoromethyl)piperidin-1-yl) pyridazin-3-yl)-1,3,4-oxadiazol-2-yl) methanol | 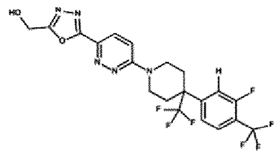 | | 492.0 |
| 153 | 1-(6-(5-(Hydroxymethyl)-1,3,4-thiadiazol-2-yl)pyridazin-3-yl)-4-phenylpiperidine-4-carbonitrile | 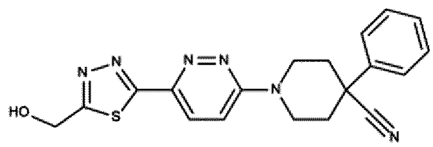 | | 379.0 |
| 154 | 4-Benzyl-1-(6-(3-(hydroxymethyl)-1,2,4-oxadiazol-5-yl)pyridazin-3-yl)piperidine-4-carbonitrile | 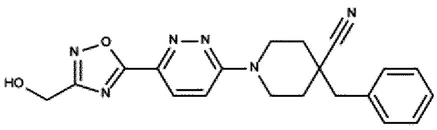 | | 377.1 |
| 155 | 1-(6-(3-(Hydroxymethyl)-1,2,4-thiadiazol-5-yl)pyridazin-3-yl)-4-(4-nitrophenyl) piperidine-4-carbonitrile | 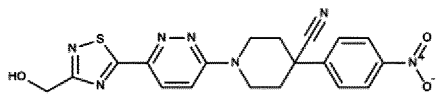 | | 424.1 |
| 156 | 1-(6-(3-(Hydroxymethyl)-1,2,4-oxadiazol-5-yl)pyridazin-3-yl)-4-phenylpiperidine-4-carbonitrile | 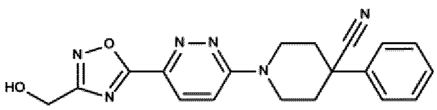 | | 363.2 |
| 157 | 4-(4-Aminophenyl)-1-(6-(3-(hydroxymethyl)-1,2,4-thiadiazol-5-yl) pyridazin-3-yl)piperidine-4-carbonitrile | 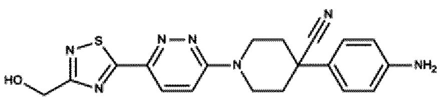 | | 394.2 |
| 160 | 1-(6-(3-Hydroxymethyl)-1,2,4-oxadiazol-5-yl)pyridazin-3-yl)-4-(4-nitrophenyl) piperidine-4-carbonitrile | 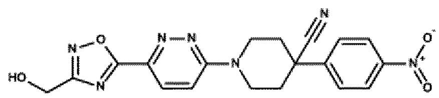 | | 408.1 |

[Table 1-17]

| Example No. | IUPAC name | Structural Formula | Salt | MS |
|---|---|---|---|---|
| 161 | 4-(4-Aminophenyl)-1-(6-(3-(hydroxymethyl)-1,2,4-oxadiazol-5-yl) pyridazin-3-yl)piperidine-4-carbonitrile | 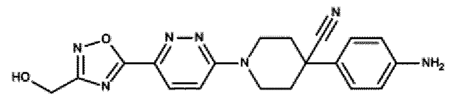 | | 378.2 |
| 162 | 1-(6-(3-(Hydroxymethyl)-1,2,4-oxadiazol-5-yl)pyridazin-3-yl)-4-(4-((trifluoromethyl)sulfonyl)phenyl)piperidine -4-carbonitrile | 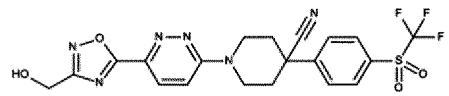 | | 495.1 |
| 163 | 1-(6-(5-(Hydroxymethyl)-1,3,4-oxadiazol-2-yl)pyridazin-3-yl)-4-(4-((trifluoromethyl)sulfonyl)phenyl)piperidine -4-carbonitrile | 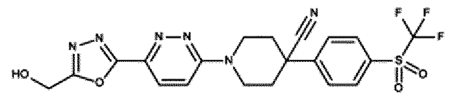 | | 495.1 |
| 164 | 1-(6-(3-(Hydroxymethyl)-1,2,4-thiadiazol-5-yl)pyridazin-3-yl)-4-(4-((trifluoromethyl)sulfonyl)phenyl)piperidine -4-carbonitrile | 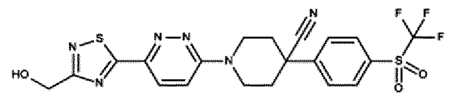 | | 511.1 |

Formulation Example 1

Each medicament containing the compound of the present invention as an active ingredient can be produced, for example, according to the following recipe:

[Expression 1]

| 1. Capsule | |
|---|---|
| (1) Compound obtained in Example 1 | 40 mg |
| (2) Lactose | 70 mg |
| (3) Microcrystalline cellulose | 9 mg |
| (4) Magnesium stearate | 1 mg |
| One capsule | 120 mg |

The components (1), (2) and (3) and a 1/2 amount of the component (4) are mixed and then granulated. The remaining amount of the component (4) is added thereto, and the whole is encapsulated in a gelatin capsule shell.

[Expression 2]

| 2. Tablet | |
|---|---|
| (1) Compound obtained in Example 1 | 40 mg |
| (2) Lactose | 58 mg |
| (3) Corn starch | 18 mg |
| (4) Microcrystalline cellulose | 3.5 mg |
| (5) Magnesium stearate | 0.5 mg |
| One tablet | 120 mg |

The components (1), (2) and (3), a 2/3 amount of the component (4) and a 1/2 amount of the component (5) are mixed and then granulated. The remaining amounts of the components (4) and (5) are added to this granule, which is then molded into a tablet by compression.

Formulation Example 2

50 mg of the compound obtained in Example 1 is dissolved in 50 mL of Japanese Pharmacopoeia distilled water for injection, and then, the amount of the solution is adjusted to 100 mL by the addition of Japanese Pharmacopoeia distilled water for injection. This solution is filtered under sterile conditions. Next, vials for injection are filled with this solution at 1 mL/vial under sterile conditions, freeze-dried, and hermetically sealed.

Test Example 1 (Testing method: Measurement of SCD inhibitory activity using microsome (TLC detection system))

A microsome fraction was produced and preserved as follows: first, the liver was excised from a rat, thoroughly washed with PBS and then chopped. A microsome preparation buffer (0.25 M sucrose, 10 mM Tris-HCl, pH 7.4, protease inhibitor cocktail (F. Hoffmann-La Roche, Ltd.)) was added to the chopped liver, and the cells were disrupted using a POLYTORON homogenizer on ice. The homogenate thus obtained by the disruption was centrifuged at 10,000 × g at 4°C for 30 minutes to collect a supernatant. The centrifugation operation described above was repeated again for the obtained supernatant to obtain a supernatant again. The obtained supernatant was centrifuged at 40,000 × g at 4°C for 60 minutes, and the obtained precipitate other than the glycogen layer was used as the microsome fraction. The protein concentration of the microsome fraction was measured using BCA Protein Assay Kit (Pierce/Thermo Fisher Scientific, Inc.), and the microsome fraction was cryopreserved at -80°C until use.

The SCD inhibitory activity of each test compound was measured as follows: first, the test compound was diluted into 10 mM with DMSO to obtain a primary dilution. The obtained primary dilution was diluted 3/1000 by the addition of 3 × buffer (300 mM potassium phosphate, pH 7.4, 450 mM KCl, 30 mM NaF, 9 mM $MgCl_2$, 4.5 mM glutathione reduced form (Wako Pure Chemical Industries, Ltd.), 0.3% BSA fatty acid free (Sigma-Aldrich Corp.) to obtain a secondary dilution. The secondary dilution was dispensed at 10 μL/well to a polypropylene 96-deep well block. Then, the microsome fraction diluted using a microsome buffer (10 mM Tris-HCl, pH 7.5, 250 mM sucrose) was added thereto at 10 μL/well (containing 10 μg of the protein). An enzyme reaction solution containing [$^{14}$C]stearoyl-CoA (American Radiolabeled Chemicals, Inc.) diluted into 10 μCi/mL using 9 mM NADH (Sigma-Aldrich Corp.) was further added thereto at 10 μL/well.

30 minutes thereafter, 2.5 N NaOH was added thereto at 10 μL/well. After the completion of the addition, the deep well block was provided with a plate seal and then left standing overnight in a heat sterilizer set to 55°C. Subsequently to the standing, fatty acid extraction from the sample was carried out in accordance with the Bligh & Dyer method. Specifically, 200 μL of a formic acid:methanol:chloroform (1:6:3) solution was added to the sample thus left standing, and then, the mixture was stirred while the single-layer state was maintained. Further, 120 μL of pure water was added to the mixture to separate it into two layers. After the separation, a 10 μL aliquot was separated from the lower layer, spotted on a reverse-phase TLC plate (RP-18, 1154230001, Merck KGaA), and developed with an acetonitrile:pure water:acetic acid (95:4.5:0.5) solution. After drying, the TLC plate was transferred to Imaging Plate (Fuji Photo Film) for 5 hours or longer. Detection was carried out using FLA7000 (GE Healthcare Japan Corp.), and the obtained spot images were digitalized using ImageQuant TL (GE Healthcare Japan Corp.) to determine the rate of inhibition of SCD activity (%).

The test results are shown in Table 2.

[Table 2]

Table 2

| Example No. | Rate of inhibition of SCD activity at compound concentration of 1 μM |
|---|---|
| 58 | 92% |
| 82 | 95% |
| 84 | 93% |
| 93 | 94% |
| 108 | 95% |
| 109 | 93% |
| 110 | 94% |
| 111 | 96% |
| 112 | 96% |
| 113 | 95% |

These results demonstrated that the compound of the present invention strongly inhibits SCD activity.

Test Example 2 Measurement of SCD inhibitory activity using human large intestine cancer cell line (HCT116 cell)

HCT116 (ATCC (American Type Culture Collection)) was inoculated at $1 \times 10^5$ cells/well to a 96-well plate and cultured overnight in DMEM (Dulbecco's modified Eagle medium) containing 10% FBS (fetal bovine serum) (AusGeneX PTY LTD.). After the culture, each well was washed twice with phosphate-buffered saline (PBS) (200 μL). Then, a reaction medium (DMEM, penicillin/streptomycin, 0.3% BSA) containing 1.5 μM of the test compound was added thereto at 40 μL/well, and the plate was left standing for 15 minutes in a $CO_2$ incubator (37°C). A reaction medium containing 0.1 μCi of [$^{14}$C]stearic acid was added at 20 μL/well to the plate thus left standing, and the plate was left standing for 3 hours in a $CO_2$ incubator (37°C). Each well of the plate thus left standing was washed twice with 200 μL of PBS. Then, 2.5 N NaOH was added thereto at 20 μL/well, and the plate was provided with a plate seal and then incubated overnight in a heat sterilizer set to 55°C. A formic acid:methanol:chloroform (1:16:4) solution was added at 200 μL/well to the plate thus left standing. Further, the mixture in each well was separated into two layer by the addition of 200 μL of pure water. After the separation, a 10 μL aliquot was separated from the lower layer, spotted on a reverse-phase TLC plate (RP-18, 1154230001, Merck KGaA), and developed with an acetonitrile:pure water:acetic acid (95:4.5:0.5) solution. After drying, the TLC plate was transferred to Imaging Plate (Fuji Photo Film) for 5 hours or longer. Detection was carried out using FLA7000 (GE Healthcare Japan Corp.), and the obtained spot images were digitalized using ImageQuant TL (GE Healthcare Japan Corp.) to determine the rate of inhibition of SCD activity (%).

The test results are shown in Table 3.

[Table 3]

Table 3

| Example No. | Rate of inhibition of SCD activity at compound concentration of 1 μM |
|---|---|
| 58 | 97% |

(continued)

| Example No. | Rate of inhibition of SCD activity at compound concentration of 1 μM |
|---|---|
| 82 | 99% |
| 84 | 99% |
| 93 | 99% |
| 108 | 100% |
| 109 | 96% |
| 110 | 98% |
| 111 | 101% |
| 112 | 97% |
| 113 | 99% |

These results demonstrated that the compound of the present invention strongly inhibits SCD activity in large intestine cancer cells.

Test Example 3 (Measurement of growth inhibitory activity using HCT116 cell)

Each compound was evaluated for its growth inhibitory activity against HCT116 cells (ATCC) by the following method: the HCT116 cells were inoculated at 900 cells/40 μL/well to a 384-well white plate (Greiner Bio-one) and maintained in an assay medium (RPMI medium (Wako Pure Chemical Industries, Ltd.) containing 2% fetal bovine serum, 50 units/mL penicillin and 50 μg/mL streptomycin (Invitrogen Corp.)). On the next day, the test compound dissolved in dimethyl sulfoxide (DMSO) was dispensed to the plate using TECAN D300 digital dispenser (Tecan Trading AG). Then, the concentration of DMSO in the medium was adjusted to 0.1% by the addition of DMSO. The HCT116 cells supplemented with the compound were incubated at 37°C in a 5% $CO_2$ environment (test compound addition group).

Also, the same reaction as above was carried out without the addition of the test compound (test compound non-addition group).

Further, the same reaction as above was carried out on condition that neither the HCT116 cells nor the test compound were added (control group).

After 3 days, 20 μL of CellTiter-Glo reagent (Promega Corp.) was added to each well, and the mixture was stirred for 10 minutes. Then, the light emission of each well was measured using ARVO MX1420 (PerkinElmer, Inc.).

The rate of inhibition of HCT116 cell growth (%) was determined according to the following calculation expression:

Rate of inhibition of cell growth = (1 - (Light emission of the test compound addition group - Light emission of the control group) / (Light emission of the test compound non-addition group - Light emission of the control group)) × 100

The test results are shown in Table 4.

[Table 4]

Table 4

| Example No. | Rate of inhibition of cell growth at compound concentration of 1 μM |
|---|---|
| 58 | 88% |
| 82 | 89% |
| 84 | 88% |
| 93 | 85% |
| 108 | 90% |

(continued)

| Example No. | Rate of inhibition of cell growth at compound concentration of 1 μM |
|---|---|
| 109 | 88% |
| 110 | 89% |
| 111 | 86% |
| 112 | 90% |
| 113 | 84% |

These results demonstrated that the compound of the present invention strongly inhibits the growth of large intestine cancer cells.

Test Example 4 Measurement of human SCD1 binding activity using (5-(6-(1'H-spiro[1-benzofuran-3,3'-pyrrolidin]-1'-yl)pyridazin-3-yl)-1,2,4-oxadiazol-3-yl)[$^3H_2$]methanol

The SCD1 binding activity of the compound of the present invention was measured as the activity of inhibiting the binding of (5-(6-(1'H-spiro[1-benzofuran-3,3'-pyrrolidin]-1'-yl)pyridazin-3-yl)-1,2,4-oxadiazol-3-yl)[$^3H_2$]methanol (radiolabeled form of the compound disclosed as a SCD inhibitor in Example 157 of International Publication No. WO2008/096746; in the present specification, also referred to as compound Z) to SCD1.

Specifically, first, Human liver cDNA library (Takara Bio Inc.) was cloned as a template by PCR using KOD-Plus- (Toyobo Co., Ltd.) DNApolymerase. The primers used were 5'-agcctcagccccctggaaagt-3' and 5'-ggaccccaaactcagccactct-3'. The amplification fragment was temporarily transferred to a pCR Blunt II-TOPO vector using Zero Blunt PCR Cloning Kit (Life Technologies, Inc.) to obtain pCR Blunt II-TOPO/hSCD1. A fragment obtained by the cleavage of this pCR Blunt II-TOPO/hSCD1 with BamHI and NotI was transferred to pFastBac1 (Life Technologies, Inc.) to construct a recombinant plasmid pFastBac1/hSCD1 for hSCD1 insect cell expression systems.

Baculovirus was prepared using the pFastBac1/hSCD1 plasmid according to the protocol of Bac-to-Bac Baculovirus Expression System (Life Technologies, Inc.).

The insect cells for use in expression were High Five cells (Life Technologies, Inc.) subcultured in a PSFM-J1 medium (Wako Pure Chemical Industries, Ltd.) containing 5% FBS, 10 U/L heparin (Sigma-Aldrich Corp.) and 50 μg/mL gentamicin.

The High Five cells were inoculated at $0.8 \times 10^6$ cells/mL and cultured until the following day. When the cell density reached $2.0 \times 10^6$ cells/mL, Ammonium Iron(III) Sulfate 12-Water (Wako Pure Chemical Industries, Ltd.) was added thereto at a final concentration of 10 μM. The cells were further infected with the baculovirus and cultured for 3 days.

After the completion of the culture, the culture solution was centrifuged (4°C, 4,000 rpm, 10 min) and frozen at -80°C to obtain hSCD1-expressing insect cells. Next, the frozen hSCD1-expressing insect cells were suspended in buffer A (50 mM Tris-HCl (pH 7.5) containing 250 mM sucrose and cOmplete Protease Inhibitor Cocktail Tablets (F. Hoffmann-La Roche, Ltd.)), and the cells were disrupted (4°C, 20,000 rpm, 20 sec × 2) using a POLYTORON homogenizer (Central Scientific Commerce, Inc.).

This cell lysate was centrifuged (4°C, 850 × g, 10 min), and the supernatant was further ultracentrifuged (4°C, 186,000 × g, 60 min). The supernatant was discarded, and buffer A was added to the precipitate. The resultant was suspended using POLYTRON (4°C, 10,000 rpm, 10 sec × 2) and passed through a 40-μm cell strainer to prepare a human SCD1-expressing membrane fraction (hereinafter, also simply referred to as human SCD1). The protein concentration was measured using BCA Protein Assay Kit (Thermo Fisher Scientific, Inc.) with BSA used as standards.

The human SCD1 binding activity of each test compound was measured as follows: first, the test compound was diluted into 10 mM with DMSO to obtain a primary dilution. The obtained primary dilution was diluted 250-fold by the addition of an assay buffer (10 mM Tris-HCl, pH 7.5, 100 mM KCl, 10 mM NaF, 3 mM $MgCl_2$, 0.005% Tween 20, 1 mM GSH) to obtain a secondary dilution. The human SCD1 diluted using an assay buffer was dispensed at 100 μL/well (containing 0.2 μg of the protein) to a polypropylene 96-well plate, and then, the secondary dilution of the compound was added thereto at 50 μL/well. Further, a solution of 12 nM compound Z diluted with an assay buffer was added thereto at 50 μL/well, and the plate was left standing at room temperature for 2 hours. Then, the contents in each well were transferred to each well of UniFilter-96 GF/C PEI coated plate (PerkinElmer, Inc.) wetted with a washing buffer, followed by washing five times with a washing buffer (10 mM Tris-HCl, pH 7.5). After drying of the filter plate, Microscint-O (PerkinElmer, Inc.) was added thereto at 25 μL/well, and the obtained radioactivity was measured using TopCount (PerkinElmer, Inc.). Also, the same reaction as above was carried out on condition that only DMSO was added without the addition of the test compound (test compound non-addition group). Further, the same reaction as above was carried

out on condition that 10 μM 5-(6-(1'H-spiro[1-benzofuran-3,3'-pyrrolidin]-1'-yl)pyridazin-3-yl)-1,2,4-oxadiazol-3-yl)methanol (unlabeled form of compound Z) was added (control group). The activity of inhibiting the binding of compound Z to human SCD1 by the compound of the present invention (the rate of inhibition of SCD1 binding (%)) was determined as follows:

$$\text{Rate of inhibition of binding (\%)} = (1 - (\text{Radioactivity of the test compound addition group} - \text{Radioactivity of the control group}) / (\text{Radioactivity of the test compound non-addition group} - \text{Radioactivity of the control group})) \times 100$$

The test results are shown in Table 5.

[Table 5]

| Example No. | Rate of inhibition of SCD1 binding (%) at compound concentration of 1 μM |
|---|---|
| 38 | 95 |
| 58 | 89 |
| 82 | 99 |
| 84 | 99 |
| 93 | 98 |
| 108 | 99 |
| 109 | 97 |
| 110 | 100 |
| 111 | 93 (Rate of inhibition of SCD1 binding at compound concentration of 0.1 μM) |
| 112 | 99 |
| 113 | 100 |

These results demonstrated that the compound of the present invention has SCD1 binding activity.
In addition, the results of Test Examples 1, 2 and 4 demonstrated that the compound of the present invention is useful as a SCD1 inhibitor.

Test Example 5

The compound of the present invention was evaluated for its antitumor effect on human large intestine cancer cell line HCT116 cancer-bearing mice by the following method.
The human large intestine cancer cell line HCT116 (purchased from ATCC (American Type Culture Collection)) was transplanted to each 6-week-old BALB/c female nude mouse (BALB/cA Jcl nu/nu: CLEA Japan Inc.) by the subcutaneous injection of $1.0 \times 10^6$ cells. After the transplantation, successfully engrafted tumor was observed, and the tumor size was measured 10 to 14 days after the transplantation. The tumor volume was calculated according to the following expression:

$$\text{Tumor volume} = \text{Major axis} \times \text{Minor axis} \times \text{Minor axis} \times (1/2)$$

Individuals in which the volume of the engrafted tumor reached approximately 150 $mm^3$ were selected and used in the experiment as groups each involving 5 to 8 individuals. Each test compound was suspended in a 0.5% methylcellulose solution and orally administered to each individual for 14 days using the dose and the number of doses shown in the table below. Finally, in order to measure drug effects, the tumor volume was calculated from the tumor sizes at the day before the start of the administration and at the final day of the administration, and tumor growth in the test compound administration group compared with a control administration group given only a 0.5% methylcellulose solution was calculated as T/C according to the following expression:

T/C (%) = (Tumor volume after the completion of the administration of the test compound administration group - Tumor volume at the day before the start of the administration of the test compound administration group) / (Tumor volume after the completion of the administration of the control administration group - Tumor volume at the day before the start of the administration of the control administration group) × 100

The table below shows T/C (%) of each test compound (specifically, the compounds shown in Examples 38, 58, 84, 93, 110, 112 and 113) against the human large intestine cancer cell line HCT116 cancer-bearing mice vs. the number of animals in each administration group, the dose and the number of doses.

The test results are shown in Table 6.

[Table 6]

| Example No. | The number of animals in administration group | Dose (mg/kg) | The number of doses per day | T/C (%) |
|---|---|---|---|---|
| **38** | **6** | **10** | **2** | **85.6** |
| **58** | **6** | **10** | **2** | **65.3** |
| **84** | **7** | **10** | **2** | **58.4** |
| **93** | **5** | **10** | **2** | **66.1** |
| **110** | **6** | **10** | **2** | **64.8** |
| **112** | **5** | **10** | **2** | **53.7** |
| **113** | **6** | **10** | **2** | **67.9** |

These results demonstrated that the compound of the present invention strongly inhibits the growth of human large intestine cancer cells *in vivo.*

Industrial Applicability

The compound of the present invention has an effect of specifically inhibiting SCD1 with high selectivity. Thus, the compound of the present invention can be used as a SCD1 inhibitor and is useful as a prophylactic or therapeutic agent for SCD1-related diseases including cancer and the like.

**Claims**

**1.** A compound represented by a formula:

[Formula 1]

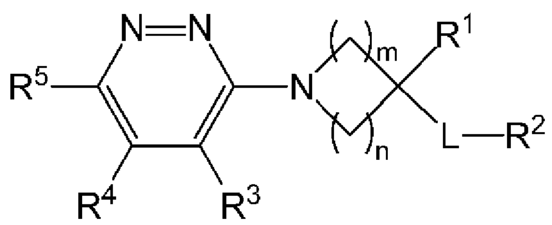

(I)

wherein

$R^1$ represents $R^6$, CN or $OR^7$;
$R^2$ represents an optionally substituted aromatic hydrocarbon group or an optionally substituted aromatic het-

erocyclic group;
one of $R^3$ and $R^4$ represents a hydrogen atom, and the other represents a hydrogen atom or a substituent;
$R^5$ represents an optionally substituted aromatic heterocyclic group;
$R^6$ represents an optionally halogenated $C_{1-6}$ alkyl group, an optionally substituted $C_{3-6}$ cycloalkyl group, an optionally substituted $C_{2-6}$ alkenyl group or an optionally substituted $C_{2-6}$ alkynyl group;
$R^7$ represents an optionally substituted $C_{3-6}$ cycloalkyl group or an optionally substituted $C_{1-6}$ alkyl group;
L represents a bond or a chain linker with a principal chain having 1 to 3 atoms; and
m and n are the same or different and each represents 1 or 2,

or a salt thereof.

**2.** The compound or a salt thereof according to claim 1, wherein $R^1$ is an optionally halogenated $C_{1-6}$ alkyl group.

**3.** The compound or a salt thereof according to claim 1, wherein $R^2$ is a $C_{6-14}$ aryl group optionally substituted by 1 to 3 substituents selected from

(1) a halogen atom,
(2) a $C_{1-6}$ alkyl group optionally substituted by 1 to 3 halogen atoms,
(3) a $C_{2-6}$ alkynyl group,
(4) a $C_{1-6}$ alkoxy group optionally substituted by 1 to 3 halogen atoms, and
(5) a sulfanyl group optionally substituted by 1 to 5 halogen atoms.

**4.** The compound or a salt thereof according to claim 1, wherein both of $R^3$ and $R^4$ are a hydrogen atom.

**5.** The compound or a salt thereof according to claim 1, wherein $R^5$ is a nitrogen-containing 5-membered aromatic heterocyclic group optionally substituted by 1 to 3 substituents selected from

(1) a $C_{1-6}$ alkyl group,
(2) a hydroxy-$C_{1-6}$ alkyl group,
(3) a $C_{1-6}$ alkyl-carbonyloxy-$C_{1-6}$ alkyl group,
(4) a $C_{6-14}$ aryl-carbonyloxy-$C_{1-6}$ alkyl group,
(5) a $C_{1-6}$ alkoxy-$C_{7-16}$ aralkyloxy-$C_{1-6}$ alkyl group, and
(6) a $C_{1-6}$ alkoxy-$C_{1-6}$ alkyl group.

**6.** The compound or a salt thereof according to claim 1, wherein L is a bond.

**7.** The compound or a salt thereof according to claim 1, wherein both of m and n are 2.

**8.** The compound or a salt thereof according to claim 1, wherein
$R^1$ is CN, $R^6$ or $OR^7$;
$R^2$ is a $C_{6-14}$ aryl group optionally substituted by 1 to 3 substituents selected from

(1) a halogen atom,
(2) a $C_{1-6}$ alkyl group optionally substituted by 1 to 3 halogen atoms,
(3) a $C_{2-6}$ alkynyl group,
(4) a $C_{1-6}$ alkoxy group optionally substituted by 1 to 3 halogen atoms, and
(5) a sulfanyl group optionally substituted by 1 to 5 halogen atoms;

each of $R^3$ and $R^4$ is a hydrogen atom;
$R^5$ is a nitrogen-containing 5-membered aromatic heterocyclic group optionally substituted by 1 to 3 substituents selected from

(1) a $C_{1-6}$ alkyl group,
(2) a hydroxy-$C_{1-6}$ alkyl group,
(3) a $C_{1-6}$ alkyl-carbonyloxy-$C_{1-6}$ alkyl group,
(4) a $C_{6-14}$ aryl-carbonyloxy-$C_{1-6}$ alkyl group,
(5) a $C_{1-6}$ alkoxy-$C_{7-16}$ aralkyloxy-$C_{1-6}$ alkyl group, and
(6) a $C_{1-6}$ alkoxy-$C_{1-6}$ alkyl group;

$R^6$ is

(1) a $C_{1-6}$ alkyl group optionally substituted by 1 to 3 halogen atoms, or
(2) a $C_{2-6}$ alkynyl group;

$R^7$ is a $C_{1-6}$ alkyl group;
L is a bond or a chain linker having a principal chain formed from 1 to 3 atoms selected from the group consisting of carbon, oxygen, nitrogen and sulfur; and
m and n are the same or different and each represents 1 or 2.

**9.** (5-(6-(4-(Trifluoromethyl)-4-(4-(trifluoromethyl)phenyl)piperidin-1-yl)pyridazin-3-yl)-1,3,4-oxadiazol-2-yl)methanol, or a salt thereof

**10.** (5-(6-(4-(Trifluoromethyl)-4-(4-(trifluoromethyl)phenyl)piperidin-1-yl)pyridazin-3-yl)-1,2,4-thiadiazol-3-yl)methanol, or a salt thereof

**11.** (5-(6-(4-(Trifluoromethyl)-4-(4-(trifluoromethyl)phenyl)piperidin-1-yl)pyridazin-3-yl)-1,3,4-thiadiazol-2-yl)methanol, or a salt thereof

**12.** A medicament comprising a compound or a salt thereof according to claim 1.

**13.** The medicament according to claim 12, wherein the medicament is a SCD1 inhibitor.

**14.** The medicament according to claim 12, wherein the medicament is a prophylactic or therapeutic agent for cancer.

**15.** A method for inhibiting SCD1 in a mammal, comprising administering an effective amount of a compound or a salt thereof according to claim 1 to a mammal.

**16.** A method for preventing or treating cancer in a mammal, comprising administering an effective amount of a compound or a salt thereof according to claim 1 to a mammal.

**17.** The compound or a salt thereof according to claim 1 for use in the prevention or treatment of cancer.

**18.** Use of a compound or a salt thereof according to claim 1 for the production of a prophylactic or therapeutic agent for cancer.

**INTERNATIONAL SEARCH REPORT**

International application No. PCT/JP2015/057122

A. CLASSIFICATION OF SUBJECT MATTER

*C07D413/14*(2006.01)i, *A61K31/501*(2006.01)i, *A61P35/00*(2006.01)i, *A61P43/00*(2006.01)i, *C07D401/14*(2006.01)i, *C07D417/14*(2006.01)i

According to International Patent Classification (IPC) or to both national classification and IPC

B. FIELDS SEARCHED

Minimum documentation searched (classification system followed by classification symbols)

C07D413/14, A61K31/501, A61P35/00, A61P43/00, C07D401/14, C07D417/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

CAplus/REGISTRY(STN), Science Direct, Wiley Online Library, ACS PUBLICATIONS, JSTPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2010-506859 A (Merck Frost Canada Ltd.), 04 March 2010 (04.03.2010), claims; paragraphs [0001], [0005], [0080]; examples 13 to 18, 20 to 21, 30, 32, 35<br>& US 2010/0004245 A1 & WO 2008/046226 A1<br>& EP 2076509 A1 & CA 2664849 A1<br>& AU 2007312866 A | 1-4,6-7, 12-14,17-18<br>1-14,17-18 |
| Y | JP 2009-501733 A (Merck Frost Canada Ltd.), 22 January 2009 (22.01.2009), claims; particularly, claim 17; paragraph [0097]<br>& US 2009/0118296 A1 & WO 2007/009236 A1<br>& EP 1910352 A1 & CA 2615045 A1<br>& AU 2006272334 A | 1-14,17-18 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 14 May 2015 (14.05.15) | 26 May 2015 (26.05.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No. PCT/JP2015/057122

C (Continuation). DOCUMENTS CONSIDERED TO BE RELEVANT

| Category* | Citation of document, with indication, where appropriate, of the relevant passages | Relevant to claim No. |
|---|---|---|
| Y | US 2013/0096181 A1 (GENENTECH, INC.), 18 April 2013 (18.04.2013), paragraphs [0257], [0260], [0262]; examples 8 to 9; claims 1 to 9, 39 to 40<br>& JP 2015-501301 A & WO 2013/056148 A2<br>& EP 2766000 A2 & CA 2850836 A<br>& CN 103998027 A & KR 10-2014-0084164 A<br>& MX 2014004426 A | 1-14,17-18 |

Form PCT/ISA/210 (continuation of second sheet) (July 2009)

**INTERNATIONAL SEARCH REPORT**

International application No.
PCT/JP2015/057122

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 15,16
because they relate to subject matter not required to be searched by this Authority, namely:
(See extra sheet)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2015/057122

Continuation of Box No.II-1 of continuation of first sheet(2)

Claims 15 and 16 pertain to methods for treatment of the human body or animal body by surgery or therapy and thus relate to a subject matter on which this International Searching Authority is not required to carry out an international search under the provisions of PCT Article 17(2)(a)(i) and PCT Rule 39.1(iv).

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010007120 A
- US 5001125 A
- WO 2008062276 A
- WO 2006034338 A
- WO 0142241 A
- WO 0023444 A
- EP 320032 A
- WO 2007009236 A
- WO 2008096746 A
- WO 2013056148 A
- WO 2013134546 A
- WO 2010142801 A
- WO 2011066803 A
- WO 2007115315 A
- WO 2009123316 A
- WO 2012122368 A
- WO 2013010453 A
- WO 20060084808 A
- WO 2000076514 A
- WO 2010130424 A
- WO 20070123591 A
- WO 2008099019 A


### Non-patent literature cited in the description


- **LIU et al.** *Journal of Medicinal Chemistry,* 2007, vol. 50 (13), 3086-3100
- **ISABEL et al.** *Bioorganic & Medicinal Chemistry Letters,* 01 January 2011, vol. 21 (1), 479-483
- The Fifth Series of Experimental Chemistry. vol. 13-19
- Shin Jikken Kagaku Koza. vol. 14-15
- Syntheses in the Organic Chemistry Laboratory. L. F. Tietze, Th. Eicher, Nankodo Co., Ltd,
- The Reaction Mechanism and Essence, Revised. Hideo Tougo, Kodansha Ltd,
- Organic Syntheses Collective. John Wiley & Sons, Inc, vol. I-II
- **JIE JACK LI.** Modern Organic Synthesis in the Laboratory: A Collection of Standard Experimental Procedures. Oxford University Press
- Comprehensive Heterocyclic Chemistry III. Elsevier Japan KK, vol. 1-14
- Strategic Applications of Named Reactions in Organic Synthesis. Kagaku-Dojin Publishing Company, Inc,
- Comprehensive Organic Transformations. VCH Publishers, Inc, 1989
- **THEODORA W. GREENE ; PETER G. M. WUTS.** Protective Groups in Organic Synthesis. Wiley-Interscience, 2007
- **P.J. KOCIENSKI.** Protecting Groups. Thieme Medical, 2004
- *Bioorganic & Medicinal Chemistry Letters,* 2012, vol. 22 (20), 6373-6376
- *Journal of Medicinal Chemistry,* 2007, vol. 50 (4), 685-695
- *Journal of Medicinal Chemistry,* 1984, vol. 27 (12), 1559-65
- *Journal of Organic Chemistry,* 2010, vol. 75 (10), 3507-3510
- *Tetrahedron Letters,* 1988, vol. 29 (47), 6125-6
- *Journal of Organic Chemistry,* 2001, vol. 66 (22), 7500-7504
- *Tetrahedron: Asymmetry,* 2009, vol. 20 (19), 2205-2210
- *Heterocycles,* 1994, vol. 39 (2), 801-9
- *Synlett,* 2002, vol. 3, 431-434
- *Tetrahedron Letters,* 2009, vol. 50 (21), 2497-2500
- *Journal of Medicinal Chemistry,* 2003, vol. 46 (25), 5512-5532
- *Journal of Organic Chemistry,* 1982, vol. 47 (8), 1445-51
- *Journal of the American Pharmaceutical Association (1912-1977),* 1950, vol. 39, 451-4
- *Journal of the American Chemical Society,* 1993, vol. 115 (1), 77-81
- *Journal of the American Chemical Society,* 1958, vol. 80, 3915-23
- *Journal of Organic Chemistry,* 1987, vol. 52 (24), 5480-2
- *Organic Syntheses,* 1959, vol. 39, 54-5
- *Organic Syntheses,* 1956, vol. 36, 28-30
- Development of Pharmaceuticals in English. **IYAKUHIN NO KAIHATSU.** Molecular Design. Hirokawa Shoten Ltd, 1990, vol. 7, 163-198